# EUROPEAN PATENT SPECIFICATION

(11) **EP 0 763 114 B1**
(45) Date of publication and mention of the grant of the patent: **05.12.2001**
(21) Application number: 95922085.6
(22) Date of filing: 19.05.1995
(51) Int. Cl.: C12N 15/40, C07K 14/18, A61K 39/29, G01N 33/576, C12Q 1/68, C12Q 1/70

(54) **HEPATITIS G VIRUS AND MOLECULAR CLONING THEREOF**
HEPATITIS G VIRUS UND SEINE MOLEKULARE KLONIERUNG
VIRUS DE L'HEPATITE G ET SON CLONAGE MOLECULAIRE

(30) Priority: 20.05.1994 US 246985; 03.08.1994 US 285543; 03.08.1994 US 285558; 26.10.1994 US 329729; 23.11.1994 US 344271; 16.12.1994 US 357509; 15.02.1995 US 389886
(43) Date of publication of application: 19.03.1997
(73) Proprietor: GENELABS TECHNOLOGIES, INC., Redwood City, CA 94063 (US)
(72) Inventor: KIM, Jungsuh P., Palo Alto, CA 94306 (US); FRY, Kirk E., Palo Alto, CA 94303 (US); YOUNG, Lavonne Marie, Palo Alto, CA 94306 (US); LINNEN, Jeffrey M., Foster City, CA 94404 (US); WAGES, John, San Carlos, CA 94070 (US)
(74) Representative: VOSSIUS & PARTNER
(86) International application number: US9506169
(87) International publication number: WO9532291

(56) References cited:
- WO-A-90/00597
- WO-A-94/18217
- WO-A-95/21922
- THE AMERICAN JOURNAL OF GASTROENTEROLOGY, vol. 89, no. 1, January 1994 BALTIMORE,MD, pages 57-61, S.K.KUWADA ET AL. 'Non-A, non-B fulminant hepatitis is also non-E and non-C'

## Description

### FIELD OF INVENTION

This invention relates to nucleic acid, polypeptide, antigen, epitope, vaccine and antibody compositions related to a NonA/NonB/NonC/NonD/NonE (N-(ABCDE)) hepatitis-associated viral agent (HGV). The invention also relates to diagnostic and therapeutic methods.

### REFERENCES

Abstracts, *The 1992 San Diego Conf.:* Genetic *Recognition, Clin. Chem.* 39(4):705 (1993).

Alexander, W. A., *et al., J. Virol.* 66:2934-2942 (1992).

Alter, H.J., *et al., New Eng. J. Med.* 321:1494-1500 (1989a).

Alter. M.J., *et al., N. Engl. J. Med.* 327:1899 (1989b).

Alter, H.J., *Abstracts of Int. Symp. on Viral Hepatitis and Liver Dis., p.* 47 (1993).

Altschul, S., et *al., J. Mol. Biol.* 215:403-10 (1990).

Ascadi, G., *et al., Nature* 352:815 (1991).

Ausubel, F.M., *et al.,* CURRENT PROTOCOLS IN MOLECULAR BIOLOGY, John Wiley and Sons, Inc., Media PA.

Barany, *F., PCR Methods Appl.* 1:5 (1991).

Barham, W. B., *et al., J. Med. Virol.* 42:129-132 (1994).

Baron, S., *et al., JAMA* 266:1375 (1991).

Bazan, J. *F., et al., Virology* 171:637-639 (1989).

Beames, *et al., Biotechniques* 11:378 (1991).

Belyavsky, A., *et al., Nuc. Acids Res.* 17:2919-2932 (1989).

Blackburn, G.F., *et al.,* Clin. Chem. 37:1534-1539 (1991).

Bradley, D.W., *et al., J. Infec. Dis.,* 148:2 (1983).

Bradley, D.W., *et al., J Gen. Virol.,* 69:1 (1988).

Bradley, D.W. *et al., Proc. Nat. Acad. Sci., USA,* 84:6277 (1987).

Briand, J.-P., *et al., J. Immunol. Meth.* 156:255 (1992).

Cahill, P., *et al., Clin. Chem.* 37:1482 (1991).

Carter, J.M., *et al., Methods Mol. Biol.* 36:207-223 (1994).

Chambers, T.J., *et al., Ann. Rev. Microbiol.* 44:649 (1990a).

Chambers, T.J., *et al., PNAS* 87:8898 (1990b).

Chomczynski *et al, Anal. Biochem.* 162:159 (1987).

Christian, R.B., *et al., J. Mol. Biol.* 227:771 (1992).

Commandaeur, *et* al., *Virology* 198:282-287 (1994).

Crea, R., U.S. Patent No. 4,888,286, issued December 19, 1989.

DeGraaf, M.E., *et al.*, Gene 128:13 (1993).

DiBisceglie, A.M., *et al., Hepatology* 16:649 (1992).

DiBisceglie, A.M., *et al., NEJM* 321:1506 (1989).

DiCesare, J., *et al.,* Biotechniques 15:152-157 (1993).

Dienstag, J.L., *et al, Sem Liver Disease* 6:67 (1986).

Earl, P. L., *et al.,* "Expression of proteins in mammalian cells using vaccinia" In *Current Protocols in Molecular Biology* (F. M. Ausubel, *et al.* Eds.), Greene Publishing Associates & Wiley Interscience, New York (1991).

Eaton, M. A. W., *et al*., U.S. Patent No. 4,719,180, issued Jan. 12, 1988.

Egholm, *et al*., *Nature* 365:566 (1993).

Elroy-Stein, O., *et al*., *Proc*. *Natl*. *Acad*. *Sci*. *USA.* 86:6126-6130 (1989).

EPO patent application 88310922.5, filed 11/18/88.

Falkner, F.G., *et al*., *J*. *Virol.* 62:1849-1854 (1988).

Farci, P., *et al*., *NEJM* 330:88 (1994).

Felgner and Rhodes, *Nature* 349:251 (1991).

Fickett, J.W., *Nuc*. *Acids Res.* 10:5303-5318 (1982).

Fling, S. P., *et al*., *Analytical Biochem.* 155:83-88 (1986).

Folgori, A., *et al.,* EMBO J. 13:2236 (1994).

Francki, R.I.B., *et al.*, *Arch. Virol.* Suppl2:223 (1991).

Frank, R., and Doring, R., *Tetrahedron* 44:6031-6040 (1988).

Frohman, M.A., *et al., Proc. Natl. Acad. Sci. USA* 85:8998-9002 (1988).

Fuerst, T. R., *et al., Proc. Natl. Acad. Sci. USA* 83:8122-8126 (1986).

Gellissen, G., *et al., Antonie Van Leeuwenhoek,* 62(1-2):79-93 (1992).

Geysen, M., *et al., Proc. Natl. Acad. Sci. USA* 81:3998-4002 (1984).

Gingeras, T.R., *et al., Ann. Biol. Clin.* 48:498 (1990).

Gingeras, T.R., *et al., J. Inf. Dis.* 164:1066 (1991).

Goeddel, D.V., *Methods in Enzymology* 185 (1990).

Grakoui, A., *et al., J. Virol.* 67:2832 (1993).

Grakoui, A., *et al., J. Virol.* 67:1385-1395 (1993).

Guatelli, J.C., et *al., Proc. Natl. Acad. Sci. USA* 87:1874 (1990).

Gubler, U., *et al*, Gene, 25:263 (1983).

Guthrie, C., and G.R. Fink, *Methods in Enzymology* 194 (1991).

Gutterman, J.U., *PNAS* 91:1198 (1994).

Harlow, E., et *al.*, ANTIBODIES: A LABORATORY MANUAL, Cold Spring Harbor Laboratory Press (1988).

Haynes, J., *et al.*, *Nuc. Acid. Res.* 11:687-706 (1983).

Hieter, P.A., *et al., Cell* 22:197-207 (1980).

Hijikata, M., *et al., PNAS* 88:5547 (1991).

Hochuli, E., in GENETIC ENGINEERING, PRINCIPALS AND PRACTICE, VOL. 12 (J. Stelow Ed.) Plenum, NY, pp. 87-98 (1990).

Holodniy, M., *et al.*, *Biotechniques* 12:36 (1992).

Hopp, T.P., *et al., Proc. Natl. Acad. Sci. USA* 78:3824-3828 (1981).

Horn, T., and Urdea, M.S., *Nuc. Acids. Res.* 17:6959 (1989).

Houghten, R.A., *Proc. Natl. Acad. Sci. USA* 82:5131 (1985).

Hudson, D., *J. Org. Chem.* 53:617 (1988).

Irwin, M.J., *et al., J. Virol.* 58:5036 (1994).

Jacob, J.R., *et al.,* in THE MOLECULAR BIOLOGY OF HCV, Section 4, pages 387-392 (1991).

Jacob, J.R., *et al., Hepatology* 10:921-927 (1989).

Jacob, J.R., *et al., J. Infect. Dis.* 161:1121-1127 (1990).

Janknecht, R., *et al., Proc. Natl. Acad. Sci. USA* 88:8972-8976 (1991).

Kaufman, R. J., "Selection and coamplification of heterologous genes in mammalian cells," in *Methods in Enzymology,* vol. 185, pp537-566. Academic Press, Inc., San Diego CA (1991).

Kakumu, S., *et al*., *Gastroenterol.* 105:507 (1993).

Katz, E.D., and Dong, M., *Biotechniques* 8:546 (1990).

Kawasaki, E.S., *et al*., in PCR TECHNOLOGY: PRINCIPLES AND APPLICATIONS OF DNA AMPLIFICATION (H.A. Erlich, ed.) Stockton Press (1989).

King, L. A., *et al*., *The baculovirus expression system. A laboratory guide,* Chapman & Hall, London, New York, Tokyo, Melbourne, Madras, 1992.

Kyte, J., & Doolittle, R. F., *J. Mol*. *Biol.* 157:105-132 (1982).

Koonin, E.V., and Dolja, V.V., *Critical Reviews in Biochem*. *& Mol*. *Biol.* 28:375-430 (1993).

Krausslich, H.G., *et al*., VIRAL PROTEINASES AS TARGETS FOR CHEMOTHERAPY (Cold Spring Harbor Press, Plainville, NY) (1989).

Kumar, R., *et al*., *AIDS Res*. *Human Retroviruses* 5(3):345-354 (1989).

Lanford, R.E., *et al*., *In Vitro Cell. Dev*. *Biol.* 25:174-182 (1989).

Larder, B.A., and Kemp, S.D., *Science* 246:1155 (1989).

Lau, Y.F., *et al*., *Mol. Cell. Biol.* 4:1469-1475 (1984).

Lomell, H., *et al., Clin. Chem.* 48:492 (1990).

Maniatis, T., *et al.,* MOLECULAR CLONING: A LABORATORY MANUAL, Cold Spring Harbor Laboratory (1982).

Marshall, W.S., and Caruthers, M.H., *Science* 259:1564 (1993).

Messing, J., *Methods in Enzymol.* 101:20 (1983).

Michelle, *et al., International Symposium on Viral Hepatitis.*

Miller, J. H., EXPERIMENTS IN MOLECULAR GENETICS, Cold Spring Harbor Laboratories, Cold Spring Harbor, NY (1972).

Morrissey, D.V., *et al., Anal. Biochem.* 181:345 (1989).

Moss, B., *et al.,* CURRENT PROTOCOLS IN MOLECULAR BIOLOGY (Section IV, Unit 16) (1991).

Moss, B., *et al.,* U.S. Patent Number 5,135,855, issued 4 August 1992.

Mullis, K.B., U.S. Patent No. 4,683,202, issued 28 July 1987.

Mullis, K.B., *et al.*, U.S. Patent No. 4,683,195, issued 28 July 1987.

Obeid, O.E., *et al.*, *Virus Research* 32:69-84 (1994).

Osikowicz, G., *et al.*, *Clin. Chem.* 36:1586 (1990).

Patterson, J.L., and Fernandez-Larsson, R., *Rev. Infect. Dis.* 12:1139 (1990).

Pearson, W.R. and Lipman, D.J., *PNAS* 85:2444-2448 (1988).

Pearson, W.R., *Methods in Enzymology* 183:63-98 (1990).

Pitha, *Biochem Biophys Acta,* 204:39 (1970a).

Pitha, *Biopolymers,* 9:965 (1970b).

Porath, J., *Protein Exp. and Purif.* 3:263 (1992).

Pritchard, C.G., and Stefano, J.E., *Ann. Biol. Chem.* 48:492 (1990).

Reichard, O., *et al., Lancet* 337:1058 (1991).

Reilly, P.R., *et al.,* BACULOVIRUS EXPRESSION VECTORS: A LABORATORY MANUAL (1992).

Reyes, G., *et al*, *Science,* 247:1335 (1990).

Reyes, G., *et al*., *Molecular and Cellular Probes*

5:473-481 (1991).

Rice, C.M., *et al*., *New Biol.* 1:285-296 (1989).

Roberts, N.A., *et al*., *Science* 248:358 (1990).

Romanos, M.A., *et al*., *Yeast* 8(6):423-488 (1992).

Sanger, *et al*., *Proc*. *Natl*. *Acad*. *Sci.* 74:5463 (1977).

Sambrook, J., *et al*., In MOLECULAR CLONING: A LABORATORY MANUAL, Cold Spring Harbor Laboratory Press, Vol. 2 (1989).

Saiki, R.K., *et al*., *Science* 239:487-491 (1988).

Schagger, H., *et al*., *Anal*. *Biochem.* 166:368-379 (1987).

Scharf, S. J., *et al*., *Science* 233:1076 (1986).

Schuler, G.D., *et al*., *Proteins: Struc*., *Func*. *and* Genet. 9:180 (1989).

Scott, J.K., and Smith, G.P., *Science* 249:386 (1990).

Scott, J.K., *et al*., *Proc*. *Natl*. *Acad*. *Sci*. *USA* 89:5398 (1992).

Smith, D.B., *et al*., *Gene* 67:31 (1988).

Smith, J.P., *Curr*. *Opin*. *Biotechnol*. 2:668 (1991).

Sreenivasan, M.A., *et al*., *J. Gen. Virol.* 65:1005 (1984).

Sumiyoshi, H., *et al*., *J*. *Virol.* 66:5425-5431 (1992).

Summerton, J., *et al*., U.S. Patent No. 5,142,047, issued 08/25/92.

Summerton, J., *et al*., U.S. Patent No. 5,185,444 issued 02/09/93.

Tam, A., *et al*., *Virology* 185:120 (1991).

Tam, J.P., *Proc*. *Natl*. *Acad. Sci. USA* 85:5409 (1988).

Tessier, D. C., Gene 98:177-183 (1991).

Tonkinson, J.L., and Stein, C.A., *Antiviral Chem. and Chemother.* 4(4):193-200 (1993).

Ulmer, *et al., Science* 259:1745 (1993).

Urdea, M., *Clin*. *Chem.* 39:725 (1993).

Urdea, M., *et al*., *AIDS* 7:S11 (1993).

Wages, J.M., *et al.,* Amplifications 10:1-6 (1993).

Walker, G.T., *PCR Methods Appl*. 3:1-6 (1993).

Wang, A.M., *et al.* in PCR PROTOCOLS: A GUIDE TO METHODS AND APPLICATIONS (M.A. Innis, *et al.,* eds.) Academic Press (1990).

Wang, B., *et al., Proc. Natl. Acad. Sci. USA* 90:4156 (1993).

Whetsell, *A.J., et al., J. Clin. Micro.* 30:845 (1992).

Wolf, J.A., *et al., Nature* 247:1465 (1990).

Vacca, J.P., *et al., PNAS* 91:4096 (1994).

VanGemen, B., *et al., J. Virol. Methods* 43:177 (1993).

Valenzuela, P., *et al., Nature* 298:344 (1982).

Valenzuela, P., *et al.,* in HEPATITIS B, eds. I. Millman, *et al.,* Plenum Press, pages 225-236 (1984).

Yarbrough, *et al., J. Virol.* 65:5790 (1991).

Yoo, B.J., *et al., J. Virol.* 69:32-38 (1995).

Yoshio, T., *et al.,* U.S. Patent No. 4,849,350, issued July 18, 1989.

Zhang, Y., *et al., J. Virol.* 65:6101-6110 (1991).

### BACKGROUND OF THE INVENTION

Viral hepatitis resulting from a virus other than hepatitis A virus (HAV) and hepatitis B virus (HBV) has been referred to as non-A, non-B hepatitis (NANBH). NANBH can be further defined based on the mode of transmission of an individual type, for example, enteric versus parenteral.

One form of NANBH, known as enterically transmitted NANBH or ET-NANBH, is contracted predominantly in poor-sanitation areas where food and drinking water have been contaminated by fecal matter. The molecular cloning of the causative agent, referred to as the hepatitis E virus (HEV), has recently been described (Reyes *et al.,* 1990; Tam *et al*.).

A second form of NANB, known as parenterally transmitted NANBH, or PT-NANBH, is transmitted by parenteral routes, typically by exposure to blood or blood products. The rate of this hepatitis varied by (i) locale, (ii) whether ALT testing was done in blood banks, and (iii) elimination of high-risk patients for AIDS. Appoximately 10% of transfusions caused PT-NANBH infection and about half of those went on to a chronic disease state (Dienstag). After implementation of anti-HCV testing, HCV seroconversion per unit transfused was decreased to less than 1% among heart surgery patients (Alter).

Human plasma samples documented as having produced post-transfusion NANBH in human recipients have been used successfully to produce PT-NANBH infection in chimpanzees (Bradley). RNA isolated from infected chimpanzee plasma has been used to construct cDNA libraries in an expression vector for immunoscreening with serum from human subjects with chronic PT-NANBH infection. This procedure identified a PT-NANBH specific cDNA clone and the viral sequence was then used as a probe to identify a set of overlapping fragments making up 7,300 contiguous basepairs of a PT-NANBH viral agent. The sequenced viral agent has been named the hepatitis C virus (HCV) (for example, the sequence of HCV is presented in EPO patent application 88310922.5, filed 11/18/88). The full-length sequence (∼ 9,500 nt) of HCV is now available.

Primate transmission studies conducted at the Centers for Disease Control (CDC; Phoenix, AZ, 1973-1975; 1978-1983) originally provided substantial evidence for the existence of multiple agents of non-A, non-B hepatitis (NANBH): the primary agents associated with the majority of cases of NANBH are now recognized to be HCV and HEV (see above), for PT-NANBH and ET-NANBH, respectively. Later epidemiologic studies conducted at the CDC (Atlanta, GA, 1989-present) using both research (prototype) and commercial tests for anti-HCV antibody showed that approximately 20% of all community-acquired NANBH was also non-C. Further testing of these samples for the presence of HEV (Reyes, *et al.*, WO A 9115603 (Genelabs Inc.) 17 October 1991) have indicated that these cases of community-acquired non-A, non-B, non-C hepatitis were also non-E.

Liver biopsy specimens, sera and plasma of Sentinel County patients (study of Drs. Miriam Alter and Kris Krawczynski) also showed that many *bona fide* cases of NANBH were also non-C hepatitis (serologically and by Reverse Transcriptase-Polymerase Chain Reaction (RT-PCR; Kawasaki, *et al.;* Wang, *et al.*, 1990) negative for all markers of HCV infection) developed subsequently into chronic hepatitis with presentation of chronic persistent hepatitis (CPH) or chronic active hepatitis (CAH) consistent with a viral infection.

### SUMMARY OF THE INVENTION

The invention is characterised by the features of the independent claims, and pertains to the characterization and isolation of a newly discovered NonA/NonB/NonC/NonD/NonE (N-(ABCDE)) hepatitis-associated viral agent, herein designated Hepatitis G Virus (HGV). Disclosed here is a family of cDNA replicas of portions of HGV genome. Also disclosed are methods for the isolation and characterization of further HGV sequences and sequences of HGV variants.

The present invention includes HGV genomic polynucleotides, cDNAs thereto and complements thereof. With respect to polynucleotides, some aspects of the invention include: a purified Hepatitis G Virus genomic polynucleotide; HGV derived RNA and DNA polynucleotides; recombinant HGV polynucleotides; a recombinant polynucleotide making up a sequence derived from HGV or HGV variant cDNA or complementary sequences thereof; a recombinant polynucleotide encoding an epitope of HGV; a recombinant vector including any of the above recombinant polynucleotides, and a host cell transformed with any of these vectors. Another aspect of the invention is a polynucleotide probe for HGV and/or its variants.

Current studies on the nature of the genome of HGV, utilizing sequence information to compare HGV to other viral sequences, suggest that HGV is a member of the Flaviviridae family of viruses.

Portions of the HGV-derived cDNA sequences are effective as probes to isolate variants of the virus which occur naturally, or to determine the presence of virus in samples. These cDNAs also make available HGV-encoded polypeptide sequences, including HGV-specific polypeptide antigens. These coding sequences allow the production of polypeptides which are useful as reagents in diagnostic tests and/or as components of vaccines, or as standards. Further, it is possible to isolate and sequence other portions of the HGV genome by utilizing probes derived from these cDNAs, therefore giving rise to additional probes and polypeptides useful in the prophylactic, therapeutic and diagnosis applications.

Other aspects of the invention include: a recombinant expression system which incorporates an open reading frame (ORF) derived from HGV cDNA or complements thereof, wherein the ORF is linked operably to a control sequence which is compatible with a desired host, a cell transformed with the recombinant expression system, and a polypeptide produced by the transformed cell.

Yet another aspect of the invention are purified HGV particles; a preparation of polypeptides from the purified HGV; a purified HGV polypeptide; a purified HGV peptide; and a purified polypeptide which comprises an epitope immunologically identifiable with an epitope contained in HGV or an HGV variant.

Included aspects of the invention are an HGV polypeptide; a recombinant polypeptide consisting of a sequence derived from a HGV genome, HGV cDNA or complements thereof; a recombinant polypeptide made of an HGV epitope; and a fusion polypeptide comprised of an HGV polypeptide.

Both polyclonal and monoclonal antibodies directed against HGV epitopes contained within the polypeptide sequences are also useful as therapeutic agents, for diagnostic tests, for the isolation of the HGV agent from which these cDNAs derive, and for screening of antiviral agents.

Also included in the invention are a purified preparation of polyclonal antibodies directed against an HGV epitope; and monoclonal antibodies directed against HGV epitopes.

Some aspects of the invention pertaining to kits are those for: investigating samples for the presence of polynucleotides derived from HGV which comprise a polynucleotide probe including a nucleotide sequence from HGV of approximately 8 or more nucleotides, in an appropriate container; analyzing samples for the presence of antibodies directed against an HGV antigen made up of a polypeptide which contains an HGV epitope present in the HGV antigen, in a suitable container; and analyzing samples for the presence of HGV antigens made up of an anti-HGV antibody, in a suitable container.

Still other aspects of the invention include a polypeptide comprised of an HGV epitope, which is attached to a solid substrate; and an antibody to an HGV epitope, which is attached to a solid substrate.

Other aspects of the invention are: a technique for the production of an HGV polypeptide, which includes incubating host cells which are transformed with an expression vector, containing a sequence encoding an HGV polypeptide, under conditions which allow expression of said polypeptide; and a polypeptide which has been produced by this method (containing, for example, an HGV epitope).

Also included in the invention are a method for the detection of HGV nucleic acids in samples comprising reacting nucleic acids of the sample with a probe for an HGV polynucleotide, under conditions allowing the creation of a polynucleotide duplex between the probe and the HGV nucleic acid from the sample; as well as detecting a polynucleotide duplex containing the probe. The invention includes the following hybridization based detection methods: reporter labeling; polymerase chain reaction; self-sustained sequence replication; ligase chain reaction; and strand displacement amplification. Further, detection methods include signal amplification (*e.g.*, branch-chained DNA probes and the Q-beta replicase method).

The invention also includes immunoassays, including an immunoassay for detecting HGV, comprising the incubation of a sample (which is suspected of being infected with HGV) with a probe antibody directed against an antigen/epitope of HGV, to be detected under conditions allowing the formation of an antigen-antibody complex; and detecting the antigen-antibody complex which contains the probe antibody. An immunoassay for the detection of antibodies which are directed against an HGV antigen comprising the incubation of a sample suspected of containing HGV with a probe polypeptide including an epitope of HGV, under conditions that allow the formation of an antibody-antigen complex; and distinguishing the antibody-antigen complex which contains the probe antigen.

Also forming part of the invention are HGV vaccines, for the treatment and/or prevention of HGV infection, comprising an immunogenic peptide containing an HGV epitope, or an inactivated preparation of HGV, or a reduced preparation of HGV.

In still another aspect, the invention includes a tissue culture grown cell, infected with HGV. In one embodiment, the tissue culture grown cells are primate liver cells.

Another aspect of the invention is a method for producing antibodies to HGV, comprising administering to a test subject an immunogenic polypeptide containing HGV epitopes in an adequate amount to elicit an immune response.

The present invention also includes an HGV mosaic polypeptide, where the mosaic polypeptide contains at least two epitopes of HGV, and, where the polypeptide substantially lacks amino acids normally intervening between the epitopes in the native HGV coding sequence. Such mosaic polypeptides are useful in the applications and methods discussed above.

The present invention further includes a random peptide epitope (mimitope) that mimics a natural HGV antigenic epitope during epitope presentation. Such mimitopes are useful in the applications and methods discussed above. Also included in the present invention is a method of identifying a random peptide HGV epitope. In the method, a library of random peptide epitopes is generated or selected. The library is contacted with an anti-HGV antibody. Mimitopes are identified that are specifically immunoreactive with the antibody. Sera (containing anti-HGV antibodies) or antibodies generated by the methods of the present invention can be used. Random peptide libraries can, for example, be displayed on phage or generated as combinatorial libraries.

In another aspect, the present invention includes therapeutic compounds and methods for the prevention and/or treatment of HGV infection.

These and other objects and features of the invention will be more fully appreciated when the following detailed description of the invention is read in conjunction with the accompanying drawings.

### BRIEF DESCRIPTION OF THE FIGURES

Figure 1: the relationship of the SEQ ID NO:14 open reading frame to the 470-20-1 clone.

Figure 2: shows an exemplary protein profile from gradient fractions eluted from a glutathione affinity column.

Figure 3: shows an exemplary Sodium dodecyl sulfate polyacrylamide gel electrophoresis analysis of fraction samples from Figure 2.

Figure 4A: shows an exemplary protein profile from gradient fractions eluted from an anion exchange column.

Figures 4B and 4C: show exemplary Sodium dodecyl sulfate polyacrylamide gel electrophoresis analysis of fraction samples from Figure 4A.

Figures 5A and 5B: amino acid alignments of HGV with two other members of Flaviviridae family -- Hog Cholera Virus and Hepatitis C Virus.

Figure 6 shows a map of a portion of the vector pGEX-Hisb-GE3-2, a bacterial expression plasmid carrying an HGV epitope.

Figures 7A to 7D show the results of Western blot analysis of the purified HGV GE3-2 protein.

Figures 8A to 8D show the results of Western blot analysis of the purified HGV Y5-10 antigen.

Figures 9A to 9D show the results of Western blot analysis of the following antigens: Y5-5, GE3-2 and Y5-10.

Figures 10A to 10F show the results of Western blot analysis of antigens GE-NS2b and GE-NS5a.

Figure 11 presents a Kyte-Doolittle hydrophobicity plot of the coding sequence of HGV.

Figure 12 shows the results of Western blot analysis of HGV pET clones with anti-T7.Tag monoclonal antibody.

Figures 13A to 13D show the results of Western blot analysis of HGV pET clone GE-NS5b. Figure 13E shows a corresponding coomassie stained gel.

Figures 14A to 14C show the results of Western blot analysis of HGV pET clone GE-E2. Figure 14D shows a corresponding coomassie stained gel.

Figures 15A to 15C show the results of Western blot analysis of HGV pET clone GE-NS5b. Figure 15D shows a corresponding coomassie stained gel.

Figure 16 shows a schematic representation of the coding regions of HGV.

### DETAILED DESCRIPTION OF THE INVENTION

### I. DEFINITIONS

The terms defined below have the following meaning herein:
1. "nonA/nonB/nonC/nonD/nonE hepatitis viral agent {N-(ABCDE)}," herein provisionally designated HGV, means a virus, virus type, or virus class which (i) is transmissible in some primates, including, mystax, chimpanzees or humans, (ii) is serologically distinct from hepatitis A virus (HAV), hepatitis B virus (HBV), hepatitis C virus (HCV), hepatitis D virus, and hepatitis E (HEV) (although HGV may co-infect a subject with these viruses), and (iii) is a member of the virus family Flaviviridae.
2. "HGV variants" are defined as viral isolates that have at least about 40%, preferably 55% or 65%, or more preferably 80% global sequence homology, that is, sequence identity over a length of the viral genome polynucleotide sequence, to the HGV polynucleotide sequences disclosed herein (*e.g*., SEQ ID NO:14).
   "Sequence homology" is determined essentially as follows. Two polynucleotide sequences of similar length (preferably, the entire viral genome) are considered to be homologous to one another, if, when they are aligned using the ALIGN program, over 40%, preferably 55% or 65%, or more preferably 80% of the nucleic acids in the highest scoring alignment are identically aligned using a ktup of 1, the default parameters and the default PAM matrix.
   The ALIGN program is found in the FASTA version 1.7 suite of sequence comparison programs (Pearson, *et al.,* 1988; Pearson, 1990; program available from William R. Pearson, Department of Biological Chemistry, Box 440, Jordan Hall, Charlottesville, VA).
   In determining whether two viruses are "highly homologous" to each other, the complete sequence of all the viral proteins (or the polyprotein) for one virus are optimally, globally aligned with the viral proteins or polyprotein of the other virus using the ALIGN program of the above suite using a ktup of 1, the default parameters and the default PAM matrix. Regions of dissimilarity or similarity are not excluded from the analysis. Differences in lengths between the two sequences are considered as mismatches. Alternatively, viral structural protein regions are typically used to determine relatedness between viral isolates. Highly homologous viruses have over 40%, or preferably 55% or 65%, or more preferably 80% global polypeptide sequence identity.
3. Two nucleic acid fragments are considered to be "selectively hybridizable" to an HGV polynucleotide, if they are capable of specifically hybridizing to HGV or a variant thereof (*e.g.*, a probe that hybridizes to HGV nucleic acid but not to polynucleotides from other members of the virus family Flaviviridae) or specifically priming a polymerase chain reaction: (i) under typical hybridization and wash conditions, as described, for example, in Maniatis, *et al.,* pages 320-328, and 382-389, (ii) using reduced stringency wash conditions that allow at most about 25-30% basepair mismatches, for example: 2 x SSC, 0.1% SDS, room temperature twice, 30 minutes each; then 2 x SSC, 0.1% SDS, 37°C. once, 30 minutes; then 2 x SSC room temperature twice, 10 minutes each, or (iii) selecting primers for use in typical polymerase chain reactions (PCR) under standard conditions (for example, in Saiki, R.K, *et al.*), which result in specific amplification of sequences of HGV or its variants.
   Preferably, highly homologous nucleic acid strands contain less than 20-30% basepair mismatches, even more preferably less than 5-20% basepair mismatches. These degrees of homology can be selected by using wash conditions of appropriate stringency for identification of clones from gene libraries (or other sources of genetic material), as is well known in the art.
4. An "HGV polynucleotide," as used herein, is defined as follows. For polynucleotides greater than about 100 nucleotides, HGV polynucleotides encompass polynucleotide sequences encoded by HGV variants and homologous sequences as defined in "2" above. For polynucleotides less than about 100 nucleotides in length, HGV polynucleotide encompasses sequences that selectively hybridizes to sequences of HGV or its variants. Further, HGV polynucleotides include polynucleotides encoding HGV polypeptides (see below).
   The term "polynucleotide" as used herein refers to a polymeric molecule having a backbone that supports bases capable of hydrogen bonding to typical nucleic acids, where the polymer backbone presents the bases in a manner to permit such hydrogen bonding in a sequence specific fashion between the polymeric molecule and a typically nucleic acid (*e.g.*, single-stranded DNA). Such bases are typically inosine, adenosine, guanosine, cytosine, uracil and thymidine. Numerous polynucleotide modifications are known in the art, for example, labels, methylation, and substitution of one or more of the naturally occurring nucleotides with an analog.
   Polymeric molecules include double and single stranded RNA and DNA, and backbone modifications thereof, for example, methylphosphonate linkages. Further, such polymeric molecules include alternative polymer backbone structures such as, but not limited to, polyvinyl backbones (Pitha, 1970a/b), morpholino backbones (Summerton, et al., 1992, 1993). A variety of other charged and uncharged polynucleotide analogs have been reported. Numerous backbone modifications are known in the art, including, but not limited to, uncharged linkages (*e.g.*, methyl phosphonates, phosphotriesters, phosphoamidates, and carbamates) and charged linkages (*e.g.*, phosphorothioates and phosphorodithioates). In addition linkages may contain the following exemplary modifications: pendant moieties, such as, proteins (including, for example, nucleases, toxins, antibodies, signal peptides and poly-L-lysine); intercalators (*e.g.,* acridine and psoralen), chelators (*e.g.,* metals, radioactive metals, boron and oxidative metals), alkylators, and other modified linkages (*e.g.*, alpha anomeric nucleic acids).
5. An "HGV polypeptide" is defined herein as any polypeptide homologous to an HGV polypeptide. "Homology," as used herein, is defined as follows. In one embodiment, a polypeptide is homologous to an HGV polypeptide if it is encoded by nucleic acid that selectively hybridizes to sequences of HGV or its variants.
   In another embodiment, a polypeptide is homologous to an HGV polypeptide if it is encoded by HGV or its variants, as defined above, polypeptides of this group are typically larger than 15, preferable 25, or more preferable 35, contiguous amino acids. Further, for polypeptides longer than about 60 amino acids, sequence comparisons for the purpose of determining "polypeptide homology" are performed using the local alignment program LALIGN. The polypeptide sequence, is compared against the HGV amino acid sequence or any of its variants, as defined above, using the LALIGN program with a ktup of 1, default parameters and the default PAM.
   Any polypeptide (typically a polypeptide not specifically immunoreactive with HGV antibodies) with an optimal alignment longer than 60 amino acids and greater than 65%, preferably 70%, or more preferably 80% of identically aligned amino acids is considered to be a "homologous polypeptide." The LALIGN program is found in the FASTA version 1.7 suite of sequence comparison programs (Pearson, *et al.*, 1988; Pearson, 1990; program available from William R. Pearson, Department of Biological Chemistry, Box 440, Jordan Hall, Charlottesville, VA).
6. A polynucleotide is "derived from" HGV if it has the same or substantially the same basepair sequence as a region of an HGV genome, cDNA of HGV or complements thereof, or if it displays homology as noted under "2", "3" or "4" above.
   A polypeptide or polypeptide "fragment" is "derived from" HGV if it is (i) encoded by an open reading frame of an HGV polynucleotide, or (ii) displays homology to HGV polypeptides as noted under "2" and "5" above, or (iii) is specifically immunoreactive with HGV positive sera.
7. "Substantially isolated" and "purified" are used in several contexts and typically refer to at least partial purification of an HGV virus particle, component (*e*.*g*., polynucleotide or polypeptide), or related compound (*e.g.*, anti-HGV antibodies) away from unrelated or contaminating components (*e.g.*, serum cells, proteins, non-HGV polynucleotides and non-anti-HGV antibodies). Methods and procedures for the isolation or purification of compounds or components of interest are described below (*e.g.*, affinity purification of fusion proteins and recombinant production of HGV polypeptides).
8. In the context of the present invention, the phrase "nucleic acid sequences," when referring to sequences which encode a protein, polypeptide, or peptide, is meant to include degenerative nucleic acid sequences which encode homologous protein, polypeptide or peptide sequences as well as the disclosed sequence.
9. An "epitope" is the antigenic determinant defined as the specific portion of an antigen with which the antigen binding portion of a specific antibody interacts.
10. An antigen or epitope is "specifically immunoreactive" with HGV positive sera when the epitope/antigen binds to antibodies present in the HGV infected sera but does not bind to antibodies present in the majority (greater than about 90%, preferably greater than 95%) of sera from individuals who are not or have not been infected with HGV. "Specifically immunoreactive" antigens or epitopes may also be immunoreactive with monoclonal or polyclonal antibodies generated against specific HGV epitopes or antigens.

An antibody or antibody composition (*e.g.,* polyclonal antibodies) is "specifically immunoreactive" with HGV when the antibody or antibody composition is immunoreactive with an HGV antigen but not with HAV, HBV, HCV, HDV or HEV antigens. Further, "specifically immunoreactive antibodies" are not immunoreactive with antigens typically present in normal sera obtained from subjects not infected with or exposed to HGV, HAV, HBV, HCV, HDV or HEV.

### II. N-(ABCDE) SERA.

Availability of a serologic test for anti-HCV and the development of an RT-PCR assay for HCV-RNA (Kawasaki, *et al.;* Wang, *et al.,* 1990) allowed the identification of several cases of both post-transfusion and community acquired non-HCV hepatitis. The human hepatitis case, PNF 2161, was originally identified as having NANB hepatitis (NANBH) through the Sentinel Counties Study of community acquired hepatitis, sponsored by the Centers for Disease Control and Prevention (Alter, *et al.,* 1989b). PNF 2161 was a sample obtained from an elderly Caucasian male patient who developed acute hepatitis approximately 8 weeks following a blood transfusion, with a peak serum ALT level of 1141 IU (normal, ≤45 IU). Following resolution of the episode of acute hepatitis, he had fluctuating, but persistently elevated ALT levels over the next seven years, consistent with chronic hepatitis, although histopathologic confirmation of this diagnosis was not obtained.

The plasma specimen used to clone HGV (as described herein) was obtained in June 1989, approximately 4^{1/2} years following the episode of acute hepatitis, and cryo-preserved. Patient PNF 2161 was initially believed not to be infected with HCV, based on consistently negative results with a first generation immunoassay test (Ortho HCV ELISA Test System; Ortho Diagnostics, Raritan, NJ). However, subsequent testing using a second generation HCV immunoassay (Ortho) and PCR with HCV 5'-non-coding region primers demonstrated that the patient was infected with HCV.

### III. ISOLATION OF HGV ASSOCIATED SEQUENCES.

As one approach toward identifying clones containing HGV sequences, a cDNA library was prepared from infected-HGV sera in the expression vector lambda gt11 (Example 1). Polynucleotide sequences were then selected for the expression of peptides which are immunoreactive with serum PNF 2161. First round screening was typically performed using the PNF 2161 serum (used to generate the phage library). It is also possible to screen with other suspected N-(ABCDE) sera.

Recombinant proteins identified by this approach provide candidates for peptides which can serve as substrates in diagnostic tests. Further, the nucleic acid coding sequences identified by this approach serve as useful hybridization probes for the identification of additional HGV coding sequences.

The sera described above were used to generate cDNA libraries in lambda gt11 (Example 1). In the method illustrated in Example 1, infected serum was precipitated in 8% PEG without dilution, and the libraries were generated from the resulting pelleted virus. Sera from infected human sources were treated in the same fashion.

As an advantageous alternative to PEG precipitation, ultracentrifugation can be used to pellet particulate agents from infected sera or other biological specimens. To isolate viral particles from which nucleic acids could be extracted, serum, ranging up to 2 ml, is diluted to approximately 10 ml with PBS, spun at 3K for 10 minutes, and the supernatant is centrifuged for a minimum of 2 hours at 40,000 rpm (approximately 110,000 x g) in a Ti70.1 rotor (Beckman Instruments, Fullerton, CA) at 4°C. The supernatant is then aspirated and the pellet extracted by standard nucleic acid extraction techniques.

cDNA libraries were generated using random primers in reverse transcription reactions with RNA extracted from pelleted sera as starting material. The resulting molecules were ligated to Sequence Independent Single Primer Amplification (SISPA; Reyes, *et al.*, 1991) linker primers and expanded in a non-selective manner, and then cloned into a suitable vector, for example, lambda gt11, for expression and screening of peptide antigens. Alternatively, the lambda gt10 vector may also be used.

Lambda gt11 is a particularly useful expression vector which contains a unique *Eco*RI insertion site 53 base pairs upstream of the translation termination codon of the β-galactosidase gene. Thus, an inserted sequence is expressed as a β-galactosidase fusion protein which contains the N-terminal portion of the β-galactosidase gene product, the heterologous peptide, and optionally the C-terminal region of the β-galactosidase peptide (the C--terminal portion being expressed when the heterologous peptide coding sequence does not contain a translation termination codon).

This vector also produces a temperature-sensitive repressor (cI857) which causes viral lysogeny at permissive temperatures, *e.g.*, 32°C, and leads to viral lysis at elevated temperatures, *e.g.*, 42°C. Advantages of this vector include: (1) highly efficient recombinant clone generation, (2) ability to select lysogenized host cells on the basis of host-cell growth at permissive, but not non-permissive, temperatures, and (3) production of recombinant fusion protein. Further, since phage containing a heterologous insert produces an inactive β-galactosidase enzyme, phage with inserts are typically identified using a colorimetric substrate conversion reaction employing β-galactosidase.

Example 1 describes the preparation of a cDNA library for the N-(ABCDE) hepatitis sera PNF 2161. The library was immunoscreened using PNF 2161 (Example 3). A number of lambda gt11 clones were identified which were immunoreactive. Immunopositive clones were plaque-purified and their immunoreactivity retested. Also, the immunoreactivity of the clones with normal human sera was also tested.

These clones were also examined for the "exogenous" nature of the cloned insert sequence. This basic test establishes that the cloned fragment does not represent a portion of human or other potentially contaminating nucleic acids (*e.g.*, *E. coli, S. cerevisiea* and mitochondrial). The clone inserts were isolated by *Eco*RI digestion following polymerase chain reaction amplification. The inserts were purified then radiolabelled and used as hybridization probes against membrane bound normal human DNA, normal mystax DNA and bacterial DNA (control DNAs) (Example 4A).

Clone 470-20-1 (PNF2161 cDNA source) was one of the clones isolated by immunoscreening with the PNF 2161 serum. The clone was not reactive with normal human sera. The clone has a large open reading frame (203 base pairs; SEQ ID NO:3), in-frame with the β-galactosidase gene of the lambda gt11 vector. The clone is exogenous by genomic DNA hybridization analysis and genomic PCR analysis, using human, yeast and *E. coli* genomic DNAs (Example 4B).

The sequence was present in PNF2161 serum as determined by RT-PCR (Example 4C). RT-PCR of serially diluted PNF 2161 RNA suggested at least about 10⁵ copies of 470-20-1 specific sequence per ml. The sequence was also detected in sucrose density gradient fractions at densities consistent with the sequence banding in association with a virus-like particle (Example 5).

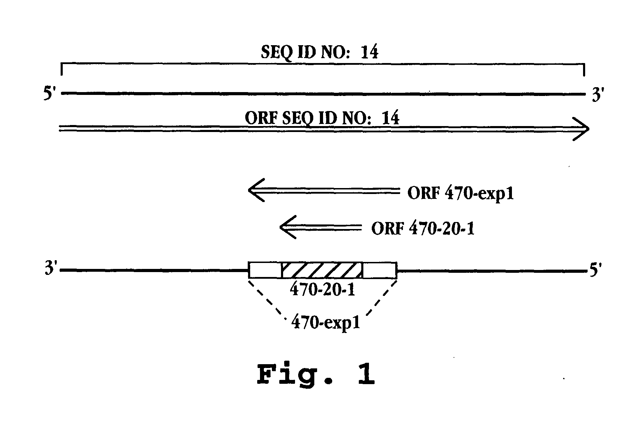

Bacterial lysates of *E. coli* expressing a second clone, clone 470-exp1, (SEQ ID NO:37) were also shown to be specifically immunoreactive with PNF 2161 serum at comparable levels to clone 470-20-1. The coding sequence of 470-exp1 was flanked by termination codons (based on sequence comparisons to SEQ ID NO:14, also see Figure 1) and had an internal methionine.

Further sequences contained in SEQ ID NO:14, adjacent to clone 470-20-1, were obtained by anchor polymerase chain reaction (Anchor PCR) using primers from clone 470-20-1 (Example 6). In this case a PNF 2161 2-cDNA source library was used as template, where the cDNA/complement double-stranded DNA products were ligated to lambda arms, but the mixture was not packaged.

470-20-1 specific primers were used in amplification reactions with SISPA-amplified PNF 2161 cDNA as a template (Example 4). The identity of the amplified DNA fragments were confirmed by (i) size and (ii) hybridization with a 470-20-1 specific oligonucleotide probe (SEQ ID NO:16). The 470-20-1 specific signal was detected in cDNA amplified by PCR from SISPA-amplified PNF 2161, demonstrating the presence of the 470-20-1 sequences in the source material.

The 470-20-1 specific primers were also used in amplification reactions with the following RNA sources as substrate: normal mystax liver RNA, normal tamarin (*Sanguins laboriatis)* liver RNA, and MY131 liver RNA (Example 4). The results from these experiments demonstrate the 470-20-1 sequences are present in the parent serum sample (PNF 2161) and in an RNA liver sample from an animal challenged with the PNF 2161 sample (MY131). Both normal control RNAs were negative for the presence of 470-20-1 sequences.

Further, PNF 2161 serum and other cloning source or related source materials were directly tested by PCR using primers from selected cloned sequences. Specific amplification products were detected by hybridization to a specific oligonucleotide probe 470-20-1-152F (SEQ ID NO:16). A specific signal was reproducibly detected in multiple extracts of PNF 2161, with the 470-20-1 specific primers.

The disease association between HGV and liver disease is further supported by the data presented in Example 4F. Sera from hepatitis patients and from blood donors with abnormal liver function were assessed for the presence of HGV by RT-PCR screening, using HGV specific primers. HGV specific sequence were detected in 6/152 of these sera samples. No HGV positives were detected among the control samples (n = 11).

The results presented above indicate the isolation of a viral agent associated with N-(ABCDE) viral infection of liver (*i.e*., hepatitis) and/or infection, and resulting disease, of other tissue and cell types.

### IV. FURTHER CHARACTERIZATION OF HGV RECOMBINANT ANTIGENS.

### A. SCREENING RECOMBINANT LIBRARIES.

Further candidate HGV antigens can be obtained from the libraries of the present invention using the screening methods described above. The cDNA library described above has been deposited with the American Type Culture Collection, 12301 Parklawn Dr., Rockville, MD, 20852, and has been assigned the following designation: PNF 2161 cDNA source, ATCC 75268.

A second PNF 2161 cDNA library has been generated essentially as described for the first PNF 2161 cDNA library, except that second PNF 2161 cDNA source library was ligated to lambda gt11 arms but was not packaged. This non-packaged library was used to obtain the extension clones described below. A packaged version of this second library (PNF 2161 2-cDNA source library) has been deposited with the American Type Culture Collection, 12301 Parklawn Drive, Rockville, MD, 20852, and has been assigned the following designation: PNF 2161 2-cDNA source, ATCC 75837.

In addition to the recombinant libraries generated above, other recombinant libraries from N-(ABCDE) hepatitis sera can likewise be generated and screened as described herein.

### B. EPITOPE MAPPING, CROSS HYBRIDIZATION AND ISOLATION OF GENOMIC SEQUENCES.

Antigen encoding DNA fragments can be identified by (i) immunoscreening, as described above, or (ii) computer analysis of coding sequences (*e.g.*, SEQ ID NO:14) using an algorithm (such as, "ANTIGEN," Intelligenetics, Mountain View, CA) to identify potential antigenic regions. An antigen-encoding DNA fragment can be subcloned. The subcloned insert can then be fragmented by partial DNase I digestion to generate random fragments or by specific restriction endonuclease digestion to produce specific subfragments. The resulting DNA fragments can be inserted into the lambda gt11 vector and subjected to immunoscreening in order to provide an epitope map of the cloned insert.

In addition, the DNA fragments can be employed as probes in hybridization experiments to identify overlapping HGV sequences, and these in turn can be further used as probes to identify a set of contiguous clones. The generation of sets of contiguous clones allows the elucidation of the sequence of the HGV's genome.

Any of the above-described clone sequences *(e.g.,* derived from SEQ ID NO:14 or clone 470-20-1) can be used to probe the cDNA and DNA libraries, generated in a vector such as lambda gt10 or "LAMBDA ZAP II" (Stratagene, San Diego, CA). Specific subfragments of known sequence may be isolated by polymerase chain reaction or after restriction endonuclease cleavage of vectors carrying such sequences. The resulting DNA fragments can be used as radiolabelled probes against any selected library. In particular, the 5' and 3' terminal sequences of the clone inserts are useful as probes to identify additional clones.

Further, the sequences provided by the 5' end of cloned inserts are useful as sequence specific primers in first-strand cDNA or DNA synthesis reactions (Maniatis *et al.;* Scharf *et al.*). For example, specifically primed PNF 2161 cDNA and DNA libraries can be prepared by using specific primers derived from SEQ ID NO:14 on PNF 2161 nucleic acids as a template. The second-strand of the new cDNA is synthesized using RNase H and DNA polymerase I. The above procedures identify or produce DNA/cDNA molecules corresponding to nucleic acid regions that are 5' adjacent to the known clone insert sequences. These newly isolated sequences can in turn be used to identify further flanking sequences, and so on, to identify the sequences composing the entire genome for HGV. As described above, after new HGV sequences are isolated, the polynucleotides can be cloned and immunoscreened to identify specific sequences encoding HGV antigens.

Extension clone sequences (SEQ ID NO:14), containing further sequences of interest, have been obtained for clone PNF 470-20-1 (SEQ ID NO:3) using the "Anchor PCR" method described in Example 6. Briefly, the strategy consists of ligating PNF 2161 SISPA cDNA to lambda gt11 arms and amplifying the ligation reaction with a gt11-specific primer and one of two 470-20-1 specific primers.

The amplification products are electrophoretically separated, transferred to filters and the DNA bound to the filters is probed with a 470-20-1 specific probe. Bands corresponding to hybridization positive band signals were gel purified, cloned and sequenced.

### C. PREPARATION OF ANTIGENIC POLYPEPTIDES AND ANTIBODIES.

The recombinant peptides of the present invention can be purified by standard protein purification procedures which may include differential precipitation, molecular sieve chromatography, ion-exchange chromatography, isoelectric focusing, gel electrophoresis and affinity chromatography.

In one embodiment of the present invention, the polynucleotide sequences of the antigens of the present invention have been cloned in the plasmid p-GEX (Example 7A) or various derivatives thereof (pGEX-GLI). The plasmid pGEX (Smith, *et al.*, 1988) and its derivatives express the polypeptide sequences of a cloned insert fused in-frame to the protein glutathione-S-transferase (sj26). In one vector construction, plasmid pGEX-hisB, an amino acid sequence of 6 histidines is introduced at the carboxy terminus of the fusion protein.

The various recombinant pGEX plasmids can be transformed into appropriate strains of *E*. *coli* and fusion protein production can be induced by the addition of IPTG (isopropyl-thio galactopyranoside) as described in Example 7A. Solubilized recombinant fusion protein can then be purified from cell lysates of the induced cultures using glutathione agarose affinity chromatography (Example 7A).

Insoluble fusion protein expressed by the plasmid pGEX-hisB can be purified by means of immobilized metal ion affinity chromatography (Porath) in buffers containing 6M Urea or 6 M guanidinium isothiocyanate, both of which are useful for the solubilization of proteins. Alternatively insoluble proteins expressed in pGEX-GLI or derivatives thereof can be purified using combinations of centrifugation to remove soluble proteins followed by solubilization of insoluble proteins and standard chromatographic methodologies, such as ion exchange or size exclusion chromatography, and other such methods are known in the art.

In the case of β-galactosidase fusion proteins (such as those produced by lambda gt11 clones) the fused protein can be isolated readily by affinity chromatography, by passing cell lysis material over a solid support having surface-bound anti-β-galactosidase antibody. For example, purification of a β-galactosidase/fusion protein, derived from 470-20-1 coding sequences, by affinity chromatography is described in Example 7B.

Also included in the invention is an expression vector, such as the lambda gt11 or pGEX vectors described above, containing HGV coding sequences and expression control elements which allow expression of the coding regions in a suitable host. The control elements generally include a promoter, translation initiation codon, and translation and transcription termination sequences, and an insertion site for introducing the insert into the vector.

The DNA encoding the desired antigenic polypeptide can be cloned into any number of commercially available vectors to generate expression of the polypeptide in the appropriate host system. These systems include, but are not limited to, the following: baculovirus expression (Reilly, *et al.;* Beames, *et al.;* Pharmingen; Clontech, Palo Alto, CA), vaccinia expression (Earl, 1991; Moss, *et al.),* expression in bacteria (Ausubel, *et al.;* Clontech), expression in yeast (Gellissen, 1992; Romanos, 1992; Goeddel; Guthrie and Fink), expression in mammalian cells (Clontech; Gibco-BRL, Ground Island, NY), *e.g.*, Chinese hamster ovary (CHO) cell lines (Haynes, 1983, Lau, 1984, Kaufman, 1990). These recombinant polypeptide antigens can be expressed directly or as fusion proteins. A number of features can be engineered into the expression vectors, such as leader sequences which promote the secretion of the expressed sequences into culture medium.

Expression of large HGV polypeptides using several of these systems is described in Example 16.

Expression in yeast systems has the advantage of commercial production. Recombinant protein production by vaccinia and CHO cell line have the advantage of being mammalian expression systems. Further, vaccinia virus expression has several advantages including the following: (i) its wide host range; (ii) faithful post-transcriptional modification, processing, folding, transport, secretion, and assembly of recombinant proteins; (iii) high level expression of relatively soluble recombinant proteins; and (iv) a large capacity to accommodate foreign DNA.

The recombinant expressed polypeptide produced HGV polypeptide antigens are typically isolated from lysed cells or culture media. Purification can be carried out by methods known in the art including salt fractionation, ion exchange chromatography, and affinity chromatography. Immunoaffinity chromatography can be employed using antibodies generated based on the HGV antigens identified by the methods of the present invention.

HGV polypeptide antigens may also be isolated from HGV particles (see below).

Continuous antigenic determinants of polypeptides are generally relatively small, typically 6 to 10 amino acids in length. Smaller fragments have been identified as antigenic regions, for example, in conformational epitopes. HGV polypeptide antigens are identified as described above. The resulting DNA coding regions of either strand can be expressed recombinantly either as fusion proteins or isolated polypeptides. In addition, amino acid sequences can be conveniently chemically synthesized using commercially available synthesizer (Applied Biosystems, Foster City, CA) or "PIN" technology (Applied Biosytems).

In another embodiment, the present invention includes mosaic proteins that are composed of multiple epitopes. An HGV mosaic polypeptide typically contains at least two epitopes of HGV, where the polypeptide substantially lacks amino acids normally intervening between the epitopes in the native HGV coding sequence. Synthetic genes (Crea; Yoshio *et al.;* Eaton *et al*.) encoding multiple, tandem epitopes can be constructed that will produce mosaic proteins using standard recombinant DNA technology using polypeptide expression vector/host system described above.

Further, multiple antigen peptides can be synthesized chemically by methods described previously (Tam, J.P., 1988; Briand *et al.*). For example, a small immunologically inert core matrix of lysine residues with α- and e- amino groups can be used to anchor multiple copies of the same or different synthetic peptides (typically 6-15 residues long) representing epitopes of interest. Mosaic proteins or multiple antigen peptide antigens give higher sensitivity and specificity in immunoassays due to the signal amplification resulting from distribution of multiple epitopes.

Antigens obtained by any of these methods can be used for antibody generation, diagnostic tests and vaccine development.

In another aspect, the invention includes specific antibodies directed against the polypeptide antigens of the present invention. Antigens obtained by any of these methods may be directly used for the generation of antibodies or they may be coupled to appropriate carrier molecules. Many such carriers are known in the art and are commercially available (*e.g.,* Pierce, Rockford IL). Typically, to prepare antibodies, a host animal, such as a rabbit, is immunized with the purified antigen or fused protein antigen. Hybrid, or fused, proteins may be generated using a variety of coding sequence derived from other proteins, such as glutathione-S-transferase or β-galactosidase. The host serum or plasma is collected following an appropriate time interval, and this serum is tested for antibodies specific against the antigen. Example 8 describes the production of rabbit serum antibodies which are specific against the 470-20-1 antigen in the Sj26/470-20-1 hybrid protein. These techniques are equally applicable to all immunogenic sequences derived from HGV, including, but not limited to, those derived from the coding sequence presented as SEQ ID NO:14.

The gamma globulin fraction or the IgG antibodies of immunized animals can be obtained, for example, by use of saturated ammonium sulfate precipitation or DEAE Sephadex chromatography, affinity chromatography, or other techniques known to those skilled in the art for producing polyclonal antibodies.

Alternatively, purified antigen or fused antigen protein may be used for producing monoclonal antibodies. Here the spleen or lymphocytes from an immunized animal are removed and immortalized or used to prepare hybridomas by methods known to those skilled in the art. To produce a human-derived hybridoma, a human lymphocyte donor is selected. A donor known to be infected with a HGV may serve as a suitable lymphocyte donor. Lymphocytes can be isolated from a peripheral blood sample. Epstein-Barr virus (EBV) can be used to immortalize human lymphocytes or a suitable fusion partner can be used to produce human-derived hybridomas. Primary *in vitro* sensitization with viral specific polypeptides can also be used in the generation of human monoclonal antibodies.

Antibodies secreted by the immortalized cells are screened to determine the clones that secrete antibodies of the desired specificity, for example, by using the ELISA or Western blot method (Example 10; Ausubel *et al.*).

Using HGV-positive serum or plasma, or the antibodies of the present invention, other antigenic peptides and epitopes can be isolated. For example, a number of different techniques have been developed for the simultaneous synthesis of many peptides (Geysen, *et al.*; Houghten; Frank and Doring; Hudson). The method developed by Geysen, *et al.,* is especially useful because of the relative simplicity with which large numbers of different peptide sequences can be generated and tested for antigenicity. In the Geysen method (also referred to as MULTI-PIN peptide synthesis), the peptides are synthesized on polyacrylamide acid grafted polyethylene rods attached to a micro-titer plate. The MULTI-PIN strategy allows large numbers of syntheses (96 peptides per plate) to be immunologically screened using the polyclonal or monoclonal antibodies of the present invention and commercially available reagents and instrumentation. Immunoreactive peptides are identified and characterized.

It has been reported that up to 6,000 oligopeptides can be synthesized in a two week period, thus making it practical (by synthesizing all of the possible overlapping amino acid sequences of a particular antigen) to screen viral antigen sequences for epitopes to the resolution of a single amino acid (Geysen, *et al.*).

An alternative method of scanning for immunodominate peptides is to synthesize longer peptides (*e.g*., 10 to 30 amino acids) corresponding to HGV coding sequences using conventional automated peptide synthesis (Carter, *et al.,* 1994; Obeid, *et al.,* 1994; Commandaeur, *et al.,* 1994). This method has the advantage that the longer peptides can fold into shapes that mimic conformational epitopes.

Also, HGV antibodies, in particular, monoclonals, can be used to identify random polypeptides that mimic their virus-encoded target polypeptides (Scott and Smith, 1990; Smith, 1991). For example, random peptide libraries displayed on phage (RPL) (Scott and Smith, 1990) can be used as a source of peptide ligands for antibody generation or for vaccine development. RPL approach allows the expression of peptide-ligand containing fusion proteins on the phage surface and enrichment of these ligand encoding phages by affinity selection using antibodies (Smith, J.P., 1991; Christian, *et al.;* Scott, *et al.*, 1992; Folgori, *et al.*). These random peptide epitopes detected by specific antibodies mimic the natural antigenic epitopes (mimotopes) during epitope presentation. HGV antigenic mimics (mimotopes) can be isolated from RPL. Hexa- to decapeptide phagotope (mimotope displayed on phage) expressing RPL can be made by published methods (Scott and Smith; Smith, J.P, 1991; Christian, *et al.;* Scott, *et al.;* DeGraaf, *et al.;* Folgori, *et al.*) and screened by HGV-associated human sera or the antibodies of the present invention.

One example of the use of RPL for isolation of 470-20-1 mimotopes is as follows. The random decapeptide-pIII fusion phage display library is constructed according to the methods described previously (DeGraaf, *et al.,* 1993). Briefly, a chemically synthesized single-stranded degenerate insert is annealed to shorter oligonucleotides which generate SfiI restriction overhangs. Annealed DNA is ligated into SfiI-cut fUSE-5 vector DNA.

*E. coli* MC1061 is transformed with the ligated DNA. The library is amplified through approximately ten population doublings in LB medium with 20 mg/ml tetracycline. This library is affinity selected using one or more of 470-20-1 immunoreactive sera (or antibodies of the present invention). Polystyrene beads (Precision Plastic Ball Company, Chicago. Il) are coated with ammonium sulfate fractionated positive serum (e.g., PNF 2161) in 50 mM NaHCO3, pH 9.6 overnight at 4 C. Antibody coated beads are thoroughly washed with PBS and blocked with BSA.

These serum coated, blocked beads are pre-incubated with an excess of M13K07-UV killed phage for 4 hours at 4°C. Library phage are then added to the above preincubation mixture and incubated for 12 hours at 4°C. Unbound phage are removed and the beads are washed extensively with TTB (50 mM Tris, pH 7.5, 150 mM NaCl, 0.5% "TWEEN 20"(v/v), 1 mg/ml BSA) buffer. Bound phage are eluted with elution buffer (0.1M HCl adjusted to pH 2.2 with 2M Tris-HCl, pH 9.0). Eluted, enriched phage are screened with a second positive serum (e.g., Mys 136 sera) by plaque immunoscreening.

Further screening of the selected phagotopes can be carried out using large panels of positive and negative sera or specific HGV monoclonal antibodies. Selected phagotopes can be used directly in ELISA assay or antibody generation. Alternatively, the sequences of the phagotope encoding nucleotides can be determined and expressed in conventional vector/host system and used as antigen.

Mimic polypeptides identified as described above can in turn can serve as antigens in detection assays or can be used for the generation of antigen-specific antibodies.

### D. ELISA AND PROTEIN BLOT SCREENING.

When HGV antigens are identified, typically through plaque immunoscreening as described above, the antigens can be expressed and purified. The antigens can then be screened rapidly against a large number of suspected HGV hepatitis sera using alternative immunoassays, such as, ELISAs or Protein Blot Assays (Western blots) employing the isolated antigen peptide. The antigen polypeptides fusion can be isolated as described above, usually by affinity chromatography to the fusion partner such as *β*-galactosidase or glutathione-S-transferase. Alternatively, the antigen itself can be purified using antibodies generated against it (see below).

A general ELISA assay format is presented in Example 10. Harlow, *et al.,* describe a number of useful techniques for immunoassays and antibody/antigen screening.

The purified antigen polypeptide or fusion polypeptide containing the antigen of interest, is attached to a solid support, for example, a multiwell polystyrene plate. Sera to be tested are diluted and added to the wells. After a period of time sufficient for the binding of antibodies to the bound antigens, the sera are washed out of the wells. A labelled reporter antibody is added to each well along with an appropriate substrate: wells containing antibodies bound to the purified antigen polypeptide or fusion polypeptide containing the antigen are detected by a positive signal.

A typical format for protein blot analysis using the polypeptide antigens of the present invention is presented in Example 10. General protein blotting methods are described by Ausubel, *et al.* In Example 10, the 470-20-1/sj26 fusion protein was used to screen a number of sera samples. The results presented in Example 10 demonstrate that several different source N-(ABCDE) hepatitis sera are immunoreactive with the polypeptide antigen.

The results presented above demonstrate that the polypeptide antigens of the present invention can, by these methods, be rapidly screened against panels of suspected HGV infected serum samples for the detection of HGV.

### E. CELL CULTURE SYSTEMS, ANIMAL MODELS AND ISOLATION OF HGV.

HGV infectivity studies have been carried out in chimpanzees, cynomolgus monkey and four mystax subjects (Example 4H). These studies have yielded further information about HGV infectivity in these animal models. The HGV described in the present specification have the advantage of being capable of infecting tamarins, cynomologous monkeys and chimpanzees.

Alternatively, primary hepatocytes obtained from infected animals (chimpanzees, baboons, monkeys, or humans) can be cultured *in vitro.* A serum-free medium, supplemented with growth factors and hormones, has been described which permits the long-term maintenance of differentiated primate hepatocytes (Lanford, *et al.;* Jacob, *et al.,* 1989, 1990, 1991). In addition to primary hepatocyte cultures, immortalized cultures of infected cells may also be generated. For example, primary liver cultures may be fused to a variety of cells (like HepG2) to provide stable immortalized cell lines. Primary hepatocyte cell cultures may also be immortalized by introduction of oncogenes or genes causing a transformed phenotype. Such oncogenes or genes can be derived from a number of sources known in the art including SV40, human cellular oncogenes and Epstein Barr Virus.

Further, the un-infected hepatocytes (*e.g.*, primary or continuous hepatoma cell lines) may be infected by exposing the cells in culture to the HGV either as partially purified particle preparations (prepared, for example, from infected sera by differential centrifugation and/or molecular sieving) or in infectious sera. These infected cells can then be propagated and the virus passaged by methods known in the art. In addition, other cell types, such as lymphoid cell lines, may be useful for the propagation of HGV.

Protein similarity studies of HGV have detected amino acid regions similar to other viruses in the family Flaviviridae. It is known that members of this family of viruses can be propagated in a variety of tissue culture systems (ATCC-Viruses catalogue, 1990). By analogy it is likely that HGV can be propagated in one or more of the following tissue culture systems: Hela cells, primary hamster kidney cells, monkey kidney cells, vero cells, LLC-MK2 (rhesus monkey kidney cells), KB cells(human oral epidermoid carcinoma cells), duck embryo cells, primary sheep leptomeningeal cells, primary sheep choroid plexus cells, pig kidney cells, bovine embryonic kidney cells, bovine turbinate cells, chick embryo cells, primary rabbit kidney cells, BHD-21 cells, or PK-13 cells.

In addition to expression of HGV, regions of HGV polynucleotide sequences, cDNA or *in vitro* transcribed RNA can be introduced by recombinant means into tissue culture cells. Such recombinant manipulations allow the individual expression of individual components of the HGV.

RNA samples can be prepared from infected tissue or, in particular, from infected cell cultures. The RNA samples can be fractionated on gels and transferred to membranes for hybridization analysis using probes derived from the cloned HGV sequences.

HGV particles may be isolated from infected sera, infected tissue, the above-described cell culture media, or the cultured infected cells by methods known in the art. Such methods include techniques based on size fractionation (*i.e.,* ultrafiltration, precipitation, sedimentation), using anionic and/or cationic exchange materials, separation on the basis of density, hydrophilic properties, and affinity chromatography. During the isolation procedure the HGV can be identified (i) using the anti-HGV hepatitis associated agent antibodies of the present invention, (ii) by using hybridization probes based on identified HGV nucleic acid sequences (*e.g.,* Example 5) or (iii) by RT-PCR.

Antibodies directed against HGV can be used in purification of HGV particles through immunoaffinity chromatography (Harlow, *et al.;* Pierce). Antibodies directed against HGV polypeptides or fusion polypeptides (such as 470-20-1) are fixed to solid supports in such a manner that the antibodies maintain their immunoselectivity. To accomplish such attachment of antibodies to solid support bifunctional coupling agents (Pierce; Pharmacia, Piscataway, NJ) containing spacer groups are frequently used to retain accessibility of the antigen binding site of the antibody.

HGV particles can be further characterized by standard procedures including, but not limited to, immunofluorescence microscopy, electron microscopy, Western blot analysis of proteins composing the particles, infection studies in animal and/or cell systems utilizing the partially purified particles, and sedimentation characteristics. The results presented in Example 5 suggest that the viral particle of the present invention is more similar to an enveloped viral particle than to a non-enveloped viral particle.

HGV particles can be disrupted to obtain HGV genomes. Disruption of the particles can be achieved by, for example, treatment with detergents in the presence of chelating agents. The genomic nucleic acid can then be further characterized. Characterization may include analysis of DNase and RNase sensitivity. The strandedness (Example 4I) and conformation (*e.g.,* circular) of the genome can be determined by techniques known in the art, including visualization by electron microscopy and sedimentation characteristics.

The isolated genomes also make it possible to sequence the entire genome whether it is segmented or not, and whether it is an RNA or DNA genome (using, for example RT-PCR, chromosome walking techniques, or PCR which utilizes primers from adjacent cloned sequences). Determination of the entire sequence of HGV allows genomic organization studies and the comparison of the HGV sequences to the coding and regulatory sequences of known viral agents.

### F. SCREENING FOR AGENTS HAVING ANTI-HGV HEPATITIS ACTIVITY.

The use of cell culture and animal model systems for propagation of HGV provides the ability to screen for anti-hepatitis agents which inhibit the production of infectious HGV: in particular, drugs that inhibit the replication of HGV. Cell culture and animal models allow the evaluation of the effect of such anti-hepatitis drugs on normal cellular functions and viability. Potential anti-viral agents (including natural products or synthetic compounds; for example, small molecules, complex mixtures such as fungal extracts, and anti-sense oligonucleotides) are typically screened for anti-viral activity over a range of concentrations. The effect on HGV replication and/or antigen production is then evaluated, typically by monitering viral macromolecular synthesis or accumulation of macromolecules (*e.g.*, DNA, RNA or protein). This evaluation is often made relative to the effect of the anti-viral agent on normal cellular function (DNA replication, RNA transcription, general protein translation, etc.).

The detection of the HGV can be accomplished by many methods including those described in the present specification. For example, antibodies can be generated against the antigens of the present invention and these antibodies used in antibody-based assays (Harlow, *et al.*) to identify and quantitate HGV antigens in cell culture. HGV antigens can be quantitated in culture using competition assays: polypeptides encoded by the cloned HGV sequences can be used in such assays. Typically, a recombinantly produced HGV antigenic polypeptide is produced and used to generate a monoclonal or polyclonal antibody. The recombinant HGV polypeptide is labelled using a reporter molecule. The inhibition of binding of this labelled polypeptide to its cognate antibody is then evaluated in the presence of samples (*e.g.,* cell culture media or sera) that contain HGV antigens. The level of HGV antigens in the sample is determined by comparison of levels of inhibition to a standard curve generated using unlabelled recombinant proteins at known concentrations.

The HGV sequences of the present invention are particularly useful for the generation of polynucleotide probes/primers that may be used to quantitate the amount of HGV nucleic acid sequences produced in a cell culture system. Such quantification can be accomplished in a number of ways. For example, probes labelled with reporter molecules can be used in standard dot-blot hybridizations or competition assays of labelled probes with infected cell nucleic acids. Further, there are a number of methods using the polymerase chain reaction to quantitate target nucleic acid levels in a sample (Osikowicz, *et al.*).

Protective antibodies can also be identified using the cell culture and animal model systems described above. For example, polyclonal or monoclonal antibodies are generated against the antigens of the present invention. These antibodies are then used to pre-treat an infectious HGV-containing inoculum (*e.g.*, serum) before infection of cell cultures or animals. The ability of a single antibody or mixtures of antibodies to protect the cell culture or animal from infection is evaluated. For example, in cell culture and animals the absence of viral antigen and/or nucleic acid production serves as a screen. Further in animals, the absence of HGV hepatitis disease symptoms, *e.g.*, elevated ALT values, is also indicative of the presence of protective antibodies.

Alternatively, convalescent sera can be screened for the presence of protective antibodies and then these sera used to identify HGV hepatitis associated agent antigens that bind with the antibodies. The identified HGV antigen is then recombinantly or synthetically produced. The ability of the antigen to generate protective antibodies is tested as above.

After initial screening, the antigen or antigens identified as capable of generating protective antibodies, either singly or in combination, can be used as a vaccine to inoculate test animals. The animals are then challenged with infectious HGV. Protection from infection indicates the ability of the animals to generate antibodies that protect them from infection. Further, use of the animal models allows identification of antigens that activate cellular immunity.

In animal model studies, a protective immune response in response to challenge by a viral preparation (*e.g.*, infected serum) (i) protects the animal from infection or (ii) prevents manifestation of disease.

### G. VACCINES AND THE GENERATION OF PROTECTIVE IMMUNITY.

Vaccines can be prepared from one or more of the immunogenic polypeptides identified by the method of the present invention. Genomic organization similarities between the isolated sequences from HGV and other known viral proteins may provide information concerning the polypeptides that are likely to be candidates for effective vaccines. In addition, a number of computer programs can be used for to identify likely regions of isolated sequences that encode protein antigenic determinant regions (for example, Hopp, *et al.;* "ANTIGEN," Intelligenetics, Mountain View CA).

Vaccines containing immunogenic polypeptides as active ingredients are typically prepared as injectables either as solutions or suspensions. Further, the immunogenic polypeptides may be prepared in a solid or lyophilized state that is suitable for resuspension, prior to injection, in an aqueous form. The immunogenic polypeptides may also be emulsified or encapsulated in liposomes. The polypeptides are frequently mixed with pharmaceutically acceptable excipients that are compatible with the polypeptides. Such excipients include, but are not limited to, the following and combinations of the following: saline, water, sugars (such as dextrose and sorbitol), glycerol, alcohols (such as ethanol [EtOH]), and others known in the art. Further, vaccine preparations may contain minor amounts of other auxiliary substances such as wetting agents, emulsifying agents (*e.g.,* detergents), and pH buffering agents. In addition, a number of adjuvants are available which may enhance the effectiveness of vaccine preparations. Examples of such adjuvants include, but are not limited to, the following: the group of related compounds including N-acetyl-muranyl-L-threonyl-D-isoglutamine and N-acetyl-nor-muranyl-L-alanyl-D-isoglutamine, and aluminum hydroxide.

The immunogenic polypeptides used in the vaccines of the present invention may be recombinant, synthetic or isolated from, for example, attenuated HGV particles. The polypeptides are commonly formulated into vaccines in neutral or salt forms. Pharmaceutically acceptable organic and inorganic salts are well known in the art.

HGV hepatitis associated agent vaccines are parenterally administered, typically by subcutaneous or intramuscular injection. Other possible formulations include oral and suppository formulations. Oral formulations commonly employ excipients (*e.g*., pharmaceutical grade sugars, saccharine, cellulose, and the like) and usually contain within 10-98% immunogenic polypeptide. Oral compositions take the form of pills, capsules, tablets, solutions, suspensions, powders, etc., and may be formulated to allow sustained or long-term release. Suppository formulations use traditional binders and carriers and typically contain between 0.1% and 10% of the immunogenic polypeptide.

In view of the above information, multivalent vaccines against HGV hepatitis associated agents can be generated which are composed of one or more structural or non-structural viral-agent polypeptide(s). These vaccines can contain, for example, recombinant expressed HGV polypeptides, polypeptides isolated from HGV virions, synthetic polypeptides or assembled epitopes in the form of mosaic polypeptides. In addition, it may be possible to prepare vaccines, which confer protection against HGV hepatitis infection through the use of inactivated HGV. Such inactivation might be achieved by preparation of viral lysates followed by treatment of the lysates with appropriate organic solvents, detergents or formalin.

Vaccines may also be prepared from attenuated HGV strains. Such attenuated HGV may be obtained utilizing the above described cell culture and/or animal model systems. Typically, attenuated strains are isolated after multiple passages *in vitro* or *in vivo.* Detection of attenuated strains is accomplished by methods known in the art. One method for detecting attenuated HGV is the use of antibody probes against HGV antigens, sequence-specific hybridization probes, or amplification with sequence-specific primers for infected animals or assay of HGV-infected *in vitro* cultures.

Alternatively, or in addition to the above methods, attenuated HGV strains may be constructed based on the genomic information that can be obtained from the information presented in the present specification. Typically, a region of the infectious agent genome that encodes, for example, a polypeptide that is related to viral pathogenesis can be deleted. The deletion should not interfere with viral replication. Further, the recombinant attenuated HGV construct allows the expression of an epitope or epitopes that are capable of giving rise to protective immune responses against the HGV. The desired immune response may include both humeral and cellular immunity.The genome of the attenuated HGV is then used to transform cells and the cells grown under conditions that allow viral replication. Such attenuated strains are useful not only as vaccines, but also as production sources of viral antigens and/or HGV particles.

Hybrid particle immunogens that contain HGV epitopes can also be generated. The immunogenicity of HGV epitopes may be enhanced by expressing the epitope in eucaryotic systems (e.g., mammalian or yeast systems) where the epitope is fused or assembled with known particle forming proteins. One such protein is the hepatitis B surface antigen. Recombinant constructs where the HGV epitope is directly linked to coding sequence for the particle forming protein will produce hybrid proteins that are immunogenic with respect to the HGV epitope and the particle forming protein. Alternatively, selected portions of the particle-forming protein coding sequence, which are not involved in particle formation, may be replaced with coding sequences corresponding to HGV epitopes. For example, regions of specific immunoreactivity to the particle-forming protein can be replaced by HGV epitope sequences.

The hepatitis B surface antigen has been shown to be expressed and assembled into particles in the yeast *Saccharomyces cerevisiea* and in mammalian cells (Valenzuela, *et al.,* 1982 and 1984; Michelle, *et al.*). These particles have been shown to have enhanced immunoreactivity. Formation of these particles using hybrid proteins, *i.e.,* recombinant constructs with heterologous viral sequences, has been previously disclosed (EPO 175,261, published 26 March 1986). Such hybrid particles containing HGV epitopes may also be useful in vaccine applications.

The vaccines of the present invention are administered in dosages compatible with the method of formulation, and in such amounts that will be pharmacologically effective for prophylactic or therapeutic treatments. The quantity of immunogen administered depends on the subject being treated, the capacity of the treatment subject's immune system for generation of protective immune response, and the desired level of protection.

HGV vaccines of the present invention can be administered in single or multiple doses. Dosage regimens are also determined relative to the treatment subject's needs and tolerances. In addition to the HGV immunogenic polypeptides, vaccine formulations may be administered in conjunction with other immunoregulatory agents.

In an additional approach to HGV vaccination, DNA constructs encoding HGV proteins under appropriate regulatory control are introduced directly into mammalian tissue, *in vivo*. Introduction of such constructs produces "genetic immunization". Similar DNA constructs have been shown to be taken up by cells and the encoded proteins expressed (Wolf, *et al.*; Ascadi, *et al*.). Injected DNA does not appear to integrate into host cells chromatin or replicate. This expression gives rise to substantial humoral and cellular immune responses, including protection from *in vivo* viral challenge in animal systems (Wang, *et al.,* 1993; Ulmer, *et al.*). In one embodiment, the DNA construct is injected into skeletal muscle following pre-treatment with local anesthetics, such as, bupivicaine hydrochloride with methylparaben in isotonic saline, to facilitate cellular DNA uptake. The injected DNA constructs are taken up by muscle cells and the encoded proteins expressed.

Compared to vaccination with soluble viral subunit proteins, genetic immunization has the advantage of authentic in vivo expression of the viral proteins. These viral proteins are expressed in association with host cell histocompatibility antigens, and other proteins, as would occur with natural viral infection. This type of immunization is capable of inducing both humoral and cellular immune responses, in contrast to many soluble subunit protein vaccines. Accordingly, this type of immunization retains many of the beneficial features of live attenuated vaccines, without the use of infectious agents for vaccination and attendant safety concerns.

Direct injection of plasmid or other DNA constructs encoding the desired vaccine antigens into *in vivo* tissues is one delivery means. Other means of delivery of the DNA constructs can be employed as well. These include a variety of lipid-based approaches in which the DNA is packaged using liposomes, cationic lipid reagents or cytofectins (such as, lipofectin). These approaches facilitate *in vivo* uptake and expression, as summarized by Felgner and Rhodes (1991). Various modifications to these basic approaches include the following: incorporation of peptides, or other moieties, to facilitate (i) targeting to particular cells, (ii) the intracellular disposition of the DNA construct following uptake, or (iii) to facilitate expression. Alternatively, the sequences encoding the desired vaccine antigens may be inserted into a suitable retroviral vector. The resulting recombinant retroviral vector inoculated into the subject for *in vivo* expression of the vaccine antigen. The antigen then induces the immune responses. As noted above, this approach has been shown to induce both humoral and cellular immunity to viral antigens (Irwin, *et al*.).

Further, the HGV vaccines of the present invention may be administered in combination with other vaccine agents, for example, with other hepatitis vaccines.

### H. SYNTHETIC PEPTIDES.

Using the coding sequences of HGV polypeptide, synthetic peptides can be generated which correspond to these polypeptides. Synthetic peptides can be commercially synthesized or prepared using standard methods and apparatus in the art (Applied Biosystems, Foster City CA).

Alternatively, oligonucleotide sequences encoding peptides can be either synthesized directly by standard methods of oligonucleotide synthesis, or, in the case of large coding sequences, synthesized by a series of cloning steps involving a tandem array of multiple oligonucleotide fragments corresponding to the coding sequence (Crea; Yoshio *et al.;* Eaton *et al.*). Oligonucleotide coding sequences can be expressed by standard recombinant procedures (Maniatis *et al.;* Ausubel et *al.*).

### V. CHARACTERIZATION OF THE VIRAL GENOME.

As shown in Example 4, the HGV genome appears to be an RNA molecule and has the closest sequence similarity to viral sequences that are catagorized in the Flaviviridae family of viruses. This family includes the Flaviviruses, Pestiviruses and an unclassified Genus made up of one member, Hepatitis C virus. The HGV virus does not have significant global (*i.e.*, over the length of the virus) sequence identity with other recognized members of the Flaviviridae -- with the exception of the protein motifs discussed below.

In general members of the Flaviviridae are enveloped viruses that have densities in sucrose gradients between 1.1 and 1.23 g/ml and are sensitive to heat, organic solvents and detergents. As shown in Example 5, HGV has density characteristics similar to an enveloped Flaviviridae virus (HCV). The integrity of the HGV virion also appears to be sensitive to organic solvents (Example 5).

Flaviviridae virions contain a single molecule of linear single-stranded (ss) RNA which also serves as the only mRNA that codes for the viral proteins. The ssRNA molecule is typically between the size of 9 and 12 kilobases long.

Viral proteins are derived from one polyprotein precursor that is subsequently processed to the mature viral proteins. Most members of the Flaviviridae do not contain poly(A) tails at their 3' ends. Virions are about 15-20% lipid by weight.

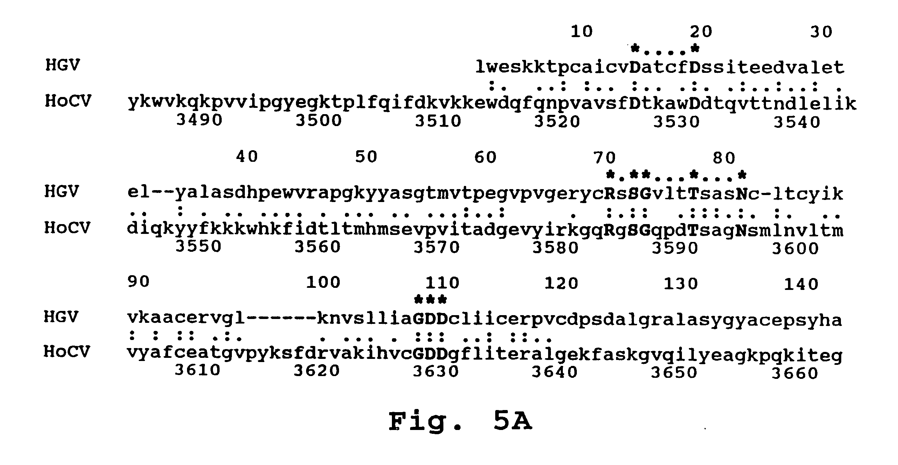

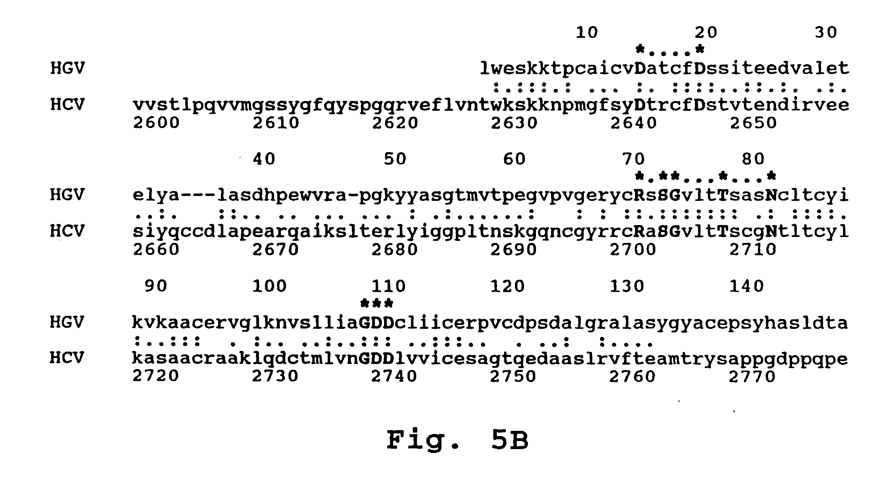

Members in the Flaviviridae family have a core protein and two or three membrane-associated proteins. The analogous structural proteins of members in the three genera Flavivirus family show little similarity to one another at the sequence level. The nonstructural proteins contain conserved motifs for RNA dependent RNA polymerase (RDRP), helicase, and a serine protease. These short blocks of conserved amino acids or motifs can be detected using computer algorithms known in the art such as "MACAW" (Schuler, *et al.*). These motifs are presumably related to constraints imposed by substrates processed by these proteins (Koonin and Dolja). The order of these motifs is conserved in all members of the Flaviviridae family. The genome of HGV contains protein motifs found in members of the Flaviviridae family, for example, (i) the helicase gene, (ii) the serine-like protease domain, and (iii) the RNA dependent RNA polymerase (RDRP) of (see Figure 5, "GDD" sequence);

Sequence information is disclosed herein on several different strains/isolates of HGV. This information can be used by one skilled in the art to isolate new stains/isolates using the techniques of hybridization, primer extension, and RT-PCR as described herein (*e.g.*, using degenerate primers based on the disclosed HGV variant sequences).

In the present case, HGV is an new isolate believed to be a member of the family Flaviviridae. Within this virus family, examination of the structural proteins encoded by a virus allows the most definitive determination of whether a viral isolate is a member of a distinct species of virus. Non-structural proteins are most conserved between different species of viruses within a family of virus species. This is believed to be the result of the necessity for preserving enzymatic functions, such as, the following: the proteolytic cleavage of a viral polyprotein, and replication of the RNA genome by viral helicase and RNA dependent RNA polymerase of the virus.

Examination of several species within any genus of the Flaviviridae family, *e.g.*, the flavivirus genus, demonstrates that the genes for these conserved functions are more highly conserved between species than the structural proteins. Accordingly, one of the major determining factors of whether a virus isolate represents a new species, versus a "variant isolate" of a known species, is a determination of global homology of the structural proteins between known viral species and the new virus isolate.

Local homologies found within regions about 200 amino acids or less which are found in non-structural proteins are indeterminant indicators of whether an isolate is a variant or a new species. Typically, virus isolates having global structural protein homologies of less than or about 40% are classified as either different species (viruses) or different genuses. The structural regions of HGV each have homologies lower than 40% compared with any virus described in "GENBANK" (comparisons carried out by methods standard in the art). Accordingly, HGV is considered to be a new species and possibly a new genus of positive strand RNA virus.

Another important region that is examined in determining the phylogenetic placement of a viral isolate is the 5' and 3' untranslated regions (UTRs). These regions are compared between viral isolates. For example, all the members HCV, an unclassified genus of Flaviviridae, have 5' untranslated regions that are greater than about 90% conserved with all other members in the genus. Further, the members of the HCV share 3' untranslated regions between about 24 and about 50 nucleotides long.

No significant alignments are found with any virus in "GENBANK" (Ver. 86) when the 5'-untranslated region is used as a query sequence with FASTA on BLASTN. Further, HGV contains a 3' untranslated region that is at least about 250 nucleotides long that also contains little homology to any other known virus.

Members of the Flaviviridae family are known to replicate in a wide variety of animals ranging from (i) hematophagous arthropod vectors (ticks and mosquitoes), where they do not cause disease, to (ii) a large range of vertebrate hosts (humans, primates, other mammals, marsupials, and birds). Over 30 members of the Flaviviridae family cause diseases in man, ranging from febrile illness, or rash, to potentially fatal diseases such as hemorrhagic fever, encephalitis, or hepatitis. At least 10 members of the Flaviviridae family cause severe and economically important diseases in domestic animals.

### VI. Utility

### A. THE INVENTION.

In one aspect, the invention pertains to polynucleotides derived from a Hepatitis G Virus (HGV) polynucleotide in substantially isolated form. In one embodiment the HGV polynucleotide is characterized by (i) transmission in primates, (ii) serologically distinguishable from hepatitis A virus (HAV), hepatitis B virus (HBV), hepatitis C virus (HCV), hepatitis D virus, and hepatitis E virus (HEV), and (iii) membership of the virus family Flaviviridae. Polynucleotides of the invention may be comprised of DNA or RNA (or analogs or variants thereof) and may be produced recombinantly, isolated, or synthesized according to methods known in the art.

Generally, HGV polynucleotides of the invention will be at least 10 nucleotides in length. In an alternative embodiment, the HGV polynucleotide will be at least 15 nucleotides in length. In still a further alternative embodiment, the HGV polynucleotide will be at least 20 nucleotides in length.

In a more specific embodiment, polynucleotides of the invention include cDNA or cDNA complements of the HGV genome. In a more specific embodiment, such a cDNA or cDNA complement will have at least a 40% sequence homology to a polynucleotide selected from the group consisting of SEQ ID NO:14, SEQ ID NO:37, and SEQ ID NO:19, or complements thereof. In yet another embodiment such cDNA's will exhibit at least 55% sequence homology to a polynucleotide selected from the group consisting of SEQ ID NO:14, SEQ ID NO:37, and SEQ ID NO:19, or complements thereof. In more specific embodiments, cDNA or cDNA complement polynucleotides of the invention will have sequences derived from sequences selected from the group consisting of SEQ ID NO:14, SEQ ID NO:37, and SEQ ID NO:19, or complements thereof.

In another general embodiment, polynucleotides of the invention are polynucleotide probes that specifically hybridize with HGV. In yet another general embodiment, polynucleotides of the invention will encode an epitope of HGV. More specifically, such epitope encoding polynucleotides may include sequences derived from SEQ ID NO:14, SEQ ID NO:19 or SEQ ID NO:37.

In another general embodiment, the polynucleotide of the invention includes a contiguous sequence of nucleotides that is capable of selectively hybridizing to an HGV polynucleotide. In this regard, HGV is characterized as a genome comprising an open reading frame (ORF) encoding an amino acid sequence having at least 40% sequence homology to one of the following amino acid sequences: the 2873 amino acid sequence of SEQ ID NO:15, the 190 amino acid sequence of SEQ ID NO:38, or the 67 amino acid sequence of SEQ ID NO:20. More particularly, the polynucleotide probe will specifically hybridize with HGV. Such a polynucleotide probe may carry detection labels or other modifications or be fixed to a solid support.

DNA polynucleotides as described above may also encode an HGV specifically immunoreactive antigenic determinants. In this regard, HGV is characterized as having a genome, cDNA or complements thereof comprising an open reading frame (ORF) encoding an amino acid sequence. Such, an amino acid sequence having at least 40% sequence homology to one of the following amino acid sequences: the 2873 amino acid sequence of SEQ ID NO:15, the 190 amino acid sequence of SEQ ID NO:38, or the 67 amino acid sequence of SEQ ID NO:20.

In another specific embodiment, an HGV-encoding DNA polynucleotide that is specifically reactive with an HGV antigenic determinant will, in accordance with the invention, include an amino acid sequence having at least 55% sequence homology to the 2873 amino acid sequence of SEQ ID NO:15 or to the 190 amino acid sequence of SEQ ID NO:38 or to the 67 amino acid sequence of SEQ ID NO:20.

In yet another specific embodiment, the DNA polynucleotide may exhibit at least 40% sequence homology to a polynucleotide selected from the group consisting of SEQ ID NO:14, SEQ ID NO:37, and SEQ ID NO:19, or complements thereof.

In still a further embodiment, the invention includes a DNA polynucleotide that encodes an HGV-derived polypeptide. More particularly, the polypeptide encoded by the polynucleotide will include a contiguous sequence of at least 15-60 amino acids having 55% sequence homology to a contiguous sequence of at least 15-60 amino acids encoded by an HGV genome, cDNA or complements thereof.

In a specific embodiment, HGV-polypeptide encoding polynucleotides may be encoded within the PNF 2161 cDNA source lambda gt11 library. In yet another specific embodiment, the DNA polynucleotide may encode an epitope of HGV. In still a further embodiment, the polynucleotide may be a probe that specifically hybridizes with HGV.

In a related aspect, the invention includes a recombinant vector that contains a DNA polynucleotide that encodes an HGV polypeptide. In another related aspect, the invention includes a cell transformed with such a vector.

In still another related aspect, the invention includes a polynucleotide probe that specifically hybridizes with an HGV hepatitis virus genome, cDNA or complements thereof. In a more specific embodiment, the polynucleotide probe sequence has at least 40% homology to a sequence derived from SEQ ID NO:19, SEQ ID NO:37, or SEQ ID NO:14, or complements thereof. In another specific embodiment, the polynucleotide probe is derived from SEQ ID NO:19, SEQ ID NO:37, or SEQ ID NO:14, or complements thereof.

In another related aspect, the invention includes a method of detecting an HGV hepatitis virus nucleic acid in a test subject. According to the method a nucleic acid-containing sample is obtained from the subject. The sample is then combined with and at least one polynucleotide probe that specifically hybridizes with the HGV hepatitis viral genome. HGV nucleic acid/probe complexes, formed by hybridization of the HGV nucleic acid with probe, are then detected. Such detecting may be accomplished by hybridization of a probe containing at least one reporter moiety to the HGV nucleic acid.

In a more specific embodiment, the above-described method includes the use of HGV nucleic acid specific probes where the two probes (primers) define an internal region of the HGV nucleic acid. In this embodiment, each probe has one strand containing a 3'-end internal to the HGV nucleic acid internal region. The nucleic acid/probe hybridization complexes are then converted to double-strand probe containing fragments by primer extension reactions. Probe-containing fragments are amplified by successively repeating the steps of (i) denaturing the double-strand fragments to produce single-strand fragments, (ii) hybridizing the single strands with the probes to form strand/probe complexes, (iii) generating double-strand fragments from the strand/probe complexes in the presence of DNA polymerase and all four deoxyribonucleotides, and (iv) repeating steps (i) to (iii) until a desired degree of amplification has been achieved. Amplification products are then identified according to established procedures. The method of the invention may further include a third polynucleotide probe capable of selectively hybridizing to the internal region described above but not to the specific probe/primer sequences used for amplification.

In another specific embodiment, detection of HGV nucleic acid/probe complexes is accomplished by a target amplification method, such as by self-sustained sequence replication, ligase chain reaction, or strand displacement amplification. In a further specific embodiment detection is accomplished employing a signal amplification technique such as branch-chained DNA probes or the Q-beta replicase method.

In still another related aspect, the invention includes a kit for analyzing samples for the presence of polynucleotides derived HGV hepatitis virus. In a general embodiment, the kit includes at least one polynucleotide probe containing a nucleotide sequence that will specifically hybridize with an HGV polynucleotide and a suitable container. In a specific embodiment, the kit includes two polynucleotide probes defining an internal region of the HGV polynucleotide, where each probe has one strand containing a 3'-end internal to the region. In a further embodiment, the probes may be useful as primers for polymerase chain reaction amplification.

In still a further related aspect, the invention includes the HGV hepatitis virus particle in substantially isolated form.
The invention also includes a polypeptide or a preparation of polypeptides from the HGV hepatitis virus in substantially isolated form. In this regard, the HGV virus is characterized as follows: (i) it is transmissible in primates; (ii) it is serologically distinct from hepatitis A virus (HAV), hepatitis B virus (HBV), hepatitis C virus (HCV), hepatitis D virus, and hepatitis E virus (HEV); and (iii) it is a member of the virus family Flaviviridae. HGV polypeptides, as defined above, may be prepared by conventional means, including chemical synthesis and recombinant DNA expression. Such polypeptides may also be fixed to a solid phase.

In a specific embodiment the polypeptide is specifically immunoreactive with at least one anti-HGV antibody. In still a further specific embodiment, the polypeptide comprises an antigenic determinant specifically immunoreactive with HGV. In this context, HGV is characterized by having a genome comprising an open reading frame (ORF) encoding an amino acid sequence having at least 40% sequence homology to the 2873 amino acid sequence of SEQ ID NO:15 or to the 190 amino acid sequence of SEQ ID NO:38 or to the 67 amino acid sequence of SEQ ID NO:20. In a more specific embodiment, the ORF encodes amino acid sequence has at least 55% sequence homology to one of the aforementioned amino acid sequences. In still a further embodiment, the polypeptide sequence is derived from the 2873 amino acid sequence of SEQ ID NO:15, or fragments thereof, the 190 amino acid sequence of SEQ ID NO:38, or fragments thereof, or the 67 amino acid sequence of SEQ ID NO:20, or fragments thereof.

In another specific embodiment, the polypeptide from the HGV hepatitis virus includes a contiguous sequence of at least about 60 amino acids encoded by an HGV genome, cDNA or complements thereof. More specifically, such peptide sequence may be encoded by the PNF 2161 cDNA source lambda gt11 library.

Recombinantly expressed HGV polypeptides may, in a more specific embodiment, include a polypeptide sequence derived from SEQ ID NO:20, SEQ ID NO:38, or SEQ ID NO:15. In another embodiment such a polypeptide may be encoded by a sequence derived from SEQ ID NO:14, or from the complement of SEQ ID NO:14.

In a further related embodiment, in accordance with the invention, an HGV hepatitis virus polypeptide may be a fusion polypeptide comprising an HGV polypeptide and a second polypeptide. More specifically, such a fusion polypeptide may include, as a second polypeptide signal sequences, β-galactosidase or glutathione-S-transferase protein sequences. Alternatively, the second polypeptide may comprise a particle forming protein.

The above-described polypeptides may be derived from structural or non-structural viral proteins.

In still a further related aspect, the invention includes a cloning vector capable of expressing, under suitable conditions, an open reading frame (ORF) of cDNA derived from HGV hepatitis virus genome, cDNA or complements thereof. In this aspect of the invention, the ORF is operably linked to a control sequence compatible with a desired host. In a related aspect, the invention includes a cell transformed with such a vector. In a more specific embodiment of the vector, the ORF may be derived from SEQ ID NO:14 or its complement. In yet further specific embodiments, the ORF may be derived from SEQ ID NO:37 or SEQ ID NO:19.

In a related aspect, the invention includes a method of producing an HGV hepatitis virus polypeptide. The method includes culturing cells containing the above-described vectors under conditions suitable to achieve expression of the open reading frame (ORF) sequence. In a more specific embodiment, the ORF sequence encodes a polypeptide sequence selected from the group of polypeptide sequences, or fragments thereof, consisting of SEQ ID NO:15, SEQ ID NO:38 and SEQ ID NO:20. Further, the ORF sequences may be derived from an HGV cDNA, or complement thereof. In yet another specific embodiment, the vector is a lambda gt11 phage vector expressed in *Escherichia coli* cells.

In a further related aspect, the invention includes a diagnostic kit for use in screening serum containing antibodies specific against HGV hepatitis virus infection. Such a kit may include a substantially isolated HGV polypeptide antigen comprising an epitope which is specifically immunoreactive with at least one anti-HGV antibody. Such a kit also includes means for detecting the binding of said antibody to the antigen.
In regard to such a kit, HGV is characterized by having a genome, cDNA or complements thereof comprising an open reading frame (ORF) encoding an amino acid sequence. Such an amino acid sequence typically having at least 40% sequence homology to the 2873 amino acid sequence of SEQ ID NO:15 or to the 190 amino acid sequence of SEQ ID NO:38 or to the 67 amino acid sequence of SEQ ID NO:20. In specific embodiments, the kit may include a recombinantly produced or chemically synthesized polypeptide antigen. The polypeptide antigen of the kit may also be attached to a solid support.

In a more specific embodiment, the detecting means of the above-described kit includes a solid support to which said polypeptide antigen is attached. Such a kit may also include a non-attached reporter-labelled anti-human antibody. In this embodiment, binding of the antibody to the HGV polypeptide antigen can be detected by binding of the reporter-labelled antibody the antibody.

In a related aspect, the invention includes a method of detecting HGV hepatitis virus infection in a test subject. This detection method includes reacting serum from an HGV test subject with a substantially isolated HGV polypeptide antigen, and examining the antigen for the presence of bound antibody. In a specific embodiment, the method includes a polypeptide antigen attached to a solid support, and the serum is reacted with the support. Subsequently, the support is reacted with a reporter-labelled anti-human antibody. The solid support is then examined for the presence of reporter-labelled antibody.

In a further aspect, the invention includes an HGV hepatitis virus vaccine composition. The composition includes a substantially isolated HGV polypeptide antigen, where the antigen includes an epitope which is specifically immunoreactive with at least one anti-HGV antibody. The peptide antigen may be produced according to methods known in the art, including recombinant expression or chemical synthesis. The peptide antigen is preferably present in a pharmacologically effective dose in a pharmaceutically acceptable carrier.

In still a further related aspect, the invention includes a monoclonal antibody that is specifically immunoreactive with the HGV hepatitis virus epitope. In another related aspect, the invention includes a substantially isolated preparation of polyclonal antibodies specifically immunoreactive with HGV. In a more specific embodiment, such polyclonal antibodies are prepared by affinity chromatography.

In a related aspect, the invention includes a method for producing antibodies to HGV. The method includes administering to a test subject a substantially isolated HGV polypeptide antigen, where the antigen includes an epitope which is specifically immunoreactive with at least one anti-HGV antibody. The antigen is administered in an amount sufficient to produce an immune response in the subject.

In yet another related aspect, the invention includes a diagnostic kit for use in screening serum containing HGV antigens. The diagnostic kit includes a substantially isolated antibody specifically immunoreactive with an HGV polypeptide antigen, and means for detecting the binding of the polypeptide antigen to the antibody. In one embodiment, the antibody is attached to a solid support. In a specific embodiment, the antibody may be a monoclonal antibody. The detecting means of the kit may include a second, labelled monoclonal antibody.
Alternatively, or in addition, the detecting means may include a labelled, competing antigen.

In another, related aspect, the invention includes a method of detecting HGV infection in a test subject. According to this aspect of the invention, serum from a test subject is reacted with a substantially isolated HGV specific antibody of the kit described above. The HGV specific antibody is then examined for the presence of bound antigen.

In still a further related aspect, the invention includes an *in vitro* grown cell infected with HGV. In a specific embodiment, the cell is a hepatocyte grown in tissue culture. More specifically, the tissue culture cell may be an immortalized hepatocyte, or it may be a from a cell line derived from liver of an HGV infected primate.

In a related aspect, the invention includes a method of propagating HGV. The method includes culturing *in vitro* grown, HGV-infected cells, as described above, under conditions effective to promote the propagation of HGV. In another related aspect, the invention includes HGV particles produced by such a propagation method.

In still a further aspect, the invention includes a mosaic polypeptide. Such a polypeptide may include at least two epitopes of HGV, where the polypeptide substantially lacks amino acids normally intervening between the epitopes in the native HGV coding sequence. In a more specific embodiment, the mosaic polypeptide is attached to a solid support. In still a further related aspect, the invention includes a nucleic acid that encodes the above-described mosaic polypeptide.

In another related aspect, the invention includes a method of detecting HGV infection in a test subject. The method includes contacting an antibody-containing sample from the subject with a mosaic polypeptide, as described above, and examining the antigen for the presence of bound antibody.

In still a further related aspect, the invention includes an HGV vaccine composition. The vaccine composition includes mosaic polypeptide that includes more than one HGV epitope. The mosaic polypeptide is present in a pharmacologically effective dose in a pharmaceutically acceptable carrier.

### B. IMMUNOASSAYS FOR HGV.

One utility for the antigens obtained by the methods of the present invention is their use as diagnostic reagents for the detection of antibodies present in the sera of test subjects infected with HGV hepatitis virus, thereby indicating infection in the subject; for example, 470-20-1 antigen, antigens encoded by SEQ ID NO:14 or its complement, and antigens encoded by portions of either strand of the complete viral sequence. The antigens of the present invention can be used singly, or in combination with each other, in order to detect HGV. The antigens of the present invention may also be coupled with diagnostic assays for other hepatitis agents such as HAV, HBV, HCV, and HEV.

In one diagnostic configuration, test serum is reacted with a solid phase reagent having a surface-bound antigen obtained by the methods of the present invention, e.g., the 470-20-1 antigen. After binding with anti-HGV antibody to the reagent and removing unbound serum components by washing, the reagent is reacted with reporter-labelled anti-human antibody to bind reporter to the reagent in proportion to the amount of bound anti-HGV antibody on the solid support. The reagent is again washed to remove unbound labelled antibody, and the amount of reporter associated with the reagent is determined. Typically, the reporter is an enzyme which is detected by incubating the solid phase in the presence of a suitable fluorometric or colorimetric substrate (Sigma, St. Louis, MO).

The solid surface reagent in the above assay is prepared by known techniques for attaching protein material to solid support material, such as polymeric beads, dip sticks, 96-well plate or filter material. These attachment methods generally include non-specific adsorption of the protein to the support or covalent attachment of the protein, typically through a free amine group, to a chemically reactive group on the solid support, such as an activated carboxyl, hydroxyl, or aldehyde group. Alternatively, streptavidin coated plates can be used in conjunction with biotinylated antigen(s).

Also forming part of the invention is an assay system or kit for carrying out this diagnostic method. The kit generally includes a support with surface-bound recombinant HGV antigen (*e.g.*, the 470-20-1 antigen, as above), and a reporter-labelled anti-human antibody for detecting surface-bound anti-HGV antigen antibody.

In a second diagnostic configuration, known as a homogeneous assay, antibody binding to a solid support produces some change in the reaction medium which can be directly detected in the medium. Known general types of homogeneous assays proposed heretofore include (a) spin-labelled reporters, where antibody binding to the antigen is detected by a change in reported mobility (broadening of the spin splitting peaks), (b) fluorescent reporters, where binding is detected by a change in fluorescence efficiency or polarization, (c) enzyme reporters, where antibody binding causes enzyme/substrate interactions, and (d) liposome-bound reporters, where binding leads to liposome lysis and release of encapsulated reporter. The adaptation of these methods to the protein antigen of the present invention follows conventional methods for preparing homogeneous assay reagents.

In each of the assays described above, the assay method involves reacting the serum from a test individual with the protein antigen and examining the antigen for the presence of bound antibody. The examining may involve attaching a labelled anti-human antibody to the antibody being examined (for example from acute, chronic or convalescent phase) and measuring the amount of reporter bound to the solid support, as in the first method, or may involve observing the effect of antibody binding on a homogeneous assay reagent, as in the second method.

A third diagnostic configuration involves use of HGV antibodies capable of detecting HGV-specific antigens. The HGV antigens may be detected, for example, using an antigen capture assay where HGV antigens present in candidate serum samples are reacted with a HGV specific monoclonal or polyclonal antibody. The antibody is bound to a solid substrate and the antigen is then detected by a second, different labelled anti-HGV antibody. Antibodies can be prepared, utilizing the peptides of the present invention, by standard methods. Further, substantially isolated antibodies (essentially free of serum proteins which may affect reactivity) can be generated (*e.g*., affinity purification (Harlow *et al.*)).

### C. HYBRIDIZATION ASSAYS FOR HGV.

One utility for the nucleic acid sequences obtained by the methods of the present invention is their use as diagnostic agents for HGV sequences present in sera, thereby indicating infection in the individual. Primers and/or probes derived from the coding sequences of the present invention, in particular, Clone 470-20-1 and SEQ ID NO:14, can be used singly, or in combination with each other, in order to detect HGV.

In one diagnostic configuration, test serum is reacted under PCR or RT-PCR conditions using primers derived from, for example, 470-20-1 sequences. The presence of HGV, in the serum used in the amplification reaction, can be detected by specific amplification of the sequences targeted by the primers. Example 4 describes the use of polymerase chain amplification reactions, employing primers derived from the clones of the present invention, to screen different source material. The results of these amplification reactions demonstrate the ability of primers derived from the clones of the present invention (for example, 470-20-1), to detect homologous sequences by amplification reactions employing a variety of different source templates. The amplification reactions in Example 4 included use of nucleic acids obtained directly from sera as template material.

Alternatively, probes can be derived from the HGV sequences of the present invention. These probes can then be labelled and used as hybridization probes against nucleic acids obtained from test serum or tissue samples. The probes can be labelled using a variety of reporter molecules and detected accordingly: for example, radioactive isotopic labelling and chemiluminescent detection reporter systems (Tropix, Bedford, Mass.).

Target amplification methods, embodied by the polymerase chain reaction, the self-sustained sequence replication technique ["3SR," (Guatelli, *et al.;* Gingeras, *et al.,* 1990) also known as "NASBA" (VanGemen, *et al*.)], the ligase chain reaction (Barany), strand-displacement amplification ["SDA," (Walker)], and other techniques, multiply the number of copies of the target sequence. Signal amplification techniques, exemplified by branched-chain DNA probes (Horn and Urdea; Urdea; Urdea, *et al.*) and the Q-beta replicase method (Cahill, *et al.;* Lomell, *et al.*), first bind a specific molecular probe, then replicate all of or part of this probe or in some other manner amplify the probe signal.

For the detection of the specific nucleic acid sequences disclosed in the present invention or contiguous sequences in the same or a similar (related) viral genome, amplification and detection methodologies may be employed, as alternatives to amplification by the PCR. A number of such techniques are known to the field of nucleic acid diagnostics (The 1992 San Diego Conference: Genetic Recognition, *Clin. Chem.* 39(4):705 (1993)).

### 1. SELF-SUSTAINED SEQUENCE REPLICATION.

The Self-Sustained Sequence Replication (3SR) technique results in amplification to a similar magnitude as PCR, but isothermally. Rather than thermal cycle-driven PCR, the 3SR operates as a concerted three-enzyme reaction of a) cDNA synthesis by reverse transcriptase, b) RNA strand degradation by RNase H, and c) RNA transcription by T7 RNA polymerase.

As the entire reaction sequence occurs isothermally (typically at 42°C.), expensive temperature-cycling instrumentation is not required. In the absence of duplex denaturation via heating, organic solvents, or other mechanism, only single-stranded templates (i.e., predominantly RNA) are amplified.

Suitable primers for use in 3SR amplification can be selected from the viral sequences of the present invention by those having ordinary skill in the art. For example, for isothermal amplification of viral sequences by the 3SR technique, primer 470-20-1-77F (SEQ ID NO:9) is modified by the addition of the T7 promoter sequence and a preferred T7 transcription initiation site to the 5'-end of the oligonucleotide. This modification results in a suitable 3SR primer T7-470-20-1-77F (SEQ ID NO:9). Primer 470-20-1-211R (SEQ ID NO:10) can be used in these reactions either without modification or T7 promoter.

RNA extracted from PNF 2161 is incubated with AMV reverse transcriptase (30 U), RNase H (3 U), T7 RNA polymerase (100 U), in 100 ul reactions containing 20 mM Tris-HCl, pH 8.1 (at room temperature), 15 mM MgCl₂, 10 mM KCl, 2 mM spermidine HCl, 5 mM dithiothreitol (DTT), 1 mM each of dATP, dCTP, dGTP, and TTP, 7 mM each of ATP, CTP, GTP, and UTP, and 0.15 uM each primer. Amplification takes place during incubation at 42°C. for 1-2 h.

Initially, primer T7-470-20-1-77F anneals to the target RNA, and is extended by AMV reverse transcriptase to form cDNA complementary to the starting RNA strand. Following degradation of the RNA strand by RNase H, reverse transcriptase catalyzes the synthesis of the second strand DNA, resulting in a double-stranded template containing the (double-stranded) T7 promoter sequence. RNA transcription results in production of single-stranded RNA. This RNA then serves to re-enter the cycle for additional rounds of amplification, finally resulting in a pool of high-concentration product RNA. The product is predominantly single-stranded RNA of the same strand as the primer containing the T7 promoter (T7-470-20-1-77F), with much smaller amounts of cDNA.

Alternatively, the other primer (470-20-1-211R) may contain the T7 promoter, or both primers may contain the promoter, resulting in production of both strands of RNA as products of the reaction. Products of the 3SR reaction may be detected, characterized, or quantitated by standard techniques for the analysis of RNA (e.g., Northern blots, RNA slot or dot blots, direct gel electrophoresis with RNA-staining dyes). Further, the products may be detected by methods making use of biotin-avidin affinity interactions or specific hybridizations of nucleic acid probes.

In one technique for rapid and specific analysis of 3SR products, solution hybridization of the product to radiolabelled oligonucleotide 470-20-1-152R (SEQ ID NO:21) is followed by non-denaturing polyacrylamide gel electrophoresis. This assay (a gel mobility shift-type assay) results in the detection of specific probe-product hybrid as a slower-moving band than the band corresponding to unhybridized oligonucleotide.

### 2. LIGASE CHAIN REACTION (LCR)

As another example of a detection system, the HGV sequence may form the basis for design of ligase chain reaction (LCR) primers. LCR makes use of the nick-closing activity of DNA ligase to join two immediately adjacent oligonucleotides possessing adjacent 5'-phosphate ("donor" oligo) and 3'-hydroxyl ("acceptor" oligo) terminii. The property of DNA ligase to join only fully complementary ends in a template-dependent way, leads to a high degree of specificity, in that ligation will not occur unless the terminii to be linked are perfectly matched in sequence to the target strand.

As an alternative to PCR, with some advantages in terms of specificity for discrimination of single base mismatches between primer and target nucleic acid, the LCR may be used to detect or "type" strains of virus possessing homology to HGV sequences. These techniques are suitable for assessing the presence of specific mutations when such base changes are known to confer drug resistance (*e*.*g*., Larder and Kemp; Gingeras, *et al*., 1991).

In the presence of template-complementary donor and acceptor oligonucleotides and oligonucleotides complementary to the donor and acceptor, exponential amplification by LCR is possible. In this embodiment, each round of ligation generates additional template for subsequent rounds, in a cyclic reaction.

For example, primer 470-20-1-211R (SEQ ID NO:10), an adjacent oligonucleotide (**B**, SEQ ID NO:22) and cognate oligos (211R', SEQ ID NO:23, and **B'**, SEQ ID NO:24), can be used to perform LCR amplification of the sequence of this invention. Reverse transcription is first performed by standard methods to generate cDNA, which is then amplified in reactions containing 0.1-1 *µ*M each of the four LCR primers, 20 mM Tris-HCl, pH 8.3 (room temperature), 25 mM KCl, 10 mM MgCl₂, 10 mM dithiothreitol (DTT), 0.5 mM NAD+, 0.01% Triton X-100, and 5 Units of DNA ligase (Ampligase, Epicentre Technologies, Madison, WI, or other commercial supplier of thermostable DNA ligase), in 25 ul reactions.

Thermal cycling is performed at 94°C. for 1 min. 30 s; 94°C. for 1 min., 65°C. for 2 min., repeated for 25-40 cycles. Specificity of product synthesis depends on primer-template match at the 3'-terminal position. Products are detected by polyacrylamide gel electrophoresis, followed by ethidium bromide staining; alternatively, one of the acceptor oligos (211R' or B) is 5'-radiolabelled for visualization by autoradiography following gel electrophoresis.

Alternatively, a donor oligo is 3'-end-labelled with a specific bindable moiety (e.g., biotin), and the acceptor is 5'-labelled with a specific detectable group (e.g., a fluorescent dye), for solid phase capture and detection.

### 3. METHODS FOR ANALYSIS OF AMPLIFIED DNA

Numerous techniques have been described for the analysis of amplified DNA. Several such techniques are advantageous for high-throughput applications, where gel electrophoresis is impractical, for example, rapid and high-resolution HPLC techniques (Katz and Dong). However, in general, methods for infectious disease organism screening using nucleic acid probes involve a separate post-amplification hybridization step in order to assure requisite specificity for pathogen detection.

One such detection embodiment is an affinity-based hybrid capture technique (Holodniy, *et al.*). In this embodiment the PCR is conducted with one biotinylated primer. Following amplification, the double-stranded product is denatured then hybridized to a peroxidase-labelled probe complementary to the strand having incorporated the biotinylated primer. The hybridized product is then incubated in a buffer which is in contact with an avidin (or streptavidin) coated surface (e.g., membrane filter, microwell, latex or paramagnetic beads).

The mass of coated solid phase which contacts the volume of PCR product to be analyzed by this method must contain sufficient biotin-binding sites to capture essentially all of the free biotinylated primer, as well as the much lower concentration of biotinylated PCR product. Following three to four washes of the solid phase, bound hybridized product is detected by incubation with o-phenylenediamine in citrate buffer containing hydrogen peroxide.

Alternatively, capture may be mediated by probe-coated surfaces, followed by affinity-based detection via the biotinylated primer and an avidin-reporter enzyme conjugate (Whetsell, *et al*.).

### 4. ADDITIONAL METHODS

Viral sequences of the present invention may also form the basis for a signal amplification approach to detection, using branched-chain DNA probes. Branched-chain probes (Horn and Urdea; Urdea) have been described for detection and quantification of rare RNA and DNA sequences (Urdea, *et al.*). In this method, an oligonucleotide probe (RNA, DNA, or nucleic acid analogue) is synthesized with a sequence complementary to the target RNA or DNA. The probe also contains a unique branching sequence or sequences not complementary to the target RNA or DNA.

This unique sequence constitutes a target for hybridization of branched secondary detector probes, each of which contains one or more other unique sequences, serving as targets for tertiary probes. At each branch point in the signal amplification pathway, a different unique sequence directs hybridization of secondary, tertiary, etc., detection probes. The last probe in the series typically is linked to an enzyme useful for detection (*e.g.*, alkaline phosphatase). The sequential hybridization of primers eventually results in the buildup of a highly-branched structure, the arms of which terminate in enzyme-linked probes.

Enzymatic turnover provides a final amplification, and the choice of highly sensitive chemiluminescent substrates (e.g., LumiPhos, Lumigen, Detroit, MI, as a substrate for alkaline phosphatase labels) results in exquisite sensitivity, on the order of 10,000 molecules or less of original target sequence per assay. In such a detection method, amplification depends only on molecular hybridization, rather than enzymatic mechanisms, and is thus far less susceptible to inhibitory substances in clinical specimens than, for example, PCR. Thus, this detection method allows the use of crude techniques for nucleic acid release in test samples, without extensive purification before assay.

Amplification for sensitive detection of the viral sequences of the present invention may also be accomplished by the Q-β replicase technique (Cahill, *et al.;* Lomell, *et al.;* Pritchard, *et al.*). In this method, a specific probe is designed to be complementary to the target sequence. This probe is then inserted by standard molecular cloning techniques into the sequence of the replicatable RNA from Q-β phage. Insertion into a specific region of the replicon does not prevent replication by Q-β replicase.

Following molecular hybridization, and several cycles of washing, the replicase is added and amplification of the probe RNA ensues. "Reversible target capture" is one known technique for reducing the potential background from replication of unhybridized probes (Morrissey, *et al.*). Amplified replicons are detectable by standard molecular hybridization techniques employing DNA, RNA or nucleic acid analogue probes.

Additional methods for amplification and detection of rare DNA or RNA sequences are known in the literature and preferred to the PCR for some applications in the field of molecular diagnostics. These alternative techniques may form the basis for detection, characterization (e.g., sequence diversity existing as multiple related strains of the sequence described herein, genotypic changes characteristic of drug resistance), or quantification of the sequence disclosed in the present invention.

Also forming part of the invention are assay systems or kits for carrying out the amplification/hybridization assay methods just described. Such kits generally include either specific primers for use in amplification reactions or hybridization probes.

### D. THERAPEUTIC USES.

As discussed above, the HGV antigens of the present invention can be used in vaccine preparation.

Further, antibodies generated against the polypeptide antigens of the present invention can be used for passive immunotherapy or passive immunoprophylaxis. The antibodies can be administered in amounts similar to those used for other therapeutic administrations of antibody. For example, pooled gamma globulin is administered at 0.02-0.1 ml/lb body weight during the early incubation of other viral diseases such as rabies, measles and hepatitis B to interfere with establishment of infection. Thus, antibodies reactive with the HGV antigens can be passively administered alone or in conjunction with another anti-viral agent to a host infected with HGV to enhance the ability of the host to deal with the infection.

The HGV sequences disclosed herein identify HGV as a member of the Flaviviridae family (see above). The Flaviviridae are classified into 3 genera, flaviviruses, petstiviruses, and the hepatitis C virus genera (Francki, *et al.*). All Flaviviridae possess a positive strand RNA genome of 9.0 - 12 kb in length which encodes a single long polypeptide of 3000-4000 amino acids. This polypeptide is proteolytically cleaved into approximately 10 proteins, including, a viral capsid protein, viral envelope protein(s), and a minimum of 5 non-structural proteins (NS). The non-structural proteins include a chymotrypsin like serine protease, RNA helicase (NS3), and an RNA-dependent RNA polymerase (NS5). The NS3 protein of Flaviviridae is required for proteolytic cleavage of the viral polypeptide. The NS5 protein is required for replication of the viral genome (Chambers, *et al.,* 1990a).

Additionally, several cellular proteins have been identified as being involved in the replication of the Flaviviridae. For example, cellular signal peptidase enzyme may be required to cleave the viral polypeptide at several cleavage sites, to allow for expression of the viral protease (Hijikata, *et al*.).

Inhibitors which prevent these proteins from carrying out their required functions in flavivirus replication may also have therapeutic value at treating infection with HGV. Finally cytokines or other polypeptides which are known to have antiviral activity and/or modulate the human immune system may be efficacious at treating HGV infection.

One compound known to inhibit Flaviviridae RNA dependent RNA polymerases, which by analogy may be expected to inhibit the activity of the NS5 protein of HGV, is the nucleotide analogue 1-B-D-ribofuranosyl-1-2,4-triazole, 3-carboxamide, also known as ribavirin (Patterson, *et al.*). The method of action of ribavirin is thought to involve depletion of intercellular guanine pools and interference with the capping of viral RNAs (Patterson *et al*.).

In individuals infected with HCV, significant reductions in viral titer and in serum levels of alanine aminotransferase (ALT -- an indicator enzyme for liver dysfunction) were observed while ribavirin was administered (Reichard, *et al.;* Di Bisceglie, *et al.*, 1992). Ribavirin appears to have broad efficacy for treating Flaviviridae infections, accordingly, beneficial results are expected after administration of ribavirin to individuals suffering from HGV derived liver disease.

Another class of compounds known to be efficacious for treating Flaviviridae infections include the cytokines interferon α, interferon δ, and interferon γ (Baron, *et al*.; Gutterman). Interferons are thought to act as antivirals by both (i) inducing the expression of cellular proteins that interfere with the replication and translation of viral RNAs, and (ii) by the activation of components of the human cellular immune system (Baron, *et al*.). The interferons have broad applicability to the treatment of viral infections including infection with HBV, HDV, and HCV (Gutterman; Farci, *et al*.). In particular, multiple studies have indicated that the interferons, either alone or in combination with other antiviral therapies, are effective at treating infection with hepatitis C virus (Di Bisceglie, *et al.,* 1989; Kakumu, *et al*.). Due to both the apparent hepatotropic nature of HGV and its classification in the family Flaviviridae, HGV infection may be expected to respond to similar interferon therapy.

Still another class of compounds with potent anti-viral activity are inhibitors of viral proteases (Krausslich, *et al*.). All Flaviviridae encode a chymotrypsin-like serine protease which is required to cleave multiple sites of the genome polypeptide at multiple sites in the non-structural region. The amino acid residues that make up the catalytic site of this protease are well described and include a Histidine, an Aspartic acid, and a Serine residue (Grakoui, *et al.*). Furthermore studies of the flavivirus, Yellow Fever Virus have indicated that mutation of the Serine residue of the active site inhibits viral replication (Chambers, *et al.,* 1990b).

Inhibitors of the HGV NS3 protein can be designed to mimic the transition state of enzymatic cleavage. Alternatively, such inhibitors may be isolated by mass screening of previously synthesized compounds. The activity of putative HGV NS3 proteinase inhibitors can be determined through the use of *in vitro* transcription/translation systems, which are widely used in Flaviviridae research (Hijikata, *et al.*; Grakoui, *et al*.).

Alternatively, the HGV genome can be cloned into a suitable vector for eukaryotic protein expression, such a bacculovirus or vaccinia, and the efficacy of the compounds can be determined in tissue culture systems (Grakoui, *et al.*). Similar approaches have been employed successfully to obtain potent inhibitors of the HIV protease (Vacca, *et al.;* Roberts, *et al.*).

Another approach to treating disease caused by infection with the HGV relies on the synthesis of antisense oligonucleotides (Tonkinson and Stein) or oligonucleotide analogs which encode portions of the sequences of HGV disclosed in the present invention. As is true for all Flaviviridae, it would be expected that the genome of HGV is a positive strand RNA molecule of 9 - 12 kb in size. The single stranded nature of the viral genome should make HGV exquisitely sensitive to antisense oligonucleotides. Possible target sequences which might be employed to inhibit viral replication include the 5' untranslated region of HGV, the ribosome binding site of HGV or other sequences which would interfere with the translation of the HGV genome.

Antisense oligonucleotides can be synthesized using commercially available synthesizers. Preferably the oligonucleotides are synthesized using phosphorodithioate backbones which have the advantage of being resistant to nuclease cleavage (Marshall & Caruthers). Additionally other oligonucleotide analogues, such as those having a uncharged or amide type backbone (Egholm, *et al*.) may be employed. These oligonucleotides are commercially available (Biosearch, Millipore, Bedford, MA) and advantageous in that their lack of charge allows them to cross biological membranes, which are typically resist the passage of charged macromolecules.

Oligonucleotides (or analogs thereof) for antisense applications are typically greater than 8 nucleotides in length to facilitate hybridization to a target sequence within the HGV genome. Upon hybridization of, for example, DNA oligomers to viral RNA target sequences, the hybridization complex can be degraded by a cellular enzyme such as RNAse H. The reduction in HGV templates then lessens the severity of HGV associated disease.

The usefulness and efficacy of the above described therapeutic methods can be evaluated *in vitro,* using the cell systems described above, and *in vivo,* using the animal model systems described above.

The following examples illustrate, but in no way are intended to limit the present invention.

### MATERIALS AND METHODS

Synthetic oligonucleotide linkers and primers were prepared using commercially available automated oligonucleotide synthesizers. Alternatively, custom designed synthetic oligonucleotides may be purchased from commercial suppliers.

Standard molecular biology and cloning techniques were performed essentially as previously described in Ausubel, *et al.,* Sambrook, *et al.,* and Maniatis, *et al.*

Common manipulations relevant to employing antisera and/or antibodies for screening and detection of immunoreactive protein antigens were performed essentially as described (Harlow, *et al.*). Similarly ELISA and Western blot assays for the detection of anti viral antibodies were performed either as described by their manufacturer (Abbott, N. Chicago, IL, Genelabs Diagnostics, Singapore) or using standard techniques known in the art (Harlow, *et al*).

### EXAMPLES

### EXAMPLE 1

### CONSTRUCTION OF PNF2161 cDNA LIBRARIES

### A. ISOLATION OF RNA FROM SERA.

One milliliter of undiluted PNF 2161 serum was precipitated by the addition of PEG (MW 6,000) to 8% and centrifugation at 12K, for 15 minutes in a microfuge, at 4°C. RNA was extracted from the resulting serum pellet essentially as described by Chomczynski.

The pellet was treated with a solution containing 4M guanidinium isothiocyanate, 0.18% 2- mercaptoethanol, and 0.5% sarcosyl. The treated pellet was extracted several times with acidic phenol-chloroform, and the RNA was precipitated with ethanol. This solution was held at -70°C for approximately 10 minutes and then spun in a microfuge at 4°C for 10 minutes. The resulting pellet was resuspended in 100 *µ*l of DEPC-treated (diethyl pyrocarbonate) water, and 10 *µ*l of 3M NaOAc, pH = 5.2, two volumes of 100% ethanol and one volume of 100% isopropanol were added to the solution. The solution was held at -70°C for at least 10 minutes. The RNA pellet was recovered by centrifugation in a microfuge at 12,000 x g for 15 minutes at 5°C. The pellet was washed in 70% ethanol and dried under vacuum.

### B. SYNTHESIS OF CDNA

### (i) FIRST STRAND SYNTHESIS

The synthesis of cDNA molecules was accomplished as follows. The above described RNA preparations were transcribed into cDNA, according to the method of Gubler *et al.* using random nucleotide hexamer primers (cDNA Synthesis Kit, BMB, Indianapolis, IN or GIBCO/BRL).

After the second-strand cDNA synthesis, T4 DNA polymerase was added to the mixture to maximize the number of blunt-ends of cDNA molecules. The reaction mixture was incubated at room temperature for 10 minutes. The reaction mixture was extracted with phenol/chloroform and chloroform isoamyl alcohol.

The cDNA was precipitated by the addition of two volumes of 100% ethanol and chilling at -70°C for 15 minutes. The cDNA was collected by centrifugation, the pellet washed with 70% ethanol and dried under vacuum.

### C. AMPLIFICATION OF THE DOUBLE STRANDED CDNA MOLECULES.

The cDNA pellet was resuspended in 12 *µ*l distilled water. To the resuspended cDNA molecules the following components were added: 5 *µ*l phosphorylated linkers (Linker AB, a double strand linker comprised of SEQ ID NO:1 and SEQ ID NO:2, where SEQ ID NO:2 is in a 3' to 5' orientation relative to SEQ ID NO:1 -- as a partially complementary sequence to SEQ ID NO:1), 2 *µ*l 10× ligation buffer (0.66 M Tris.Cl pH=7.6, 50 mM MgCl₂, 50 mM DTT, 10 mM ATP) and 1 *µ*l T4 DNA ligase (0.3 to 0.6 Weiss Units). Typically, the cDNA and linker were mixed at a 1:100 ratio. The reaction was incubated at 14°C overnight. The following morning the reaction was incubated at 70°C for three minutes to inactivate the ligase.

To 100 *µ*l of 10 mM Tris-Cl buffer, pH 8.3, containing 1.5 mM MgCl₂ and 50 mM KCl (Buffer A) was added about 1 *µ*l of the linker-ligated cDNA preparation, 2 *µ*M of a primer having the sequence shown as SEQ ID NO:1, 200 *µ*M each of dATP, dCTP, dGTP, and dTTP, and 2.5 units of *Thermus aquaticus* DNA polymerase (Taq polymerase). The reaction mixture was heated to 94°C for 30 sec for denaturation, allowed to cool to 50°C for 30 sec for primer annealing, and then heated to 72°C for 0.5-3 minutes to allow for primer extension by *Taq* polymerase. The amplification reaction, involving successive heating, cooling, and polymerase reaction, was repeated an additional 25-40 times with the aid of a Perkin-Elmer Cetus DNA thermal cycler (Mullis; Mullis, *et al*.; Reyes, *et al.,* 1991; Perkin-Elmer Cetus, Norwalk, CT).

After the amplification reactions, the solution was then phenol/chloroform, chloroform/isoamyl alcohol extracted and precipitated with two volumes of ethanol. The resulting amplified cDNA pellets were resuspended in 20 *µ*l TE (pH=7.5).

### D. CLONING OF THE CDNA INTO LAMBDA VECTORS.

The linkers used in the construction of the cDNAs contained an *Eco*RI site which allowed for direct insertion of the amplified cDNAs into lambda gt11 vectors (Promega, Madison WI or Stratagene, La Jolla, CA). Lambda vectors were purchased from the manufacturer (Promega) which were already digested with *EcoR*I and treated with alkaline phosphatase, to remove the 5' phosphate and prevent self-ligation of the vector.

The *Eco*RI-digested cDNA preparations were ligated into lambda gt11 (Promega). The conditions of the ligation reactions were as follows: 1 *µ*l vector DNA (Promega, 0.5 mg/ml); 0.5 or 3 *µ*l of the PCR amplified insert cDNA; 0.5 *µ*l 10 × ligation buffer (0.5 M Tris-HCl, pH=7.8; 0.1 M MgCl₂; 0.2 M DTT; 10 mM ATP; 0.5 mg/ml bovine serum albumin (BSA)), 0.5 *µ*l T4 DNA ligase (0.3 to 0.6 Weiss units) and distilled water to a final reaction volume of 5 *µ*l.

The ligation reactions were incubated at 14°C overnight (12-18 hours). The ligated cDNA was packaged by standard procedures using a lambda DNA packaging system ("GIGAPAK", Stratagene, LaJolla, CA), and then plated at various dilutions to determine the titer. A standard X-gal blue/white assay was used to determine recombinant frequency of the libraries (Miller; Maniatis et *al.*).

Percent recombination in each library was also determined as follows. A number of random clones were selected and corresponding phage DNA isolated. Polymerase chain reaction (Mullis; Mullis, *et al*.) was then performed using isolated phage DNA as template and lambda DNA sequences, derived from lambda sequences flanking the *Eco*RI insert site for the cDNA molecules, as primers. The presence or absence of insert was evident from gel analysis of the polymerase chain reaction products.

The cDNA-insert phage libraries generated from serum sample PNF 2161 was deposited with the American Type Culture Collection, 12301 Parklawn Dr., Rockville MD 20852, and has been assigned the deposit designation ATCC 75268 (PNF 2161 cDNA source).

### EXAMPLE 2

### IMMUNOSCREENING OF RECOMBINANT LIBRARIES

The lambda gt11 libraries generated in Example 1 were immunoscreened for the production of antigens recognizable by the PNF 2161 serum from which the libraries were generated. The phage were plated for plaque formation using the *Escherichia coli* bacterial plating strain *E. coli* KM392. Alternatively, *E. coli* Y1090R (Promega, Madison WI) may be used.

The fusion proteins expressed by the lambda gt11 clones were screened with serum antibodies essentially as described by Ausubel, *et al.*

Each library was plated at approximately 2 x 10⁴ phages per 150 mm plate. Plates were overlaid with nitrocellulose filters overnight. Filters were washed with TBS (10 mM, Tris pH 7.5; 150 mM NaCl), blocked with AIB (TBS buffer with 1% gelatin) and incubated with a primary antibody diluted 100 times in AIB.

After washing with TBS, filters were incubated with a second antibody, goat-anti-human IgG conjugated to alkaline phosphatase (Promega). Reactive plaques were developed with a substrate (for example, BCIP, 5-bromo-4-chloro-3-indolyl-phosphate), with NBT (nitro blue tetrazolium salt (Sigma)). Positive areas from the primary screening were replated and immunoscreened until pure plaques were obtained.

### EXAMPLE 3

### SCREENING OF THE PNF 2161 LIBRARY

The cDNA library of PNF 2161 in lambda gt11 was screened, as described in Example 2, with PNF 2161 sera. The results of the screening are presented in Table 1.

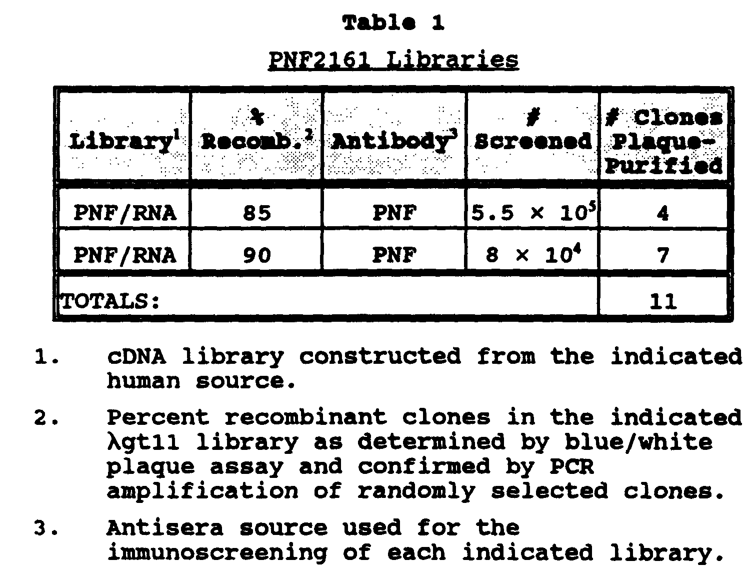

One of the clones isolated by the above screen (PNF 2161 clone 470-20-1, SEQ ID NO:3; β-galactosidase in-frame fusion translated sequence, SEQ ID NO:4), was used to generate extension clones, as described in Example 6. Clone 470-20-1 nucleic acid sequence is presented as SEQ ID NO:3 (protein sequence SEQ ID NO:4). The isolated nucleic acid sequence without the SISPA cloning linkers is presented as SEQ ID NO:19 (protein SEQ ID NO:20).

### EXAMPLE 4

### CHARACTERIZATION OF THE IMMUNOREACTIVE 470-20-1 CLONE

### A. SOUTHERN BLOT ANALYSIS OF IMMUNOREACTIVE CLONES.

The inserts of immunoreactive clones were screened for their ability to hybridize to the following control DNA sources: normal human peripheral blood lymphocyte (purchased from Stanford University Blood Bank, Stanford, California) DNA, and *Escherichia coli* KM392 genomic DNA (Ausubel, *et al.;* Maniatis, *et al.;* Sambrook, *et al.).* Ten micrograms of human lymphocyte DNA and 2 micrograms of *E. coli* genomic DNA were digested with *Eco*RI and *Hind*III. The restriction digestion products were electrophoretically fractionated on an agarose gel (Ausubel, *et al.*) and transferred to nylon or nitrocellulose membranes (Schleicher and Schuell, Keene, NH) as per the manufacturer's instructions.

Probes from the immunoreactive clones were prepared as follows. Each clone was amplified using primers corresponding to lambda gt11 sequences that flank the *Eco*RI cloning site of the gt11 vector. Amplification was carried out by polymerase chain reactions utilizing each immunoreactive clone as template. The resulting amplification products were digested with *Eco*RI, the amplified fragments gel purified and eluted from the gel (Ausubel, *et al.*). The resulting amplified fragments, derived from the immunoreactive clones, were then random prime labelled using a commercially available kit (BMB) employing ³²P-dNTPs.

The random primed probes were then hybridized to the above-prepared nylon membrane to test for hybridization of the insert sequences to the control DNAs. The 470-20-1 insert did not hybridize with any of the control DNAs.

As positive hybridization controls, a probe derivative from a human C-kappa gene fragment (Hieter) was used as single gene copy control for human DNA and a *E. coli* polymerase gene fragment was similarly used for *E. coli* DNA.

### B. GENOMIC PCR.

PCR detection was developed first to verify exogenicity with respect to several genomic DNAs which could have been inadvertently cloned during library construction, then to test for the presence of the cloned sequence in the cloning source and related specimen materials. Several different types of specimens, including SISPA-amplified nucleic acids and nucleic acids extracted from the primary source, and nucleic acids extracted from related source materials (*e.g.*, from animal passage studies), were tested.

The term "genomic PCR" refers to testing for the presence of specific sequences in genomic DNA from relevant organisms. For example, a genomic PCR for a Mystax-derived clone would include genomic DNAs as follows:
1. human DNA (1 *µ*g/rxn.)
2. Mystax DNA (0.1-1 *µ*g/rxn.)
3. *E. coli* (10-100 ng/rxn.)
4. yeast (10-100 ng/rxn.)

Human and Mystax DNAs are tested, as the immediate and ultimate source for the agent. *E. coli* genomic DNA, as a frequent contaminant of commercial enzyme preparations, is tested. Yeast is also tested, as a ubiquitous organism, whose DNA can contaminate reagents and thus, be cloned.

In addition, a negative control (*i.e*., buffer or water only), and positive controls to include approximately 10⁵c/rxn., are also amplified.

Amplification conditions vary, as may be determined for individual sequences, but follow closely the following standard PCR protocol: PCR was performed in reactions containing 10 mM Tris, pH 8.3, 50 mM KCl, 1.75 mM MgCl₂, 1.0 uM each primer, 200 uM each dATP, dCTP, and dGTP, and 300 *µ*M dUTP, 2.5 units Taq DNA polymerase, and 0.2 units uracil-N-glycosylase per 100 ul reaction. Cycling was for at least 1 minute at 94°C, followed by 30 to 40 repetitions of denaturation (92-94°C for 15 seconds), annealing (55-56°C for 30 seconds), and extension (72°C for 30 seconds). PCR reagents were assembled, and amplification reactions were constituted, in a specially-designated laboratory maintained free of amplified DNA.

As a further barrier to contamination by amplified sequences and thus compromise of the test by "false positives," the PCR was performed with dUTP replacing TTP, in order to render the amplified sequences biochemically distinguishable from native DNA. To enzymatically render unamplifiable any contaminating PCR product, the enzyme uracil-N-glycosylase was included in all genomic PCR reactions. Upon conclusion of thermal cycling, the reactions were held at 72°C to prevent renaturation of uracil-N-glycosylase and possible degradation of amplified U-containing sequences.

A "HOT START PCR" was performed, using standard techniques ("AMPLIWAX", Perkin-Elmer Biotechnology; alternatively, manual techniques were used), in order to make the above general protocol more robust for amplification of diverse sequences, which ideally require different amplification conditions for maximal sensitivity and specificity.

Detection of amplified DNA was performed by hybridization to specific oligonucleotide probes located internal to the two PCR primer sequences and having no or minimal overlap with the primers. In some cases, direct visualization of electrophoresed PCR products was performed, using ethidium bromide fluorescence, but probe hybridization was in each case also performed, to help ensure discrimination between specific and non-specific amplification products. Hybridization to radiolabelled probes in solution was followed by electrophoresis in 8-15% polyacrylamide gels (as appropriate to the size of the amplified sequence) and autoradiography.

Clone 470-20-1 was tested by genomic PCR, against human, *E*. *coli,* and yeast DNAs. No specific sequence was detected in negative control reactions, nor in any genomic DNA which was tested, and 10⁵ copies of DNA/reaction resulted in a readily-detectable signal. This sensitivity (*i.e.,* 10⁵/reaction) is adequate for detection of single-copy human sequences in reactions containing 1 ug total DNA, representing the DNA from approximately 1.5 x 10⁵ cells.

### C. DIRECT SERUM PCR

Serum or other cloning source or related source materials were directly tested by PCR using primers from selected cloned sequences. In these experiments, HGV viral particles were directly precipitated from sera with polyethylene glycol (PEG), or, in the case of PNF and certain other sera, were pelleted by ultracentrifugation. For purification of RNA, the pelleted materials were dissolved in guanidinium thiocyanate and extracted by the acid guanidinium phenol technique (Chomczynski, *et al.*).

Alternatively, a modification of this method afforded through and implemented by the use of commercially available reagents, *e.g*., "TRIREAGENT" (Molecular Research Center, Cincinnati, OH) or "TRIZOL" (Life Technologies, Gaithersburg, MD), and associated protocols was used to isolate RNA. In addition, RNA suitable for PCR analysis was isolated directly from serum or other fluids containing virus, without prior concentration or pelleting of virus particles, through the use of "PURESCRIPT" reagents and protocols (Gentra Systems, Minneapolis, MN).

Isolated DNA was used directly as a template for the PCR. RNA was reverse transcribed using reverse transcriptase (Gibco/BRL), and the cDNA product was then used as a template for subsequent PCR amplification.

In the case of 470-20-1, nucleic acid from the equivalent of 20-50 ul of PNF serum was used as the input template into each RT-PCR or PCR reaction. Primers were designed based on the 470-20-1 sequence, as follows: 470-20-1-77F (SEQ ID NO:9) and 470-20-1-211R (SEQ ID NO:10). Reverse transcription was performed using MMLV-RT (Gibco/BRL) and random hexamers (Promega) by incubation at room temperature for approximately 10 minutes, 42°C for 15 minutes, and 99°C for 5 minutes, with rapid cooling to 4°C. The synthesized cDNA was amplified directly, without purification, by PCR, in reactions containing 1.75 mM MgCl₂, 0.2-1 *µ*M each primer, 200 uM each dATP, dCTP, dGTP, and dTTP, and 2.5-5.0 units Taq DNA polymerase ("AMPLITAQ", Perkin-Elmer) per 100 ul reaction. Cycling was for at least one minute at 94°C, followed by 40-45 repetitions of denaturation (94°C for 15 seconds for 10 cycles; 92°C or 94°C for 15 seconds for the succeeding cycles), annealing (55°C for 30 seconds), and extension (72°C for 30 seconds), in the "GENEAMP SYSTEM 9600" thermal cycler (Perkin-Elmer) or comparable cycling conditions in other thermal cyclers (Perkin-Elmer; MJ Research, Watertown, MA).

Positive controls consisted of (i) previously amplified PCR product whose concentration was estimated using the Hoechst 33258 fluroescence assay, (ii) purified plasmid DNA containing the DNA sequence of interest, or (iii) purified RNA transcripts derived from plasmid clones in which the DNA sequence of interest is disposed under the transcriptional control of phage RNA promoters such as T7, T3, or SP6 and RNA prepared through the use of commercially available *in vitro* transcription kits. In addition, an aliquot of positive control DNA corresponding to approximately 10-100 copies/rxn. can be spiked into reactions containing nucleic acids extracted from the cloning source specimen, as a control for the presence of inhibitors of DNA amplification reactions. Each separate extract was tested with at least one positive control.

Specific products were detected by hybridization to a specific oligonucleotide probe 470-20-1-152F (SEQ ID NO:16), for confirmation of specificity. Hybridization of 10 ul of PCR product was performed in solution in 20 ul reactions containing approximately 1 × 10⁶ cpm of ³²P-labelled 470-20-1-152F. Specific hybrids were detected following electrophoretic separation from unhybridized oligo in polyacrylamide gels, and autoradiography.

In addition to PNF, extracted nucleic acids from normal serum was also reverse transcribed and amplified, using the "serum PCR" protocol sequence. No signal was detected in normal human serum. The specific signal in PNF serum was reproducibly detected in multiple extracts, with the 470-20-1 specific primers.

### D. AMPLIFICATION FROM SISPA UNCLONED NUCLEIC ACIDS

SISPA (Sequence-Independent Single Primer Amplification) amplified cDNA was used as templates (Example 1). Sequence-specific primers designed from selected cloned sequences were used to amplify DNA fragments of interest from the templates. Typically, the templates were the SISPA-amplified samples used in the cloning manipulations. For example, amplification primers 470-20-1-77F (SEQ ID NO:9) and 470-20-1-211R (SEQ ID NO:10) were selected from the clone 470-20-1 sequence (SEQ ID NO:3). These primers were used in amplification reactions with the SISPA-amplified PNF2161 cDNA as a template.

The identity of the amplified DNA fragments were confirmed by (i) hybridization with the specific oligonucleotide probe 470-20-1-152F (SEQ ID NO:16), designed based on the 470-20-1 sequence (SEQ ID NO:3) and/or (ii) size. The probe used for DNA blot detection was labelled with digoxygenin using terminal transferase according to the manufacturer's recommendations (BMB). Hybridization to the amplified DNA was then performed using either Southern blot or liquid hybridization (Kumar, *et al.*, 1989) analyses.

Positive control DNA used in the amplification reactions was previously amplified PCR product whose concentration was estimated by the Hoechst 33258 fluorescence assay, or, alternatively, purified plasmid DNA containing the cloned inserts of interest.

The 470-20-1 specific signal was detected in cDNA amplified by PCR from SISPA-amplified PNF2161. Negative control reactions were nonreactive, and positive control DNA templates were detected.

### E. AMPLIFICATION FROM LIVER RNA SAMPLES.

RNA was prepared from liver biopsy material following the methods of Cathal, *et al.,* wherein tissue was extracted in 5M guanidine thiocyanate followed by direct precipitation of RNA by 4M LiCl. After washing of the RNA pellet with 2M LiCl, residual contaminating protein was removed by extraction with phenol:chloroform and the RNA recovered by ethanol precipitation.

The 470-20-1 specific primers were also used in amplification reactions with the following RNA sources as substrate: normal mystax liver RNA, normal tamarin (*Sanguinus labiatus*) liver RNA, and MY131 liver RNA. MY131 is a mystax that was inoculated intravenously with 1 ml of PNF 2161 plasma. There were obvious elevations of a liver enzyme (SCID) and histological evidence of an apparent viral infection. The histological correlation was most obvious in the liver of MY131, whose liver was obtained at or near the peak of SCID activity. Mystax 131 liver RNA did not give amplified products with the noncoding primers (SEQ ID NO:7 and SEQ ID NO:8) of HCV.

The amplification reactions were carried out in duplicate for two experiments. The results of these amplification reactions are presented in Table 2.

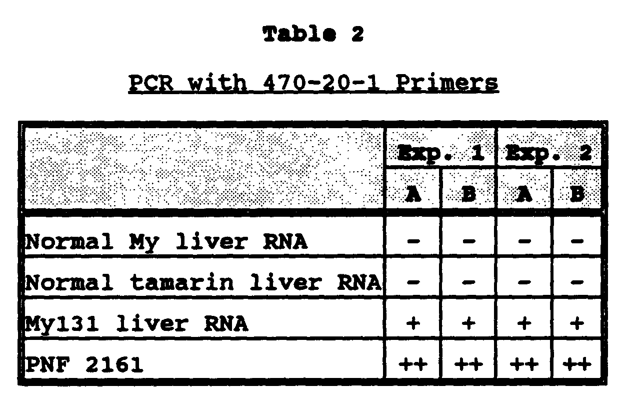

These results demonstrate the 470-20-1 sequences are present in the parent serum sample (PNF 2161) and in a liver RNA sample from a passage animal of the PNF 2161 sample (MY131). However, both control RNAs were negative for the presence of 470-20-1 sequences.

### F. SCREENING OF A SERUM PANEL FOR HGV SEQUENCES BY POLYMERASE CHAIN REACTION USING RNA TEMPLATES.

### 1. HIGH-ALT DONORS

The disease association between HGV and liver disease was assessed by polymerase chain reaction screening, using HGV specific primers, of sera from hepatitis patients and from blood donors with abnormal liver function. The latter consisted of serum from blood donations with serum ALT levels greater than 45 International Units per ml.

A serum panel consisting of 152 total sera was selected. The following sera were selected for the serum panel: 104 high-ALT sera from screened blood donations at the Stanford University Blood Bank (SUBB); 34 N-(ABCDE) hepatitis sera from northern California, Egypt, and Peru; and 14 sera from other donors suspected of having liver disease and/or hepatitis virus infection. The negative controls for the panel were as follows: 9 highly-screened blood donors (SUBB) notable for the absence of risk factors for viral infections ("supernormal" sera, *e.g.*, O-negative, Rh-negative; negative for HIV, known hepatitis agents, and CMV; whose multiple previous blood donations had been transfused without causing disease); and 2 random blood donors. These sera were assayed for the presence of HGV specific sequences by RT-PCR using the 470-20-1 primers 77F (SEQ ID NO:9) and 211R (SEQ ID NO:10).

RNA extraction and RT-PCR were performed essentially as described in Example 4C, except that the primer 470-20-1-211R was 5'-biotinylated to facilitate rapid screening of amplified products by a method involving hybridization in solution, followed by affinity capture of hybridized probe using streptavidin-coated paramagnetic beads. Methods for the analysis of nucleic acids by hybridization to specific labelled probes with capture of the hybridized sequences through affinity interactions are well known in the art of nucleic acid analysis.

Depending on the amount of serum available for testing, RNA from 30 to 50 *µ*l of serum was used per RT/PCR reaction. Each serum was tested in duplicate, with positive controls corresponding to 10, 100, or 1000 copies of RNA transcript per reaction and with appropriate negative (buffer) controls. No negative controls were reactive, and at least 10 copies per reaction were detectable in each PCR run. Indeterminate results were defined as specific hybridizing signal being present in only one of two duplicate reactions.

Efficient, highly sensitive analysis of the products from the amplification analysis of this serum panel was performed using an instrument specifically designed for affinity-based hybrid capture using electrochemiluminscent oligonucleotide probes (QPCR System 5000™, Perkin-Elmer). Assays utilizing the QPCR 5000™ have been described (DiCesare, et al; Wages, et al).

The products of each reaction were assayed by hybridization to probe 470-20-1-152F (5'-end-labelled with an electrochemiluminescent ruthenium chelate), and measurement using the "QPCR 5000." Based on a cutoff of the sum of the mean and three times the standard deviation of negative controls in a given amplification run, a total of 34 possible positives were selected for confirmatory testing.

The 34 samples were analyzed by solution hybridization and electrophoresis (Example 4C). Out of these 34 samples, 6 sera (*i.e.,* 6/152) were shown to have specific hybridizing sequences in duplicate reactions. Of these six samples, three were strongly reactive by comparison with positive controls: one High-ALT serum from SUBB, and two N-(ABCDE) sera from Egypt.

A second blood sample was obtained from the highly positive SUBB serum donor one year after the initial sample was taken. The second serum sample was confirmed to be HGV positive by the PCR methods described above. This result confirms persistant infection by HGV in a human. The serum was designated "JC." Further, the serum donor was HCV negative (determined by seroreactivity tests and PCR) and antibody negative for HAV and HBV.

In addition, a third N-(ABCDE) serum from Egypt, a northern California blood donor with N-(ABCDE) hepatitis, and a N-(ABCDE) hepatitis serum, were also shown to be weakly positive by this method. Two other sera gave indeterminate results, defined as the presence of specific sequences in one of two amplification reactions.

Subsequent PCR analysis of replicate serum aliquots from these HGV-positive and indeterminate sera resulted in HGV-positive results in 6 of 8 sera tested and indeterminate results in the remaining 2 sera.

A second primer set was used for the confirmation of HGV positive samples. This primer set (GV57-4512MF, SEQ ID NO:121, and GV57-4657MR, SEQ ID NO:122) for diagnostic amplification, was selected from a conserved region of HGV derived from the putative NS5 coding region. An approximately 2.2 kb fragment was amplified from each of 5 separate HGV isolates. The primers used for the amplification reactions were 470EXT4-2189R (SEQ ID NO:119) and 470EXT4-29F (SEQ ID NO:120). The amplified DNA fragments were sequenced and the sequences aligned. Highly conserved regions were identified from the alignment and optimal primer sequences were designed incorporating mixed base synthesis at those positions that remained divergent throughout the five sequences. The resulting NS5 primers were as follows: GV57-4512MF, SEQ ID NO:121, and GV57-4657MR, SEQ ID NO:122. These primers were used to amplify a diagnostic fragment of 165 bp from test samples.

An internal probe sequence, GV22dc-89MF (SEQ ID NO:123) was derived from another highly conserved region for detection of the specifically amplified product. The probe is also of sufficient length to allow for detection of minimally divergent HGV sequences under lowered stringency conditions.

Analysis of specimens for the presence of the diagnostic NS5 sequence followed the same conditions for sample preparation, amplification, and liquid hybridization as described for the 470-20-1 primers (Example 4C). The concordance of results for sera samples analyzed by PCR using both the 470-20-1 and NS5 primer pairs are shown in Table 3.

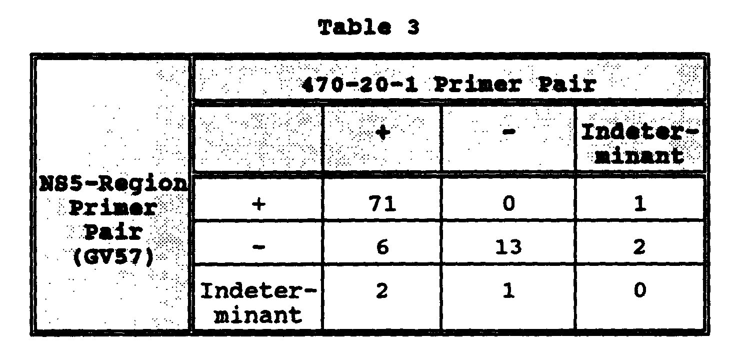

Further PCR analyses of additional aliquots obtained from the 8 sera identified above as being HGV-positive were carried out using the 470-20-1 primer set (SEQ ID NO:9 and SEQ ID NO:10) and the NS5 primer set. In these assays, the HGV PCR analyses gave consistently positive results in 5 of the 8 sera. These results are presented in Table 4.

In contrast, none of the two random donors or nine highly-screened "supernormal" sera was positive in either set of PCR analysis.

These results reinforce the disease association between HGV and liver disease.

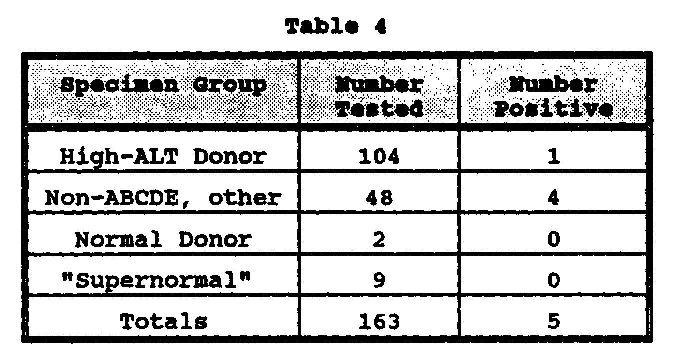

Further testing of sera from High-ALT donors has yielded the following results. A total of 495 sera have been tested, in addition to the initial panel of 104 sera described above. Of these 495 specimens, 6 were identified as HGV positive using the primer pair 470-20-1-77F (SEQ ID NO:9) and 470-20-1-211R (SEQ ID NO:10). These six sera have the following HCV profiles: R25342, HCV negative; R17749, HCV positive; J53171, HCV positive, HBV positive; J54406, HCV negative; R08074, HCV negative; and X31049, HCV negative. Positive scores are based on repeated reactivity in at least 2 separate reactions. R25342 was tested and confirmed positive by PCR using the NS5 primer pair. Accordingly, a detection rate of approximately 1.2% has been observed (7 of 599 tested).

Freshly-obtained plasma samples from blood donors with elevated ALT were also obtained from SUBB, the Peninsula Blood Bank (Burlingame, CA), and the New York Blood Center (New York, NY), for testing for HGV RNA by PCR (470-20-1 primer pair). Of 214 total donations which were tested, a total of 5 (approximately 2.3%) were HGV RNA positive. These five sera have the following HCV profiles: T55806, HCV positive; T55875, HCV negative; T56633, HCV negative; R38730, HCV negative; and 3831781, HCV negative. Subsequent donations from two of these donors, T55806 and T55875, were also HGV RNA positive. T55806, T55875 and T56633 were tested and confirmed positive by PCR using the NS5 primer pair.

### 2. SCREENING OF ACCEPTED BLOOD DONORS

To assess the prevalence of HGV in the normal blood donor population, serum was collected from screened blood donors for transfusion at SUBB. A total of 968 specimens, representing 769 unique donors, was tested for HGV RNA. The samples were screened by PCR using the 470-20-1 primer pair.

A total of 16 sera were identified as having detectable HGV RNA. Of these, 6 represent duplicates from 3 donors, such that a total of 13 unique donors of 769 tested were HGV positive by RNA PCR. All positive samples were tested and confirmed positive by PCR using the NS5 primer pair. These donors were characterized by normal ALT levels, as well as otherwise normal serology. Accordingly, approximately 1.7% of the sera tested in the normal blood donor population are HGV positive. Therefore, the presence of HGV was detected in both accepted and rejected blood donors.

### 3. SPECIMENS FROM VARIOUS GEOGRAPHIC LOCALES.

The presence of HGV infection in populations of hepatitis patients from geographically widespread sources was assessed by PCR. The PCR reactions were carried out essentially as described in Example 4C using the 470-20-1 PCR primer pairs. Serum samples from Egypt, Greece, Australia (see Example 4F-4), Peru, England, Italy, Germany, South Korea, United States and Japan were tested. HGV RNA was detectable in subsets of all populations tested.

### 4. POST-TRANSFUSION ASSOCIATED HGV INFECTION AND PARENTERAL TRANSMISSION.

HGV RNA was detected in several post-transfusion hepatitis cases (those of Japanese and European origin were included in Example 4F-3). For 4 total cases, one from Japan, two from the U.S. and one from Australia, multiple time-points were assayed for the presence of HGV RNA. For 3 of these cases, (i) pre-transfusion samples were available to estabish previous HGV status of the patient, and (ii) samples were available from individual blood donors to those three cases, to establish donor HGV status.

The first case was a Japanese patient transfused on 12/2/80. Following the transfusion the patient developed Non-B Non-C hepatitis. A total of 5 sera from this patient were tested for HGV RNA by PCR using the 470-20-1 primer pair. HGV RNA was detectable from about 2 weeks to about 8 months following transfusion. A sample taken greater than 1 year post-transfusion was indeterminate (i.e., positive in one duplicate reaction only). No pre-transfusion sample was available for testing.

Cases BIZ and STO (Tables 5 and 6, respectively) were from a prospectively-followed heart surgery study (Alter, *et al.*, 1989) conducted at the NIH. For each of these patients, pre-transfusion sera were available and were determined to be negative for HGV RNA by PCR using the 470-20-1 primer pair. BIZ tested positive for HGV RNA from day one post-transfusion to week 198 post-transfusion. Of 9 total blood donors to BIZ, 2 out of 8 tested were found to be HGV positive. STO tested positive for HGV RNA from week 5 post-transfusion through week 92 post-transfusion.

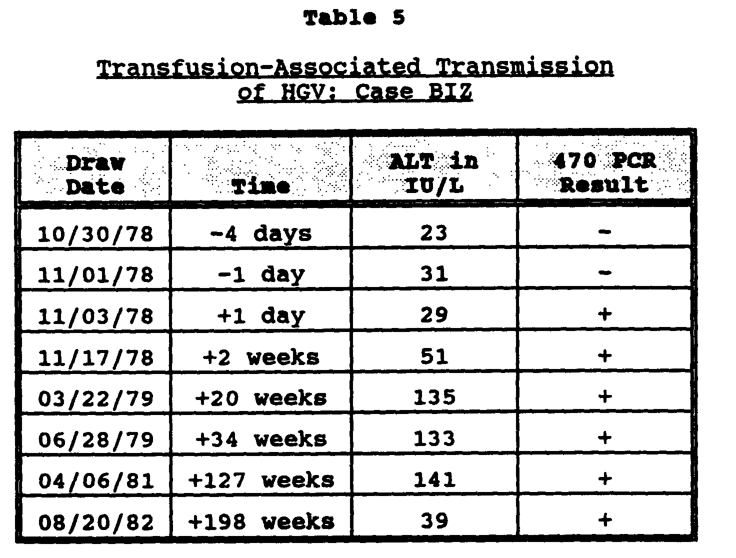

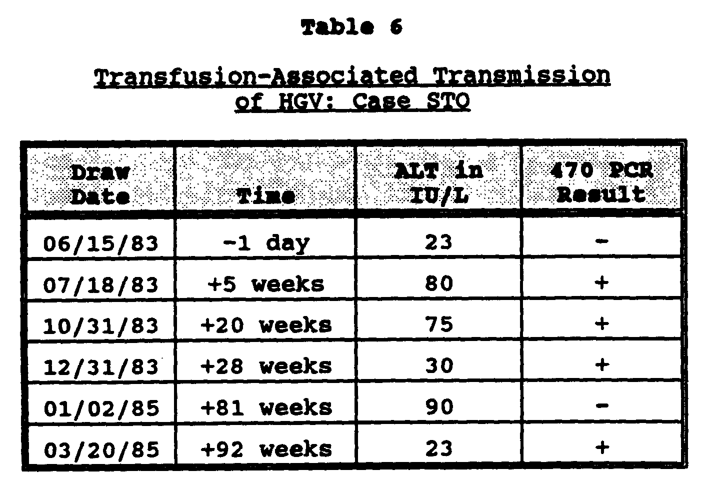

The fourth case, also prospectively-defined, was a cardiac surgery patient who participated in a post-transfusion hepatitis study conducted in Sydney, Australia. The patient (PA-124), having no other identifiable risk factors, received 14 units of blood during surgery (4 units packed red cells, 10 units of platelets). Of these 14 units one was HGV positive; the other 13 were HGV negative. HBV and HCV serologies of the 14 blood donors were negative with the exception of a reactive HCV EIA (first generation test). No other HCV test confirmed the positive finding.

In patient PA-124 (Table 7), serum ALT was elevated beginning with a sample taken two weeks post-operation, and was observed to be at least 10 times the pre-operation level for a period of 14 weeks. PCR results for HCV performed on pre-transfusion, 4 week, and 8 week sera from PA-124, were all negative. Serum from this patient was tested for HGV RNA using the 470-20-1 PCR primers. A pre-transfusion sample was negative for HGV RNA. Positive results were demonstrated following transfusion, coinciding with and succeeding the ALT elevation. The presence of HGV RNA was detected out to one year post-transfusion. These data support the conclusion that HGV may be parenterally transmitted.

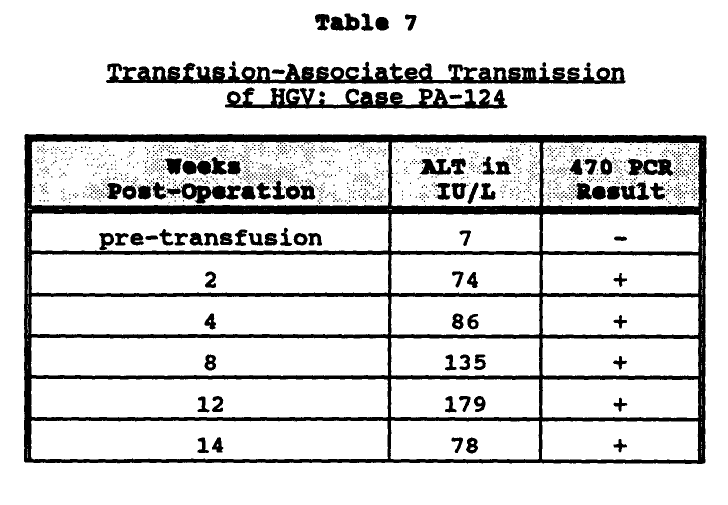

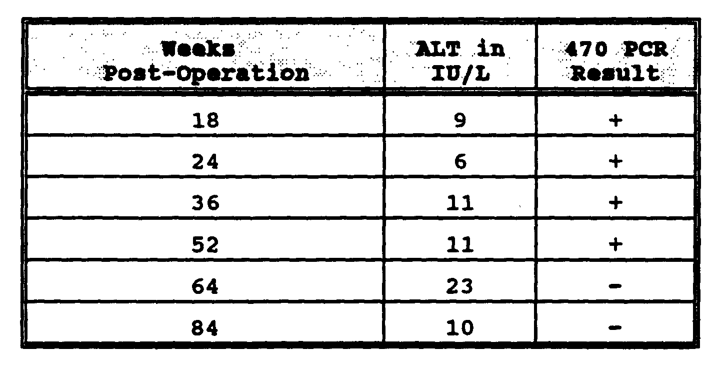

In addition to prospectively-defined post-transfusion transmission cases, additional cases of HGV infection were identified in risk groups defined by multiple transfusions and intravenous drug use (IVDU) (Table 8).

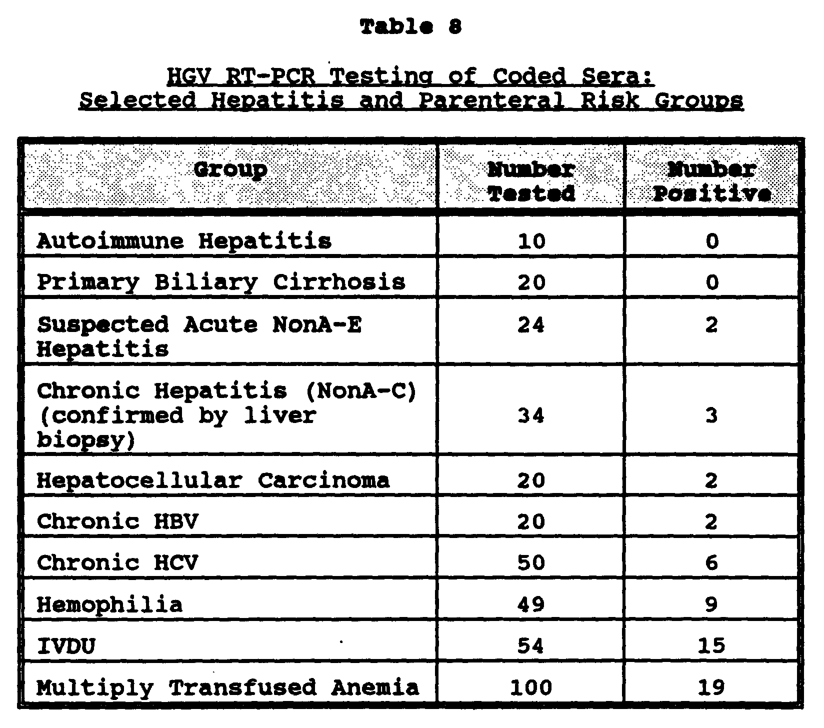

Among 100 multiply-transfused sickle cell anemia and thalassemia patients, 19 (19%) were found to have detectable serum HGV RNA. Similarly, 9 of 49 hemophilia patients (18%) were HGV positive with 470-20-1 and NS5 primers. Significantly, 15 of 54 (28%) IVDU were found to be PCR positive for HGV RNA. Infection rates in these parenteral risk groups (18-28%) appear to be higher than rates in blood donors with elevated ALT (1-2%). These results reinforce the significance of the parenteral route for HGV transmission.

### 5. PCR SCREENING OF SELECTED HEPATITIS DISEASE GROUPS

Sera from patients with acute and chronic hepatitis, hepatocellular carcinoma, HBV infection or HCV infection were tested for the presence of HGV using polymerase chain reaction (data presented in Table 8). In each of sets of specimens from patients with liver disease, HGV positive specimens were demonstrated (with the exception of specimens from patients with autoimmune hepatitis and primary biliary cirrhosis, both conditions not thought to be exclusively associated with an infectious agent).

As shown in the collections of sera from post-transfusion hepatitis patients (Example 4F-4), HGV infection is established during acute hepatitis, but circulating viral RNA continues to be detected during chronic infection for periods of time measured in months to years.

Approximately 10-20% co-infection rates were observed in patients with HBV and HCV infection. HGV infection is thus shown to be associated with hepatitis with or without co-infection with other hepatitis viruses. Co-infection may reflect similar risk factors and routes of transmission for these hepatitis viruses. As noted above, there is a higher prevalence of HGV in parenteral risk groups, such as hemophiliacs, IVDU's, and multiply transfused anemia patients (compared with other hepatitis risk groups).

### 6. PERSISTENT INFECTION BY HGV IN HUMANS

Post-transfusion hepatitis cases BIZ, STO, and PA-124 were show to have PCR-detectable viral RNA up to 3.8, 1.8, and 1.0 years, respectively, following transfusion and acute infection. Additional serum samples were obtained from donor JC (Example 4F-1), one year and 1.5 years following the initial positive sample. These follow-up serum samples were also HGV positive. Additional sera from other high-ALT donors (T55806, T55875, R25342), obtained several months following the serum sample in which HGV infection was originally detected, were also positive. Similarly, when HGV infection was established in an experimental primate (CH1356, Example 4H), HGV RNA was detected over 1.5 years following innoculation. These data establish persistent HGV viremia in humans and experimental primates.

### G. AMPLIFICATION OF LONG FRAGMENTS FROM PATIENT RNA FOR SEQUENCING.

PCR primers were designed to amplify several informative regions of the HGV genome in order to obtain sequence information on varied HGV isolates. The primers 470EXT4-2189R (SEQ ID NO:119) and 470EXT4-29F (SEQ ID NO:120) were designed to amplify a 2.2 kb fragment that contained the original 470-20-1 sequence. RNA from samples was reverse-transcribed using "SUPERSCRIPT II" reverse transcriptase (Gibco/BRL, Gaithersburg, MD). The resulting cDNA was amplified using reagents for efficient long-range PCR ("XL PCR BUFFERS" and "rTth-XL", Perkin Elmer/Applied Biosystems Div., Foster City, CA).

The amplification reaction was considered to be positive if a band of the correct size on agarose gel electrophoresis was detected. The sample was confirmed as positive by preliminary DNA sequencing of the amplification product. The following sera samples tested positive for HGV RNA by this amplification method: PNF2161; R10291 (JC); and specimens from each of the North American, Egyptian, and Japanese groups. However, no positive samples were detected from the Peruvian sera.

Successful amplification from a variety of HGV-positive specimens provides confirmation of the results obtained by PCR amplification using the 470-20-1 primer pair discussed above. Failure to obtain amplification, however, may reflect poor RNA quality or low copy number or local sequence differences among isolates such that the selected primer sets would not function universally.

In order to obtain sequence information from the putative 5'-untranslated region of the HGV genome, primers were designed to amplify fragments from the 5'-untranslated region (based on the HGV PNF 2161-variant). The two fragments were defined by the following primer sets: FV94-22F (SEQ ID NO:124) and FV94-724R (SEQ ID NO:125), yielding a 728 base pair fragment; and FV94-94F (SEQ ID NO:126) and FV94-912R (SEQ ID NO:127), yielding an 847 base pair fragment.

The conditions just described to promote efficient long-range PCR were used. Products were obtained from most of the samples tested, providing additional confirmation of the presence of HGV RNA in the samples.

### H. INFECTIVITY OF HGV IN PRIMATES.

Two chimpanzees (designated CH1323 and CH1356), six cynomolgus monkeys (CY143, CY8904, CY8908, CY8912, CY8917, and CH8918), and six Mystax (MY29, MY131, MY98, MY187, MY229, MY254) subjects were inoculated with PNF 2161. Pre-inoculation and post-inoculation sera were monitored for ALT and for the presence of HGV RNA sequences (as determined by PCR screening - described above).

One cynomologous monkey (CY8904) showed a positive RNA PCR result (39 days post-inoculation) and one indeterminant result from a total of 17 seperate blood draws. In one chimpanzee, designated CH1356, was sustained viremia observed by RT-PCR. As shown in Table 9, no significant ALT elevation was observed, and circulating virus was detected only at time points considerably after inoculation. Viremia was observed at and following 118 days post-inoculation. Suggestive reactivity was also observed in the first post-inoculation time-point (8 days), which may indicate residual inoculum.

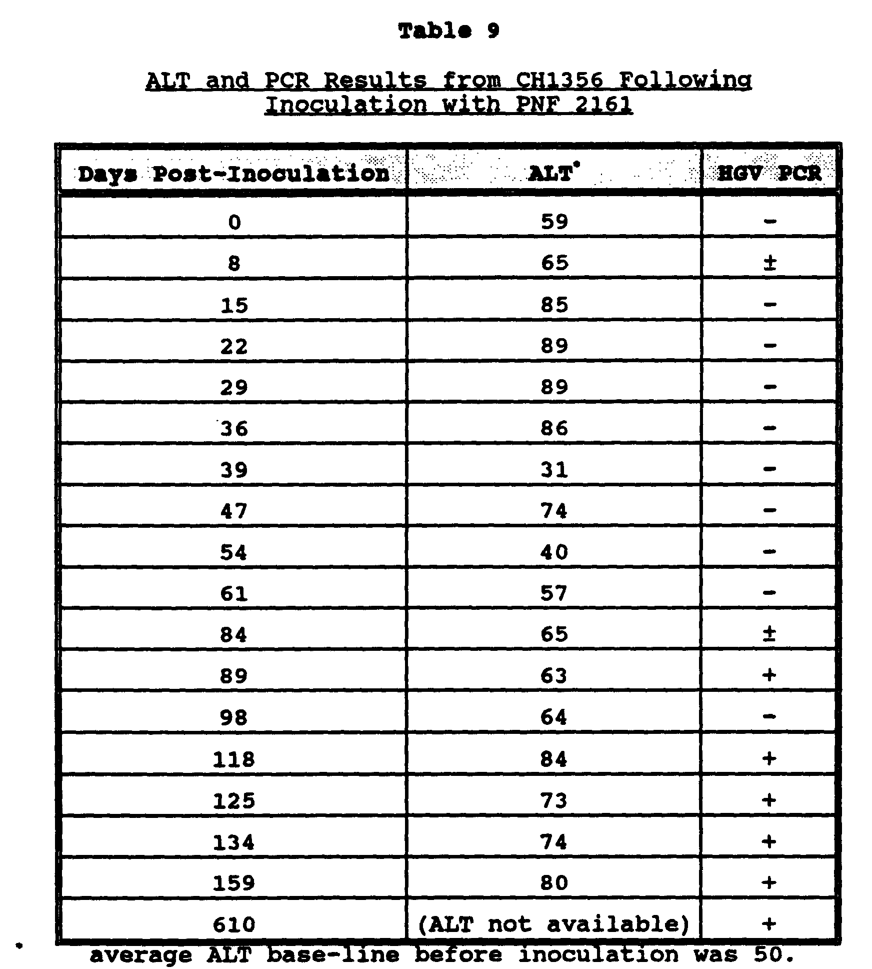

The data presented above indicate that HGV infection was persistent up to 1.7 years in an experimental primate.

### I. CHARACTERIZATION OF THE VIRAL GENOME.

The isolation of 470-20-1 from a cDNA library (Example 1) suggests that the viral genome detected in PNF 2161 is RNA. Further experiments to confirm the identity of the HGV viral genome as RNA include the following.

Selective degradation of either RNA or DNA (*e.g*., by DNase-free RNase or RNase-free DNase) in the original cloning source followed by amplification with HGV specific primers and detection of the amplification products serves to distinguish RNA from DNA templates.

An alternative method makes use of amplification reactions (nucleic acids from the original cloning source as template and HGV specific primers) that employ (i) a DNA-dependent DNA polymerase, in the absence of any RNA-dependent DNA polymerase (*i.e.*, reverse transcripase) in the reactions, and (ii) a DNA-dependent DNA polymerase and an RNA-dependent DNA polymerase in the reactions. In this method, if the HGV genome is DNA or has a DNA intermediate, then amplified product is detected in both types of amplification reactions. If the HGV genome is only RNA, the amplified product is detected in only the reverse transcriptase-containing reactions.

Total nucleic acid (*i.e*., DNA or RNA) was extracted from PNF 2161, using proteinase K and SDS followed by phenol extraction, as described in Example 4C. The purified nucleic acid was then amplified using polymerase chain reaction (PCR) where either (i) the PCR was preceded by a reverse transcription step, or (ii) the reverse transcription step was omitted. Amplification was reproducibly obtained only when the PCR reactions were preceded by reverse transcription. As a control, DNA templates were successfully amplified in separate reactions. These results demonstrate that the nature of the HGV viral genome is RNA.

The strand of the cloned, double-stranded DNA sequence that was originally present in PNF 2161 may be deduced by various means, including the following. Northern or dot blotting of the unamplified genomic RNA from an infected source serum can be performed, followed by hybridization of duplicate blots to probes corresponding to each strand of the cloned sequence. Alternatively, single-stranded cDNA probes isolated from M13 vectors (Messing), or multiple strand-specific oligonucleotide probes are used for added sensitivity. If the source serum contains single-stranded RNA, only one probe (*i.e.*, sequences from one strand of the 470-20-1 clones) yield a signal, under appropriate conditions of hybridization stringency. If the source serum contains double-stranded RNA, both strand-probes will yeild a signal.

The polymerase chain reaction, prefaced by reverse transcription using one or the other specific primer, represents a much more sensitive alternative to Northern blotting. Genomic RNA extracted from purified virions present in PNF 2161 serum is used as the input template into each RT/PCR. Rather than cDNA synthesis with random hexamers, HGV sequence-specific primers were used. One cDNA synthesis reaction was performed with a primer complementary to one strand of the cloned sequence (*e.g.,* 470-20-1-77F); a second cDNA synthesis reaction was also performed using a primer derived from the opposite strand (*e.g.*, 470-20-1-211R).

The resulting first strand cDNA was amplified in using two HGV specific primers. Controls were included for successful amplification by PCR (e.g., DNA controls). RNA transcripts from each strand of the cloned sequence was also used, to control also for the reverse transcription efficiency obtained when using the specific primers which are described.

Specific products were detected by agarose gel electrophoresis with ethidium bromide staining. DNA controls (*i.e.*, double-stranded DNA controls for the PCR amplifcation) were successfully amplified regardless of the primer used for reverse transcription. Single-stranded RNA transcripts (*i.e.*, controls for reverse transcription efficiency and strand specificity) were amplified only when the opposite-strand primer was used for cDNA synthesis.

The PNF-derived HGV polynucleotide gave rise to a specific amplified product only when the primer 470-20-1-211R was used for reverse transcription, thus indicating that the original HGV polynucleotide sequence present in the serum is complementary to 470-20-1-211R and is likely a single-strand RNA.

### EXAMPLE 5

### SUCROSE DENSITY GRADIENT SEPARATION OF PNF2161

### A. BANDING OF PNF-2161 AGENT.

A continuous gradient of 10-60% sucrose ("ULTRAPURE", Gibco/BRL) in TNE (50 mM Tris-Cl, pH 7.5, 100 mM NaCl, 1 mM EDTA) was prepared using a gradient maker from Hoefer Scientific (San Francisco, CA). Approximately 12.5 ml of the gradient was overlaid with 0.4 ml of PNF serum which had been stored at -70°C, rapidly thawed at 37°C, then diluted in TNE.

The gradient was then centrifuged in the SW40 rotor (Beckman Instruments) at 40,000 rpm (approximately 200,000 x g at rₐᵥ) at 4°C for approximately 18 hours. Fractions of volume approximately 0.6 ml were collected from the bottom of the tube, and 0.5 ml was weighed directly into the ultracentrifuge tube, for calculation of density.

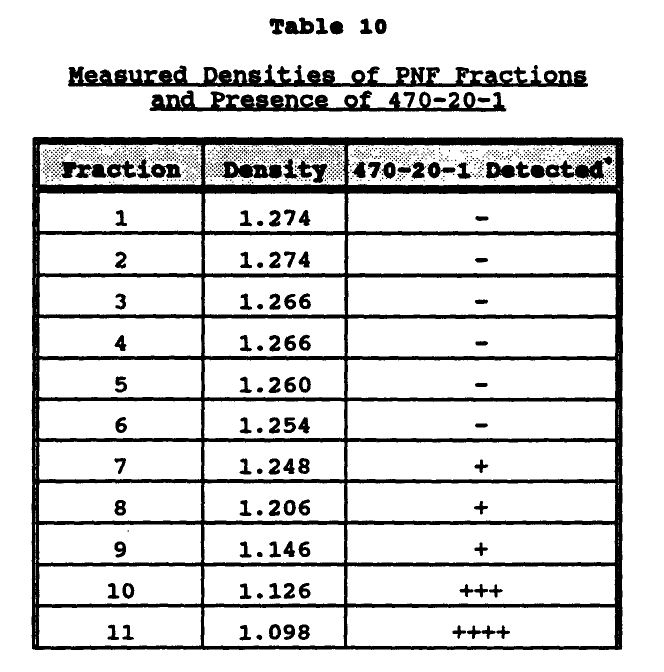

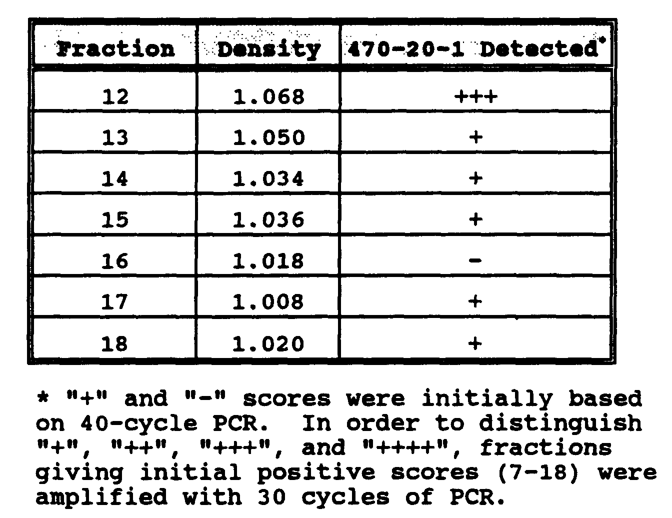

The putative viral particles were then pelleted by centrifugation at 40,000 rpm in the Ti70.1 rotor (approximately 110,000 × g) at 4°C for 2 hours, and RNA was extracted using the acid guanidinium phenol technique ("TRI REAGENT", Molecular Research Center, Cincinnati, OH), and alcohol-precipitated using glycogen as a carrier to improve recovery. The purified nucleic acid was dissolved in an RNase-free buffer containing 2 mM DTT and 1 U/*µ*l recombinant RNasin.

Analysis of the gradient fractions by RNA PCR (Example 4C) showed a distinct peak in the 470-20-1 specific signal, localized in fractions of density ranging from 1.126 to 1.068 g/ml (Table 10). The 470-20-1 signal was thus shown, under these conditions, to form a discrete band, consistent with the expected behavior of a viral particle in a sucrose gradient.

### B. RELATIVE VIRAL PARTICLE DENSITIES.

PNF 2161 has been demonstrated to be co-infected with HCV (see above). In order to compare the properties of the 470-20-1 viral particle to other known hepatitis viral particles, the serum PNF 2161 and a sample of purified Hepatitis A Virus were layered on a sucrose gradient (as described above). Fractions (0.6 ml) were collected, pelleted and the RNA extracted. The isolated RNA from each fraction was subjected to amplification reactions (PCR) using HAV (SEQ ID NO:5; SEQ ID NO:6), HCV (SEQ ID NO:7; SEQ ID NO:8) and 470-20-1 (SEQ ID NO:9, SEQ ID NO:10) specific primers.

Product bands were identified by electrophoretic separation of the amplification reactions on agarose gels followed by ethidium bromide staining. The results of this analysis are presented in Table 11.

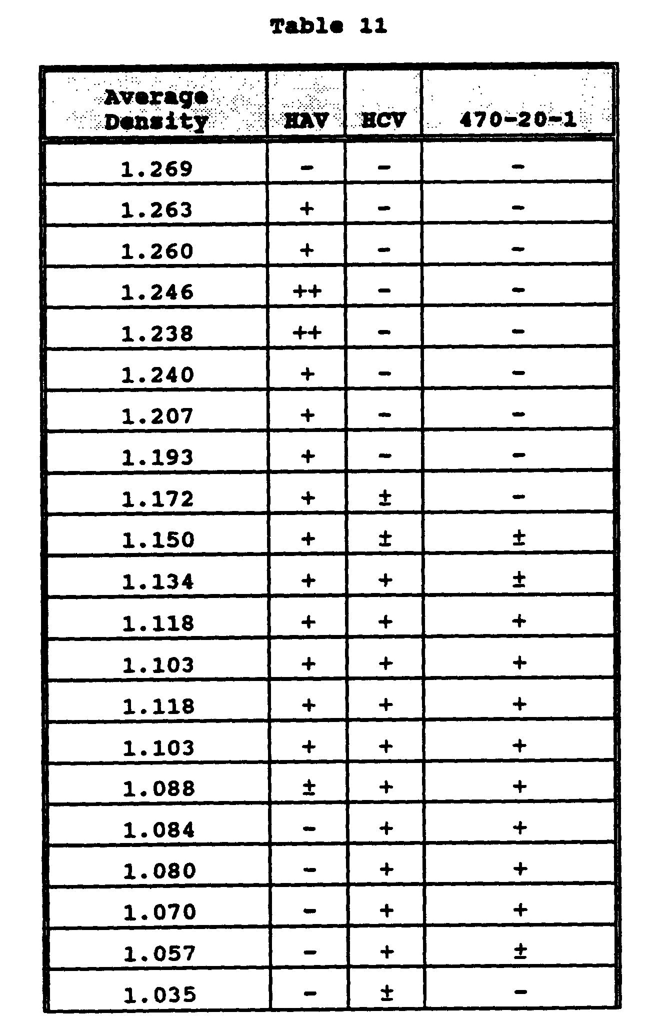

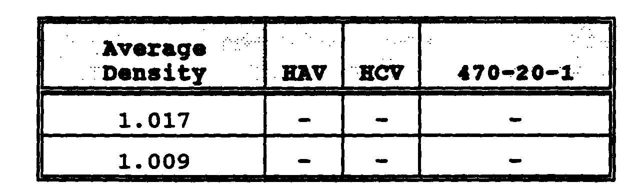

These results suggest that 470-20-1 particles are more similar to HCV particles than to HAV.

Further, serum PNF 2161 and HAV particles were treated with chloroform before sucrose gradient centrifugation. The results of these experiments suggest that 470-20-1 agent may be an enveloped virus since it has more similar properties to an enveloped Flaviviridae member (HCV) than a non-enveloped virus (HAV).

### EXAMPLE 6

### GENERATION OF 470-20-1 EXTENSION CLONES

### A. ANCHOR PCR.

RNA was extracted directly from PNF2161 serum as described in Example 1. The RNA was passed through a "CHROMA SPIN" 100 gel filtration column (Clontech) to remove small molecular weight impurities. cDNA was synthesized using a BMB cDNA synthesis kit. After cDNA synthesis, the PNF cDNA was ligated to a 50 to 100 fold excess of KL-1/KL-2 SISPA or JML-A/JML-B linkers (SEQ ID NO:11/SEQ ID NO:12, and SEQ ID NO:17/SEQ ID NO:18, respectively) and amplified for 35 cycles using either the primer KL-1 or the primer JML-A.

The 470 extension clones were generated by anchored PCR of a 1 *µ*l aliquot from a 10 *µ*l ligation reaction containing EcoRI digested (dephosphorylated) lambda gt11 arms (1 *µ*g) and EcoRI digested PNF cDNA (0.2 *µ*g). PCR amplification (40 cycles) of the ligation reaction was carried out using the lambda gt11 reverse primer (SEQ ID NO:13) in combination with either 470-20-77F (SEQ ID NO:9) or 470-20-1-211R (SEQ ID NO:10). All primer concentrations for PCR were 0.2 *µ*M.

The amplification products (9 *µ*l/100 *µ*l) were separated on a 1.5% agarose gel, blotted to "NYTRAN" (Schleicher and Schuell, Keene, NH), and probed with a digoxygenin labelled oligonucleotide probe specific for 470-20-1. The digoxygenin labeling was performed according to the manufacturer's recommendations using terminal transferase (BMB). Bands that hybridized were gel-purified, cloned into the "TA CLONING VECTOR pCR II" (Invitrogen), and sequenced.

Numerous clones having both 5' and 3' extensions to 470-20-1 were identified. All sequences are based on a consensus sequence from the sequencing of at least two independent isolates. This Anchor PCR approach was repeated in a similar manner to obtain further 5' and 3' extension sequences. These PCR amplification reactions were carried out using the lambda gt11 reverse primer (SEQ ID NO:13) in combination with HGV specific primers derived from sequences obtained from previous extension clones. The substrate for these reactions was unpackaged PNF 2161 2-cDNA source DNA.

Sequencing was carried out using "DYEDEOXY TERMINATOR CYCLE SEQUENCING" (a modification of the procedure of Sanger, *et al.*) on an Applied Biosystems model 373A DNA sequencing system according to the manufacturer's recommendations (Applied Biosystems, Foster City, CA). Sequence data is presented in the Sequence Listing. Sequences were compared with "GENBANK", EMBL database and dbEST (National Library of Medicine) sequences at both nucleic acid and amino acid levels. Search programs FASTA, BLASTP, BLASTN and BLASTX (Altschul, *et al.*) indicated that these sequences were novel as both nucleic acid and amino acid sequences.

Individual clones obtained using a selected primer pair were aligned to yeild a consensus sequence. The series of consensus sequences used to construct the sequence for the HGV-PNF 2161 varient was as follows: 4E3, SEQ ID NO:26; 3E3, SEQ ID NO:27; 2E5, SEQ ID NO:28; 1E5, SEQ ID NO:29; 4E5, SEQ ID NO:30; 3E5, SEQ ID NO:31; 2E3, SEQ ID NO:32; 1E3, SEQ ID NO:33; 4E5-20, SEQ ID NO:34; 5E3, SEQ ID NO:39; 6E3, SEQ ID NO:40; 7E3, SEQ ID NO:42; 5E5, SEQ ID NO:43; 6E5(44F), SEQ ID NO:44; 8E3, SEQ ID NO:98; 9E3, SEQ ID NO:109; 10E3, SEQ ID NO:110; 11E3, SEQ ID NO:116; 12E3, SEQ ID NO:118; 5'-end, SEQ ID NO:175; and 3'-END, SEQ ID NO:167.

The individual consensus sequences were aligned, overlapping sequences identified and a consensus sequence for the HGV-PNF 2161 variant was determined. This consensus sequence was compared with the sequences obtained for four other HGV variants: JC (SEQ ID NO:182), BG34 (SEQ ID NO:176), T55806 (SEQ ID NO:178), and EB20-2 (SEQ ID NO:180).

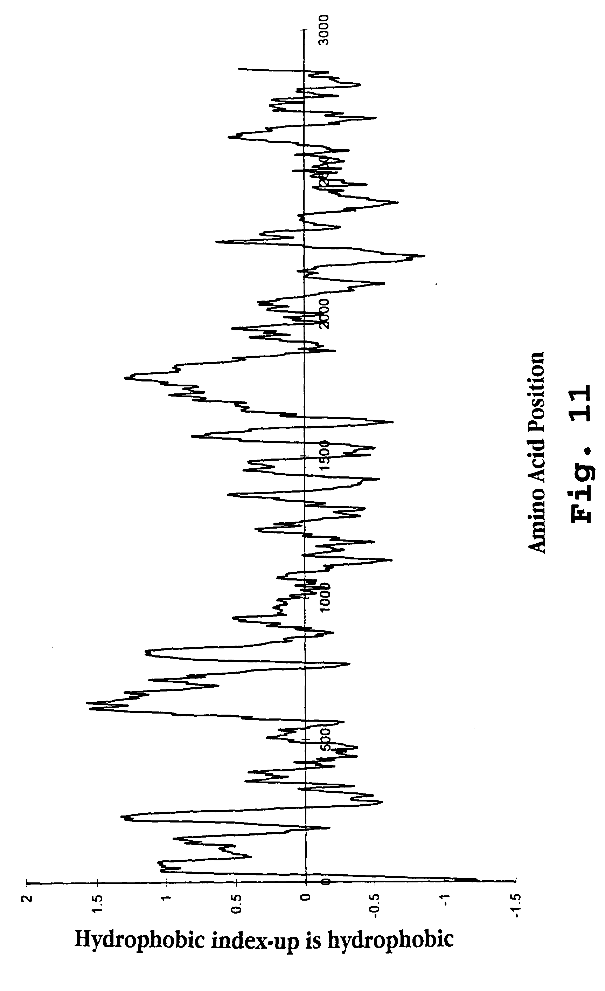

The consensus sequence of the HGV-PNF 2161 variant consists of 9391 base pairs presented as SEQ ID NO:14. This sequence represents a continuous open reading frame (SEQ ID NO:15). A Kyte-Doolittle hydrophobicity plot of the polyprotein is presented as Figure 11.

The relationship between the original 470-20-1 clone and the sequences obtained by extension is shown schematically in Figure 1. As seen in the figure, the DNA strand having opposite polarity to the protein coding sequence of 470-20-1 comprising a long continuous open reading frame.

The amino acid sequence of HGV was compared against the sequences of all viral sequence in the PIR database (IntelliGenetics, Inc., Mountain View, CA) of protein sequences. The comparison was carried out using the "SSEARCH" program of the "FASTA" suite of programs version 1.7 (Pearson, *et al*.). Regions of local sequence similarities were found between the HGV sequences and two viruses in the Flaviviridae family of viruses. The similarity alignments are presented in Figures 5A and 5B.

Present in these alignments are motifs for the RNA dependent RNA polymerase (RDRP) of these viruses. Conserved RDRP amino acid motifs are indicated in Figures 5A and 5B by stars and uppercase, bold letters (Koonin and Dolja). These alignments demonstrate that this portion of the HGV coding sequence correspond to RDRP. This alignment data combined with the data concerning the RNA genome of HGV supports the placement of HGV as a member of the Flaviviridae family.

The global amino acid sequence identities of the HGV polyprotein (SEQ ID NO:15) with HoCV (Hog Cholera Virus) and HCV are 17.1% and 25.5%, respectively. Such levels of global sequence identity demonstrates that HGV is a separate viral entity from both HoCV and HCV. To illustrate, in two members of the Flaviviridae family of viruses BVDV (Bovine Diarrhea Virus) and HCV, 16.2% of the amino acids can be globally aligned with HGV.

Members within a genus generally show high homology when aligned globally, for example, BVDV vs. HoCV show 71.2% identity. Various members (variants) of the unnamed genus of which HCV is a member are between 65% and 100% identical when globally aligned.

### B. RACE PCR: 5' END CLONING.

Clones representing the 5'-end of the HGV genome were obtained by a modified Anchor PCR approach that utilized RACE (Rapid Amplification of cDNA Ends) technology. The RACE method was originally described by Frohman, *et al.*, (1988) and Belyausky, *et al.*, (1989). Briefly, the 5'-end clones of HGV were obtained as follows.

First-strand cDNA synthesis was primed using random hexamers and synthesis was carried out using either "SUPERSCRIPT II" or "rTth" reverse transcriptase (GIBCO/BRL). After first-strand synthesis, the RNA template was degraded by base hydrolysis (NaOH). The cDNA sample was neutralized by the addition of acetic acid and purified by absorption to a glass matrix support ("GENO-BIND," Clontech, Palo Alto, CA). Following purification, the cDNA was concentrated by ethanol precipitation and washed twice with 80% ethanol.

The originally described RACE method was modified as follows. A single-stranded oligonucleotide anchor (SEQ ID NO:174) (Clontech) was ligated to the 3' end of the first-strand cDNA using T4 RNA ligase in the presence of cobalt chloride. The oligonucleotide anchor was obtained from the manufacturer with two modifications: (i) the 3'-end of the anchor was modified with an amino group which prevents concatamer formation, and (ii) the 5'-end contains a phosphate group which allows ligation to the first-strand cDNA.

After ligation of the anchor, the cDNA was used as a template for PCR amplification using several HGV-specific primers in combination with a primer complementary to the anchor sequence (AP primer, SEQ ID NO:134). The resulting amplification products were separated by agarose gel electrophoresis, transferred to filters and hybridized with a nested, HGV-specific oligonucleotide probe. Bands that hybridized to the HGV-probe were isolated, cloned into "pCR-II" (Invitrogen, San Diego, CA) and sequenced.

### C. HGV 3' END CLONING.

Clones representing the 3'-end of the HGV genome were obtained by a modified anchored RT-PCR method. Briefly, poly A polymerase (GIBCO/BRL, Gaithersburg, MD) was used to catalyze the addition of a poly(A) tail to PNF 2161 RNA prior to cDNA synthesis. The poly(A) addition was performed according to the manufacturer's recommendations. Following purification of the poly(A) modified RNA, reverse transcription with "SUPERSCRIPT II" (GIBCO/BRL) was carried out using primer GV-5446IRT (SEQ ID NO:184). The resulting cDNA was amplified by PCR using the following primer set: GV59-5446F (SEQ ID NO:171) and GV-5446IR (SEQ ID NO:172).

After amplification, the products were separated by agarose gel electrophoresis, transferred to filters and hybridized with a digoxigenin-labelled oligonucleotide probe (E5-7-PRB, SEQ ID NO:173). Products that hybridized with the oligonucleotide were isolated, purified, cloned into "pCR-II" and sequenced. The two clones isolated by this method were MP3-3 (SEQ ID NO:168) and MP3-7 (SEQ ID NO:169).

### EXAMPLE 7

### ISOLATION OF 470-20-1 FUSION PROTEIN

### A. EXPRESSION AND PURIFICATION OF 470-20-1/GLUTATHIONE-S-TRANSFERASE FUSION PROTEIN

Expression of a glutathione-S-transferase (sj26) fused protein containing the 470-20-1 peptide was achieved as follows. A 237 base pair insert (containing 17 nucleotides of SISPA linkers on both sides) corresponding to the original lambda gt11 470-20-1 clone was isolated from the lambda gt11 470-20-1 clone by polymerase chain reaction using primers gt11 F(SEQ ID NO:25) and gt11 R(SEQ ID NO:13) followed by *Eco RI* digestion.

The insert was cloned into a modified pGEX vector, pGEX MOV. pGEX MOV encodes sj26 protein fused with six histidines at the carboxy terminal end (sj26his). The 470-20-1 polypeptide coding sequences were introduced into the vector at a cloning site located downstream of sj26his coding sequence in the vector. Thus, the 470-20-1 polypeptide is expressed as sj26his/470-20-1 fusion protein. The sj26 protein and six histidine region of the fusion protein allow the affinity purification of the fusion protein by dual chromatographic methods employing glutathione-conjugated beads (Smith, D.B., *et al*.) and immobilized metal ion beads (Hochula; Porath).

E. coli strain W3110 (ATCC catalogue number 27352) was transformed with pGEX MOV and pGEX MOV containing 470-20-1 insert. Sj26his protein and 470-20-1 fusion protein were induced by the addition of 2 mM isopropyl-β-thiogalactopyranoside (IPTG). The fusion proteins were purified either by glutathione-affinity chromatography or by immobilized metal ion chromatography (IMAC) according to the published methods (Smith, D.B., *et al.;* Porath) in conjunction with conventional ion-exchange chromatography.

The purified 470-20-1 fusion protein was immunoreactive with PNF 2161. However, purified sj26his protein was not immunoreactive with PNF 2161, indicating the presence of specific immunoreaction between the 470-20-1 peptide and PNF 2161.

### B. ISOLATION OF 470-20-1/B-GALACTOSIDASE FUSION PROTEIN

KM392 lysogens infected either with lambda phage gt11 or with gt11/470-20-1 are incubated in 32°C until the culture reaches to an O.D. of 0.4. Then the culture is incubated in a 43°C water bath for 15 minutes to induce gt11 peptide synthesis, and further incubated at 37°C for 1 hour. Bacterial cells are pelleted and lysed in lysis buffer (10 mM Tris, pH 7.4, 2 % "TRITON X-100" and 1% aprotinin). Bacterial lysates are clarified by centrifugation (10K, for 10 minutes, Sorvall JA20 rotor) and the clarified lysates are incubated with Sepharose 4B beads conjugated with anti-β-galactosidase (Promega).

Binding and elution of β-galactosidase fusion proteins are performed according to the manufacturer's instruction. Typically binding of the proteins and washing of the column are done with lysis buffer. Bound proteins are eluted with 0.1 M carbonate/bicarbonate buffer, pH 10. The purified 470-20-1/b-galactosidase protein is immunoreactive with both PNF2161 and anti-b-galactosidase antibody. However, β-galactosidase, expressed by gt11 lysogen and purified, is not immunoreactive with PNF2161 but immunoreactive with antiβ-galactosidase antibody.

### EXAMPLE 8

### PURIFICATION OF THE 470-20-1 FUSION PROTEIN AND PREPARATION OF ANTI-470-20-1 ANTIBODY

### A. GLUTATHIONE AFFINITY PURIFICATION

Materials included 50 ml glutathione affinity matrix reduced form (Sigma), XK 26/30 Pharmacia column, 2.5 × 10 cm Bio-Rad "ECONO-COLUMN" (Richmond, CA), Gilson (Middleton, WI) HPLC, DTT (Sigma), glutathione reduced form (Sigma), urea, and sodium phosphate dibasic.

The following solutions were used in purification of the fusion protein:
Buffer A: phosphate buffer saline, pH 7.4, and
Buffer B: 50 mM Tris Ph 8.5, 8 mM glutathione, (reduced form glutathione)
Strip buffer: 8 M urea, 100 mM Tris pH 8.8, 10 mM glutathione, 1.5 NaCl.

*E. coli* carrying the plasmid pGEX MOV containing 470-20-1 insert, were grown in a fermentor (20 liters). The bacteria were collected and lysed in phosphate buffered saline (PBS) containing 2 mM phenylmethyl sulfonyl fluoride (PMSF) using a micro-fluidizer. Unless otherwise noted, all of the following procedures were carried out at 4°C.

The crude lysate was prepared for loading by placing lysed bacteria into "OAKRIDGE" tubes and spinning at 20K rpms (40k x g) in a Beckman model JA-20 rotor. The supernatant was filtered through a 0.4 *µ*m filter and then through a 0.2 *µ*m filter.

The 2.5 × 10 cm "ECONO-COLUMN" was packed with the glutathione affinity matrix that was swelled in PBS for two hours at room temperature. The column was brought into equilibrium by washing with 4 bed volumes of PBS.

The column was loaded with the crude lysate at a flow rate of 8 ml per minute. Subsequently, the column was washed with 5 column volumes of PBS at the same flow rate.

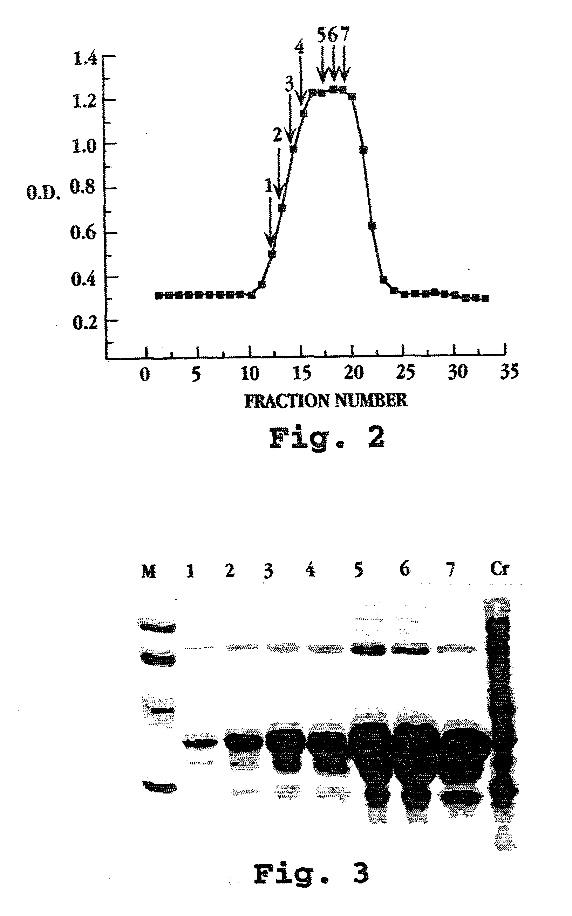

The column was eluted by setting the flow rate to 0.75-1 ml/min. and introducing Buffer B. Buffer B was pumped through the column for 5 column volumes and two-minute fractions were collected. An exemplary elution profile is shown in Figure 2. The content and purity of the proteins present in the fractions were assessed by standard SDS PAGE (Figure 3). The 470-20-1/sj26his fusion protein was identified based on its predicted molecular weight and its immunoreactivity to PNF 2161 serum. For further manipulations, the protein can be isolated from fractions containing the fusion protein or from the gel by extraction of gel regions containing the fusion protein.

### B. PURIFICATION OF CLONE 470-20-1 FUSION PROTEIN BY ANION EXCHANGE.

Solutions include the following:
Buffer A (10 mM sodium phosphate pH 8.0, 4 M urea, 10 mM DTT);
Buffer B (10 mM sodium phosphate pH 8.0, 4 M urea, 10 mM DTT, 2.0 M NaCl); and
Strip Buffer (8 M urea, 100 mM Tris pH 8.8, 10 mM glutathione, 1.5 NaCl).

Crude lysate (or other protein source, such as pooled fractions from above) was loaded onto "HIGH-Q-50" (Biorad, Richmond, CA) column at a flow rate of 4.0 ml/min. The column was then washed with Buffer A for 5 column volumes at a flow rate of 4.0 ml/min.

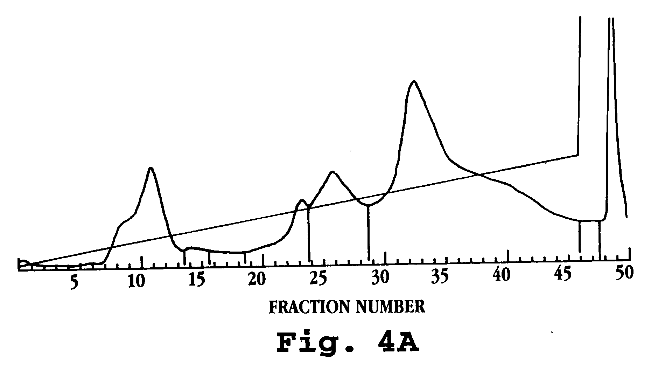

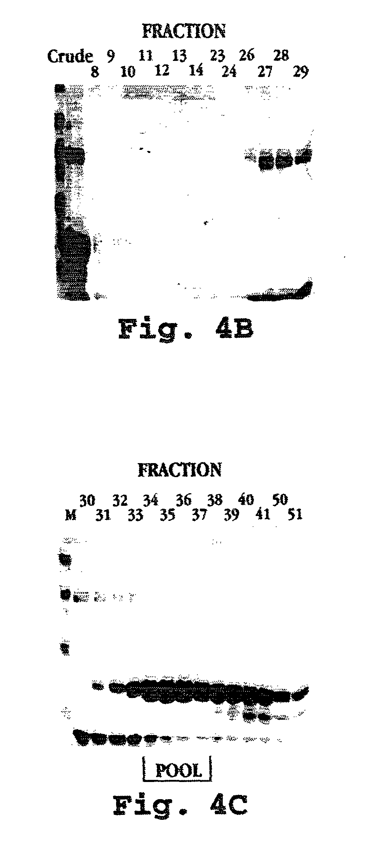

After these washes, a gradient was started and ran from Buffer A to Buffer B in 15 column volumes. The gradient then stepped to 100% Buffer B for one column volume. An exemplary gradient is shown in Figure 4A. Fractions were collected every 10 minutes. Purity of the 470-20-1/sj26his fusion protein was assessed by standard SDS-PAGE (Figures 4B and 4C) and relevant fractions were pooled (approximately fractions 34 through 37, Figure 4C).

### C. PREPARATION OF ANTI-470-20-1 ANTIBODY

The purified 470-20-1/sj26his fusion protein is injected subcutaneously in Freund's adjuvant in a rabbit. Approximately 1 mg of fusion protein is injected at days 0 and 21, and rabbit serum is typically collected at 6 and 8 weeks.

A second rabbit is similarly immunized with purified sj26his protein.

Minilysates are prepared from bacteria expressing the 470-20-1/sj26his fusion protein, sj26his protein, and β-galactosidase/470-20-1 fusion protein. The lysates are fractionated on a gel and transfered to a membrane. Separate Western blots are performed using the sera from the two rabbits.

Serum from the animal immunized with 470-20-1 fusion protein is immunoreactive with all sj26his fusion protein in minilysates of IPTG induced E. coli W3110 that are transformed either with pGEX MOV or with pGEX MOV containing 470-20-1 insert. This serum is also immunoreactive with the fusion protein in the minilysate from the 470-20-1 lambda gt11 construct.

The second rabbit serum is immunoreactive with both sj26his and 470-20-1/sj26his fusion proteins in the minilysates. This serum is not expected to immunoreactive with 470-20-1/β-galactosidase fusion protein in the minilysate from the 470-20-1 lambda gt11 construct. None of the sera are expected to be immunoreactive with β-galactosidase.

Anti-470-20-1 antibody present in the sera from the animal immunized with the fusion protein is purified by affinity chromatography (using the 470-20-1 ligand).

Alternatively, the fusion protein can be cleaved to provide the 470-20-1 antigen free of the sj-26 protein sequences. The 470-20-1 antigen alone is then used to generate antibodies as described above.

### EXAMPLE 9

### Rabbit Anti-Peptide Sera

Peptides were designed to cover the entire HGV sequence, in particular, to cover each of the functional groups in the non-structural and structural genes. Peptides were synthesized commercially by conventional techniques. Representative peptides are presented in Table 12.

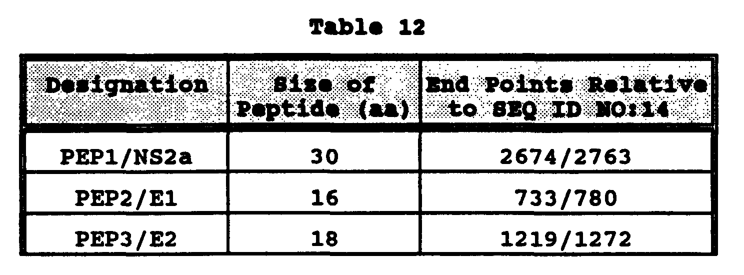

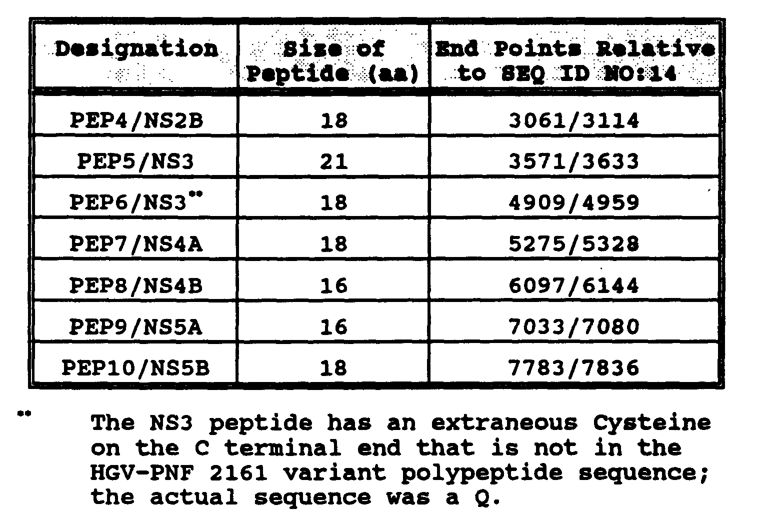

The peptides were coupled to KLH. Using rabbits as host, the conjugated peptides were injected subcutaneously at multiple sites. Anti-peptide rabbit serum were generated by a commercial facility. A two-week immunization protocol was used with bleeds taken at alternate weeks.

Rabbit anti-peptide sera were shown to be peptide specific and to have high titer. Rabbit anti-peptide sera also recognize corresponding recombinant proteins expressed in *E. coli* and baculovirus. Antibody endpoint titers range from 1:50,000 dilution to 1:625,000 dilution. Rabbit anti-peptide 7 (NS4a) had low end point titers of only 1:1,000. Accordingly, rabbit anti-serum to the NS4a protein expressed in, for example, the baculovirus system may be a more useful reagent.

Rabbit anti-peptide sera are useful for immunoprecipitating corresponding HGV proteins expressed, for example, in baculovirus and vaccinia. Rabbit anti-peptide sera are also useful as capture antibody in EIAs to detect HGV antigen. Rabbit anti-peptide sera are further useful in the characterization of the HGV proteins.

### EXAMPLE 10

### SEROLOGY

### A. WESTERN BLOT ANALYSIS OF SERA PANELS

The 470-20-1 fusion antigen (described above) was used to screen panels of sera. Many of the panels were of human sera derived both from individuals suffering from hepatitis and uninfected controls.

Affinity purified 470-20-1 fusion antigen (Example 8) was loaded onto a 12% SDS-PAGE at 2 *µ*g/cm. The gel was run for two hours at 200V. The antigen was transfered from the gel to a nitrocellulose filter.

The membrane was then blocked for 2 hours using a solution of 1% bovine serum albumin, 3% normal goat serum, 0.25% gelatin, 100 mM NaPO₄, 100 mM NaCl, and 1% nonfat dry milk. The membrane was then dried and cut into 1-2 mm strips; each strip contained the 470-20-1 fusion antigen. The strip was typically rehydrated with TBS (150 mM NaCl; 20 mM Tris HCl, pH 7.5) and incubated in panel sera (1:100) overnight with rocking at room temperature.

The strips were washed twice for five minutes each time in TBS plus "TWEEN 20" (0.05%), and then washed twice for five minutes each time in TBS. The strips were then incubated in secondary antibody (Promega anti-human IgG-Alkaline Phosphatase conjugate, 1:7500), for 1 hour with rocking at room temperature. The strips were then washed twice × 5 minutes in TBS + "TWEEN 20", then twice × 5 minutes in TBS.

Bound antibody was detected by incubating the strips in a substrate solution containing BCIP (Example 2) and NBT (Example 2) in pH 9.5 buffer (100 mM Tris, 100 mM NaCl, 5 mM MgCl₂). Color development was allowed to proceed for approximately 15 minutes at which point color development was halted by 3 washes in distilled H₂O.

Test sera were derived from the following groups of individuals: (i) blood donors, negative for HBV Ab, surface Ag, negative for HCV, HIV, HTLV-1 Abs; (ii) HBV, sera from individuals who are infected with Hepatitis B virus; (iii) HCV, sera from individuals infected with Hepatitis C virus by virtue of being reactive in a second-generation HCV ELISA assay; and (iv) HXV, individuals serologically negative for HAV, HBV, HCV, or HEV.

The results of these screens are presented in Table 13.

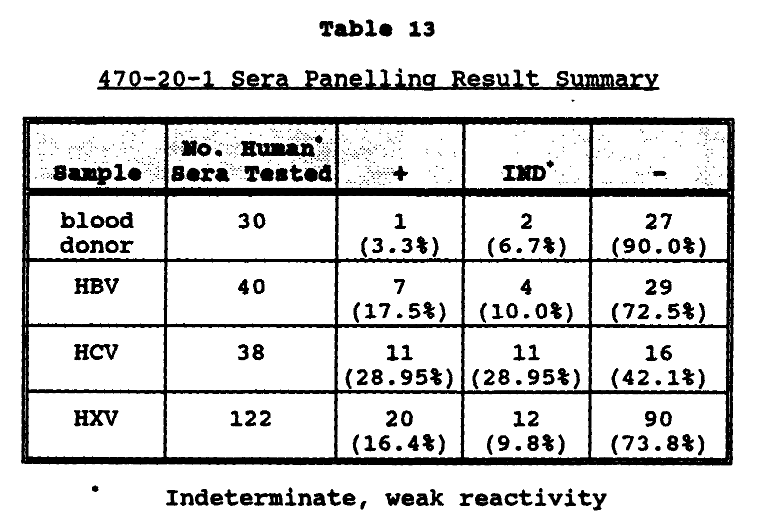

These results suggest the presence of the 470-20-1 antigen in a number of different sera samples. The antigen is not immunoreactive with normal human sera.

### B. GENERAL ELISA PROTOCOL FOR DETECTION OF ANTIBODIES

Polystyrene 96 well plates ("IMMULON II" (PGC)) are coated with 5 *µ*g/ml (100 *µ*L per well) antigen in 0.1 M sodium bicarbonate buffer, pH 9.5. Plates are sealed with "PARAFILM" and stored at 4°C overnight.

Plates are aspirated and blocked with 300 uL 10% normal goat serum and incubated at 37°C for 1 hr.

Plates are washed 5 times with PBS 0.5% "TWEEN-20".

Antisera is diluted in 1 × PBS, pH 7.2. The desired dilution(s) of antisera (0.1 mL) are added to each well and the plate incubated 1 hour at 37°C. The plates are then washed 5 times with PBS 0.5% "TWEEN-20".

Horseradish peroxidase (HRP) conjugated goat anti-human antiserum (Cappel) is diluted 1/5,000 in PBS. 0.1 mL of this solution is added to each well. The plate is incubated 30 min at 37°C, then washed 5 times with PBS.

Sigma ABTS (substrate) is prepared just prior to addition to the plate.

The reagent consists of 50 ml 0.05 M citric acid, pH 4.2, 0.078 ml 30% hydrogen peroxide solution and 15 mg ABTS. 0.1 ml of the substrate is added to each well, then incubated for 30 min at room temperature. The reaction is stopped with the addition of 0.050 mL 5% SDS (w/v). The relative absorbance is determined at 410 nm.

### EXAMPLE 11

### Expression of Selected HGV Antigens

The entire coding sequence of HGV was subcloned into greater than 50 distinct overlapping cDNA fragments. The length of most cDNA fragments ranged from about 200 bp to about 500 bp. The cDNA fragments were cloned separately into the expression vector, pGEX-HisB. This vector is similar to pGEX-MOV, described above.

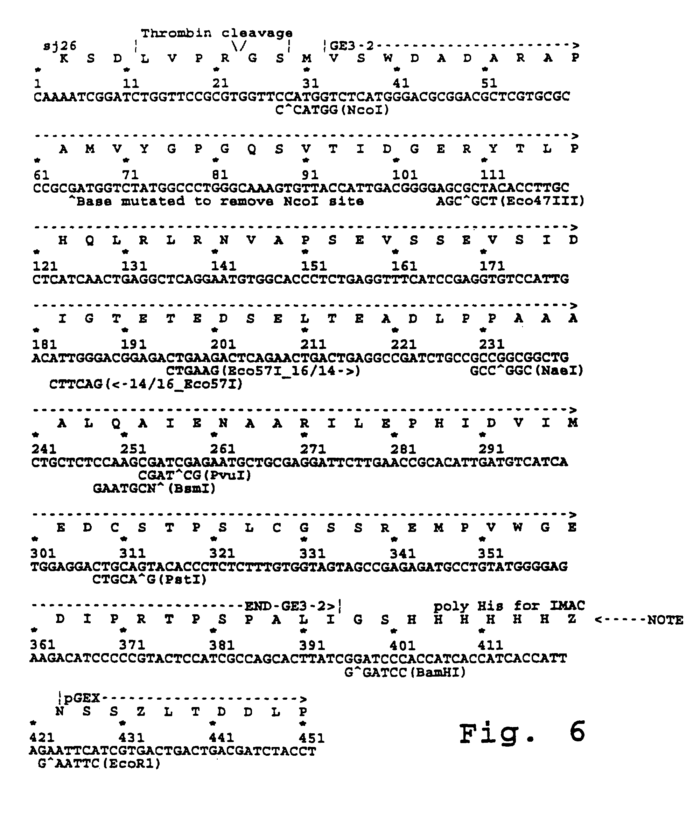

pGEX-hisB is a modification of pGEX-2T (Genbank accession number A01438; a commercially available expression vector). The vector pGEX-2T has been modified by insertion of a *NcoI* site directly downstream from the thrombin cleavage site. This site is followed by a *BamHI* site, which is followed by a poly-histidine (six histidines) encoding sequence, followed by the *EcoRI* site found in pGEX-2T. Coding sequences of interest are typically inserted between the *NcoI* site and the *BamHI* site. In Figure 6 (SEQ ID NO:115), the inserted sequence encodes the GE3-2 antigen. The rest of the vector sequence is identical to pGEX-2T. Expression of fusion protein is carried out essentially as described above with other pGEX-derived expression vectors.

Cloning of all 50 fragments was carried out essentially as described below, where specific primers were selected for each of the 50 coding regions. Each HGV insert DNA is PCR amplified from RNA extracted from PNF 2161 or other HGV(+) sera using a specific set of primers as described in Example 4C. Typically, the 5' primer contained a *NcoI* restriction site and the 3' primer contained a *BamHI* restriction site. The *NcoI* primers in the amplified fragments allowed in-frame fusion of amplified coding sequences to the GST-Sj26 coding sequence in the expression vectors pGEX-Hisb or pGEX MOV.

Amplified HGV insert DNA is digested with restriction enzymes NcoI and Bam HI. Digested insert DNA is gel purified and ligated with *Nco*I and *BamHI* digested pGEX hisB or pGEX MOV. *E. coli* strain W3110 (ATCC #27325, American Type Culture Collection, Rockville, MD) was transformed with the ligation product. Ampicillin resistant colonies were selected. Presence of the insert was confirmed by the PCR amplification of the insert from the ampicillin resistant colony using primers homologous to pGEX vector sequences flanking the inserted molecules (primers GLI F (SEQ ID NO:235) and GLI R (SEQ ID NO:236).

The size of the PCR amplification product is the insert size plus approximately 160 bp derived from vector. Transformants with appropriate inserts were selected and subjected to protein induction by IPTG as described in Example 7. Expressed recombinant proteins were analyzed for specific immunoreactivity against putative HGV-infected human sera by Western blot.

Eight fragments designated GE3, GE9, GE15, GE17, GE4, EXP3, GE1-N and GE-57 encoded antigens that gave a clear immunogenic response when reacted with putative HGV-infected human sera.

### A. CLONING OF GE3, GE9, GE15, GE17, GE4, EXP3, GE1-N AND GE57.

The coding sequence inserts for clones GE3, GE9, GE15, GE17, GE4, EXP3, GE1-N and GE57 were generated by polymerase chain reaction from SISPA-amplified double-stranded cDNA or RNA obtained from PNF 2161 or T55806 using PCR primers specific for each fragment. Following Table 14 lists the coordinates of each clone relative to SEQ ID NO:14 and the primer sets used for generation of each clone insert.

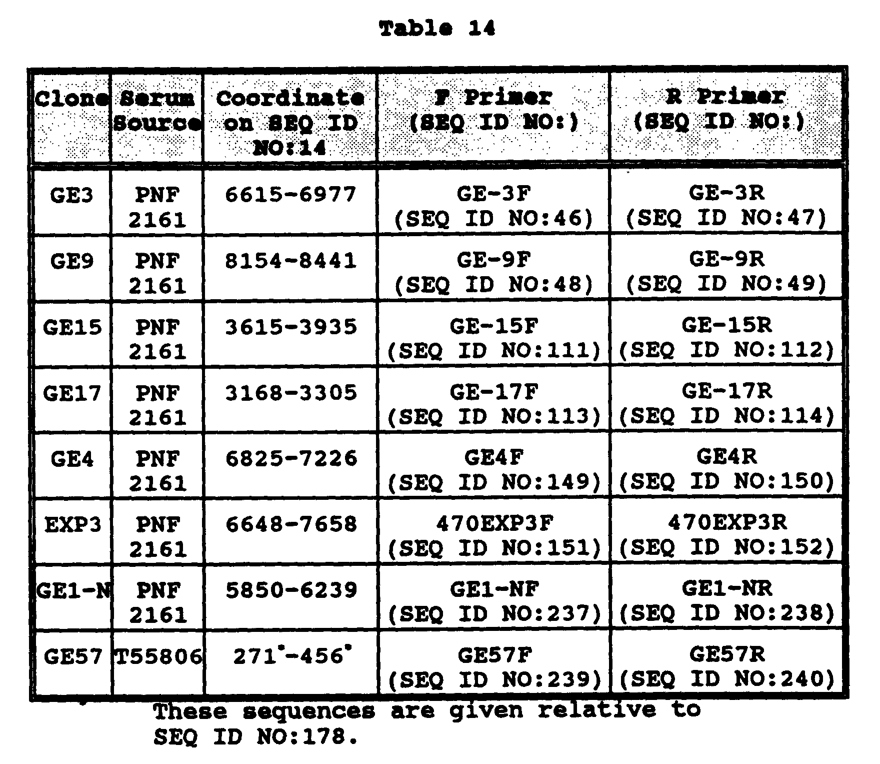

The amino acid sequence of GE57 is presented as SEQ ID NO:241.

In the GE3-5' primer (GE-3F, SEQ ID NO:46) a silent point mutation was introduced to modify a natural *NcoI* restriction site. Using the above-described primers, PCR amplification products were generated. The amplification products were gel purified, digested with *NcoI* and *BamHI,* and gel purified again. The purified *NcoI/BamHI* GE3, GE9, GE15, GE17, GE4, GE1-N and GE57 fragments were independently ligated into dephosphorylated, *NcoI/BamHI* cut pGEX-HisB vectors. The purified *NcoI/BamHI* EXP3 fragment was ligated into dephosphorylated, *NcoI/BamHI* cut pGEX-MOV vector.

Each ligation mixture was transformed into *E. coli* W3110 strain and ampicillin resistant colonies were selected. The ampicillin resistant colonies were resuspended in a Tris/EDTA buffer and analyzed by PCR, using primers GLI F (SEQ ID NO:235) and GLI R (SEQ ID NO:236) to confirm the presence of insert sequences. Eight candidate clones were designated GE3-2, GE9-2, GE15-1, GE17-2, GE4-8, EXP3-7, GE1-N and GE57, respectively.

### B. Expression of the GE3-2, GE9-2, GE15-1, GE17-2, GE4-8, EXP3-7, GE1-N and GE57 Fusion Proteins.

Colonies of ampicillin resistant bacteria carrying GE3-2, GE9-2, GE15-1, and GE17-2, GE4-8, EXP3-7, GE1-N and GE57 containing-vectors were individually inoculated into LB medium containing ampicillin. The cultures were grown to OD of 0.8 to 0.9 at which time IPTG (isopropylthio-beta-galactoside; Gibco-BRL) was added to a final concentration of 0.3 to 1 mM, for the induction of protein expression. Incubation in the presence of IPTG was continued for 3 to 4 hours.

Bacterial cells were harvested by centrifugation and resuspended in SDS sample buffer (0.0625 M Tris, pH 6.8, 10% glycerol, 5% mercaptoethanol, 2.3% SDS). The resuspended pellet was boiled for 5 min. and then cleared of insoluble cellular debris by centrifugation. The supernatants obtained from IPTG-induced cultures of GE3-2, GE9-2, GE15-1, GE17-2, GE4-8, EXP3-7, GE1-N and GE57 were analyzed by SDS-polyacrylamide gel electrophoresis (PAGE) together with uninduced lysates. The proteins from these gels were then transferred to nitrocellulose filters (*i.e.*, by Western blotting).

The filters were first incubated with rabbit polyclonal antibody or mouse monoclonal antibody (RM001 from Sierra Biosource, CA) directed to GST protein to detect the expression of appropriate size GST-fusion protein expression. Expected protein sizes of above clones are 40, 38, 39, 32, 42, 64, 42 and 33 KDa, respectively. Immunoreactivity of RM001 with bands at the appropriate molecular weight for the fusion proteins demonstrated the successful expression of the fusion proteins of above clones by the bacterial cells. Expression of the clone proteins were also monitored by the appearance of over-expressed proteins of appropriate sizes upon IPTG induction on the Coomassie brilliant blue stained gel.

### C. WESTERN BLOT ANALYSIS OF HGV PROTEINS.

Once the expression of the HGV clone protein was confirmed by Western blot analysis with anti-GST antibody a second set of filters, prepared as above, were then exposed to several HGV(+) and HGV(-) human sera. Human sera used for Western blot analyses of whole cell lysates were pre-absorbed with the lambda-gt11-nitrocellulose filters. Lambda-gt11-nitrocellulose filters were prepared as follows. Briefly, an overnight culture of KM392 culture was prepared in LB. The culture was diluted 10 fold in fresh LB containing 0.2% maltose and incubated for 1 hour at 37°C with shaking.

After 1 hour the culture was mixed with an equal volume of MgCa solution (0.01M MgCl₂ and 0.01M CaCl₂). To this mixture lambda gt11 was added to a titer of 2 x 10⁴ PFU/ml and incubated for 30 min without shaking. After 30 minutes (per each ml of this phage/E.coli mixture) 15 ml of molten (55°C) LB top agar (LB with 0.8% agar) was added: 8 ml of this mixture was spread onto each 15 cm LB agar plate. After the top agar solidified the plate was incubated at 37°C for 3-5 hr.

After plaques developed, a nitrocellulose filter was placed on the plate and the plate further incubated at 37°C overnight. The nitrocellulose filter was removed and washed thoroughly with TBS (50 mM Tris-HCl, pH 7.5, 150 mM NaCl) plus 0.05 % "TWEEN 20." The washed filter was then blocked with 1% gelatin in TBS overnight. The filter was washed three times (5 minutes each wash) with TBS.

For the pre-absorption of human sera each serum was diluted 100 fold in blocking solution (described in Example 10). Ten mls. of diluted serum was then incubated overnight with two lambda gt11 filters prepared as above. Lambda gt11 filters were removed and the pre-absorbed serum used for Western blot analysis.

Western blot analyses demonstrated that clones GE3-2, GE9-2, GE15-1, GE17-2, GE4-8, EXP3-7, GE1-N and GE57 showed specific immunoreactivity toward HGV(+) sera. The GE-4-8 protein was immunoreactive with J21689 serum. J21689 is HGV (+) serum as determined by HGV PCR (Example 4) and HCV (+) as determined by HCV PCR and serological analyses. The EXP3-7 protein was immunoreactive with JC and T55806. JC is the HGV-positive serum identified in Example 4F that was rejected by the blood bank for being high ALT. A second JC sample, taken one year after the initial serum sample, was also positive for HGV by PCR analysis. T55806 is also the HGV-positive serum identified in Example 4F that was rejected by the blood bank for being High ALT. This serum is co-positive with HCV.

Further, GE15-1 and GE-17 showed weak but specific immunoreactivity toward PNF 2161 and T55806. GE1-N was immunoreactive with PNF2161, JC, T55806, T56633, T27034 and R0001. T56633, T27034 and R0001 are HGV (+) sera identified in Example 4F. GE57 was immunoreactive with E57963 and R0001. E57963 is HGV and HCV co-positive serum. GE3-2 and GE9-2 were also immunoreactive with HGV sera specifically. However, none of the eight antigens were immunoreactive with HGV negative sera T43608 and R05072.

The GE3-2 and GE9-2 fusion proteins were purified from bacterial cell lysates essentially as in Example 7 using dual chromatographic methods employing glutathione-conjugated beads (Smith, D.B., *et al.*) and immobilized metal ion beads (Hochuli; Porath). The purified proteins were subjected to Western blot analysis as follows.

Various amounts of the purified HGV proteins (*e.g*., GE3-2 and GE9-2 proteins) were loaded on 12% acrylamide gels. Following PAGE, proteins were transferred from the gels to nitrocellulose membranes, using standard procedures. Individual membranes were incubated with one of a number of human or mouse sera. Excess sera were removed by washing the membranes.

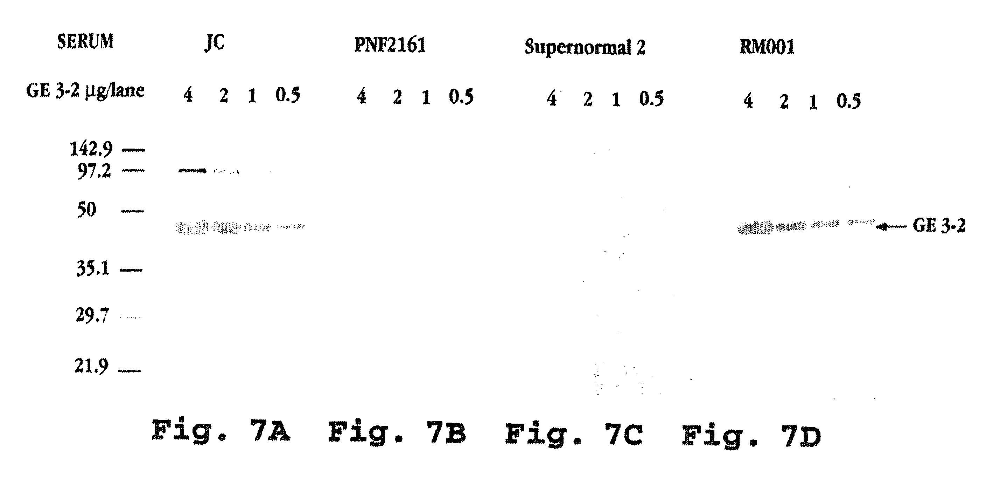

These membranes were incubated with alkaline phosphatase-conjugated goat anti-human antibody (Promega) or alkaline phosphatase-conjugated goat anti-mouse antibodies (Sigma), depending on the serum being used for screening. The membranes were washed again, to remove excess goat anti-human IgG antibody, and exposed to NBT/BCIP. Photographs of exemplary stained membranes having the GE3 fusion protein are shown in Figures 7A to 7D.

The Figures show the results of Western blot analysis of the purified GE3-2 protein using the following sera: N-(ABCDE) human (JC) serum (Figure 7A), N-(ABDE) human (PNF 2161) serum (Figure 7B), a super normal (SN2) serum (Figure 7C), and mouse monoclonal antibody (RM001) directed against GST-Sj26 protein (Figure 7D).

In each of the figures, lane 1 contains pre-stained molecular weight standards(Bio-Rad), and lanes 2-5 contain, respectively, the following amounts of the GE3-2 fusion protein: 4 *µ*g, 2 *µ*g, 1 *µ*g, and 0.5 *µ*g. Numbers represent loading amounts in micrograms per 0.6 centimeter of gel (well size). Dilutions of the human JC, PNF 2161 and Super Normal 2 sera were 1:100. The anti-sj26 dilution was 1:1000. The band seen at about 97K in the JC blot is reactivity against a minor contaminant in the GE3.2 fusion protein preparation. Protein marker sizes are 142.9, 97.2, 50, 35.1, 29.7 and 21.9 KD.

As shown in Figures 7A to 7D, GE3-2 showed specific immunoreactivity with JC serum. GE3-2 reacted weakly with PNF 2161 serum and would be scored as an indeterminant or negative.

In parallel experiments, GE9-2 showed weak but specific immunoreactivity toward PNF 2161 serum.

### EXAMPLE 12

### Construction of Exemplary Epitope Libraries

### A. THE Y5 LIBRARY.

Polymerase Chain Reactions were employed to amplify 3 overlapping DNA fragments from PNF 2161 SISPA-amplified cDNA. The PNF 2161 SISPA-amplified cDNA was prepared using the JML-A/B linkers (SEQ ID NO:54 and SEQ ID NO:55). One microliter of this material was re-amplified for 30 cycles (1 minute at 94°C, 1.5 minutes at 55°C and 2 minutes at 72°C) using 1 *µ*M of the JML-A primers. The total reaction volume was 100 *µ*l. The products from 3 of these amplifications were combined and separated from excess PCR primers by a single pass through a "WIZARD PCR COLUMN" (Promega) following the manufacturer's instructions. The "WIZARD PCR COLUMN" is a silica based resin that binds DNA in high ionic strength buffers and will release DNA in low ionic strength buffers. The amplified DNA was eluted from the column with 100 *µ*l distilled H20.

The eluted DNA was fractionated on a 1.5% Agarose TBE gel (Maniatis, *et al*.) and visualized with UV light following ethidium bromide staining. A strong smear of DNA fragments between 150 and 1000 bp was observed. One microliter of the re-amplified cDNA was used as for template in PCR reactions with each primer pair presented in Table 15.

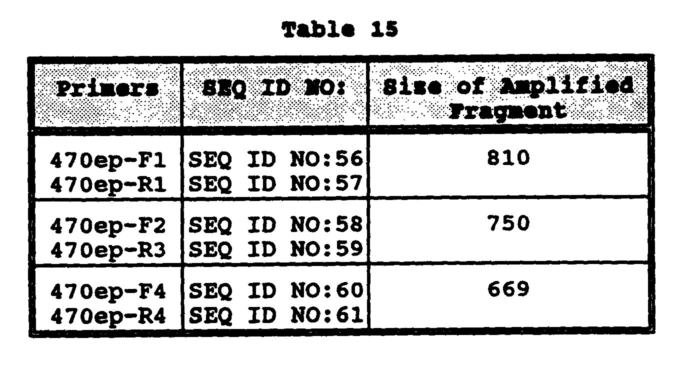

The primers were designed to result in the amplification of HGV specific DNA fragments of the sizes indicated in Table 15. In the amplification reactions, the primer pairs were used at a concentration of 1 *µ*M. Amplifications were for 30 cycles of 1 minute at 94, 1.5 minutes at 54°C and 3 minutes at 72°C in a total reaction volume of 100 *µ*l. Each of the three different primer pair PCR reactions resulted in the specific amplification of products having the expected sizes. For each primer pair reaction, amplification products from 3 independent PCR reactions were combined and purified using a "WIZARD PCR COLUMN" as described above. The purified products were eluted in 50 *µ*l dH20.

Samples from each purified product (14 *µ*l, containing approximately 1 - 2 *µ*g of each primer-pair amplified DNA fragment) were combined. The combined sample of all three different amplified fragments was added to 5 *µ*l of 10X DNAse Digestion buffer (500 mM Tris PH 7.5, 100 mM MnCl₂) and 2 *µ*l of dH20. From this digestion mixture, a 10 *µ*l sample was removed and placed in a tube containing 5 *µ*l of Stop solution (100 mM EDTA, pH 8.0). This sample was the 0 "minutes of digestion" time point. The rest of the digestion reaction was placed at 25°C. To the digestion mixture 1 *µ*l of 1/25 diluted RNase-free DNAse I (Stratagene) was added. At various time points 10 *µ*l aliquots were withdrawn and mixed with 5 *µ*l of Stop solution. The DNAse I digested DNA products were analyzed on a 1.5% Agarose TBE gel.

The results of several digestion experiments showed that 40 minutes of digestion provided a good distribution of DNA fragments in the size range of 100 - 300 bp. A DNAse I digestion was then repeated with the entire digestion being left for 40 minutes at room temperature. The digestion was stopped by the addition of 18 *µ*l of Stop Buffer and the digested DNA products were purified using a "WIZARD PCR COLUMN." The "WIZARD-PCR COLUMN" was eluted with 50 *µ*l of dH20 and the eluted DNA added to the following reaction mixture: 7 *µ*l of Restriction Enzyme Buffer C (Promega, 10 mM MgCl₂, 1 mM DTT, 50 mM NaCl, 10 mM Tris, pH 7.9, 1X concentration); 11 *µ*l of 1.25 mM dNTPs; and 2 *µ*l T4 DNA Polymerase (Boehringer-Mannhiem). This reaction mixture was held at 37°C for 30 minutes, at which point 70 *µ*l of pH 8.0 phenol/CHCl₃ was added and mixed. The phenol/CHCl₃ was removed and extracted once to yield a total aqueous volume of 150 *µ*l containing the DNA sample. The DNA was ethanol precipitated using 2 volumes of absolute ethanol and 0.5 volume of 7.5 M NH₄-acetate. The DNA was pelleted by centrifugation for 15 minutes at 14,000 rpm in an "EPPENDORF MICROFUGE", dried for 5 minutes at 42°C and resuspended in 25 *µ*l of dH20.

The DNA was ligated to 5' phosphorylated SISPA linkers KL1 (SEQ ID NO:62) and KL2 (SEQ ID NO:63). Several different concentrations of SISPA linkers and DNA was tested. The highest level of ligation (assessed as described below) occurred under the following ligation reaction conditions: 6 *µ*l of DNA, 2 *µ*l of 5.0 x 10 -12 M KL1/KL2 linkers, 1 *µ*l of 10X ligase buffer (New England Biolabs), and 1 *µ*l of 400 Units/*µ*l T4 DNA Ligase (New England Biolabs) in a total reaction volume of 10 *µ*l. Ligations were carried out overnight at 16°C.

Two reactions were run in parallel as follows. A 2 *µ*l sample of the ligated material was amplified using the KL1 SISPA primer in a total reaction volume of 100 *µ*l (25 cycles of 1 minute at 94°C, 1.5 minutes at 55°C and 2 minutes at 72°C). The degree of ligation was assessed by separating 1/5 of the PCR reaction amplified products by electrophoresis using a 1.5% agarose TBE gel. The gel was stained with ethidium bromide and the bands visualized with UV light.

The amplification products from the duplicate reactions were purified using "WIZARD PCR COLUMNS" and the purified DNA eluted in 50 *µ*l of dH20. A twenty-five microliter aliquot of the PCR KL1/KL2 amplified DNA was digested with 36 Units of *EcoRI* (Promega) in a total volume of 30 *µ*l. The reaction was carried out overnight at 37°C. The Digested DNA was purified using a "SEPHADEX G25" spin column.

The *EcoRI* digested DNA was ligated in overnight reactions to λgt11 arms that were pre-digested with *EcoRI* and treated with calf intestinal alkaline phosphatase (Stratagene, La Jolla, CA). The ligation mixture was packaged using a "GIGAPACK GOLD PACKAGING EXTRACT" (Stratagene) following manufacturer's instructions. Titration of the amount of recombinant phage obtained was performed by plating a 1/10 dilution of the packaged phage on a lawn of KM-392, where the plate contained 20 *µ*l of a 100 mg/ml solution of x-gal (5-Bromo-4-chloro-3-indolyl-β-D-galactoside; Sigma) and 20 *µ*l of a 0.1 M solution of IPTG (Isopropyl-1-thio-β-D-galactoside; Sigma). A titer was obtained of 1.2 × 10⁶ phage/ml containing over 75% recombinant phage.

The percentage of recombinant plaques was confirmed by PCR analysis of 8 randomly picked plaques using primers 11F (SEQ ID NO:25) and 11R (SEQ ID NO:13). This packaged library containing the DNA fragments derived from the digestion of the amplified DNAs F1/R1, F2/R3, and F4/R4 amplified DNAs and was designated library Y5.

### B. THE ENV LIBRARY.

An expression library, designated the ENV library, was generated as follows. One microliter of PNF 2161 SISPA amplified DNA was used as the template in polymerase chain amplification reactions utilizing the following primer pairs: GEP-F15 (SEQ ID NO:128) and GEP-R15 (SEQ ID NO:129), which generate a 525 nucleotide HGV fragment; and GEP-F17 (SEQ ID NO:130) and GEP-R16 (SEQ ID NO:131), which generate a 765 nucleotide HGV fragment.

PCR amplification was for 35 cycles of 94°C for 1 min, 52°C for 1.5 minutes, and 72°C for 3 minutes. The amplified products were purified and digested with DNAse I. Ligation of KL1 and KL2 linkers to cDNA, amplification of DNA fragments and construction of libraries in lambda gt11 were performed essentially as described in Example 12A. The recombinant frequency of the library was greater than 70%. Analysis of the inserts by polymerase chain reaction using primers derived from the flanking regions of lambda gt11 confirmed the recombinant frequency and indicated that the insert size range was 150-500 nucleotides.

### C. THE NS3 LIBRARY.

An expression library designated NS3 was constructed as follows. A first fragment was amplified by polymerase chain reaction using the primers 470ep-F9 (SEQ ID NO:132) and 470ep-R9 (SEQ ID NO:133) and, as template, PNF 2161 SISPA amplified nucleic acids. The predicted product of this amplification reaction was 777 base pairs. The amplified fragment was gel purified by separation on a TAE gel. The fragment was further purified using "GENECLEAN" (Bio 101, La Jolla, CA).

Fragment F9/R9 was also amplified using the extension clone GE3L-11 (SEQ ID NO:41) as source material. Approximately 25 ng of GE3L-11 was used as template with the F9 and R9 primers in amplification reactions.

Both of the F9/R9 amplifications were for 30 cycles of 94°C, for 1 minute, 52°C for two minutes, and 72°C for 3 minutes, using "TAQ START" (Clonetech, Palo Alto, CA). The amplification products from both reactions were combined. The products were digested with DNAse I (10 *µ*l GE3L product and 25 ul of PNF SISPA product). The GE3L-based amplification product represented the majority of the amplification product starting material. Ligation of KL1 and KL2 linkers to cDNA, amplification of DNA fragments and construction of libraries in lambda gt11 were performed essentially as described in Example 12A.

The titer obtained was 2.5 × 10⁶ phage/ml and the percent recombinant phage was determined to be greater than 99%. Polymerase chain reaction analysis of the insert sizes confirmed the recombinant frequency and indicated an insert size range of 150 to 550 nucleotides.

In addition, a second fragment was also amplified using the GEP-F10/GEP-R10 primers (SEQ ID NO:135 and SEQ ID NO:136, respectively). One microliter of PNF 2161 SISPA amplified nucleic acids was used as template. The predicted fragment size of 570 nucleotides was obtained. The resulting amplification products were manipulated as just described for the F9/R9 amplifications. The titer obtained for this fragment when inserted in lambda gt11 was 1.47 X 10⁶ phage/ml, with a recombinant frequency of 90%.

### D. THE NS2 LIBRARY.

The NS2 epitope library was constructed using the methodologies described in Example 12A. Four DNA fragments containing all or part of the HGV proteins NS2, NS3, and NS5b were amplified from 1 ul of PNF 2161 SISPA DNA (prepared essentially as described in Example 12A). The library was generated using the primers given in Table 16 and SISPA amplified PNF 2162 DNA as template.

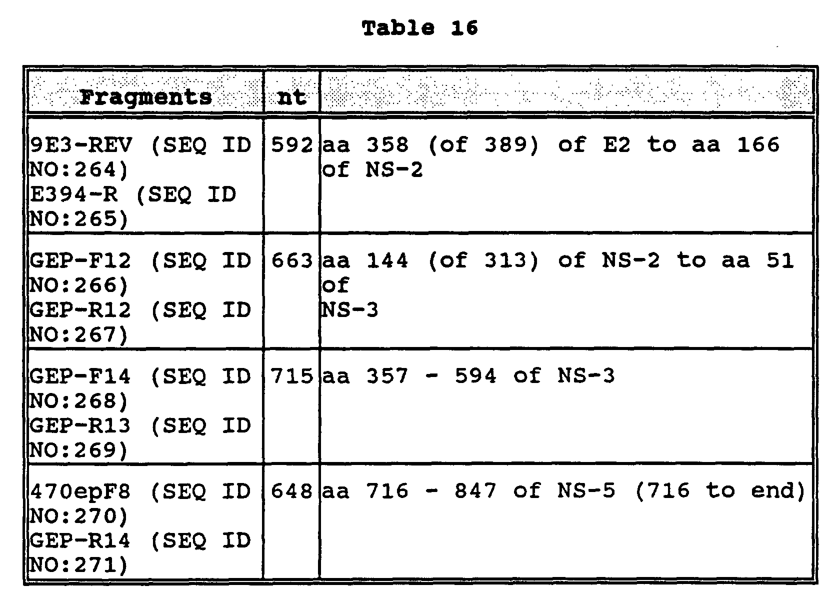

All amplifications were for 35 cycles of 94°C/1 minute, 48°C/2 minutes, and 73°C/3 minutes. All amplifications yielded at least a fragment of the expected size. The amplified products were mixed and in an approximately 1:1:1:1 ratio and partially digested with DNase I. As above, the digestion products were ligated to KL1 SISPA linkers, amplified and *EcoRI* digested. The digested fragments were ligated into lambda gt11. The ligation reactions were packaged.

The packaged ligation products were plated. The resulting library was determined to contain ∼70% recombinant phage with an observed insert size of 150 to 500 nucleotides.

### E. THE VNS5A LIBRARY.

Primers 470EXT4-2189R (SEQ ID NO:119) and 470EXT4-29F (SEQ ID NO:120) were used to isolate a 2.1 kb DNA fragment that contains the entire coding sequences for the HGV proteins NS4b and NS5a, as well as the 3' end of NS4a and the 5' end of NS5b. PCR amplifications using these primers were performed as described in Example 4G. Successful amplification was observed with multiple HGV-infected sera including the following: T56633 was from a blood donor whose donation was rejected due to an ALT value above the cutoff; samples E21-A and E20 were derived from Egyptian individuals suffering from hepatitis; and sample AH0591 is derived from an Australian individual who developed fulminant hepatitis.

The amplified products of E21-A and E20 were cloned into the T overhang site of the vector T/A (obtained from InVitrogen, San Diego, CA) essentially as described in Example 6. The 2.1 kb HGV inserts from these 2 plasmids were then isolated by the digestion of approximately 20 ug of plasmid DNA with approximately 150 units of the restriction enzyme EcoRI. After incubation overnight at 37°C, the products of the digestion were separated by TAE agarose gel electrophoresis. The products were excised from the section of the agarose gel containing the fragment of interest. The agarose was melted and extraction of the liberated DNA was carried out using the "GENECLEAN II" kit according to the manufacturers instructions (Bio 101, La Jolla, CA).

The purified 2.1 kb fragments derived from the E21-A and E20 samples, as well as the DNA fragments obtained from PCR amplification of samples T56633 and AH0591, were digested separately with DNAse I as described in Example 12A. For all 4 samples digestion conditions were determined that resulted in the isolation of fragments of between 100 to 1000 nts in size. After purification and trimming (Example 12A) the fragments derived from each of the 4 HGV infected samples were ligated separately to different sets of SISPA linkers. After ligation the DNAs were SISPA amplified.

The amplified DNAs were separately digested overnight at 37°C with approximately 100 units of *Eco*RI. The digested DNAs were then purified by spin column chromatography using G25 resin (5'3' Inc, Boulder, CO). Digested DNA from the samples T56633, AH0591, and E21-A were combined at a ratio of 1:1:1 and the mixture of DNAs was ligated into the *Eco*RI site of λgt11 as described in Example 12A. After packaging using the "GIGAPACK III XL" extract (Stratagene, LaJolla, CA), the resulting library was plated in the presence of IPTG and XGAL and determined to have a titer of approximately 1.0 x 10⁶ phage/ml and a recombinant frequency of approximately 70%.

### EXAMPLE 13

### Immunoscreening of the Epitope Libraries

### A. ISOLATION OF IMMUNOREACTIVE Y5 CLONES.

Two HGV positive sera, PNF2161 and JC, were used for immunoscreening of the Y5 library, essentially as described in Example 2. The Y5 phage library was plated onto 20 plates at approximately 15,000 phage per plate. The plates were incubated for approximately 5 hours and were overlaid with nitrocellulose filters (Schleicher and Schuell) overnight. The filters were blocked by incubation in AIB (1% gelatin plus 0.02% Na azide) for approximately 6 hours. The blocked filters were washed once with TBS.

Ten Y5 library filters were incubated overnight, with agitation, with PNF2161 serum and ten filters with JC serum. Both sera were diluted 1:10 in AIB. In order to reduce non-specific antibody binding, the diluted sera had been pre-treated by incubation overnight with nitrocellulose filters to which wild type λgt11 were adsorbed.

The filters were removed from the sera, washed 3 times with TBS and incubated with goat anti-human alkaline phosphatase-conjugated secondary antibody (Promega; diluted 1/7500 in AIB) for one hour. The filters were washed 4 times with TBS. Bound secondary antibody was detected by incubation of the filters in AP buffer (100 mM NaCl, 5 mM MgCl₂, 100 mM Tris pH 9.5) containing NBT and BCIP.

Plaques that tested positive in the initial screen were picked and eluted in 500 *µ*l of PDB (100 mM NaCl, 8.1 mM MgSO₄, 50 mM Tris pH 7.5, 0.02% Gelatin). The immunoreactive phage were purified by replating the eluted phage at a total density of 100 - 500 plaques per 100 mm plate. The plates were re-immunoscreened with the appropriate HGV-positive sera, essentially as described above. After color development several isolated, positive plaques were picked and put into 500 *µ*l of PDB. After 1 hour of incubation, 2 *µ*l of the re-purified phage PDB solution was used as template in a PCR reaction containing the 11F (SEQ ID NO:25) and 11R (SEQ ID NO:13) PCR primers. These primers are homologous to sequences located 70 nucleotides (nt) 5' and 90 nt 3' of the *EcoRI* site of λgt11. The PCR reactions were amplified through 30 cycles of 94°C for 1 minute, 55°C for 1.5 minutes and 72°C for 2 minutes.

The PCR amplification reactions were size-fractionated on agarose gels. PCR amplification of purified plaques resulted in a single band for each single-plaque amplification reaction, where the amplified fragment contained the DNA insert plus approximately 140 bp of 5' and 3' phage flanking sequences. The amplified products, from PCR reactions resulting in single bands, were purified using a "S-300 HR" spin column (Pharmacia), following manufacturers instructions. The DNA was quantitated and DNA sequenced employing an Applied Biosystems automated sequencer 373A and appropriate protocols.

The above-described screening of the Y5 library with JC sera resulted in the purification and DNA sequencing of the positive-strand clones presented in Table 17. Positive-strand clones correspond to the 5' to 3' translation of the HGV sequence presented in SEQ ID NO:14 -- the polyprotein reading frame.

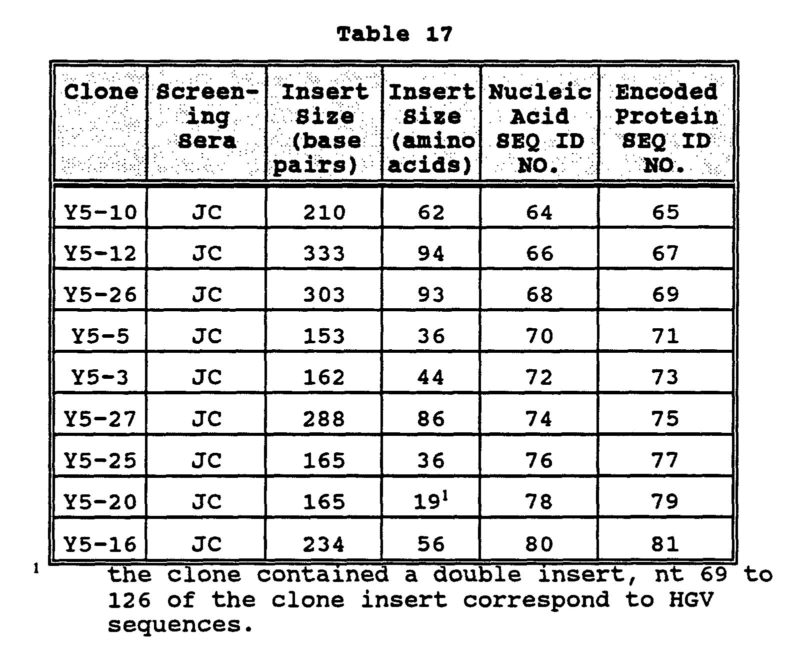

These clones delineated 2 immunogenic regions within the putative NS5 protein of HGV. These two region, relative to the sequence presented as SEQ ID NO:14 are positions 6636 to 6821 and 7278 to 7385.

Further, screening of the Y5 library with PNF 2161 sera resulted in the purification and DNA sequencing of the following negative-strand clones presented in Table 18. Negative-strand clones correspond to the 5' to 3' translation of the sequence complementary to the HGV sequence presented in SEQ ID NO:14.

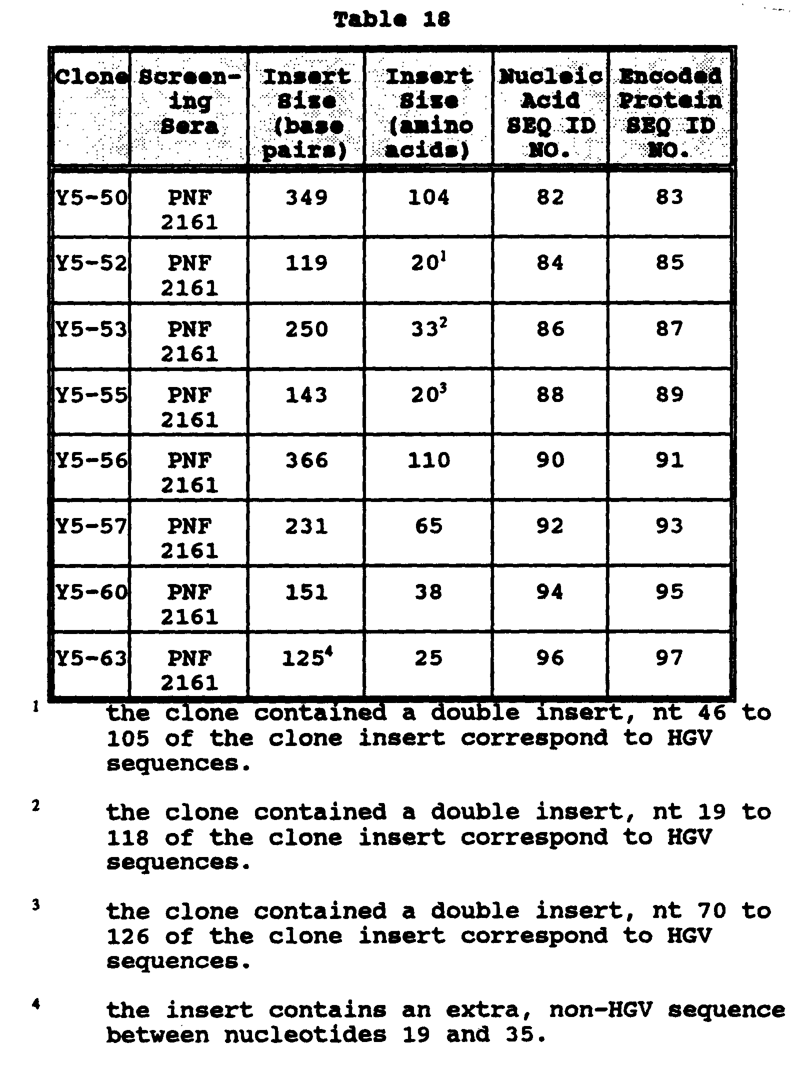

All of these sequences contain portions of the original HGV clone 470-20-1 isolated using the PNF 2161 serum.

Additional epitope clones from the Y5 library were isolated as follows. The Y5 library was screened with the HGV infected sera J21689 and T56633 using the methods described in Example 13. Greater than 400 positive plaques were obtained, indicating the presence of a strongly immunogenic sequence recognized by both of these HGV infected sera. Ten of these positive plaques were purified and DNA sequenced. The results obtained from the DNA sequencing are delineated in Table 19.

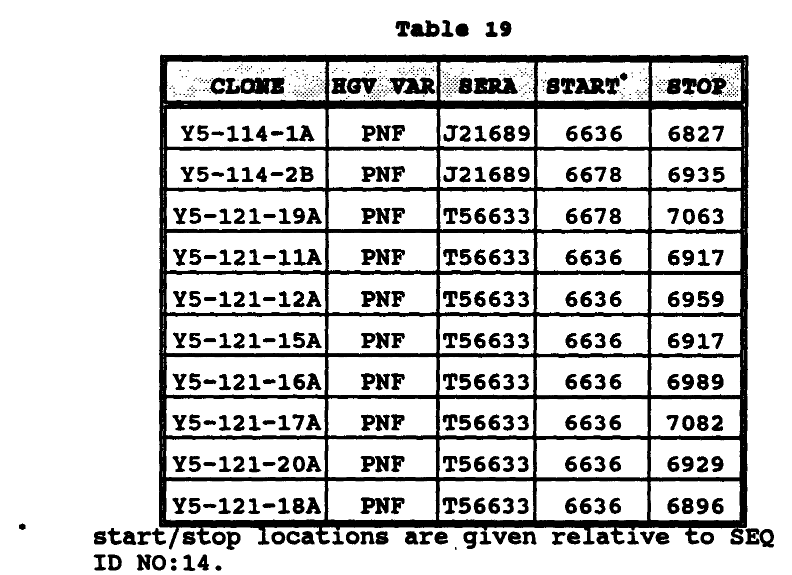

Comparison of these sequences with those obtained previously from screening this library indicated that these clones all contained the same epitope(s) that are contained in the previously isolated epitope clone Y5-10. Two of the clones, Y5-114-2B and Y5-121-19A are distinguished by the fact that their 5' ends are located 14 amino acids closer to the carboxy terminal of NS5a than the previously observed start of clones Y5-10, Y5-12, and Y5-26. None of the above clones has its 3' end interior to that observed in the clone Y5-10. Thus a minimal sequence of this epitope is contained within amino acid sequence (SEQ ID NO:272).

### B. ANTIGENIC CLONES FROM THE ENV LIBRARY.

The ENV library was screened with HGV serum J21094. This serum (J21094) was identified as HCV positive based on the first generation (c-100) HCV test. Subsequent testing of the initial J21094 serum sample, and of subsequently obtained J21094 samples, by PCR and with other HCV antigens confirmed that the source individual for the serum was HCV infected. Evidence for the presence of HGV nucleic acid was obtained via PCR analysis using the 470-20-1 and NS5 primer sets.

A number of phage clones were identified as immunoreactive with J21094 serum. The phage were plaque purified and sequenced. Seven of the clones (Q7-12-1, Q7-16-2-2, Q7-15-2, Q7-17-2-1, Q7-19-1, and Q7-19-2-1) contained the same insert. The nucleotide sequence for Q7-12-1 is presented as SEQ ID NO:143 (polypeptide sequence, SEQ ID NO:144).

One additional clone, Q7-16-1, obtained by the method just described, has the same 5' end as Q7-12-1, but is 26 amino acids shorter at the 3' end.

### C. ANTIGENIC CLONES FROM THE NS3 LIBRARY.

A one to one mixture of the F9/R9 phage and F10/R10 phage were screened using the following sera: PNF 2161, J21689 and E57963. Both J21689 and E57963 are sera that test co-positive for HCV and HGV by PCR (using multiple primers). Each immunoscreening was of 10 plates or approximately 150,000 phage. Some of the immunopositive clones identified in these screens are as follows.

Clone Y12-10-3 (polynucleotide sequence, SEQ ID NO:145; polypeptide sequence, SEQ ID NO:146) was identified by its immunoreactivity with J21689 serum. The clone expresses an 88 amino acid insert from HGV NS3.

Clone Y12-15-1 (polynucleotide sequence, SEQ ID NO:147; polypeptide sequence, SEQ ID NO:148) was identified by its immunoreactivity with E57963 serum. The clone expresses a 64 amino acid insert from the NS3 protein of HGV. This sequence is located approximately 70 amino acids 5' to clone Y12-10-3.

### D. ANTIGENIC CLONES FROM THE NS2 LIBRARY.

Multiple positive plaques were isolated by screening the NS2 library with HGV-positive serum T56633. Eleven of these plaques were subsequently purified and DNA sequenced. The locations of the inserts contained within these plaques (relative to SEQ ID NO:14) are delineated in Table 20.

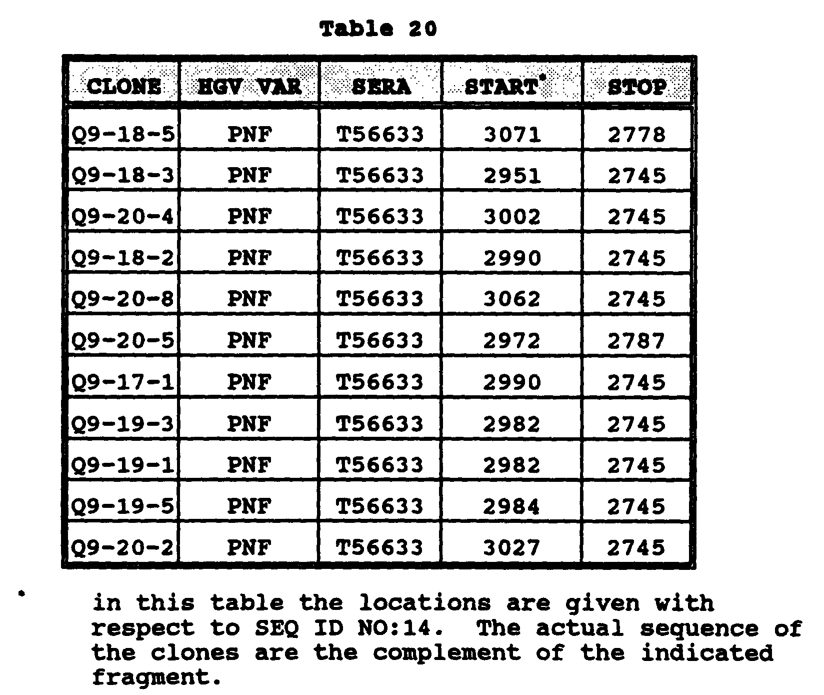

All of the immunoclones express portions of the same open reading frame (ORF). This reading frame is encoded by the HGV polynucleotide strand that is complementary to the sequence encoding the polyprotein. This ORF extends between nts 6322 and 6865 of the sequence complementary to SEQ ID NO:14. There is a Methionine that could serve as a site of translation initiation located at nt 6388 of the complementary strand that would allow for the production of a 159 amino acid protein.

The smallest amino acid sequence common to all of the 11 sequenced clones is located between nts 6342 to 6606 (relative to the complementary strand of SEQ ID NO:14). The amino acid sequence encoded by this region of the negative strand of HGV-PNF 2161 is presented as SEQ ID NO:273.

The subcloning and subsequent Western blot analysis of immunoreactive negative strand regions is described below.

### E. ANTIGENIC CLONES FROM THE VNS5A LIBRARY.

Approximately 1.5 X 10⁵ phage from the VNS5a library was plated out and subsequently screened with the HGV-positive serum J29374 using the procedures described in Example 13. Immunoscreening of the VNS5a library with J29374 resulted in the isolation of multiple positive plaques. Six of these plaques were purified and subsequently DNA sequenced. The original strain of the DNA sequence obtained could be determined by which of the SISPA linker sequences was present at the 5' and 3' ends of the clones. The locations of the starts and stops of the obtained clones (relative to SEQ ID NO:14) and their source sera are summarized in Table 21.

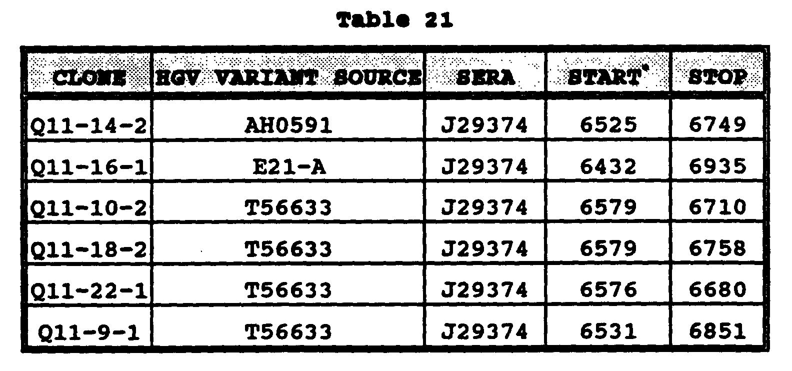

All of these clones contain the sequence of the clone Q11-22-1 in common (SEQ ID NO:274). This amino acid sequence is located immediately 5' to the minimal sequence of the Y5-10 epitope. Thus it defines an additional unique epitope in HGV NS5a (along with Y5-10 and Y5-5). Comparison of the observed amino acid sequence of these 3 HGV variants with the sequence of the PNF-2161 and JC isolates reveals few amino acid substitutions.

### EXAMPLE 14

### Further Characterization of Immunoreactive Clones

### A. SUBCLONING.

### 1. Y5 CLONES.

Clones Y5-10, Y5-16, and Y5-5 were selected for subcloning into the expression vector pGEX-HisB. PCR primers were designed which removed the extraneous linker sequences at the end of these clones. These primers also introduced (i) a *NcoI* site at the 5' end (relative to the coding sequence) of each insert, and (ii) a *BamHI* site at the 3' end of each insert. Using these primers (see Table 22), the DNA fragments were amplified from 2 *µ*l of the plaque pure stocks.

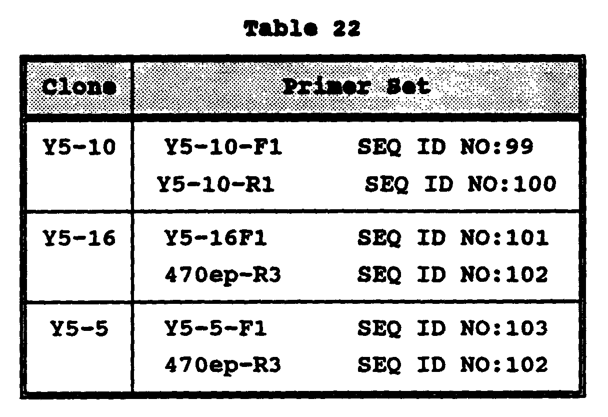

Amplifications were performed as follows: 30 cycles of 94°C for 1 minute, 50°C for 1.5 minutes, and 72°C for 2 minutes. After amplification the resulting DNAs were purified using "WIZARD PCR," spin columns, the samples eluted in 50 *µ*l, and digested overnight with *NcoI* and *BamHI.* A minimum of 30 units of each enzyme was used in the restriction endonuclease digestions (*NcoI,* Boehringer Mannhiem; *BamHI,* Promega).

The digested PCR fragments were ligated overnight to expression vector pGEX-HisB that had been digested with *NcoI* and *BamHI.* Each set of ligated plasmids was independently used to transform *E. coli* strain W3110, using a heat shock protocol (Ausubel, *et al.;* Maniatis, *et al.*). Transformants were selected on LB plates containing 100 *µ*g/ml ampicillin and resistant colonies were used to inoculate 2 mls of LB containing 100 *µ*g/ml ampicillin. Cultures expressing non-recombinant sj26/his protein were also prepared.

After incubation overnight at 37°C the cultures were diluted 1/10 into 2 mls of fresh LB plus ampicillin and grown for an additional 1 hour at 37°C. IPTG was added to a final concentration of 0.2 mM and the cultures were grown for an additional 3 hours at 37°C. The bacteria were pelleted by centrifugation and the bacterial pellet was resuspended in 100 *µ*l PBS. To the pellet, 100 *µ*l of 2X SDS sample buffer (0.125 M Tris, pH 6.8, 10% glycine, 5% β-mercaptoethanol, 2.3% SDS) was added. The resulting lysates were vortexed and heated to 100°C for 5 minutes. Aliquots (15 *µ* l) of each lysate were loaded onto a 12% acrylamide SDS-PAGE gel.

The expressed proteins were size-fractionated by electrophoresis. The separated proteins were transferred from the gel to nitrocellulose filters using standard techniques (Harlow, *et al*.). An additional gel containing the expressed proteins was stained using coomasie blue protein stain.

Transformants carrying plasmids Y5-10, Y5-5 and Y5-16 expressed significant amounts of correctly sized recombinant fusion proteins. The identity of the recombinant fusions were confirmed by incubating a Western blot (prepared above) with a murine monoclonal antibody that is specifically immunoreactive with sj26 (Sierra BioSource, Gilroy, CA).

Additional confirmation that the picked colonies contained the appropriate insert was obtained as follows. A phage solution for each colony was prepared by inoculating 40 *µ*l of TE solution with a toothpick containing a small amount of bacteria putatively expressing a recombinant clone had been inoculated. A 5 *µ*l sample was taken from each solution and separately PCR amplified.

The amplifications employed the appropriate forward primer, (*e.g.*, Y5-10 F for a colony putatively expressing Y5-10) and a reverse primer (SEQ ID NO:104) homologous to a sequence located 3' to the cloning sites of the plasmid pGEX-HisB. The PCR amplifications were for 25 cycles as follows: 94°C for 1 minute, 50°C for 1.5 minutes and 72°C for 2 minutes. All of the colonies selected for further analysis produced a correctly sized DNA band with no other obvious bands under these conditions.

The immunoreactivity of the antigens expressed from the Y5-10, Y5-16, & Y5-5 inserts (expressed as sj26-his fusion proteins) was determined as follows. Aliquots (15 *µ*l) of the crude lysates prepared above were size-fractionated by SDS-PAGE using a 12% acrylamide gel. The proteins were electro-blotted ("NOVEX MINICELL MINIBLOT II," San Diego, CA) onto nitrocellulose filters. The filters were then individually incubated with one of the following sera: JC, PNF 2161, and super normal serum 4 (SN4) (R05072) as a negative control. In addition, one filter was incubated with anti-sj26 monoclonal antibodies (RM001; Sierra BioSource).

As expected, the recombinant protein produced by the bacteria expressing the antigens encoded by the Y5-10, Y5-5, and Y5-16 inserts all reacted with JC sera. No reactivity was observed with either PNF 2161 or SN4 sera. All proteins appeared to be expressed at similar levels as determined by their reactivity to the anti-sj26 monoclonal antibody. The Y5-5 and Y5-10 encoded proteins were selected for further purification.

*E. coli* carrying Y5-5- and Y5-10- containing pGEX-HisB vectors were cultured and expression of the fusion protein induced as described above. The cells were lysed in PBS, containing 2 mM PMSF, using a French Press at 1500 psi. The crude lysate was spun to remove cellular debris. The supernatant was loaded onto the glutathione affinity column at a high flow rate and the column was washed with 10 column volumes of PBS. The Y5-5 and Y5-10 fusion proteins were eluted with 10 mM Tris pH 8.8 containing 10 mM glutathione.

Each of the fusion protein samples was diluted 1/10 with Buffer A (10 mM Tris pH 8.8, containing 8 M urea) and loaded onto a nickel charged-chelating "SEPHAROSE" fast flow column. Each column was repeatedly washed with Buffer A until no further contaminants were eluted. The fusion proteins were eluted using a gradient of imidazole in buffer A. An imidazole gradient was run from 0 to 0.5 M imidazole in 20 column volumes. Fractions were collected.

Each set of fractions was analyzed by standard SDS-PAGE using 12% polyacrylamide gels. Pools of the Y5-5 and Y5-10 fusion protein-containing fractions were separately made.

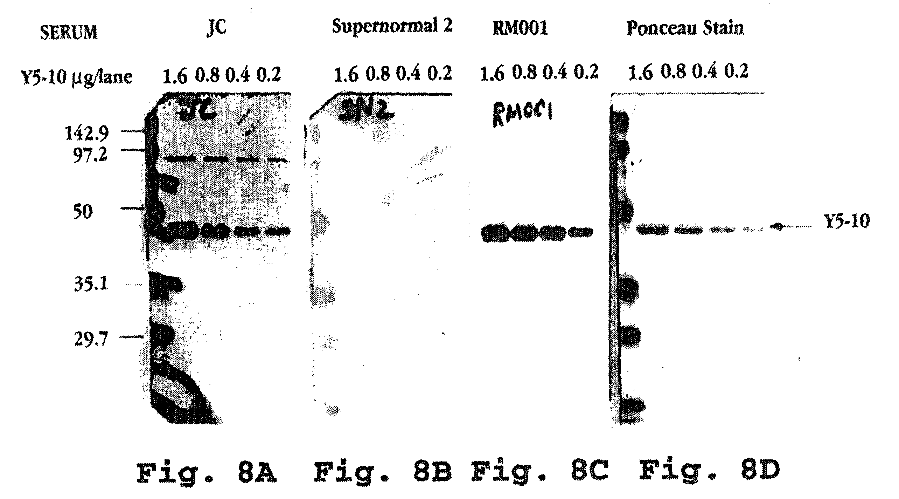

Figures 8A to 8D show the results of Western blot analysis of the following samples (*µ*g/lane): lane 1, Y5-10 antigen 1.6 *µ*g; lane 2, Y5-10 antigen 0.8 *µ*g; lane 3, Y5-10 antigen 0.4 *µ*g; and lane 4, Y5-10 antigen 0.2 *µ*g. Human serum JC (Figure 8A) and Super Normal 2 serum (Figure 8B) were diluted 1:100. The anti-GST mouse monoclonal antibody RM001 (Figure 8C) was diluted 1:1000. Figure 8D shows the Y5-10 antigen resolved by SDS-PAGE, transferred onto the nitrocellulose membrane and stained with Ponceau S protein stain (Kodak, Rochester, NY; Sigma). Arrow indicates the location of Y5.10 antigen. These results demonstrate that Y5-10 is specifically immunoreactive with N-(ABCDE) human serum JC.

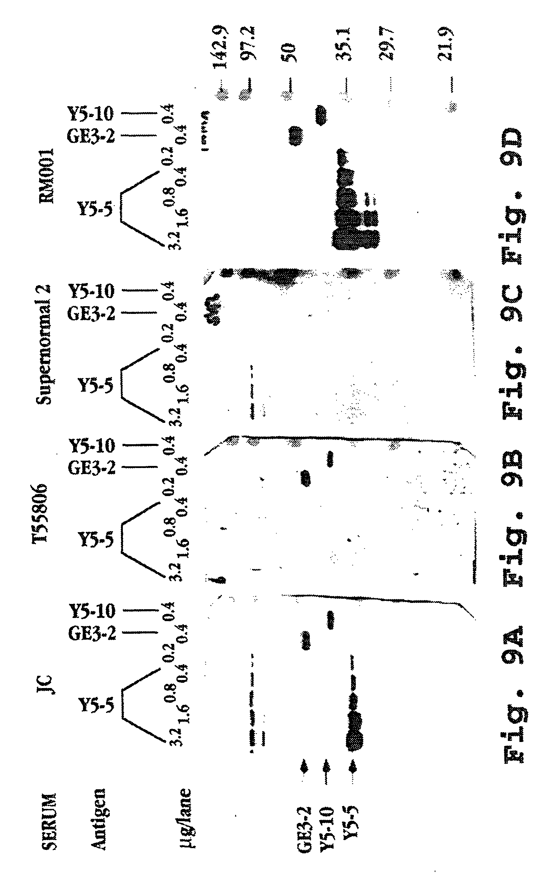

Figures 9A to 9D show the results of Western blot analysis of the following samples: lane 1, Y5-5 antigen 3.2 *µ*g; lane 2, Y5-5 antigen 1.6 *µ*g; lane 3, Y5-5 antigen 0.8 *µ*g; lane 4, Y5-5 antigen 0.4 *µ*g; lane 5, Y5-5 antigen 0.2 *µ*g; lane 6, GE3-2 antigen 0.4 *µ*g; and lane 7, Y5-10 antigen 0.4 *µ*g. Human serum JC (Figure 9A), T55806 (Figure 9B), and Super Normal 2 serum (Figure 9C) were diluted 1:100. RM001, the anti-GST mouse monoclonal antibody, (Figure 9D) was diluted 1:1000. Arrows indicate the locations of antigens Y5.5, GE3.2 and Y5.10. These results show specific immunoreactivity of the Y5-5 antigen with the JC serum. Further, the antigens GE3-2 and Y5-10 were reactive with T55806. However, the Y5-5 antigen was not reactive with the HGV-positive sera T55806.

The Y5-10 antigen was also size-fractionated by SDS polyacrylamide gel electrophoresis. The gel was stained using coomasie blue protein stain. The gel was scanned for purity with a laser densitometer. The purity of the Y5-10 fusion protein was approximately 95%.

### 2. ENV CLONES.

The immunoclone Q7-12-1 was originally isolated by screening the ENV epitope library with the HGV positive sera J21094. Sequence specific primers were employed to isolate the HGV insert contained within the Q7-12-1 λgt11 clone. The Q7-12-1 insert was excised and cloned into pGEX-Nde. The sequence of the insert was confirmed by the DNA sequencing (SEQ ID NO:275).

### 3. NS3 CLONES.

The immunoclone Y12-15-1 was originally isolated by screening the NS3 epitope library with the HGV positive sera E57963. Sequence specific primers were employed to isolate the HGV insert contained within the Y12-15-1 λgt11 clone. The Y12-15-1 insert was excised and cloned into pGEX-Nde. The sequence of the insert was confirmed by the DNA sequencing (SEQ ID NO:276).

The immunoclone Y12-10-3 was originally isolated by screening the NS3 epitope library with the HGV positive sera J21689. Sequence specific primers were employed to isolate the HGV insert contained within the Y12-10-3 λgt11 clone. The Y12-10-3 insert was excised and cloned into pGEX-Nde. Production of fusion proteins by selected clone was evaluated by Western blot analysis. The sequence of the insert was confirmed by the DNA sequencing (SEQ ID NO:277).

### 4. NS2 CLONES.

Multiple negative strand immunoclones derived from sequences complementary to the sequences of the NS2 region of SEQ ID NO:14 were isolated. There are at least 2 significant ORFs encoded by the negative strand of HGV. The first of these ORFs, represented by the Q9 series of clones was described above. The second of these ORFs is located between nts 6723 and 7259 of the complement of SEQ ID NO:14 and also possess a 5' methionine at nt 6774. The second ORF encodes a 162 amino acid protein.

Selected portions of the sequences of both of these negative strand ORFs were cloned into the expression vector pGEX-Nde. All of these subclones were obtained by the PCR amplification of PNF 2161 SISPA material using appropriate oligonucleotide primers, thus they contain the sequence of the HGV-PNF 2161 variant. Table 23 indicates the names, size of the ORF and locations relative to the complement of SEQ ID NO:14.

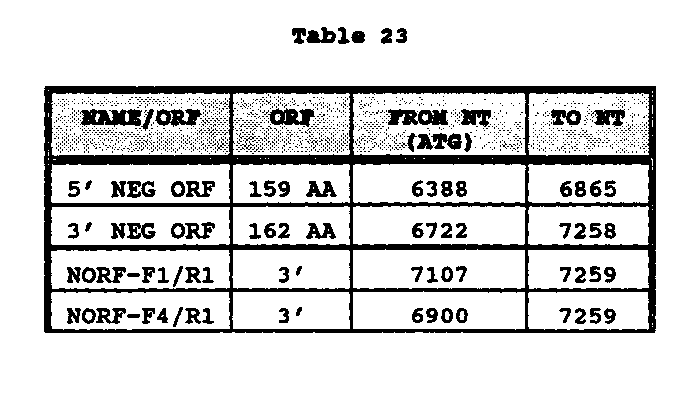

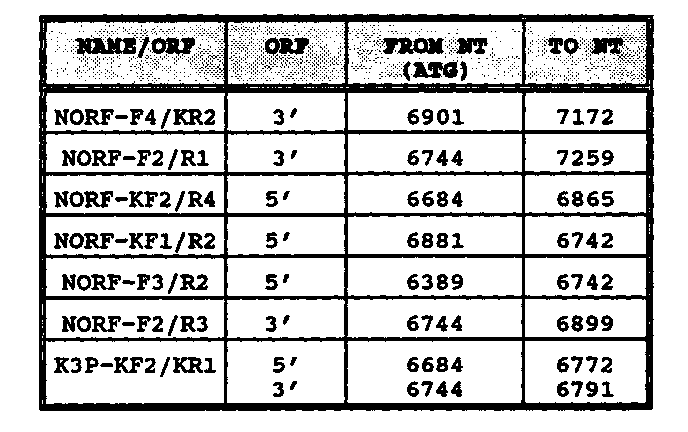

The first 2 lines of this table identify the locations of the NS2 region 5' and 3' negative strand ORFs relative to the complement of SEQ ID NO:14. The remaining lines indicate the specific nucleotide sequences expressed by all of the 9 clones. Note that several of the clones express amino acids located 5' to the hypothetical HGV initiating methionine of the ORF. Also note that the last clone listed, K3p-KF2/KR1, is a chimera expressing the indicated portions of the 5' ORF followed by the indicated portions of the 3' ORF.

All of the DNA fragments were subsequently cloned into pGEX-Nde. Insert containing clones were also identified and confirmed.

### 5. NS5A CLONES.

Table 24 lists a number of NS5a clones and the regions of SEQ ID NO:14 to which they correspond.

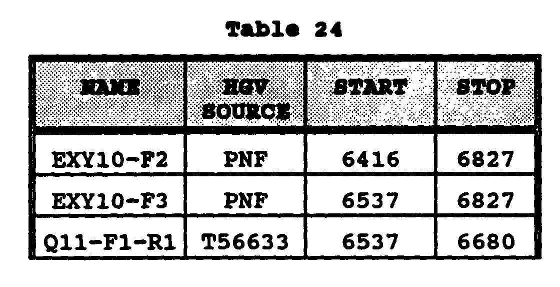

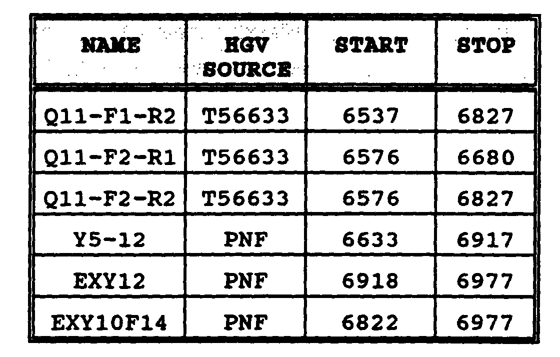

These sequences were cloned into the vector pGEX-Nde for expression of the encoded protein antigens.

### B. WESTERN BLOT ANALYSIS OF SELECTED HGV SUBCLONES.

To determine the reactivity of both the negative and positive strand constructs described above whole cell lysates from bacteria expressing the various HGV subclones were prepared essentially as described in Example 13B. Aliquots of the expressed proteins were then fractionated by SDS-PAGE, the proteins transferred to nitrocellulose filters, and the filters probed with HGV-positive or control sera (*e.g.,* anti-SJ26 MAB RM01). The blots were incubated with an appropriate reporter antibody.

With respect to the HGV proteins tested, clear immunoreactivity to the protein NORF-F3/R2 was detected with the HGV sera J21689 and T56633. The NORF-F3/R2 subclone expresses the amino acid sequences that were also encoded by the Q9 series of negative strand epitope clones. The observed strong reactivity with HGV sera T56633 confirms the immunoreactivity of this region of the negative strand of HGV. Reactivity to the NORF-F3/R2 protein was not observed with the sera from the HGV negative individual R04316 or any of 5 other HGV negative supernormal sera tested.

Additional blots indicated that the other major 5' ORF clone NORF KF2-R4, which expresses amino acids of the carboxy terminal half of the 5' negative strand ORF located does not react with the HGV-positive sera T56633. This observation in conjunction with the locations of the Q9 epitope clones described above suggest that the immunogenic epitope of this portion of the negative strand is contained within the 55 amino acid delineated above (SEQ ID NO:273). The fact that this sequence is recognized by other HGV antisera, including J21689, indicates that immunoreactivity towards this sequence is relatively widespread among HGV infected individuals.

Further, clear immunoreactivity with the Y12-10-3 protein was observed with the HGV-infected sera J21689, J29374, and E57963. The specificity of this reactivity is additionally supported by the failure to observe immunoreactivity with the HGV antisera J29374 or E57963 in the absence of the induction of Y12-10-3 protein expression by IPTG. No reactivity to Y12-10-3 was observed with any of 7 supernormal sera tested.

### EXAMPLE 15

### A Multi-Antigen HGV Diagnostic Assay

Although the epitope clones described above do not appear to be reactive with all HGV PCR-positive sera, many of these clones react with a substantial fraction of the HGV infected sera they have been tested against. Additionally these proteins have not exhibited substantial cross reactivity with HGV-negative sera. It is therefore possible to construct a diagnostic assay in which several of these proteins are combined so that the individual reactivities of the protein are summed. Such an assay is expected to have a relatively high sensitivity for the detection of HGV-positive sera and a relatively low background reactivity with HGV-negative sera.

Exemplary epitopes/antigens useful in such an assay include, but are not limited to, NORF-F3/R2 (NS2-Neg strand), Y12-10-3 (NS3), Q11-F2-R1 (NS5a), Y5-10 (NS5a), Y5-5 (NS5a), Q11-F2-R2 (combines 2 epitopes of NS5a).

For this assay, individual antigens are typically selected that contain different unique epitopes that recognized different subset of HGV-positive sera. Further, such antigens typically do not significantly react with HGV-negative sera. Following the guidance of the present invention, additional useful immunogenic clones can be isolated.

A multi-antigen diagnostic assay can take many formats. In one embodiment, the assay might entail immobilizing each of, et al., 5 HGV proteins and control proteins at separate locations on a nitrocellulose strip or other convenient solid phase format. Alternatively the non-viral portions of, for example, an HGV-fusion protein could be modified, either by insertions or deletions such that they would naturally migrate to easily distinguishable locations upon SDS PAGE and subsequent Western blot analysis. Strips are then incubated in test sera. After detection of bound antibody, a serum may then be scored based on (i) the number of antigens with which it is immunoreactive, and (ii) the strength of the immunological reactions. Reactivity to a non-HGV control protein would render a serum un-typeable. Reactivity with no HGV protein would classify a serum as HGV-negative.

ELISA-based screening assay can be formed by combining purified antigen proteins in a single reaction zone or by creating protein constructs that express 2 or more of the reactive epitopes as a single protein (*e.g*., a HGV mosaic polypeptide). The methods to construct mosaic polypeptides is described herein. Q11-F2-R2 construct described above, in fact, represents a "matrix protein" that encodes 2 individual epitopes in a single polypeptide chain. Western blot assays may serve as a confirmatory assay for such an ELISA screening test.

Alternatively or in addition, full length HGV proteins, such as E2, NS5a and NS3 might be placed in a single reaction zone. Sera reactive with such proteins may also be confirmed as HGV positive by Western blot assay.

### EXAMPLE 16

### Expression of Large HGV Polypeptides

### A. Expression of Larger HGV Antigens in E. coli

### 1. Cloning and Expression.

To identify conformational HGV epitopes (not covered by small overlapping HGV constructs or by phage library screening) larger HGV protein constructs were generated in the pET-21a(+) vector (Novagen, WI) based on the prediction of cleavage sites (Bazan, *et al.,* 1989; Chambers, *et al.*, 1990b; Grakoui, *et al.,* 1993; Kyte and Doolittle, 1982). Individual HGV protein constructs were generated in a similar fashion to HGV sequences cloned into pGEX vectors.

Briefly, selected HGV sequences were RT-PCR amplified from a HGV(+) human sera source using HGV sequence specific primers. The primers were engineered to contain appropriate restriction sites for cloning manipulations in the pET vector. Coding sequences of interest were typically inserted between the *Eco*RI site and the *Hind*III sites in the vector to produce 5' in-frame fusions with T7.*Tag* leader sequence and 3' in-frame fusion with a hexamer histidine sequence. T7.*Tag* (an 11 amino acid sequence) allows the detection of the fusion proteins using an anti-T7.*Tag* monoclonal antibody (Novagen, WI). The histidine hexamer at the carboxyl end of the fusion protein allows the purification of the protein using immobilized metal ion affinity chromatography.

HGV fragments were ligated into appropriately digested pET-21a(+) vectors. Ligated products were transformed into competent E.coli (HMS174; Novagen, WI). Plasmid DNA from transformed HMS174 was analyzed for the presence of HGV sequences by PCR, using primers T7F(SEQ ID NO:157) and T7R(SEQ ID NO:158), which are homologous to pET-21a(+) vector sequences flanking the inserted molecule. The size of the PCR product was the insert size plus approximately 260 bp derived from the vector.

For each construct the PCR results confirmed the presence of the insert sequences. Transformants with appropriate inserts were selected, plasmid DNAs with HGV inserts prepared and introduced into HMS174(DE3) competent E.coli (Novagen, WI) for the expression of HGV proteins.

Expression of HGV proteins was induced with 1 mM IPTG. Expression of the T7.*Tag* fusion proteins was monitored by the appearance of the predicted size proteins on the Coomassie blue stained gel. Expression of the fusion proteins was confirmed by Western blot analysis using anti-T7.*Tag* antibody (Novagen, WI). HGV proteins expressed in pET-21a(+) vector are shown in the Table 25. The start and end points of the expressed sequences are given relative to SEQ ID NO:14. The amino acid sequence of GE-Cap is shown in SEQ ID NO:185.

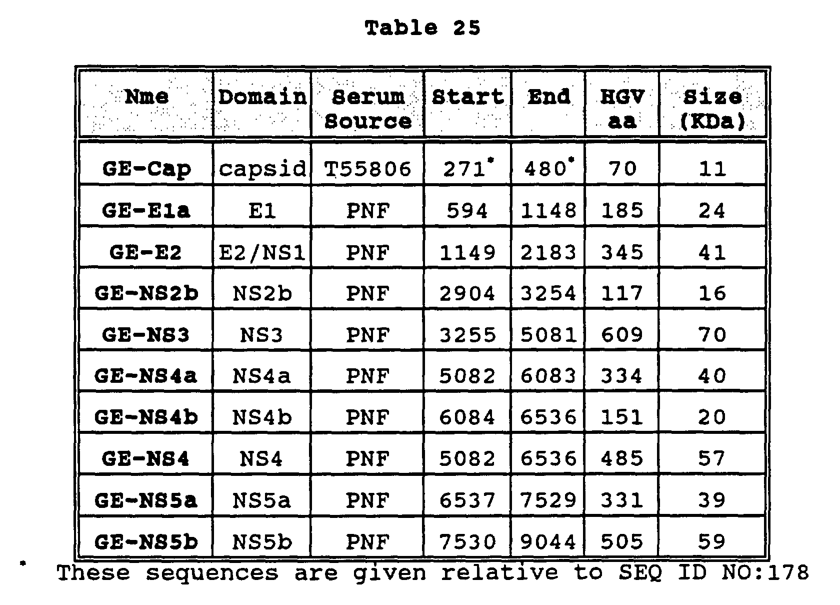

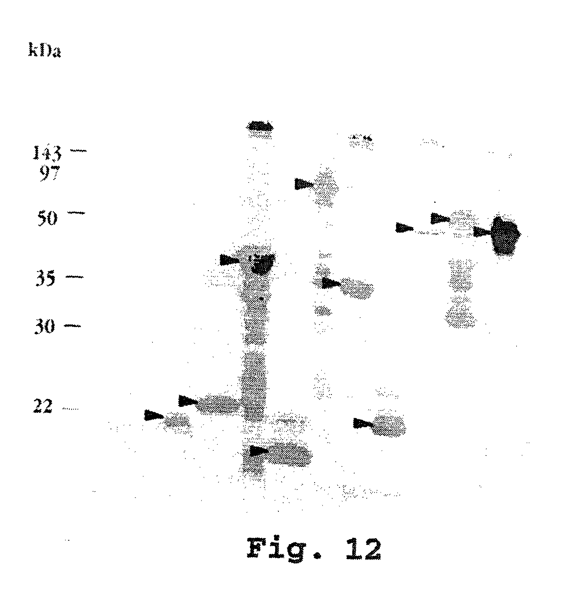

Figure 12 shows the expression of each HGV proteins demonstrated by Western blot analysis with T7.*Tag* monoclonal antibody. The lanes in Figure 12 are as follows: Lane 1, pre-stained molecular weight marker (Bio-Rad); Lane 2, uninduced GE-Cap lysate; Lanes 3-11, IPTG induced lysates of GE-Cap, E1a, E2, NS2b, NS3, NS4a, NS4b, NS4, and NS5b lysate, respectively. Lane 12 contained 1 *µ*g of purified NS5a. Locations of each antigen are marked with arrow heads. As shown in Figure 12 all the HGV proteins were expressed in *E.coli.*

### 2. Western Blot Analyses of HGV proteins expressed in pET vector

Western blot analyses of the HGV protein expressed in pET vector were performed as described in Example 11C using *E. coli* whole cell lysates and pre-absorbed sera. The results of these analyses demonstrated that several of pET HGV proteins are specifically immunoreactive with HGV-positive human sera but not with HGV-negative human sera. GE-NS2b-1 protein was immunoreactive with J21689 serum. The GE-NS5a-3 protein was immunoreactivity with several HGV (+) sera on Western blot analysis, including JC, T55806, T56633, J21689, E57963 and R0001. Among these sera T55806, J21689 and E57963 are HCV co-positive (by the PCR analysis). Neither GE-NS2b-1 nor GE-NS5a-3 were immunoreactive with several HGV negative sera tested.

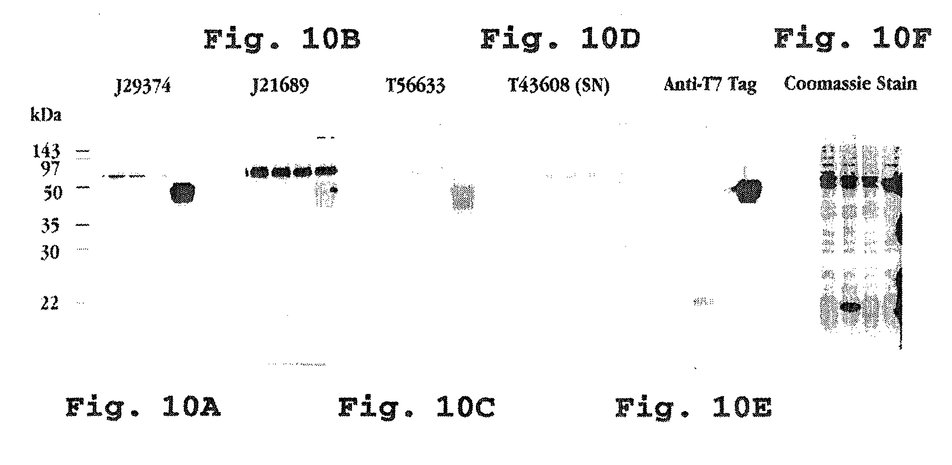

Figures 10A to 10F show the exemplary results of a series of Western blot experiments examining the reactivity of antigens GE-NS2b and GE-NS5a3. The lanes in each blot of Figures 10A to 10F are as follows: Lane 1, uninduced GE-NS2b lysate; Lane 2, IPTG induced GE-NS2b lysate; Lane 3, uninduced GE-NS5a lysate; and Lane 4, IPTG induced GE-NS5a lysate. Each blot was incubated with a human serum or mouse monoclonal antibody: Figure 10A, J29374; Figure 10B, J21689; Figure 10C, T56633; Figure 10D, T43608 (super normal serum); Figure 10E, Anti-T7.Tag; and Figure 10F, coomassie stained gel. The serum or monoclonal antibody that was used is indicated above each blot. Human sera were diluted 1:100 and anti-T7.Tag mouse monoclonal antibody was diluted 1:1000.

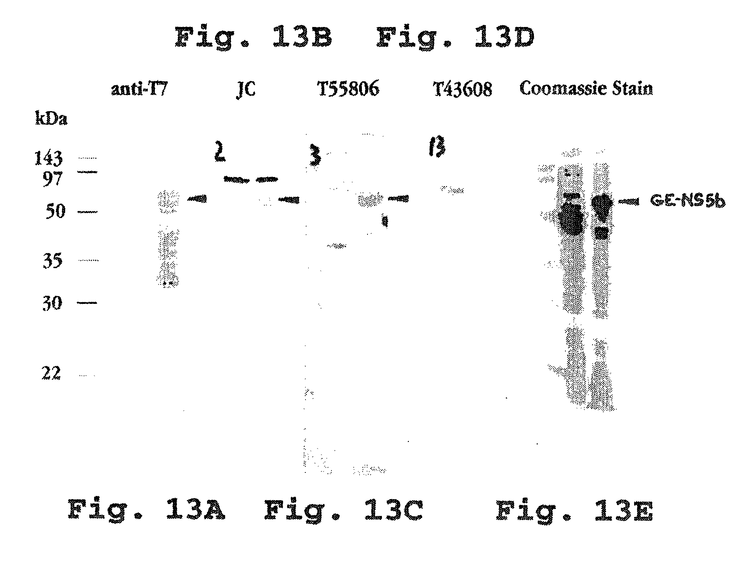

In addition to the sera listed above, additional HGV-PCR positive sera have been screened using GE-NS5a. The results of all these analyses have demonstrated the reactivity of the GE-NS5a antigen with multiple HGV-infected sera. GE-NS5b was immunoreactive with HGV(+) sera JC and T55806 but was not immunoreactive with HGV(-) negative sera tested. Figures 13A to 13E show the results of a series of Western blot experiments examining the reactivity of antigen GE-NS5b. The lanes in each blot the figures are as follows: Lane 1, pre-stained molecular weight marker (Bio-Rad); Lane 2, uninduced GE-NS5b lysate; Lane 3, IPTG induced GE-NS5b lysate.

Each blot was incubated with a human serum or mouse monoclonal antibody: Figure 13A, anti-T7.Tag monoclonal antibody; Figure 13B, JC; Figure 13C, T55806; and Figure 13D, T43608 (super normal serum). Figure 13E is a Coomassie Stain.

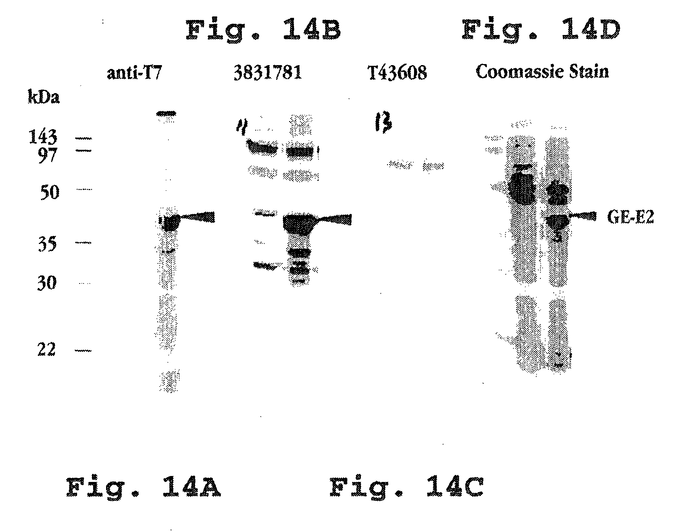

Figures 14A to 14D show the results of a series of Western blot experiments examining the reactivity of antigen GE-E2. The lanes in each of Figures 14A to 14D are as follows: Lane 1, pre-stained molecular weight marker (Bio-Rad); Lane 2, uninduced GE-E2 lysate; Lane 3, IPTG induced GE-E2 lysate. Each blot was incubated with a human serum or mouse monoclonal antibody: Figure 14A, anti-T7.Tag monoclonal antibody; Figure 14B, 3831781; and Figure 14C, T43608 (super normal serum). Figure 14D is Coomassie Stain. The serum or monoclonal antibody that was used is indicated above each blot. GE-E2 protein was immunoreactive with HGV-positive serum 3831781 but was not immunoreactive with supernormal serum T43608 (Figures 14B and 14C, respectively).

Antigens GE-Cap and GE-NS4a were also specifically immunoreactive with HGV(+) serum J21689.

### B. Expression larger HGV Antigens in Insect Cells.

Expression of proteins using recombinant baculoviruses offers the following advantages (i) a high level of recombinant protein expression, and (ii) the benefits of a higher eucaryotic system, including efficient protein translocation and modification. This system is particularly useful for expression of translocated proteins, *e.g.,* HGV E1, E2 and NS2a.

### 1. Cloning and Expression.

*Spodoptera frugiperda* insect cell culture *Sf21* and a derivative of *Autografa californica* nuclear polyhedrosis virus "BACULOGOLD" (Pharmingen, San Diego, CA) were used for expression of HGV polypeptides. Established protocols were used for insect cell cultivation and for generation of recombinant baculoviruses by co-transfection of baculovirus plasmid transfer vectors with linearized baculovirus DNA (King, 1992). Conventional techniques were used for construction of baculovirus plasmid transfer vectors (Maniatis, *et al.;* Sambrook, *et al.*).

The baculovirus transfer vector pAcYM1 (King, *et al.,* 1992) was modified by ligating a double-stranded oligonucleotide coding for a Histidine hexamer into the vector's *Bam*HI cloning site (vector designated pAcYMIH). A stop codon (TAA) was placed after the Histidine hexamer sequence. This provides a histidine hexamer on the carboxy-termini of expressed proteins. The *Bam*HI cloning site of the pAcYMI parent vector remained intact in the pAcYMIH and could be used for cloning various genes in-frame with the Histidine hexamer. The histidine hexamer provides a method of rapid and efficient purification of the expressed protein (Janknecht, *et al.,* 1991).

A second baculovirus transfer vector, pVT-Bac, was also modified in a similar manner to provide a histidine hexamer on the carboxy-termini of expressed proteins. pVT-Bac like the pAcYMI vector contains a strong late polyhedrin promoter. In addition, pVT-Bac also provides a strong insect translocation signal sequence to ensure efficient translocation of the expressed proteins (Tessier, *et al*., 1991). The pVT-Bac vector was modified by ligating a double-stranded oligonucleotide coding for a histidine hexamer into the vector's BamHI cloning site (yielding the pVT-BacH vector). The BamHI cloning site of the pVT-Bac parent vector remains intact in the obtained pVT-BacH vector and can be used for cloning genes in-frame with the insect leader sequence and the histidine hexamer sequence.

DNA fragments coding for various HGV genes were obtained by reverse transcription PCR. Regions of the HGV genome were selected according to predicted cleavage sites (Bazan, *et al.,* 1989; Chambers, *et al.,* 1990b; Grakoui, *et al*., 1993; Kyte and Doolittle, 1982). The following primer pairs were used in RT-PCR amplification reactions using PNF 2161 source nucleic acid: E1, SEQ ID NO:242, SEQ ID NO:243; E2B (HGV signal sequence), SEQ ID NO:244, SEQ ID NO:245; E2C (insect signal sequence), SEQ ID NO:246, SEQ ID NO:247; NS2a, SEQ ID NO:248, SEQ ID NO:249; NS2b, SEQ ID NO:250, SEQ ID NO:251; NS3, SEQ ID NO:252, SEQ ID NO:253; NS4a, SEQ ID NO:254, SEQ ID NO:255; NS4b, SEQ ID NO:256, SEQ ID NO:257; NS5a, SEQ ID NO:258, SEQ ID NO:259; NS5b, SEQ ID NO:260, SEQ ID NO:261; and E1-E2-NS2a, SEQ ID NO:262, SEQ ID NO:263.

Amplified DNA fragments were digested with *Bam*HI or *Bgl*II endonucleases and cloned into *Bam*HI cut pAcYMI, pAcYMIH, pVT-Bac or pVT-BacH vectors. Sequences coding for the E1 and E2 carboxy-terminal anchors as well as a hydrophobic sequence at the carboxy-terminus of NS5b were deleted in order to facilitate subsequent protein purification.

The recombinant baculovirus plasmid transfer vectors containing HGV sequences were co-transfected with linearized baculovirus DNA and the recombinant viruses were selected as white foci in presence of X-gal (King, *et al.,* 1992). Recombinant viruses were twice plaque-purified and propagated. Monolayers of Sf21 cells were infected with the recombinant baculoviruses at the multiplicity 5 p.f.u. per cell and incubated at 27°C for 60h. The cells were washed with PBS and lysed in TNN buffer (50 mM Tris-HCl pH 8.0, 150 mM NaCl, 0.5% "NONIDET-P40"). Inclusion bodies were isolated by spinning the cell samples at 14k for 5 minutes. The inclusion bodies were resuspended in protein dissociation buffer (10% 2-mercaptoethanol, 10% SDS, 25% glycerol, 10mM Tris-HCl pH 6.8, 0.02% Bromphenol blue) and incubated at 100°C for 10 minutes.

The protein expression patterns analyzed by SDS-PAGE. Proteins were separated by 0.1% SDS-18% PAGE and stained with Coomassie brilliant blue. The majority of the HGV proteins were expressed to a high level and could be easily detected on the Coomassie blue stained gels. NS5a and NS2a polypeptides were detected by ³⁵S methionine protein labeling (King, *et al.,* 1992).

HGV E2 protein glycosylation was examined as follows. Sf21 cells were infected with recombinant baculoviruses and processed as described above. Proteins were separated by 0.1% SDS-12% PAGE, electroblotted onto an "IMMOBILON-P" membrane (Millipore, Bedford, MA) and reacted with *Galanthus nivalis* agglutinin (Boehringer Mannheim DIG Glycan differentiation kit) which is specific for mannose residues. The HGV E2 protein that was expressed with its own signal sequence was extensively glycosylated, indicating that the predicted E2 signal sequence can function as such.

### 2. Immunofluorescence Assay Analysis.

SF21 insect cells were infected with the baculovirus-HGV constructs described above. Cells were harvested, spun at 1.5K rpm for 3 minutes, washed in 1X PBS, and spun again.

For Immunofluorescence Assay (IFA) (King, *et al.,* 1992) the cells were resuspended in PBS and layered into the wells of glass slides such that the cells formed a sub-confluent layer in the wells of the slides. The slides were air-dried. The cells fixed with pre-chilled - 70°C acetone for 10 minutes and rehydrated with PBS for 5 minutes. The excess PBS was removed by blotting. The fixed cells were treated for one hour with the following "Blocking" buffer: 40mM Tris-HCl pH 7.5, 3% goat serum, 1% BSA, 1% nonfat milk and 0.1% gelatin.

Primary antibody was then added to the fixed cells. Primary antibodies included a series of human HGV-positive sera and a positive control monoclonal antibody. Before use, the sera were pre-absorbed for non-specific proteins using insect cell lysate. Pre-absorption was carried out overnight at 4°C. Uninfected SF21 cells were used as a negative control. After addition of a selected primary antibody (sera), the slides were incubated for 2 hours then washed several times with PBS and excess buffer removed. A secondary antibody conjugated with fluorescein (0.5 *µ*g/ml conc.) was then added to the samples on the slides. The incubation time and temperature for the secondary antibody was the same as for the primary antibody. After incubation, slides were washed in PBS and mounted with a cover slip. The fluorescence of the cells was then determined using a fluorescence microscope.

The results of this analysis were as follows. Cells expressing HGV antigen E1-E2-NS2a were immunoreactive with 4/10 HGV-positive sera and weakly immunoreactive with an additional 2/10 sera. Cells expressing E1 were weakly immunoreactive with 1/10 sera. Cells expressing E2 were immunoreactive with 3/10 sera and weakly immunoreactive with 1/10 sera. None of the cells carrying HGV antigens were immunoreactive with supernormal control sera.

### 3. Western Blot Analyses of HGV proteins expressed in baculo vector

Western blot analyses of the HGV proteins expressed in recombinant baculo virus infected Sf21 insect cells were also performed. Inclusion bodies were prepared as described above and subjected to Western blot analysis. Western blot analysis was performed using pre-absorbed sera. The results of the analyses demonstrated that E2 proteins (one variant having the endogenous HGV signal sequence, E2B, and a second variant carrying an insect signal sequence, E2C) were specifically immunoreactive with HGV(+) serum 3831781.

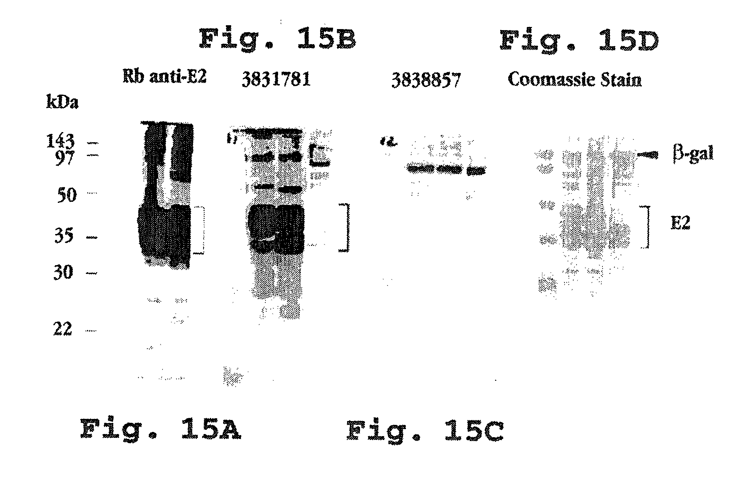

Figures 15A to 15D show the results of a series of Western blot experiments examining the reactivity of baculo antigens E2B and E2C. The lanes in each blot of Figures 15A to 15D were as follows: Lane 1, pre-stained molecular weight marker (Bio-Rad); Lane 2, E2B lysate; Lane 3, E2C lysate; Lane 4, β-galactosidase lysate. Each blot was incubated with a human or rabbit serum : Figure 15A, rabbit anti-E2 antibody; Figure 15B, 3831781 (an HGV-PCR-positive serum); Figure 15C, 3838857 (an HGV-negative serum). Figure 15D a Coomassie Stain. The serum or rabbit antibody that was used is indicated above each blot. Human sera were diluted 1:100 and rabbit serum was diluted 1:1000.

Further, HGV antigen NS2b protein expressed in insect cells was immunoreactive with J21689. These results are consistent with the results obtained with pET expressed HGV proteins.

### C. Expression of Larger Antigens in Vaccinia.

### 1. Cloning and Expression.

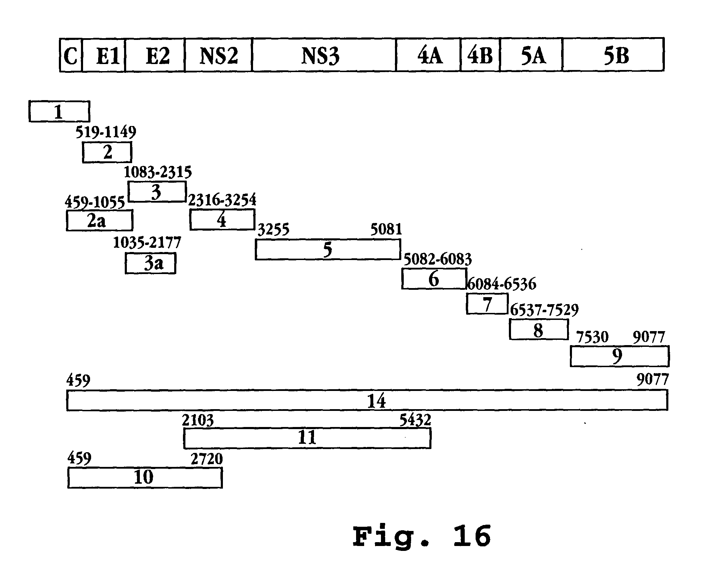

Various regions of HGV genome were integrated into vaccinia virus genome for expression. An exemplary HGV polypeptide expression strategy is given in Figure 16. HGV (PNF 2161 variant) proteins expressed in vaccinia virus are schematically illustrated in Figure 16. Full length polyprotein is drawn (not to scale) by an open box indicating regions of predicted proteins: C=highly basic protein, 4A=NS4A, 4B=NS4B, 5a=NS5A, 5b=NS5B. The individual boxes with nucleotide locations (below the polyprotein) represents exemplary regions of HGV for expression in vaccinia virus. The number in the box stands for recombinant virus nomenclature. Virus #1 was derived from the highly basic protein region of HGV Stain T55806 (SEQ ID NO:185).

Two sets of recombinant viruses were generated. The first set contained HGV sequences that correspond to individual protein domains based on sequence analysis of HGV cDNA (Figure 16, fragments #1 to #9). The second set contained HGV sequences that spanned multiple protein domains, up to full length of HGV genome (Figure 16, #10, #11, #14).

The various regions of the HGV genome were cloned into the multicloning site of the vaccinia expression vector. A recombinant vaccinia virus expression system was used that included bacterial phage T7 system and *E. coli lac* repressor for high level inducible expression (Fuerst, 1986; Elroy-Stein, 1989; Alexander, 1992; Moss, *et al.*). Therefore, recombinant protein is expressed only in the presence of an inducer, such as isopropyl beta-D-thiogalactoside (IPTG). Both direct cloning and PCR were used for plasmid construction. In the latter, restriction endonuclease sites suitable for cloning into the vaccinia vector were incorporated into primers used to amplify individual DNA fragment.

A polyhistidine tag was also incorporated into every clone covering individual domains of HGV for use in purifying the expressed proteins. HGV-PCR amplification products were digested with the appropriate restriction enzymes and ligated into the vaccinia vector. Target HGV cDNA fragments were integrated into vaccinia virus genome through homologous recombination and drug (mycophenolic acid) selection (Falkner, 1988, Earl, 1991). Recombinant virus were plaque purified 4 times before a viral stock was generated.

The length of each clone in nucleotides is indicated in Figure 16. The group of smaller clones (#1 to #9) are useful for HGV epitope mapping. The larger clones (*e*.*g*., #10, #11 and #14) are also useful for mapping the HGV polyprotein cleavage sites experimentally. In addition to the clones shown in Figure 16, additional recombinant viruses covering multiple domains from NS3 to NS5b can be constructed.

Expression plasmids were transfected into mammalian cells which had been infected with a parent vaccinia virus. CV-1 and BS-C-1 cells were maintained in Minimum Essential Medium (MEM) supplemented with 10% fetal bovine serum. The cells were used for transfection (CV-1) and recombinant virus selection and propagation (BS-C-1).

### 2. Evaluation of recombinant protein expression.

BS-C-1 cells were infected with recombinant virus in the presence or absence of IPTG for 7 hours after which cells were labeled with ³⁵S-methionine for another one hour (Zhang, 1991). Briefly, 1 × 10⁶ BS-C-1 cells were infected with recombinant virus at a multiplicity of infection (MOI) of 10 plaque forming unit (PFU) per cell for 1 h and then supplemented with medium in the presence or absence of 5 mM IPTG for another 6 h. Cells were pulse-labeled with 600 ul Methionine-free medium supplemented with 2.5% dialyzed fetal bovine serum plus 60 uCi 355-methionine ("TRAN ³⁵S-LABEL", ICN, Costa Mesa, CA) in the presence or absence of 5 mM IPTG for another 60 min. Labeled cells were then lysed on ice for 10 min in the presence of 100 mM Tris pH8.0, 150 mM NaCl, and 1% "TRITON X-100." Nuclei were spun down and supernatant was collected for analysis.

Cell lysate was analyzed by SDS-polyacrylamide gel electrophoresis (Fling, 1986; Schagger, 1987). Gels were fixed with 50% methanol and 10% acetic acid before they were treated with a fluorograph solution "AMPLIFY" (Amersham, Arlington Heights, IL). Gels were dried and exposed to X-ray film.

Using this method, expression of HGV polypeptides by viruses containing inserts #4 to #11, and #14 (Figure 16) has been confirmed. Expression of polypeptides corresponding to other regions is confirmed in a similar manner. For example, in a NS5a construct, upon induction by IPTG, a unique polypeptide was produced that migrated just below a 46 KDa protein standard. This protein was not seen in the infection in the absence of IPTG induction, establishing the identity of the protein as NS5a recombinant protein.

Further, limited immunoprecipitations using HGV region-specific antisera (for example, rabbit anti-sera raised against an isolated HGV polypeptide from the region of interest) against ³⁵S-Met labeled cell lysate from individual virus infections was carried out to evaluate the protein expression from recombinant viruses. For example, expression of NS2, NS3, NS4B, NS5A and NS5B has been confirmed. An alternative method, to evaluate recombinant protein expression is to perform western blot analysis with HGV-region-specific antisera.

When the full length HGV polyprotein was expressed in #14 virus (Figure 16), processed products of NS2, NS3 and NS5A were detected using immunoprecipitation with HGV region-specific antisera, demonstrating the usefulness of the full length HGV clone to evaluate polyprotein processing.

Using an expression strategy similar to that shown in Figure 16, candidate HGV proteins/antigens can be expressed in yeast or CHO cells. Yeast offers high level of expression, economical operation, and ease of scaling up for commercial production. CHO cell lines allow secretion of the recombinant proteins into growth media for large scale protein production and purification useful, for example, for vaccine development.

### EXAMPLE 17

### HGV Encoded Highly Basic Proteins

### A. DETERMINATION OF THE METHIONINE USED FOR INITIATION IN THE TRANSLATION OF HGV FROM PNF AND T55806.

The methionine located at nucleotide (nt) 459 (relative to SEQ ID NO:14) in the HGV-PNF 2161 variant is in-frame with the polyprotein. The "capsid" region appears to be 32 amino acid long. In other HGV isolates, such as T55806, this region is longer (*e.g.*, about 83 amino acids). The methionine located at nt 349 (relative to SEQ ID NO:14) in HGV-PNF 2161 variant is not in-frame with the polyprotein sequence, but a methionine at the same position in HGV-T55806 variant is in frame with the polyprotein. To see if there is a read-through or a ribosomal frame shift at this position in HGV-PNF 2161, the following experiments were carried out.

Constructs were made containing (i) HGV genomic sequences having all the MET codons upstream of the HGV E1 region (*e.g.*, in HGV-PNF 2161 there are six such METs and five such in T55806), (ii) two different 3' ends for each construct to allow determination of whether a ribosome shift of read-through occurs. For a given genomic DNA, if both translated products are the same size, that suggests they are terminated prematurely at the stop codon. On the other hand, if read-through or frameshift occurs two products that differ by 55 amino acids are expected.

A total of 21 constructs containing sequences from variants HGV-PNF 2161 and HGV-T55806 were subcloned in a pGEX vector and corresponding proteins expressed in *E. coli.* Sizes of the resulting translation products were determined by both Coomassie stained gels and Westerns that were blotted with monoclonal anti-GST antibody. Induced and un-induced samples were prepared for each construct.

The results demonstrated that the size of the protein products corresponded to that expected by translation initiating at the first MET in-frame with the polyprotein. There was no evidence of frame-shifting or read-through.

### B. ALTERNATIVE ENCODED HIGHLY BASIC PROTEINS.

The method of Fickett (1982) was used to scan the genomic sequences HGV-PNF 2161 and HGV-JC for sequences that potentially encode proteins (i) alternative to the previously described polyprotein, (ii) showing conservation between HGV-PNF 2161 and HGV-JC, and (iii) having predicted isoelectric points in excess of pH 10. Two such potential proteins were identified.

The first protein is encoded by residues 628 through 882 (relative to SEQ ID NO:14) in HGV-PNF 2161 and by residues 556 through 810 (relative to SEQ ID NO:182) in HGV-JC. This protein is 85 amino acids long, is greater than 75% homologous between HFV94-1 and JC9B, and has a predicted pI of 11.6-12.3.

The second protein is encoded by residues 6844 through 7125 in HGV-PNF 2161 (relative to SEQ ID NO:14) and by 6772 to 7053 in HGV-JC (relative to SEQ ID NO:182). This protein is 94 amino acids long, is greater than 88% homologous between HGV-PNF 2161 and HGV-JC, and has a predicted pI of 12.4-12.7.

These exemplary two proteins represent potentially expressed highly basic proteins of HGV.

### EXAMPLE 18

### Cloning Further HGV Isolates and Design of Diagnostic Primers

### A. CONSTRUCTION OF A CDNA CLONE OF HGV-PNF 2161.

A cDNA clone of the nearly full-length HGV genome from PNF 2161 was constructed by cloning three overlapping PCR products into the plasmid vector pGEM3Z (Promega, Madison, WI). The PCR products used in this construction were obtained by reverse transcription with "SUPERSCRIPT II" (Gibco/BRL, Gaithersburg, MD) followed by PCR using reaction conditions that allowed for the amplification of long target sequences ("rTth-XL" polymerase and "XL PCR BUFFERS", Applied Biosystems, Foster City, CA). The rTth enzyme used for these "long-range" PCR reactions has proof-reading activity (i.e. 3' to 5' exonuclease activity) that corrects mis-incorporated nucleotides, thus providing for high fidelity PCR.

The three products used to construct the HGV genome included (i) an internal 6.7 kb product (nt 2101 to 8834 of SEQ ID NO:14) amplified using the primers GV75-36FE (SEQ ID NO:228) and GV75-7064RLE (SEQ ID NO:229), (ii) a 2.8 kb 5'-end product (nt 38 to 2899 of SEQ ID NO:14) amplified using 28F (SEQ ID NO:230) and FV94-2864R (SEQ ID NO:231), and (iii) a 2.9 kb 3'-end product (nt 6449 to 9366 of SEQ ID NO:14) amplified using FV94-6439F (SEQ ID NO:232) and FV94-9331R (SEQ ID NO:233).

Initially, the 6.7 kb internal fragment was cloned into the "TA-vector" pCRII to create the clone HGV7. Subsequently, a 6.1 kb *Kpn*I/*Eco*RI fragment was removed from HGV7 and combined with the *Kpn*I/*Xba*I digested 2.8 kb 5'-end product (the primer 28F contains an artificial XbaI site) and cloned into *Xba*I/*Eco*RI digested pGEM3Z. This 8.8 kb clone, which lacks about 0.6 kb of the 3' portion of the HGV genome, was designated HGV-KEX-2. To construct the nearly full-length HGV genome, the 3'-end HGV product was digested with *Nhe*I and *Eco*RI (the primer FV94-9331R contains an artificial *Eco*RI site) and cloned into *Nhe*I/*Eco*RI digested HGV-KEX-2 plasmid creating a cloned HGV-PNF2161 sequence of 9329 nt (nt 38 to 9366 of SEQ ID NO:14) that is designated 3Z-HGV94-6. The complete sequence of 3Z-HGV94-6 is presented as SEQ ID NO:234.

The clone 3Z-HGV94-6 may be used to generate in vitro-transcribed full-length HGV RNA or portions thereof (*e.g.*, using SP6 polymerase). The RNA molecules can be used to transfect human cell lines. This approach could be used to map the various regions of the viral genome, study its replication, and understand the mechanisms of HGV pathogenicity in human cells (Rice, *et al.,* 1989; Sumiyoshi, *et al.,* 1992; Yoo, et al., 1995).

### B. CLONING THE JC VARIANT.

One milliliter of JC serum was spun at 40,000 rpms (Beckman, Spinco Rotor 70.1Ti) for 2 hours. The resulting pellet was extracted using "TRIREAGENT" (MRC, Cincinnati, OH), resulting in the formation of 3 phases. The upper phase contained RNA only. This phase was taken and RNA recovered by ethanol precipitation.

HGV cDNA molecules were generated from the JC sample by two methods. The first method was amplification (RT-PCR) of the JC nucleic acid sample using specific and nested primers. The primer sequences were based on the HGV sequence obtained from PNF 2161 serum. The criteria used to select the primers were (i) regions having a high G/C content, and (ii) no repetitious sequences.

The second method used to generate HGV cDNA molecules was amplification using HGV (PNF 2161) specific primers followed by identification of HGV specific sequences with ³²P-labelled oligonucleotide probes. Such DNA hybridizations were carried out essentially as described by Sambrook, *et al.* (1989). The PCR derived clones were either (i) cloned into the "TA" vector (Invitrogen, San Diego, CA) and sequenced with vector primers (TAR and TAF), or (ii) sequenced directly after PCR amplification. Both the probe and primer sequences were based on the HGV variant obtained from the PNF 2161 serum.

These two approaches yielded multiply-overlapping HGV fragments from the JC serum. Each of these fragments were cloned and sequenced. The sequences were aligned to obtain the HGV (JC-variant) consensus sequence presented as SEQ ID NO:182 (polypeptide sequence, SEQ ID NO:183). The sequence of each region of the HGV (JC-variant) virus was based on a consensus from at least three different, overlapping, independent clones.

### C. OTHER HGV VARIANTS.

In addition to the HGV PNF 2161-variant and JC-variant sequences, three partial HGV isolates have been obtained from the sera BG34, T55806 and EB20 by methods similar to those described above. The partial sequences of these isolates are presented as SEQ ID NO:176 (BG34 nucleic acid), SEQ ID NO:177 (BG34 polypeptide), SEQ ID NO:178 (T55806 nucleic acid), SEQ ID NO:179 (T55806 polypeptide), SEQ ID NO:180 (EB20-2 nucleic acid) and SEQ ID NO:181 (EB20-2 polypeptide).

### D. ALTERNATIVE PRIMERS FOR DIAGNOSTIC PCR.

PCR primers and corresponding assay development may be derived from regions of the HGV genome(s) typically based on the analysis of conserved regions. Based on comparisons of the HGV-JC variant and the HGV-PNF 2161 variant, the 5' untranslated region of HGV was selected as one such region for development of a further PCR-based diagnostic test for the detection of HGV isolates. Two exemplary primers are FV-94-22F (SEQ ID NO:124) and FV94-724R (SEQ ID NO:125). These primers amplify an approximately 728 bp fragment of the HGV genome.

Sequence analysis was performed on amplification products from reactions employing these two primers for 36 isolates of HGV (including PNF 2161 and JC, see Table 26). An approximately 400 bp region (nt 69 to 469 of SEQ ID NO:14) of the approximately 728 bp amplification product was used for multiple sequence alignments (Table 26) and further determination of conserved regions (see below).

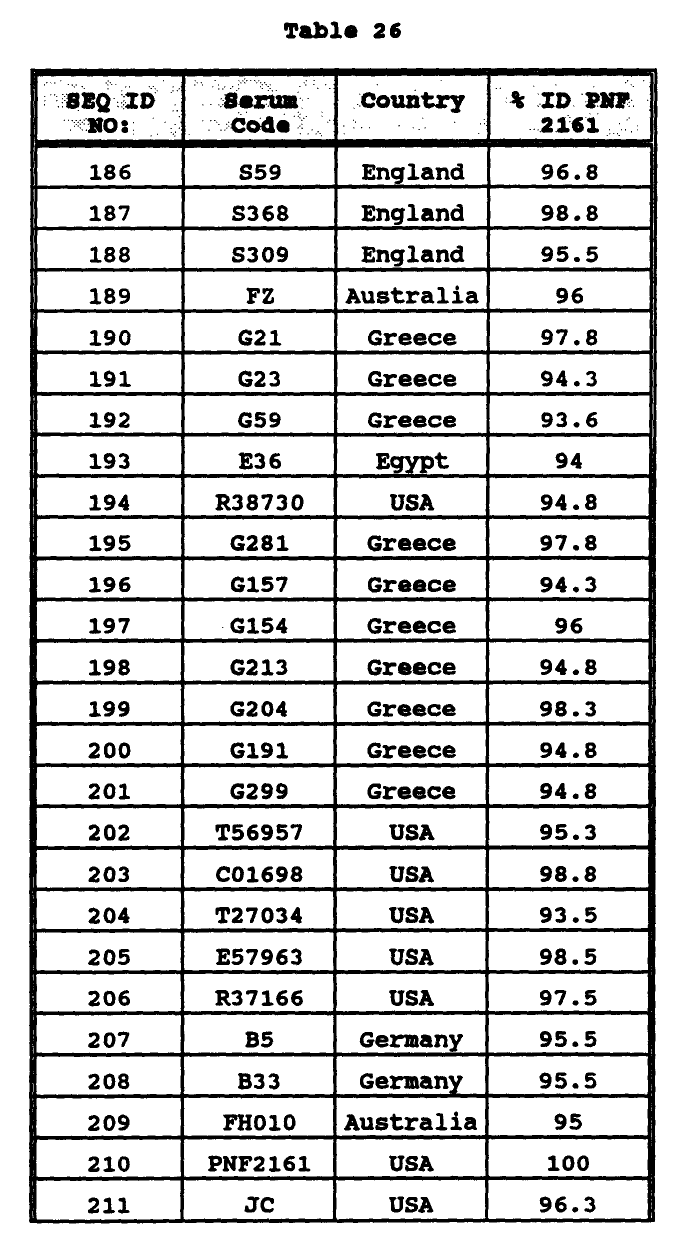

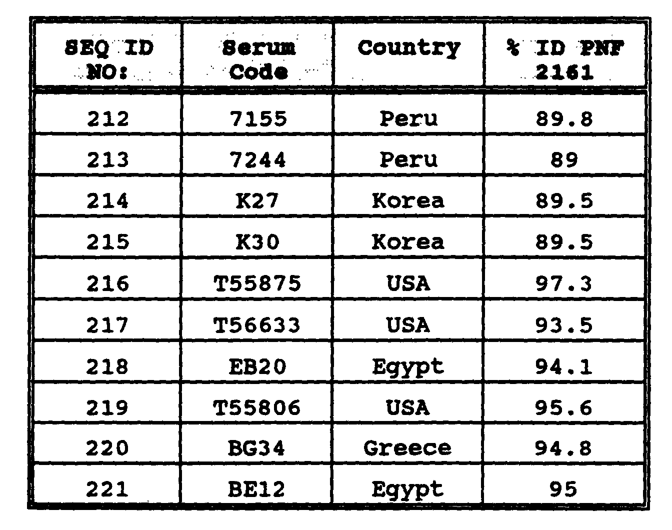

The development of an amplification-based (*e.g.*, PCR) or probe-based method/assay for the detection of HGV isolates in samples involves the selection of appropriate primer/probe sequences. Two criteria for such an assay are low copy sensitivity and specificity for HGV sequences. Alignments of sequences (such as just described) can help guide primer/probe selection and design.

Several criteria for selecting primers are as follows: (i) forward and reverse primers of a pair should not be significantly complementary in sequence, and (ii) primers should not have significant self complementarity or the potential to form secondary structures. These precautions minimize the potential for generation of primer dimers or oligomers.

Primers may optimally be designed from sequence regions showing no variation among different isolates but may also be designed from regions of less homology by incorporating mixed base synthesis or neutral bases, such as inosine, at those positions to account for known isolate divergence. The following two groups of primers are examples of primers may be employed in development of a PCR-based assay for detection of HGV genomes: forward primers SEQ ID NO:222, SEQ ID NO:223 and SEQ ID NO:224; and reverse primers SEQ ID NO:225, SEQ ID NO:226 and SEQ ID NO:227.

Various combinations of primers, may be employed in development of an HGV diagnostic assay. Optimal combinations of primers are experimentally determined and typically address considerations for assay sensitivity and specificity. Such considerations include the following: (i) a PCR product length of 100-300 bp for efficient amplification and ease of product detection; (ii) an ability to reproducibly detect at least 10 copies of target HGV, and (iii) an ability to reproducibly detect a majority of HGV variants.

In addition, probe sequences may be similarly designed with mixed base or neutral base syntheses and/or may be used at reduced stringency so as to detect a majority of HGV variants.

## Claims

1. Non-A Non-B Non-C Non-D Non-E Hepatitis Virus (HGV) in substantially isolated form, where said HGV is **characterized** as follows: (i) is transmissible in primates, (ii) is serologically distinct from hepatitis A virus (HAV), hepatitis B virus (HBV), hepatitis C virus (HCV), hepatitis D virus, and hepatitis E virus (HEV), (iii) is a member of the virus family Flaviviridae, and (iv) contains polynucleotides having at least 55% sequence homology to a polynucleotide having the sequence presented as SEQ ID NO:37, or SEQ ID NO:19, or their complements.

2. A Non-A Non-B Non-C Non-D Non-E Hepatitis Virus (HGV) polypeptide in substantially isolated form, where said HGV: (i) has the characteristics of the HGV virus of claim 1, and (ii) is further **characterized by** polypeptides whose amino acid sequences have at least 40% sequence homology to amino acid sequences selected from the 190 amino acid sequence of SEQ ID NO:38, and the 67 amino acid sequence of SEQ ID NO:20.

3. The polypeptide of claim 2, comprising an antigen which is specifically immunoreactive with at least one anti-HGV antibody, as evidenced by the ability of the antigen to immunoreact specifically with a body fluid or tissue sample from an HGV-positive subject.

4. The polypeptide of claim 2, which is a recombinant fusion polypeptide composed of an HGV polypeptide and a second polypeptide, where said second polypeptide is selected from the group consisting of β-galactosidase proteins, glutathione-S-transferase proteins, and particle-forming proteins.

5. The polypeptide of claim 2, comprising a sequence of at least 15 contiguous amino acids encoded by an HGV genome, cDNA or complements thereof, wherein said amino acid sequence is selected from the group consisting of (i) the 190 amino acid sequence of SEQ ID NO:38, or fragments thereof, (ii) the 67 amino acid sequence of SEQ ID NO:20, or fragments thereof, and (iii) an amino acid sequence encoded within the PNF 2161 cDNA source lambda gtll library.

6. A diagnostic kit for use in screening a body fluid or tissue sample containing antibodies specific against the Non-A Non-B Non-C Non-D Non-E Hepatitis Virus (HGV) of claim 1, comprising
the polypeptide antigen of claim 3, and
means for detecting an immunological complex formed by specific immunoreaction of said antigen with antibodies in said sample.

7. The diagnostic kit of claim 6, for use in screening serum.

8. A diagnostic kit for use in screening a body fluid or tissue sample containing Non-A Non-B Non-C Non-D Non-E Hepatitis Virus (HGV) antigens, comprising
a substantially isolated antibody specifically immunoreactive with the Non-A Non-B Non-C Non-D Non-E Hepatitis Virus (HGV) polypeptide antigen of claim 3, and
means for detecting the binding of said polypeptide antigen to said antibody.

9. A method of detecting Non-A Non-B Non-C Non-D Non-E Hepatitis Virus (HGV) infection in a test subject, comprising
reacting a body fluid or tissue sample from a test subject with a substantially isolated HGV specific antibody of the kit of claim 8, and
examining the antibody for the presence of bound antigen.

10. A monoclonal antibody specifically immunoreactive with the Non-A Non-B Non-C Non-D Non-E Hepatitis Virus (HGV) antigen of claim 3.

11. A substantially isolated preparation of polyclonal antibodies specifically immunoreactive with the Non-A Non-B Non-C Non-D Non-E Hepatitis Virus (HGV) antigen of claim 3.

12. A method of screening a body fluid or tissue sample containing antibodies specific against the Non-A Non-B Non-C Non-D Non-E Hepatitis Virus (HGV) of claim 1, comprising
contacting the sample with the polypeptide antigen of claim 3, and
detecting an immunological complex formed by specific immunoreaction of said antigen with antibodies in said sample.

13. Use of the HGV polypeptide of claim 2, comprising an antigen containing an epitope which is specifically immunoreactive with at least one anti-HGV antibody for the production of an immune response for the preparation of a composition for producing antibodies to the Non-A Non-B Non-C Non-D Non-E Hepatitis Virus (HGV) of claim 1.

14. A mosaic polypeptide, comprising
at least two different antigens of claim 3, where said mosaic polypeptide lacks amino acids normally intervening between said antigens in a native HGV polypeptide.

15. A Non-A Non-B Non-C Non-D Non-E Hepatitis Virus (HGV) vaccine composition, comprising
a substantially isolated HGV polypeptide of claim 3, present in a pharmacologically effective dose in a pharmaceutically acceptable carrier.

16. A Non-A Non-B Non-C Non-D Non-E Hepatitis Virus (HGV) polynucleotide in substantially isolated form, where said HGV has the characteristics of the HGV virus of claim 1.

17. The polynucleotide of claim 16, which has at least 55% sequence homology to a polynucleotide having the sequence presented as SEQ ID NO:37, or SEQ ID NO:19, or their complements.

18. The polynucleotide of claim 16, useful for PCR detection of a Non-A Non-B Non-C Non-D Non-E hepatitis Virus (HGV).

19. A method of detecting Non-A Non-B Non-C Non-D Non-E Hepatitis Virus (HGV) nucleic acid in a test subject, comprising:
obtaining a nucleic acid-containing sample from the subject,
combining the sample containing nucleic acid and a probe composed of the polynucleotide of claim 16, under suitable hybridization conditions, and
detecting the presence of HGV nucleic acid/probe complexes, formed by hybridization of the HGV nucleic acid with said probe.

20. The method of claim 19, wherein said detecting includes using HGV nucleic acid specific probes, where the two probes define an internal region of the HGV nucleic acid and each probe has one strand containing a 3'-end internal to the region, converting the nucleic acid/probe hybridization complexes to double-strand probe-containing fragments by primer extension reactions,
amplifying the number of probe-containing fragments by successively repeating the steps of (i) denaturing the double-strand fragments to produce single-strand fragments, (ii) hybridizing the single strands with the probes to form strand/probe complexes, (iii) generating double-strand fragments from the strand/probe complexes in the presence of an enzyme and all four deoxyribonucleotides, and (iv) repeating steps (i) to (iii) until a desired degree of amplification has been achieved,
identifying the amplification products.

21. A kit for analyzing samples for the presence of polynucleotides derived from the Non-A Non-B Non-C Non-D Non-E Hepatitis Virus (HGV) of claim 1, comprising
at least one polynucleotide probe containing a nucleotide sequence that will specifically hybridize with the HGV polynucleotide of claim 16, and
a suitable container.

22. A cloning vector capable of expressing, under suitable conditions, an open reading frame (ORF) of cDNA derived from a Non-A Non-B Non-C Non-D ⁻Non-E Hepatitis Virus (HGV) genome, or complements thereof, where said virus has the characteristics of claim 1, and the ORF is operably linked to a control sequence compatible with a desired host.

23. A method of producing a Non-A Non-B Non-C Non-D Non-E Hepatitis Virus (HGV) polypeptide, comprising
culturing a cell transformed with a vector of claim 22, under conditions resulting in the expression of the open reading frame (ORF) sequence.

## Patentansprüche

1. Nicht-A-Nicht-B-Nicht-C-Nicht-D-Nicht-E-Hepatitis-Virus (HGV) in im Wesentlichen isolierter Form, wobei das HGV wie folgt **gekennzeichnet** ist:
(i) übertragbar in Primaten;
(ii) serologisch unterscheidbar von Hepatitis-A-Virus (HAV), Hepatitis-B-Virus (HBV), Hepatitis-C-Virus (HCV), Hepatitis-D-Virus und Hepatitis-E-Virus (HEV);
(iii) Mitglied der Virusfamilie Flaviviridae; und
(iv) enthaltend Polynucleotide mit mindestens 55 % Sequenzhomologie zu einem Polynucleotid mit der Sequenz wie in SEQ ID NO: 37 oder in SEQ ID NO: 19 dargestellt, oder deren Komplemente.

2. Nicht-A-Nicht-B-Nicht-C-Nicht-D-Nicht-E-Hepatitis-Virus (HGV)-Polypeptid in im Wesentlichen isolierter Form, wobei das HGV:
(i) die Eigenschaften des HGV-Virus nach Anspruch 1 besitzt, und
(ii) weiter durch Polypeptide **gekennzeichnet** ist, deren Aminosäuresequenzen mindestens 40 % Sequenzhomologie mit Aminosäuresequenzen aufweisen, ausgewählt aus der 190 Aminosäuren umfassenden Sequenz mit der SEQ ID NO: 38 und der 67 Aminosäuren umfassenden Sequenz mit der SEQ ID NO: 20.

3. Polypeptid nach Anspruch 2, umfassend ein Antigen, welches spezifisch immunreaktiv mit mindestens einem Anti-HGV-Antikörper ist, bewiesen durch die Fähigkeit des Antigens eine spezifische Immunreaktivität mit einer Körperflüssigkeits- oder Gewebeprobe von einem HGV-positiven Individuum zu zeigen.

4. Polypeptid nach Anspruch 2, welches ein rekombinantes Fusionspolypeptid ist, zusammengesetzt aus einem HGV-Polypeptid und einem zweiten Polypeptid, wobei dieses zweite Polypeptid ausgewählt ist aus der Gruppe bestehend aus β-Galactosidase-Proteinen, Glutathion-S-Transferase-Proteinen und partikelbildenden Proteinen.

5. Polypeptid nach Anspruch 2, umfassend eine Sequenz von mindestens 15 angrenzenden Aminosäuren, codiert von einem HGV-Genom, cDNA oder deren Komplementen, wobei die Aminosäuresequenz ausgewählt ist aus der Gruppe bestehend aus:
(i) der 190 Aminosäuren umfassenden Sequenz von SEQ ID NO: 38 oder Fragmenten davon;
(ii) der 67 Aminosäuren umfassenden Sequenz von SEQ ID NO: 20 oder Fragmenten davon; und
(iii) einer Aminosäuresequenz, die codiert wird innerhalb der aus PNF 2161 isolierten cDNA der Lambda gt11-Genbank.

6. Diagnostischer Kit zur Verwendung bei der Durchmusterung von Körperflüssigkeits- oder Gewebeproben, enthaltend Antikörper spezifisch gegen das Nicht-A-Nicht-B-Nicht-C-Nicht-D-Nicht-E-Hepatitis-Virus (HGV) nach Anspruch 1, umfassend
das Polypeptid-Antigen nach Anspruch 3, und
Mittel zum Nachweis eines Immunkomplexes, der durch die spezifische Immunreaktion des Antigens mit Antikörpern in der Probe erzeugt wird.

7. Diagnostischer Kit nach Anspruch 6, zur Verwendung bei der Durchmusterung von Serum.

8. Diagnostischer Kit zur Verwendung bei der Durchmusterung von Körperflüssigkeits- oder Gewebeproben, die Nicht-A-Nicht-B-Nicht-C-Nicht-D-Nicht-E-Hepatitis-Virus (HGV)-Antigene enthalten, umfassend
einen im Wesentlichen isolierten Antikörper, der spezifisch immunreaktiv mit dem Nicht-A-Nicht-B-Nicht-C-Nicht-D-Nicht-E-Hepatitis-Virus (HGV)-Polypeptid-Antigen nach Anspruch 3 ist, und
Mittel zum Nachweis der Bindung des Polypeptid-Antigens an den Antikörper.

9. Verfahren zum Nachweis einer Nicht-A-Nicht-B-Nicht-C-Nicht-D-Nicht-E-Hepatitis-Virus (HGV)-Infektion in einem Testindividuum, umfassend
die Reaktion einer Körperflüssigkeits- oder Gewebeprobe eines Testindividuums mit einem im Wesentlichen isolierten, HGV-spezifischen Antikörper des Kits nach Anspruch 8, und
Überprüfung des Antikörpers auf die Gegenwart von gebundenem Antigen.

10. Monoclonaler Antikörper, der spezifisch immunreaktiv mit dem Nicht-A-Nicht-B-Nicht-C-Nicht-D-Nicht-E-Hepatitis-Virus (HGV)-Antigen nach Anspruch 3 ist.

11. Im Wesentlichen isoliertes Präparat von polyclonalen Antikörpern, die spezifisch immunreaktiv mit dem Nicht-A-Nicht-B-Nicht-C-Nicht-D-Nicht-E-Hepatitis-Virus (HGV)-Antigen nach Anspruch 3 sind.

12. Verfahren zur Durchmusterung einer Körperflüssigkeits- oder Gewebeprobe, die Antikörper spezifisch gegen das Nicht-A-Nicht-B-Nicht-C-Nicht-D-Nicht-E-Hepatitis-Virus (HGV) nach Anspruch 1 enthält, umfassend
Inkontaktbringen der Probe mit dem Polypeptid-Antigen nach Anspruch 3, und Nachweis eines immunologischen Komplexes, der durch die spezifische Immunreaktion des Antigens mit dem Antikörper in der Probe erzeugt wird.

13. Verwendung des HGV-Polypeptids nach Anspruch 2, umfassend ein Antigen, das ein Epitop enthält, welches spezifisch immunreaktiv mit mindestens einem Anti-HGV-Antikörper ist, für die Einleitung einer Immunantwort für die Herstellung einer Zusammensetzung zur Produktion von Antikörpern gegen das Nicht-A-Nicht-B-Nicht-C-Nicht-D-Nicht-E-Hepatitis-Virus (HGV) nach Anspruch 1.

14. Mosaikpolypeptid, umfassend mindestens zwei unterschiedliche Antigene nach Anspruch 3, wobei dem Mosaikpolypeptid Aminosäuren fehlen, welche üblicherweise zwischen den Antigenen im nativen HGV-Polypeptid vermitteln.

15. Nicht-A-Nicht-B-Nicht-C-Nicht-D-Nicht-E-Hepatitis-Virus (HGV)-lmpfstoffzusammensetzung, umfassend
ein im Wesentlichen isoliertes HGV-Polypeptid nach Anspruch 3, das in einer pharmakologisch wirksamen Dosis in einem pharmakologisch verträglichen Träger vorliegt.

16. Nicht-A-Nicht-B-Nicht-C-Nicht-D-Nicht-E-Hepatitis-Virus (HGV)-Polynucleotid in im Wesentlichen isolierter Form, wobei das HGV die Eigenschaften des HGV-Virus nach Anspruch 1 besitzt.

17. Polynucleotid nach Anspruch 16, welches mindestens 55 % Sequenzhomologie mit einem Polynucleotid hat, welches eine Sequenz besitzt wie in SEQ ID NO: 37 oder SEQ ID NO: 19 dargestellt, oder deren Komplemente.

18. Polynucleotid nach Anspruch 16, geeignet für den PCR-Nachweis eines Nicht-A-Nicht-B-Nicht-C-Nicht-D-Nicht-E-Hepatitis-Virus (HGV).

19. Verfahren zum Nachweis von Nicht-A-Nicht-B-Nicht-C-Nicht-D-Nicht-E-Hepatitis-Virus (HGV)-Nucleinsäure in einem Testindividuum, umfassend:
Bereitstellen einer Nucleinsäure-enthaltenden Probe von dem Individuum,
Kombinieren der Probe, welche die Nucleinsäure enthält, und einer Sonde zusammengesetzt aus dem Polynucleotid nach Anspruch 16 unter geeigneten Hybridisierungsbedingungen, und
Nachweis der Anwesenheit von HGV-Nucleinsäure/Sondenkomplexen, die erzeugt werden durch Hybridisierung der HGV-Nucleinsäure mit der Sonde.

20. Verfahren nach Anspruch 19, wobei der Nachweis die Verwendung von HGV Nucleinsäure-spezifischen Sonden umfaßt, wobei die zwei Sonden einen inneren Bereich der HGV-Nucleinsäure definieren und jede Sonde einen Strang mit einem 3'-Ende innerhalb der Region umfaßt, Umwandlung der Nucleinsäure/Sonde- Hybridisierungsskomplexe in doppelsträngige Sondenenthaltende Fragmente durch Primerverlängerungsreaktionen,
Amplifizieren der Zahl der Sonden-enthaltenden Fragmente durch nacheinander sich wiederholende Schritte von
(i) Denaturieren der Doppelstrangfragmente zur Herstellung von Einzelstrangfragmenten;
(ii) Hybridisieren der Einzelstränge mit den Sonden zur Bildung von Strang/Sondenkomplexen;
(iii) Erzeugen von Doppelstrangfragmenten aus den Strang/Sondenkomplexen in der Gegenwart eines Enzyms und aller vier Desoxyribonudeotide; und
(iv) Wiederholen der Schritte (i) bis (iii), bis der erwünschte Grad der Amplifikation erreicht ist,
Identifizieren der Amplifikationsprodukte.

21. Kit zur Analyse von Proben auf die Anwesenheit von Polynucleotiden, die vom Nicht-A-Nicht-B-Nicht-C-Nicht-D-Nicht-E-Hepatitis-Virus (HGV) nach Anspruch 1 stammen, umfassend mindestens eine Polynucleotidsonde, die eine Nucleotidsequenz enthält, die spezifisch mit dem HGV-Polynucleotid nach Anspruch 16 hybridisiert, und einen geeigneten Behälter.

22. Clonierungsvektor, der fähig ist zur Expression, unter geeigneten Bedingungen, eines offenen Leserasters (ORF) einer cDNA, die von einem Nicht-A-Nicht-B-Nicht-C-Nicht-D-Nicht-E-Hepatitis-Virus (HGV)-Genom stammt, oder Komplementen davon, wobei dieses Virus die Eigenschaften nach Anspruch 1 besitzt und das ORF mit einer Kontrollsequenz funktionell verknüpft ist, die für einen gewünschten Wirt geeignet ist.

23. Verfahren zur Herstellung eines Nicht-A-Nicht-B-Nicht-C-Nicht-D-Nicht-E-Hepatitis-Virus (HGV)-Polypeptids, umfassend das Züchten einer Zelle, welche mit einem Vektor nach Anspruch 22 transformiert ist, unter Bedingungen, die zur Expression der Sequenz in dem offenen Leseraster (ORF) führen.

## Revendications

1. Virus d'hépatite Non-A Non-B Non-C Non-D Non-E (HGV) sous forme substantiellement isolée, ledit HGV étant **caractérisé** de la manière suivante : (i) est transmissible aux primates, (ii) est sérologiquement distinct du virus de l'hépatite A (HAV), du virus de l'hépatite B (HBV), du virus de l'hépatite C (HCV), du virus de l'hépatite D et du virus de l'hépatite E (HEV), (iii) est un membre de la famille de virus Flaviviridae, et (iv) contient des polynucléotides présentant une homologie de séquence d'au moins 55 % avec un polynucléotide ayant la séquence représentée par la SEQ ID N° 37 ou la SEQ ID N° 19, ou bien leurs compléments.

2. Polypeptide de virus d'hépatite Non-A Non-B Non-C Non-D Non-E (HGV) sous forme substantiellement isolée, ledit HGV : (i) ayant les caractéristiques du virus HGV suivant la revendication 1, et (ii) étant **caractérisé en outre par** des polypeptides dont les séquences d'amino-acides présentent une homologie de séquence d'au moins 40 % avec des séquences d'amino-acides choisies entre la séquence de 190 amino-acides de la SEQ ID N° 38 et la séquence de 67 amino-acides de la SEQ ID N° 20.

3. Polypeptide suivant la revendication 2, comprenant un antigène qui est spécifiquement immunoréactif avec au moins un anticorps anti-HGV, de la manière mise en évidence par l'aptitude de l'antigène à immunoréagir spécifiquement avec un liquide corporel ou un échantillon de tissu provenant d'un sujet HGV-positif.

4. Polypeptide suivant la revendication 2, qui est un polypeptide de fusion recombinant constitué d'un polypeptide de HGV et d'un second polypeptide, dans lequel ledit second polypeptide est choisi dans le groupe consistant en des protéines β-galactosidases, des protéines glutathion-S-transférases et des protéines formant des particules.

5. Polypeptide suivant la revendication 2, comprenant une séquence d'au moins 15 amino-acides contigus codée par un génome de HGV, son ADNc ou ses compléments, dans lequel ladite séquence d'amino-acides est choisie dans le groupe consistant en (i) la séquence de 190 amino-acides de la SEQ ID N° 38 ou ses fragments, (ii) la séquence de 67 amino-acides de la SEQ ID N° 20 ou ses fragments, et (iii) une séquence d'amino-acides codée dans la banque lambda gt11 de source d'ADNc de PNF 2161.

6. Kit de diagnostic destiné à être utilisé dans l'analyse d'un liquide corporel ou d'un échantillon de tissu contenant des anticorps spécifiques contre le virus d'hépatite Non-A Non-B Non-C Non-D Non-E (HGV) suivant la revendication 1, comprenant
l'antigène polypeptidique suivant la revendication 3, et
un moyen pour détecter un complexe immunologique formé par une immunoréaction spécifique dudit antigène avec des anticorps présents dans ledit échantillon.

7. Kit de diagnostic suivant la revendication 6, destiné à être utilisé dans l'analyse d'un sérum.

8. Kit de diagnostic destiné à être utilisé dans l'analyse d'un liquide corporel ou d'un échantillon de tissu contenant des antigènes de virus d'hépatite Non-A Non-B Non-C Non-D Non-E (HGV), comprenant
un anticorps substantiellement isolé spécifiquement immunoréactif avec l'antigène polypeptidique de virus d'hépatite Non-A Non-B Non-C Non-D Non-E (HGV) suivant la revendication 3, et
un moyen pour détecter la liaison dudit antigène polypeptidique audit anticorps.

9. Procédé pour la détection d'une infection par un virus d'hépatite Non-A Non-B Non-C Non-D Non-E (HGV) chez un sujet d'essai, comprenant
la réaction d'un liquide corporel ou d'un échantillon de tissu d'un sujet d'essai avec un anticorps spécifique de HGV substantiellement isolé du kit suivant la revendication 8, et
l'étude de l'anticorps pour déterminer la présence d'un antigène lié.

10. Anticorps monoclonal spécifiquement immunoréactif avec l'antigène de virus d'hépatite Non-A Non-B Non-C Non-D Non-E (HGV) suivant la revendication 3.

11. Préparation substantiellement isolée d'anticorps polyclonaux spécifiquement immunoréactifs avec l'antigène de virus d'hépatite Non-A Non-B Non-C Non-D Non-E (HGV) suivant la revendication 3.

12. Méthode d'analyse d'un liquide corporel ou échantillon de tissu contenant des anticorps spécifiques contre le virus d'hépatite Non-A Non-B Non-C Non-D Non-E (HGV) suivant la revendication 1, comprenant
la mise en contact de l'échantillon avec l'antigène polypeptidique suivant la revendication 3, et
la détection d'un complexe immunologique formé par une immunoréaction spécifique dudit antigène avec des anticorps présents dans ledit échantillon.

13. Utilisation du polypeptide de HGV suivant la revendication 2, comprenant un antigène contenant un épitope qui est spécifiquement immunoréactif avec au moins un anticorps anti-HGV pour la production d'une réponse immunitaire pour la préparation d'une composition destinée à la production d'anticorps contre le virus d'hépatite Non-A Non-B Non-C Non-D Non-E (HGV) suivant la revendication 1.

14. Polypeptide en mosaïque, comprenant
au moins deux antigènes différents suivant la revendication 3, ledit polypeptide en mosaïque étant dépourvu des amino-acides normalement intermédiaires entre lesdits antigènes dans un polypeptide de HGV naturel.

15. Composition de vaccin contre le virus d'hépatite Non-A Non-B Non-C Non-D Non-E (HGV), comprenant
un polypeptide de HGV substantiellement isolé suivant la revendication 3, présent à une dose pharmacologiquement efficace dans un support pharmaceutiquement acceptable.

16. Polynucléotide de virus d'hépatite Non-A Non-B Non-C Non-D Non-E (HGV) sous une forme substantiellement isolée, ledit HGV ayant les caractéristiques du virus HGV suivant la revendication 1.

17. Polynucléotide suivant la revendication 16, qui présente une homologie de séquence d'au moins 55 % avec un polynucléotide ayant la séquence représentée par la SEQ ID N° 37 ou la SEQ ID N° 19, ou bien leurs compléments.

18. Polynucléotide suivant la revendication 16, utile pour une détection par PCR d'un virus d'hépatite Non-A Non-B Non-C Non-D Non-E (HGV).

19. Méthode de détection d'un acide nucléique de virus d'hépatite Non-A Non-B Non-C Non-D Non-E (HGV) chez un sujet d'essai, comprenant les étapes consistant :
à obtenir à partir du sujet un échantillon contenant un acide nucléique,
à associer l'échantillon contenant l'acide nucléique à une sonde constituée du polynucléotide suivant la revendication 16, dans des conditions d'hybridation convenables, et
à détecter la présence de complexes acide nucléique de HGV/sonde, formés par hybridation de l'acide nucléique de HGV avec ladite sonde.

20. Méthode suivant la revendication 19, dans laquelle la détection comprend l'utilisation de sondes spécifiques de l'acide nucléique de HGV, dans laquelle les deux sondes définissent une région interne de l'acide nucléique de HGV et chaque sonde possède un brin contenant une extrémité 3' interne à la région, la conversion des complexes d'hybridation acide nucléique/sonde en fragments bicaténaires contenant les sondes par des réactions d'extension d'amorce,
l'amplification du nombre de fragments contenant les sondes en répétant de manière successive les étapes consistant (i) à dénaturer les fragments bicaténaires pour produire des fragments monocaténaires, (ii) à hybrider les brins simples avec les sondes pour former des complexes brin/sonde, (iii) à engendrer des fragments bicaténaires à partir des complexes brin/sonde en présence d'une enzyme et de l'ensemble des quatre désoxyribonucléotides, et (iv) à répéter les étapes (i) à (iii) jusqu'à obtention du degré désiré d'amplification,
l'identification des produits d'amplification.

21. Kit pour l'analyse d'échantillons afin de déterminer la présence de polynucléotides dérivés du virus d'hépatite Non-A Non-B Non-C Non-D Non-E (HGV) suivant la revendication 1, comprenant
au moins une sonde polynucléotidique contenant une séquence de nucléotides qui s'hybridera spécifiquement avec le polynucléotide de HGV suivant la revendication 16, et
un récipient convenable.

22. Vecteur de clonage apte à l'expression, dans des conditions convenables, d'un cadre de lecture ouvert (ORF) d'ADNc dérivé du génome d'un virus d'hépatite Non-A Non-B Non-C Non-D Non-E (HGV), ou de ses compléments, dans lequel ledit virus présente les caractéristiques suivant la revendication 1, et le ORF est lié de manière fonctionnelle à une séquence de régulation compatible avec un hôte désiré.

23. Procédé pour la production d'un polypeptide de virus d'hépatite Non-A Non-B Non-C Non-D Non-E (HGV), comprenant
la culture d'une cellule transformée avec un vecteur suivant la revendication 22, dans des conditions ayant pour résultat l'expression de la séquence de cadre de lecture ouvert (ORF).


### SEQUENCE LISTING

(1) GENERAL INFORMATION:
   (i) APPLICANT:
      (A) NAME: Genelabs Technologies, Inc.
      (B) STREET: 505 Penobscot Drive
      (C) CITY: Redwood City
      (D) STATE: CA
      (E) COUNTRY: USA
      (F) POSTAL CODE: 94063
   (ii) TITLE OF INVENTION: Hepatitis G Virus and Molecular Cloning Thereof
   (iii) NUMBER OF SEQUENCES: 277
   (iv) CORRESPONDENCE ADDRESS:
      (A) ADDRESSEE: Dehlinger & Associates
      (B) STREET: 350 Cambridge Ave., Suite 250
      (C) CITY: Palo Alto
      (D) STATE: CA
      (E) COUNTRY: USA
      (F) ZIP: 94306
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 08/389,886
      (B) FILING DATE: 15-FEB-1995
      (C) DOCKET NUMBER: 4600-0201.35
   (vii) PRIOR APPLICATION DATA:
      (A) APPLICATION NUMBER: US 08/357,509
      (B) FILING DATE: 16-DEC-1994
      (C) DOCKET NUMBER: 4600-0201.34
   (vii) PRIOR APPLICATION DATA:
      (A) APPLICATION NUMBER: US 08/329,729
      (B) FILING DATE: 26-OCT-1994
      (C) DOCKET NUMBER: 4600-0201.33
   (vii) PRIOR APPLICATION DATA:
      (A) APPLICATION NUMBER: US 08/344,271
      (B) FILING DATE: 23-NOV-1994
      (C) DOCKET NUMBER: 4600-0202
   (vii) PRIOR APPLICATION DATA:
      (A) APPLICATION NUMBER: US 08/285,558
      (B) FILING DATE: 03-AUG-1994
      (C) DOCKET NUMBER: 4600-0201.30
   (vii) PRIOR APPLICATION DATA:
      (A) APPLICATION NUMBER: US 08/285,543
      (B) FILING DATE: 03-AUG-1994
      (C) DOCKET NUMBER: 4600-0201.32
   (vii) PRIOR APPLICATION DATA:
      (A) APPLICATION NUMBER: US 08/246,985
      (B) FILING DATE: 20-MAY-1994
      (C) DOCKET NUMBER: 4600-0201
   (viii) ATTORNEY/AGENT INFORMATION:
      (A) NAME: Fabian, Gary R.
      (B) REGISTRATION NUMBER: 33,875
      (C) REFERENCE/DOCKET NUMBER: 4600-0201.41/G100PCT
   (ix) TELECOMMUNICATION INFORMATION:
      (A) TELEPHONE: (415) 324-0880
      (B) TELEFAX: (415) 324-0960
(2) INFORMATION FOR SEQ ID NO:1:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: SISPA primer, top strand Linker AB
   (xi) SEQUENCE DESCRIPTION: SEQ ID NO:1:
   GGAATTCGCG GCCGCTCG 18
(2) INFORMATION FOR SEQ ID NO:2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: Linker AB, bottom strand
   (xi) SEQUENCE DESCRIPTION: SEQ ID NO:2:
   CGAGCGGCCG CGAATTCCTT                                                    20
(2) INFORMATION FOR SEQ ID NO:3:
   (i) SEQUENCE CHARACTERISTICS:
      (A) LENGTH: 237 base pairs
      (B) TYPE: nucleic acid
      (C) STRANDEDNESS: double
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: PNF 2161 CLONE 470-20-1
   (ix) FEATURE:
      (A) NAME/KEY: CDS
      (B) LOCATION: 1..237
   (xi) SEQUENCE DESCRIPTION: SEQ ID NO:3:
   GAA TTC GCG GCC GCT CGG GCT GTC TCG GAC TCT TGG ATG ACC TCG AAT         48
   Glu Phe Ala Ala Ala Arg Ala Val Ser Asp Ser Trp Met Thr Ser Asn
    1               5                  10                  15
   GAG TCA GAG GAC GGG GTA TCC TCC TGC GAG GAG GAC ACC GGC GGG GTC         96
   Glu Ser Glu Asp Gly Val Ser Ser Cys Glu Glu Asp Thr Gly Gly Val
                20                  25                  30
   TTC TCA TCT GAG CTG CTC TCA GTA ACC GAG ATA AGT GCT GGC GAT GGA        144
   Phe Ser Ser Glu Leu Leu Ser Val Thr Glu Ile Ser Ala Gly Asp Gly
            35                  40                  45
   GTA CGG GGG ATG TCT TCT CCC CAT ACA GGC ATC TCT CGG CTA CTA CCA        192
   Val Arg Gly Met Ser Ser Pro His Thr Gly Ile Ser Arg Leu Leu Pro
        50                  55                  60
   CAA AGA GAG GGT GTA CTG CAG TCC TCC ACG AGC GGC CGC GAA TTC            237
   Gln Arg Glu Gly Val Leu Gln Ser Ser Thr Ser Gly Arg Glu Phe
    65                  70                  75
(2) INFORMATION FOR SEQ ID NO:4:
   (i) SEQUENCE CHARACTERISTICS:
      (A) LENGTH: 79 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Glu Phe Ala Ala Ala Arg Ala Val Ser Asp Ser Trp Met Thr Ser Asn
    1               5                  10                  15
   Glu Ser Glu Asp Gly Val Ser Ser Cys Glu Glu Asp Thr Gly Gly Val
                20                  25                  30
   Phe Ser Ser Glu Leu Leu Ser Val Thr Glu Ile Ser Ala Gly Asp Gly
   35 40 45
   Val Arg Gly Met Ser Ser Pro His Thr Gly Ile Ser Arg Leu Leu Pro
   50 55 60
   Gln Arg Glu Gly Val Leu Gln Ser Ser Thr Ser Gly Arg Glu Phe
   65 70 75
(2) INFORMATION FOR SEQ ID NO:5:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: HAV-R1
   (xi) SEQUENCE DESCRIPTION: SEQ ID NO:5:
   GTTGACCAAC TGAGTCTGAA GC 22
(2) INFORMATION FOR SEQ ID NO:6:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: HAV-F1
   (xi) SEQUENCE DESCRIPTION: SEQ ID NO:6:
   GATTGGAAAT CTGATCCGTC CC 22
(2) INFORMATION FOR SEQ ID NO:7:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: HCV-LANR
   (xi) SEQUENCE DESCRIPTION: SEQ ID NO:7:
   TCGCGACCCA ACACTACTC 19
(2) INFORMATION FOR SEQ ID NO:8:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: HCV 1532
   (xi) SEQUENCE DESCRIPTION: SEQ ID NO:8:
   GGGGGCGACA CTCCACCA 18
(2) INFORMATION FOR SEQ ID NO:9:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470-20-1-77F
   (xi) SEQUENCE DESCRIPTION: SEQ ID NO:9:
   CTCTTTGTGG TAGTAGCCGA GAGAT 25
(2) INFORMATION FOR SEQ ID NO:10:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470-20-1-211R
   (xi) SEQUENCE DESCRIPTION: SEQ ID NO:10:
   CGAATGAGTC AGAGGACGGG GTAT 24
(2) INFORMATION FOR SEQ ID NO:11:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer KL-1
   (xi) SEQUENCE DESCRIPTION: SEQ ID NO:11:
   GCAGGATCCG AATTCGCATC TAGAGAT 27
(2) INFORMATION FOR SEQ ID NO:12:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer KL-2
   (xi) SEQUENCE DESCRIPTION: SEQ ID NO:12:
   ATCTCTAGAT GCGAATTCGG ATCCTGCGA 29
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: LAMBDA GT11, REVERSE PRIMER
   (xi) SEQUENCE DESCRIPTION: SEQ ID NO:13:
   GGCAGACATG GCCTGCCCGG 20
(2) INFORMATION FOR SEQ ID NO:14:
   (i) SEQUENCE CHARACTERISTICS:
      (A) LENGTH: 9392 base pairs
      (B) TYPE: nucleic acid
      (C) STRANDEDNESS: double
      (D) TOPOLOGY: unknown
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: HGV-PNF 2161 Variant
   (ix) FEATURE:
      (A) NAME/KEY: CDS
      (B) LOCATION: 459..9077
   (xi) SEQUENCE DESCRIPTION: SEQ ID NO:14:
   ACGTGGGGGA GTTGATCCCC CCCCCCCGGC ACTGGGTGCA AGCCCCAGAA ACCGACGCCT      60
   ATCTAAGTAG ACGCAATGAC TCGGCGCCGA CTCGGCGACC GGCCAAAAGG TGGTGGATGG     120
   GTGATGACAG GGTTGGTAGG TCGTAAATCC CGGTCACCTT GGTAGCCACT ATAGGTGGGT     180
   CTTAAGAGAA GGTTAAGATT CCTCTTGTGC CTGCGGCGAG ACCGCGCACG GTCCACAGGT     240
   GTTGGCCCTA CCGGTGGGAA TAAGGGCCCG ACGTCAGGCT CGTCGTTAAA CCGAGCCCGT     300
   TACCCACCTG GGCAAACGAC GCCCACGTAC GGTCCACGTC GCCCTTCAAT GTCTCTCTTG     360
   ACCAATAGGC GTAGCCGGCG AGTTGACAAG GACCAGTGGG GGCCGGGGGC TTGGAGAGGG     420
   ACTCCAAGTC CCGCCCTTCC CGGTGGGCCG GGAAATGC ATG GGG CCA CCC AGC         473
                                             Met Gly Pro Pro Ser
                                               1               5
   TCC GCG GCG GCC TGC AGC CGG GGT AGC CCA AGA ATC CTT CGG GTG AGG       521
   Ser Ala Ala Ala Cys Ser Arg Gly Ser Pro Arg Ile Leu Arg Val Arg
                    10                  15                  20
   GCG GGT GGC ATT TCC TTT TTC TAT ACC ATC ATG GCA GTC CTT CTG CTC       569
   Ala Gly Gly Ile Ser Phe Phe Tyr Thr Ile Met Ala Val Leu Leu Leu
                25                  30                  35
   CTT CTC GTG GTT GAG GCC GGG GCC ATT CTG GCC CCG GCC ACC CAC GCT       617
   Leu Leu Val Val Glu Ala Gly Ala Ile Leu Ala Pro Ala Thr His Ala
            40                  45                  50
   TGT CGA GCG AAT GGG CAA TAT TTC CTC ACA AAT TGT TGT GCC CCG GAG       665
   Cys Arg Ala Asn Gly Gln Tyr Phe Leu Thr Asn Cys Cys Ala Pro Glu
        55                  60                  65
   GAC ATC GGG TTC TGC CTG GAG GGT GGA TGC CTG GTG GCC CTG GGG TGC       713
   Asp Ile Gly Phe Cys Leu Glu Gly Gly Cys Leu Val Ala Leu Gly Cys
    70                  75                  80                  85
   ACG ATT TGC ACT GAC CAA TGC TGG CCA CTG TAT CAG GCG GGT TTG GCT       761
   Thr Ile Cys Thr Asp Gln Cys Trp Pro Leu Tyr Gln Ala Gly Leu Ala
                    90                  95                 100
   GTG CGG CCT GGC AAG TCC GCG GCC CAA CTG GTG GGG GAG CTG GGT AGC       809
   Val Arg Pro Gly Lys Ser Ala Ala Gln Leu Val Gly Glu Leu Gly Ser
               105                 110                 115
   CTA TAC GGG CCC CTG TCG GTC TCG GCC TAT GTG GCT GGG ATC CTG GGC      857
   Leu Tyr Gly Pro Leu Ser Val Ser Ala Tyr Val Ala Gly Ile Leu Gly
           120                 125                 130
   CTG GGT GAG GTG TAC TCG GGT GTC CTA ACG GTG GGA GTC GCG TTG ACG      905
   Leu Gly Glu Val Tyr Ser Gly Val Leu Thr Val Gly Val Ala Leu Thr
       135                 140                 145
   CGC CGG GTC TAC CCG GTG CCT AAC CTG ACG TGT GCA GTC GCG TGT GAG      953
   Arg Arg Val Tyr Pro Val Pro Asn Leu Thr Cys Ala Val Ala Cys Glu
   150                 155                 160                 165
   CTA AAG TGG GAA AGT GAG TTT TGG AGA TGG ACT GAA CAG CTG GCC TCC     1001
   Leu Lys Trp Glu Ser Glu Phe Trp Arg Trp Thr Glu Gln Leu Ala Ser
                   170                 175                 180
   AAC TAC TGG ATT CTG GAA TAC CTC TGG AAG GTC CCA TTT GAT TTC TGG     1049
   Asn Tyr Trp Ile Leu Glu Tyr Leu Trp Lys Val Pro Phe Asp Phe Trp
               185                 190                 195
   AGA GGC GTG ATA AGC CTG ACC CCC TTG TTG GTT TGC GTG GCC GCA TTG     1097
   Arg Gly Val Ile Ser Leu Thr Pro Leu Leu Val Cys Val Ala Ala Leu
           200                 205                 210
   CTG CTG CTT GAG CAA CGG ATT GTC ATG GTC TTC CTG TTG GTG ACG ATG     1145
   Leu Leu Leu Glu Gln Arg Ile Val Met Val Phe Leu Leu Val Thr Met
       215                 220                 225
   GCC GGG ATG TCG CAA GGC GCC CCT GCC TCC GTT TTG GGG TCA CGC CCC     1193
   Ala Gly Met Ser Gln Gly Ala Pro Ala Ser Val Leu Gly Ser Arg Pro
   230                 235                 240                 245
   TTT GAC TAC GGG TTG ACT TGG CAG ACC TGC TCT TGC AGG GCC AAC GGT     1241
   Phe Asp Tyr Gly Leu Thr Trp Gln Thr Cys Ser Cys Arg Ala Asn Gly
                   250                 255                 260
   TCG CGT TTT TCG ACT GGG GAG AAG GTG TGG GAC CGT GGG AAC GTT ACG     1289
   Ser Arg Phe Ser Thr Gly Glu Lys Val Trp Asp Arg Gly Asn Val Thr
               265                 270                 275
   CTT CAG TGT GAC TGC CCT AAC GGC CCC TGG GTG TGG TTG CCA GCC TTT     1337
   Leu Gln Cys Asp Cys Pro Asn Gly Pro Trp Val Trp Leu Pro Ala Phe
           280                 285                 290
   TGC CAA GCA ATC GGC TGG GGT GAC CCC ATC ACT TAT TGG AGC CAC GGG     1385
   Cys Gln Ala Ile Gly Trp Gly Asp Pro Ile Thr Tyr Trp Ser His Gly
   295 300 305
   CAA AAT CAG TGG CCC CTT TCA TGC CCC CAG TAT GTC TAT GGG TCT GCT 1433
   Gln Asn Gln Trp Pro Leu Ser Cys Pro Gln Tyr Val Tyr Gly Ser Ala
   310 315 320 325
   ACA GTC ACT TGC GTG TGG GGT TCC GCT TCT TGG TTT GCC TCC ACC AGT 1481
   Thr Val Thr Cys Val Trp Gly Ser Ala Ser Trp Phe Ala Ser Thr Ser
   330 335 340
   GGT CGC GAC TCG AAG ATA GAT GTG TGG AGT TTA GTG CCA GTT GGC TCT 1529
   Gly Arg Asp Ser Lys Ile Asp Val Trp Ser Leu Val Pro Val Gly Ser
   345 350 355
   GCC ACC TGC ACC ATA GCC GCA CTT GGA TCA TCG GAT CGC GAC ACG GTG 1577
   Ala Thr Cys Thr Ile Ala Ala Leu Gly Ser Ser Asp Arg Asp Thr Val
   360 365 370
   CCT GGG CTC TCC GAG TGG GGA ATC CCG TGC GTG ACG TGT GTT CTG GAC 1625
   Pro Gly Leu Ser Glu Trp Gly Ile Pro Cys Val Thr Cys Val Leu Asp
   375 380 385
   CGT CGG CCT GCC TCC TGC GGC ACC TGT GTG AGG GAC TGC TGG CCC GAG 1673
   Arg Arg Pro Ala Ser Cys Gly Thr Cys Val Arg Asp Cys Trp Pro Glu
   390 395 400 405
   ACC GGG TCG GTT AGG TTC CCA TTC CAT CGG TGC GGC GTG GGG CCT CGG 1721
   Thr Gly Ser Val Arg Phe Pro Phe His Arg Cys Gly Val Gly Pro Arg
   410 415 420
   CTG ACA AAG GAC TTG GAA GCT GTG CCC TTC GTC AAC AGG ACA ACT CCC 1769
   Leu Thr Lys Asp Leu Glu Ala Val Pro Phe Val Asn Arg Thr Thr Pro
   425 430 435
   TTC ACC ATT AGG GGG CCC CTG GGC AAC CAG GGC CGA GGC AAC CCG GTG 1817
   Phe Thr Ile Arg Gly Pro Leu Gly Asn Gln Gly Arg Gly Asn Pro Val
   440 445 450
   CGG TCG CCC TTG GGT TTT GGG TCC TAC GCC ATG ACC AGG ATC CGA GAT 1865
   Arg Ser Pro Leu Gly Phe Gly Ser Tyr Ala Met Thr Arg Ile Arg Asp
   455 460 465
   ACC CTA CAT CTG GTG GAG TGT CCC ACA CCA GCC ATT GAG CCT CCC ACC 1913
   Thr Leu His Leu Val Glu Cys Pro Thr Pro Ala Ile Glu Pro Pro Thr
   470 475 480 485
   GGG ACG TTT GGG TTC TTC CCC GGG ACG CCG CCT CTC AAC AAC TGC ATG 1961
   Gly Thr Phe Gly Phe Phe Pro Gly Thr Pro Pro Leu Asn Asn Cys Met
   490 495 500
   CTC TTG GGC ACG GAA GTG TCC GAG GCA CTT GGG GGG GCT GGC CTC ACG 2009
   Leu Leu Gly Thr Glu Val Ser Glu Ala Leu Gly Gly Ala Gly Leu Thr
   505 510 515
   GGG GGG TTC TAT GAA CCC CTG GTG CGC AGG TGT TCG AAG CTG ATG GGA 2057
   Gly Gly Phe Tyr Glu Pro Leu Val Arg Arg Cys Ser Lys Leu Met Gly
   520 525 530
   AGC CGA AAT CCG GTT TGT CCG GGG TTT GCA TGG CTC TCT TCG GGC AGG 2105
   Ser Arg Asn Pro Val Cys Pro Gly Phe Ala Trp Leu Ser Ser Gly Arg
   535 540 545
   CCT GAT GGG TTT ATA CAT GTC CAG GGT CAC TTG CAG GAG GTG GAT GCA 2153
   Pro Asp Gly Phe Ile His Val Gln Gly His Leu Gln Glu Val Asp Ala
   550 555 560 565
   GGC AAC TTC ATC CCG CCC CCG CGC TGG TTG CTC TTG GAC TTT GTA TTT 2201
   Gly Asn Phe Ile Pro Pro Pro Arg Trp Leu Leu Leu Asp Phe Val Phe
   570 575 580
   GTC CTG TTA TAC CTG ATG AAG CTG GCT GAG GCA CGG TTG GTC CCG CTG 2249
   Val Leu Leu Tyr Leu Met Lys Leu Ala Glu Ala Arg Leu Val Pro Leu
   585 590 595
   ATC TTG CTG CTG CTA TGG TGG TGG GTG AAC CAG CTG GCA GTC CTA GGG 2297
   Ile Leu Leu Leu Leu Trp Trp Trp Val Asn Gln Leu Ala Val Leu Gly
   600 605 610
   CTG CCG GCT GTG GAA GCC GCC GTG GCA GGT GAG GTC TTC GCG GGC CCT 2345
   Leu Pro Ala Val Glu Ala Ala Val Ala Gly Glu Val Phe Ala Gly Pro
   615 620 625
   GCC CTG TCC TGG TGT CTG GGA CTC CCG GTC GTC AGT ATG ATA TTG GGT 2393
   Ala Leu Ser Trp Cys Leu Gly Leu Pro Val Val Ser Met Ile Leu Gly
   630 635 640 645
   TTG GCA AAC CTG GTG CTG TAC TTT AGA TGG TTG GGA CCC CAA CGC CTG 2441
   Leu Ala Asn Leu Val Leu Tyr Phe Arg Trp Leu Gly Pro Gln Arg Leu
   650 655 660
   ATG TTC CTC GTG TTG TGG AAG CTT GCT CGG GGA GCT TTC CCG CTG GCC      2489
   Met Phe Leu Val Leu Trp Lys Leu Ala Arg Gly Ala Phe Pro Leu Ala
               665                 670                 675
   CTC TTG ATG GGG ATT TCG GCG ACC CGC GGG CGC ACC TCA GTG CTC GGG      2537
   Leu Leu Met Gly Ile Ser Ala Thr Arg Gly Arg Thr Ser Val Leu Gly
           680                 685                 690
   GCC GAG TTC TGC TTC GAT GCT ACA TTC GAG GTG GAC ACT TCG GTG TTG      2585
   Ala Glu Phe Cys Phe Asp Ala Thr Phe Glu Val Asp Thr Ser Val Leu
       695                 700                 705
   GGC TGG GTG GTG GCC AGT GTG GTA GCT TGG GCC ATT GCG CTC CTG AGC      2633
   Gly Trp Val Val Ala Ser Val Val Ala Trp Ala Ile Ala Leu Leu Ser
   710                 715                 720                 725
   TCG ATG AGC GCA GGG GGG TGG AGG CAC AAA GCC GTG ATC TAT AGG ACG      2681
   Ser Met Ser Ala Gly Gly Trp Arg His Lys Ala Val Ile Tyr Arg Thr
                   730                 735                 740
   TGG TGT AAG GGG TAC CAG GCA ATC CGT CAA AGG GTG GTG AGG AGC CCC      2729
   Trp Cys Lys Gly Tyr Gln Ala Ile Arg Gln Arg Val Val Arg Ser Pro
               745                 750                 755
   CTC GGG GAG GGG CGG CCT GCC AAA CCC CTG ACC TTT GCC TGG TGC TTG      2777
   Leu Gly Glu Gly Arg Pro Ala Lys Pro Leu Thr Phe Ala Trp Cys Leu
           760                 765                 770
   GCC TCG TAC ATC TGG CCA GAT GCT GTG ATG ATG GTG GTG GTT GCC TTG      2825
   Ala Ser Tyr Ile Trp Pro Asp Ala Val Met Met Val Val Val Ala Leu
       775                 780                 785
   GTC CTT CTC TTT GGC CTG TTC GAC GCG TTG GAT TGG GCC TTG GAG GAG      2873
   Val Leu Leu Phe Gly Leu Phe Asp Ala Leu Asp Trp Ala Leu Glu Glu
   790                 795                 800                 805
   ATC TTG GTG TCC CGG CCC TCG TTG CGG CGT TTG GCT CGG GTG GTT GAG      2921
   Ile Leu Val Ser Arg Pro Ser Leu Arg Arg Leu Ala Arg Val Val Glu
                   810                 815                 820
   TGC TGT GTG ATG GCG GGT GAG AAG GCC ACA ACC GTC CGG CTG GTC TCC      2969
   Cys Cys Val Met Ala Gly Glu Lys Ala Thr Thr Val Arg Leu Val Ser
               825                 830                 835
   AAG ATG TGT GCG AGA GGA GCT TAT TTG TTC GAT CAT ATG GGC TCT TTT      3017
   Lys Met Cys Ala Arg Gly Ala Tyr Leu Phe Asp His Met Gly Ser Phe
           840                 845                 850
   TCG CGT GCT GTC AAG GAG CGC CTG TTG GAA TGG GAC GCA GCT CTT GAA      3065
   Ser Arg Ala Val Lys Glu Arg Leu Leu Glu Trp Asp Ala Ala Leu Glu
       855                 860                 865
   CCT CTG TCA TTC ACT AGG ACG GAC TGT CGC ATC ATA CGG GAT GCC GCG      3113
   Pro Leu Ser Phe Thr Arg Thr Asp Cys Arg Ile Ile Arg Asp Ala Ala
   870                 875                 880                 885
   AGG ACT TTG TCC TGC GGG CAG TGC GTC ATG GGT TTA CCC GTG GTT GCG      3161
   Arg Thr Leu Ser Cys Gly Gln Cys Val Met Gly Leu Pro Val Val Ala
                   890                 895                 900
   CGC CGT GGT GAT GAG GTT CTC ATC GGC GTC TTC CAG GAT GTG AAT CAT      3209
   Arg Arg Gly Asp Glu Val Leu Ile Gly Val Phe Gln Asp Val Asn His
               905                 910                 915
   TTG CCT CCC GGG TTT GTT CCG ACC GCG CCT GTT GTC ATC CGA CGG TGC      3257
   Leu Pro Pro Gly Phe Val Pro Thr Ala Pro Val Val Ile Arg Arg Cys
           920                 925                 930
   GGA AAG GGC TTC TTG GGG GTC ACA AAG GCT GCC TTG ACA GGT CGG GAT      3305
   Gly Lys Gly Phe Leu Gly Val Thr Lys Ala Ala Leu Thr Gly Arg Asp
       935                 940                 945
   CCT GAC TTA CAT CCA GGG AAC GTC ATG GTG TTG GGG ACG GCT ACG TCG      3353
   Pro Asp Leu His Pro Gly Asn Val Met Val Leu Gly Thr Ala Thr Ser
   950                 955                 960                 965
   CGA AGC ATG GGA ACA TGC TTG AAC GGC CTG CTG TTC ACG ACC TTC CAT      3401
   Arg Ser Met Gly Thr Cys Leu Asn Gly Leu Leu Phe Thr Thr Phe His
                   970                 975                 980
   GGG GCT TCA TCC CGA ACC ATC GCC ACA CCC GTG GGG GCC CTT AAT CCC      3449
   Gly Ala Ser Ser Arg Thr Ile Ala Thr Pro Val Gly Ala Leu Asn Pro
               985                 990                 995
   AGA TGG TGG TCA GCC AGT GAT GAT GTC ACG GTG TAT CCA CTC CCG GAT      3497
   Arg Trp Trp Ser Ala Ser Asp Asp Val Thr Val Tyr Pro Leu Pro Asp
           1000                1005                1010
   GGG GCT ACT TCG TTA ACA CCT TGT ACT TGC CAG GCT GAG TCC TGT TGG      3545
   Gly Ala Thr Ser Leu Thr Pro Cys Thr Cys Gln Ala Glu Ser Cys Trp
       1015                1020                1025
   GTC ATC AGA TCC GAC GGG GCC CTA TGC CAT GGC TTG AGC AAG GGG GAC      3593
   Val Ile Arg Ser Asp Gly Ala Leu Cys His Gly Leu Ser Lys Gly Asp
   1030                1035                1040                1045
   AAG GTG GAG CTG GAT GTG GCC ATG GAG GTC TCT GAC TTC CGT GGC TCG      3641
   Lys Val Glu Leu Asp Val Ala Met Glu Val Ser Asp Phe Arg Gly Ser
                   1050                1055                1060
   TCT GGC TCA CCG GTC CTA TGT GAC GAA GGG CAC GCA GTA GGA ATG CTC      3689
   Ser Gly Ser Pro Val Leu Cys Asp Glu Gly His Ala Val Gly Met Leu
               1065                1070                1075
   GTG TCT GTG CTT CAC TCC GGT GGT AGG GTC ACC GCG GCA CGG TTC ACT      3737
   Val Ser Val Leu His Ser Gly Gly Arg Val Thr Ala Ala Arg Phe Thr
           1080                1085                1090
   AGG CCG TGG ACC CAA GTG CCA ACA GAT GCC AAA ACC ACT ACT GAA CCC      3785
   Arg Pro Trp Thr Gln Val Pro Thr Asp Ala Lys Thr Thr Thr Glu Pro
       1095                1100                1105
   CCT CCG GTG CCG GCC AAA GGA GTT TTC AAA GAG GCC CCG TTG TTT ATG      3833
   Pro Pro Val Pro Ala Lys Gly Val Phe Lys Glu Ala Pro Leu Phe Met
   1110                1115                1120                1125
   CCT ACG GGA GCG GGA AAG AGC ACT CGC GTC CCG TTG GAG TAC GAT AAC      3881
   Pro Thr Gly Ala Gly Lys Ser Thr Arg Val Pro Leu Glu Tyr Asp Asn
                   1130                1135                1140
   ATG GGG CAC AAG GTC TTA ATC TTG AAC CCC TCA GTG GCC ACT GTG CGG      3929
   Met Gly His Lys Val Leu Ile Leu Asn Pro Ser Val Ala Thr Val Arg
               1145                1150                1155
   GCC ATG GGC CCG TAC ATG GAG CGG CTG GCG GGT AAA CAT CCA AGT ATA      3977
   Ala Met Gly Pro Tyr Met Glu Arg Leu Ala Gly Lys His Pro Ser Ile
           1160                1165                1170
   TAC TGT GGG CAT GAT ACA ACT GCT TTC ACA AGG ATC ACT GAC TCC CCC      4025
   Tyr Cys Gly His Asp Thr Thr Ala Phe Thr Arg Ile Thr Asp Ser Pro
       1175                1180                1185
   CTG ACG TAT TCA ACC TAT GGG AGG TTT TTG GCC AAC CCT AGG CAG ATG      4073
   Leu Thr Tyr Ser Thr Tyr Gly Arg Phe Leu Ala Asn Pro Arg Gln Met
   1190                1195                1200                1205
   CTA CGG GGC GTT TCG GTG GTC ATT TGT GAT GAG TGC CAC AGT CAT GAC      4121
   Leu Arg Gly Val Ser Val Val Ile Cys Asp Glu Cys His Ser His Asp
                   1210                1215                1220
   TCA ACC GTG CTG TTA GGC ATT GGG AGA GTC CGG GAG CTG GCG CGT GGG      4169
   Ser Thr Val Leu Leu Gly Ile Gly Arg Val Arg Glu Leu Ala Arg Gly
               1225                1230                1235
   TGC GGG GTG CAA CTA GTG CTC TAC GCC ACC GCT ACA CCT CCC GGA TCC      4217
   Cys Gly Val Gln Leu Val Leu Tyr Ala Thr Ala Thr Pro Pro Gly Ser
           1240                1245                1250
   CCT ATG ACG CAG CAC CCT TCC ATA ATT GAG ACA AAA TTG GAC GTG GGC      4265
   Pro Met Thr Gln His Pro Ser Ile Ile Glu Thr Lys Leu Asp Val Gly
       1255                1260                1265
   GAG ATT CCC TTT TAT GGG CAT GGA ATA CCC CTC GAG CGG ATG CGA ACC      4313
   Glu Ile Pro Phe Tyr Gly His Gly Ile Pro Leu Glu Arg Met Arg Thr
   1270                1275                1280                1285
   GGA AGG CAC CTC GTG TTC TGC CAT TCT AAG GCT GAG TGC GAG CGC CTT      4361
   Gly Arg His Leu Val Phe Cys His Ser Lys Ala Glu Cys Glu Arg Leu
                   1290                1295                1300
   GCT GGC CAG TTC TCC GCT AGG GGG GTC AAT GCC ATT GCC TAT TAT AGG      4409
   Ala Gly Gln Phe Ser Ala Arg Gly Val Asn Ala Ile Ala Tyr Tyr Arg
               1305                1310                1315
   GGT AAA GAC AGT TCT ATC ATC AAG GAT GGG GAC CTG GTG GTC TGT GCT      4457
   Gly Lys Asp Ser Ser Ile Ile Lys Asp Gly Asp Leu Val Val Cys Ala
           1320                1325                1330
   ACA GAC GCG CTT TCC ACT GGG TAC ACT GGA AAT TTC GAC TCC GTC ACC      4505
   Thr Asp Ala Leu Ser Thr Gly Tyr Thr Gly Asn Phe Asp Ser Val Thr
       1335                1340                1345
   GAC TGT GGA TTA GTG GTG GAG GAG GTC GTT GAG GTG ACC CTT GAT CCC      4553
   Asp Cys Gly Leu Val Val Glu Glu Val Val Glu Val Thr Leu Asp Pro
   1350                1355                1360                1365
   ACC ATT ACC ATC TCC CTG CGG ACA GTG CCT GCG TCG GCT GAA CTG TCG      4601
   Thr Ile Thr Ile Ser Leu Arg Thr Val Pro Ala Ser Ala Glu Leu Ser
                   1370                1375                1380
   ATG CAA AGA CGA GGA CGC ACG GGT AGG GGC AGG TCT GGA CGC TAC TAC      4649
   Met Gln Arg Arg Gly Arg Thr Gly Arg Gly Arg Ser Gly Arg Tyr Tyr
               1385                1390                1395
   TAC GCG GGG GTG GGC AAA GCC CCT GCG GGT GTG GTG CGC TCA GGT CCT      4697
   Tyr Ala Gly Val Gly Lys Ala Pro Ala Gly Val Val Arg Ser Gly Pro
           1400                1405                1410
   GTC TGG TCG GCG GTG GAA GCT GGA GTG ACC TGG TAC GGA ATG GAA CCT      4745
   Val Trp Ser Ala Val Glu Ala Gly Val Thr Trp Tyr Gly Met Glu Pro
       1415                1420                1425
   GAC TTG ACA GCT AAC CTA CTG AGA CTT TAC GAC GAC TGC CCT TAC ACC      4793
   Asp Leu Thr Ala Asn Leu Leu Arg Leu Tyr Asp Asp Cys Pro Tyr Thr
   1430                1435                1440                1445
   GCA GCC GTC GCG GCT GAT ATC GGA GAA GCC GCG GTG TTC TTC TCT GGG      4841
   Ala Ala Val Ala Ala Asp Ile Gly Glu Ala Ala Val Phe Phe Ser Gly
                   1450                1455                1460
   CTC GCC CCA TTG AGG ATG CAC CCT GAT GTC AGC TGG GCA AAA GTT CGC      4889
   Leu Ala Pro Leu Arg Met His Pro Asp Val Ser Trp Ala Lys Val Arg
               1465                1470                1475
   GGC GTC AAC TGG CCC CTC TTG GTG GGT GTT CAG CGG ACC ATG TGT CGG      4937
   Gly Val Asn Trp Pro Leu Leu Val Gly Val Gln Arg Thr Met Cys Arg
           1480                1485                1490
   GAA ACA CTG TCT CCC GGC CCA TCG GAT GAC CCC CAA TGG GCA GGT CTG      4985
   Glu Thr Leu Ser Pro Gly Pro Ser Asp Asp Pro Gln Trp Ala Gly Leu
       1495                1500                1505
   AAG GGC CCA AAT CCT GTC CCA CTC CTG CTG AGG TGG GGC AAT GAT TTA      5033
   Lys Gly Pro Asn Pro Val Pro Leu Leu Leu Arg Trp Gly Asn Asp Leu
   1510                1515                1520                1525
   CCA TCT AAA GTG GCC GGC CAC CAC ATA GTG GAC GAC CTG GTC CGG AGA      5081
   Pro Ser Lys Val Ala Gly His His Ile Val Asp Asp Leu Val Arg Arg
                   1530                1535                1540
   CTC GGT GTG GCG GAG GGT TAC GTC CGC TGC GAC GCT GGG CCG ATC TTG      5129
   Leu Gly Val Ala Glu Gly Tyr Val Arg Cys Asp Ala Gly Pro Ile Leu
               1545                1550                1555
   ATG ATC GGT CTA GCT ATC GCG GGG GGA ATG ATC TAC GCG TCA TAC ACC      5177
   Met Ile Gly Leu Ala Ile Ala Gly Gly Met Ile Tyr Ala Ser Tyr Thr
   1560 1565 1570
   GGG TCG CTA GTG GTG GTG ACA GAC TGG GAT GTG AAG GGG GGT GGC GCC 5225
   Gly Ser Leu Val Val Val Thr Asp Trp Asp Val Lys Gly Gly Gly Ala
   1575 1580 1585
   CCC CTT TAT CGG CAT GGA GAC CAG GCC ACG CCT CAG CCG GTG GTG CAG 5273
   Pro Leu Tyr Arg His Gly Asp Gln Ala Thr Pro Gln Pro Val Val Gln
   1590 1595 1600 1605
   GTT CCT CCG GTA GAC CAT CGG CCG GGG GGT GAA TCA GCA CCA TCG GAT 5321
   Val Pro Pro Val Asp His Arg Pro Gly Gly Glu Ser Ala Pro Ser Asp
   1610 1615 1620
   GCC AAG ACA GTG ACA GAT GCG GTG GCA GCC ATC CAG GTG GAC TGC GAT 5369
   Ala Lys Thr Val Thr Asp Ala Val Ala Ala Ile Gln Val Asp Cys Asp
   1625 1630 1635
   TGG ACT ATC ATG ACT CTG TCG ATC GGA GAA GTG TTG TCC TTG GCT CAG 5417
   Trp Thr Ile Met Thr Leu Ser Ile Gly Glu Val Leu Ser Leu Ala Gln
   1640 1645 1650
   GCT AAG ACG GCC GAG GCC TAC ACA GCA ACC GCC AAG TGG CTC GCT GGC 5465
   Ala Lys Thr Ala Glu Ala Tyr Thr Ala Thr Ala Lys Trp Leu Ala Gly
   1655 1660 1665
   TGC TAT ACG GGG ACG CGG GCC GTT CCC ACT GTA TCC ATT GTT GAC AAG 5513
   Cys Tyr Thr Gly Thr Arg Ala Val Pro Thr Val Ser Ile Val Asp Lys
   1670 1675 1680 1685
   CTC TTC GCC GGA GGG TGG GCG GCT GTG GTG GGC CAT TGC CAC AGC GTG 5561
   Leu Phe Ala Gly Gly Trp Ala Ala Val Val Gly His Cys His Ser Val
   1690 1695 1700
   ATT GCT GCG GCG GTG GCG GCC TAC GGG GCT TCA AGG AGC CCG CCG TTG 5609
   Ile Ala Ala Ala Val Ala Ala Tyr Gly Ala Ser Arg Ser Pro Pro Leu
   1705 1710 1715
   GCA GCC GCG GCT TCC TAC CTG ATG GGG TTG GGC GTT GGA GGC AAC GCT 5657
   Ala Ala Ala Ala Ser Tyr Leu Met Gly Leu Gly Val Gly Gly Asn Ala
   1720 1725 1730
   CAG ACG CGC CTG GCG TCT GCC CTC CTA TTG GGG GCT GCT GGA ACC GCC 5705
   Gln Thr Arg Leu Ala Ser Ala Leu Leu Leu Gly Ala Ala Gly Thr Ala
   1735 1740 1745
   TTG GGC ACT CCT GTC GTG GGC TTG ACC ATG GCA GGT GCG TTC ATG GGG 5753
   Leu Gly Thr Pro Val Val Gly Leu Thr Met Ala Gly Ala Phe Met Gly
   1750 1755 1760 1765
   GGG GCC AGT GTC TCC CCC TCC TTG GTC ACC ATT TTA TTG GGG GCC GTC 5801
   Gly Ala Ser Val Ser Pro Ser Leu Val Thr Ile Leu Leu Gly Ala Val
   1770 1775 1780
   GGA GGT TGG GAG GGT GTT GTC AAC GCG GCG AGC CTA GTC TTT GAC TTC 5849
   Gly Gly Trp Glu Gly Val Val Asn Ala Ala Ser Leu Val Phe Asp Phe
   1785 1790 1795
   ATG GCG GGG AAA CTT TCA TCA GAA GAT CTG TGG TAT GCC ATC CCG GTA 5897
   Met Ala Gly Lys Leu Ser Ser Glu Asp Leu Trp Tyr Ala Ile Pro Val
   1800 1805 1810
   CTG ACC AGC CCG GGG GCG GGC CTT GCG GGG ATC GCT CTC GGG TTG GTT 5945
   Leu Thr Ser Pro Gly Ala Gly Leu Ala Gly Ile Ala Leu Gly Leu Val
   1815 1820 1825
   TTG TAT TCA GCT AAC AAC TCT GGC ACT ACC ACT TGG TTG AAC CGT CTG 5993
   Leu Tyr Ser Ala Asn Asn Ser Gly Thr Thr Thr Trp Leu Asn Arg Leu
   1830 1835 1840 1845
   CTG ACT ACG TTA CCA AGG TCT TCA TGT ATC CCG GAC AGT TAC TTT CAG 6041
   Leu Thr Thr Leu Pro Arg Ser Ser Cys Ile Pro Asp Ser Tyr Phe Gln
   1850 1855 1860
   CAA GTT GAC TAT TGC GAC AAG GTC TCA GCC GTG CTC CGG CGC CTG AGC 6089
   Gln Val Asp Tyr Cys Asp Lys Val Ser Ala Val Leu Arg Arg Leu Ser
   1865 1870 1875
   CTC ACC CGC ACA GTG GTT GCC CTG GTC AAC AGG GAG CCT AAG GTG GAT 6137
   Leu Thr Arg Thr Val Val Ala Leu Val Asn Arg Glu Pro Lys Val Asp
   1880 1885 1890
   GAG GTA CAG GTG GGG TAT GTC TGG GAC CTG TGG GAG TGG ATC ATG CGC 6185
   Glu Val Gln Val Gly Tyr Val Trp Asp Leu Trp Glu Trp Ile Met Arg
   1895 1900 1905
   CAA GTG CGC GTG GTC ATG GCC AGA CTC AGG GCC CTC TGC CCC GTG GTG 6233
   Gln Val Arg Val Val Met Ala Arg Leu Arg Ala Leu Cys Pro Val Val
   1910 1915 1920 1925
   TCA CTA CCC TTG TGG CAT TGC GGG GAG GGG TGG TCC GGG GAA TGG TTG 6281
   Ser Leu Pro Leu Trp His Cys Gly Glu Gly Trp Ser Gly Glu Trp Leu
                   1930                1935                1940
   CTT GAC GGT CAT GTT GAG AGT CGC TGC CTC TGT GGC TGC GTG ATC ACT      6329
   Leu Asp Gly His Val Glu Ser Arg Cys Leu Cys Gly Cys Val Ile Thr
               1945                1950                1955
   GGT GAC GTT CTG AAT GGG CAA CTC AAA GAA CCA GTT TAC TCT ACC AAG      6377
   Gly Asp Val Leu Asn Gly Gln Leu Lys Glu Pro Val Tyr Ser Thr Lys
           1960                1965                1970
   CTG TGC CGG CAC TAT TGG ATG GGG ACT GTC CCT GTG AAC ATG CTG GGT      6425
   Leu Cys Arg His Tyr Trp Met Gly Thr Val Pro Val Asn Met Leu Gly
       1975                1980                1985
   TAC GGT GAA ACG TCG CCT CTC CTG GCC TCC GAC ACC CCG AAG GTT GTG      6473
   Tyr Gly Glu Thr Ser Pro Leu Leu Ala Ser Asp Thr Pro Lys Val Val
   1990                1995                2000                2005
   CCC TTC GGG ACG TCT GGC TGG GCT GAG GTG GTG GTG ACC ACT ACC CAC      6521
   Pro Phe Gly Thr Ser Gly Trp Ala Glu Val Val Val Thr Thr Thr His
                   2010                2015                2020
   GTG GTA ATC AGG AGG ACC TCC GCC TAT AAG CTG CTG CGC CAG CAA ATC      6569
   Val Val Ile Arg Arg Thr Ser Ala Tyr Lys Leu Leu Arg Gln Gln Ile
               2025                2030                2035
   CTA TCG GCT GCT GTA GCT GAG CCC TAC TAC GTC GAC GGC ATT CCG GTC      6617
   Leu Ser Ala Ala Val Ala Glu Pro Tyr Tyr Val Asp Gly Ile Pro Val
           2040                2045                2050
   TCA TGG GAC GCG GAC GCT CGT GCG CCC GCC ATG GTC TAT GGC CCT GGG      6665
   Ser Trp Asp Ala Asp Ala Arg Ala Pro Ala Met Val Tyr Gly Pro Gly
       2055                2060                2065
   CAA AGT GTT ACC ATT GAC GGG GAG CGC TAC ACC TTG CCT CAT CAA CTG      6713
   Gln Ser Val Thr Ile Asp Gly Glu Arg Tyr Thr Leu Pro His Gln Leu
   2070                2075                2080                2085
   AGG CTC AGG AAT GTG GCA CCC TCT GAG GTT TCA TCC GAG GTG TCC ATT      6761
   Arg Leu Arg Asn Val Ala Pro Ser Glu Val Ser Ser Glu Val Ser Ile
                   2090                2095                2100
   GAC ATT GGG ACG GAG ACT GAA GAC TCA GAA CTG ACT GAG GCC GAT CTG      6809
   Asp Ile Gly Thr Glu Thr Glu Asp Ser Glu Leu Thr Glu Ala Asp Leu
   2105 2110 2115
   CCG CCG GCG GCT GCT GCT CTC CAA GCG ATC GAG AAT GCT GCG AGG ATT 6857
   Pro Pro Ala Ala Ala Ala Leu Gln Ala Ile Glu Asn Ala Ala Arg Ile
   2120 2125 2130
   CTT GAA CCG CAC ATT GAT GTC ATC ATG GAG GAC TGC AGT ACA CCC TCT 6905
   Leu Glu Pro His Ile Asp Val Ile Met Glu Asp Cys Ser Thr Pro Ser
   2135 2140 2145
   CTT TGT GGT AGT AGC CGA GAG ATG CCT GTA TGG GGA GAA GAC ATC CCC 6953
   Leu Cys Gly Ser Ser Arg Glu Met Pro Val Trp Gly Glu Asp Ile Pro
   2150 2155 2160 2165
   CGT ACT CCA TCG CCA GCA CTT ATC TCG GTT ACT GAG AGC AGC TCA GAT 7001
   Arg Thr Pro Ser Pro Ala Leu Ile Ser Val Thr Glu Ser Ser Ser Asp
   2170 2175 2180
   GAG AAG ACC CCG TCG GTG TCC TCC TCG CAG GAG GAT ACC CCG TCC TCT 7049
   Glu Lys Thr Pro Ser Val Ser Ser Ser Gln Glu Asp Thr Pro Ser Ser
   2185 2190 2195
   GAC TCA TTC GAG GTC ATC CAA GAG TCC GAG ACA GCC GAA GGG GAG GAA 7097
   Asp Ser Phe Glu Val Ile Gln Glu Ser Glu Thr Ala Glu Gly Glu Glu
   2200 2205 2210
   AGT GTC TTC AAC GTG GCT CTT TCC GTA TTA AAA GCC TTA TTT CCA CAG 7145
   Ser Val Phe Asn Val Ala Leu Ser Val Leu Lys Ala Leu Phe Pro Gln
   2215 2220 2225
   AGC GAC GCG ACC AGG AAG CTT ACC GTC AAG ATG TCG TGC TGC GTT GAA 7193
   Ser Asp Ala Thr Arg Lys Leu Thr Val Lys Met Ser Cys Cys Val Glu
   2230 2235 2240 2245
   AAG AGC GTC ACG CGC TTT TTC TCA TTG GGG TTG ACG GTG GCT GAT GTT 7241
   Lys Ser Val Thr Arg Phe Phe Ser Leu Gly Leu Thr Val Ala Asp Val
   2250 2255 2260
   GCT AGC CTG TGT GAG ATG GAA ATC CAG AAC CAT ACA GCC TAT TGT GAC 7289
   Ala Ser Leu Cys Glu Met Glu Ile Gln Asn His Thr Ala Tyr Cys Asp
   2265 2270 2275
   CAG GTG CGC ACT CCG CTT GAA TTG CAG GTT GGG TGC TTG GTG GGC AAT 7337
   Gln Val Arg Thr Pro Leu Glu Leu Gln Val Gly Cys Leu Val Gly Asn
   2280 2285 2290
   GAA CTT ACC TTT GAA TGT GAC AAG TGT GAG GCT AGG CAA GAA ACC TTG 7385
   Glu Leu Thr Phe Glu Cys Asp Lys Cys Glu Ala Arg Gln Glu Thr Leu
   2295 2300 2305
   GCC TCC TTC TCT TAC ATT TGG TCT GGA GTG CCG CTG ACT AGG GCC ACG 7433
   Ala Ser Phe Ser Tyr Ile Trp Ser Gly Val Pro Leu Thr Arg Ala Thr
   2310 2315 2320 2325
   CCG GCC AAG CCT CCC GTG GTG AGG CCG GTT GGC TCT TTG TTA GTG GCC 7481
   Pro Ala Lys Pro Pro Val Val Arg Pro Val Gly Ser Leu Leu Val Ala
   2330 2335 2340
   GAC ACT ACT AAG GTG TAT GTT ACC AAT CCA GAC AAT GTG GGA CGG AGG 7529
   Asp Thr Thr Lys Val Tyr Val Thr Asn Pro Asp Asn Val Gly Arg Arg
   2345 2350 2355
   GTG GAC AAG GTG ACC TTC TGG CGT GCT CCT AGG GTT CAT GAT AAG TAC 7577
   Val Asp Lys Val Thr Phe Trp Arg Ala Pro Arg Val His Asp Lys Tyr
   2360 2365 2370
   CTC GTG GAC TCT ATT GAG CGC GCT AAG AGG GCC GCT CAA GCC TGC CTA 7625
   Leu Val Asp Ser Ile Glu Arg Ala Lys Arg Ala Ala Gln Ala Cys Leu
   2375 2380 2385
   AGC ATG GGT TAC ACT TAT GAG GAA GCA ATA AGG ACT GTA AGG CCA CAT 7673
   Ser Met Gly Tyr Thr Tyr Glu Glu Ala Ile Arg Thr Val Arg Pro His
   2390 2395 2400 2405
   GCT GCC ATG GGC TGG GGA TCT AAG GTG TCG GTT AAG GAC TTA GCC ACC 7721
   Ala Ala Met Gly Trp Gly Ser Lys Val Ser Val Lys Asp Leu Ala Thr
   2410 2415 2420
   CCC GCG GGG AAG ATG GCC GTC CAT GAC CGG CTT CAG GAG ATA CTT GAA 7769
   Pro Ala Gly Lys Met Ala Val His Asp Arg Leu Gln Glu Ile Leu Glu
   2425 2430 2435
   GGG ACT CCG GTC CCC TTT ACT CTT ACT GTG AAA AAG GAG GTG TTC TTC 7817
   Gly Thr Pro Val Pro Phe Thr Leu Thr Val Lys Lys Glu Val Phe Phe
   2440 2445 2450
   AAA GAC CGG AAG GAG GAG AAG GCC CCC CGC CTC ATT GTG TTC CCC CCC 7865
   Lys Asp Arg Lys Glu Glu Lys Ala Pro Arg Leu Ile Val Phe Pro Pro
   2455 2460 2465
   CTG GAC TTC CGG ATA GCT GAA AAG CTC ATC TTG GGA GAC CCA GGC CGG 7913
   Leu Asp Phe Arg Ile Ala Glu Lys Leu Ile Leu Gly Asp Pro Gly Arg
   2470 2475 2480 2485
   GTA GCC AAG GCG GTG TTG GGG GGG GCC TAC GCC TTC CAG TAC ACC CCA 7961
   Val Ala Lys Ala Val Leu Gly Gly Ala Tyr Ala Phe Gln Tyr Thr Pro
   2490 2495 2500
   AAT CAG CGA GTT AAG GAG ATG CTC AAG CTA TGG GAG TCT AAG AAG ACC 8009
   Asn Gln Arg Val Lys Glu Met Leu Lys Leu Trp Glu Ser Lys Lys Thr
   2505 2510 2515
   CCT TGC GCC ATC TGT GTG GAC GCC ACC TGC TTC GAC AGT AGC ATA ACT 8057
   Pro Cys Ala Ile Cys Val Asp Ala Thr Cys Phe Asp Ser Ser Ile Thr
   2520 2525 2530
   GAA GAG GAC GTG GCT TTG GAG ACA GAG CTA TAC GCT CTG GCC TCT GAC 8105
   Glu Glu Asp Val Ala Leu Glu Thr Glu Leu Tyr Ala Leu Ala Ser Asp
   2535 2540 2545
   CAT CCA GAA TGG GTG CGG GCA CTT GGG AAA TAC TAT GCC TCA GGC ACC 8153
   His Pro Glu Trp Val Arg Ala Leu Gly Lys Tyr Tyr Ala Ser Gly Thr
   2550 2555 2560 2565
   ATG GTC ACC CCG GAA GGG GTG CCC GTC GGT GAG AGG TAT TGC AGA TCC 8201
   Met Val Thr Pro Glu Gly Val Pro Val Gly Glu Arg Tyr Cys Arg Ser
   2570 2575 2580
   TCG GGT GTC CTA ACA ACT AGC GCG AGC AAC TGC TTG ACC TGC TAC ATC 8249
   Ser Gly Val Leu Thr Thr Ser Ala Ser Asn Cys Leu Thr Cys Tyr Ile
   2585 2590 2595
   AAG GTG AAA GCT GCC TGT GAG AGA GTG GGG CTG AAA AAT GTC TCT CTT 8297
   Lys Val Lys Ala Ala Cys Glu Arg Val Gly Leu Lys Asn Val Ser Leu
   2600 2605 2610
   CTC ATA GCC GGC GAT GAC TGC TTG ATC ATA TGT GAG CGG CCA GTG TGC 8345
   Leu Ile Ala Gly Asp Asp Cys Leu Ile Ile Cys Glu Arg Pro Val Cys
   2615 2620 2625
   GAC CCA AGC GAC GCT TTG GGC AGA GCC CTA GCG AGC TAT GGG TAC GCG 8393
   Asp Pro Ser Asp Ala Leu Gly Arg Ala Leu Ala Ser Tyr Gly Tyr Ala
   2630 2635 2640 2645
   TGC GAG CCC TCA TAT CAT GCA TCA TTG GAC ACG GCC CCC TTC TGC TCC 8441
   Cys Glu Pro Ser Tyr His Ala Ser Leu Asp Thr Ala Pro Phe Cys Ser
   2650 2655 2660
   ACT TGG CTT GCT GAG TGC AAT GCA GAT GGG AAG CGC CAT TTC TTC CTG 8489
   Thr Trp Leu Ala Glu Cys Asn Ala Asp Gly Lys Arg His Phe Phe Leu
   2665 2670 2675
   ACC ACG GAC TTC CGG AGG CCG CTC GCT CGC ATG TCG AGT GAG TAT AGT 8537
   Thr Thr Asp Phe Arg Arg Pro Leu Ala Arg Met Ser Ser Glu Tyr Ser
   2680 2685 2690
   GAC CCG ATG GCT TCG GCG ATC GGT TAC ATC CTC CTT TAT CCT TGG CAC 8585
   Asp Pro Met Ala Ser Ala Ile Gly Tyr Ile Leu Leu Tyr Pro Trp His
   2695 2700 2705
   CCC ATC ACA CGG TGG GTC ATC ATC CCT CAT GTG CTA ACG TGC GCA TTC 8633
   Pro Ile Thr Arg Trp Val Ile Ile Pro His Val Leu Thr Cys Ala Phe
   2710 2715 2720 2725
   AGG GGT GGA GGC ACA CCG TCT GAT CCG GTT TGG TGC CAG GTG CAT GGT 8681
   Arg Gly Gly Gly Thr Pro Ser Asp Pro Val Trp Cys Gln Val His Gly
   2730 2735 2740
   AAC TAC TAC AAG TTT CCA CTG GAC AAA CTG CCT AAC ATC ATC GTG GCC 8729
   Asn Tyr Tyr Lys Phe Pro Leu Asp Lys Leu Pro Asn Ile Ile Val Ala
   2745 2750 2755
   CTC CAC GGA CCA GCA GCG TTG AGG GTT ACC GCA GAC ACA ACT AAA ACA 8777
   Leu His Gly Pro Ala Ala Leu Arg Val Thr Ala Asp Thr Thr Lys Thr
   2760 2765 2770
   AAG ATG GAG GCT GGT AAG GTT CTG AGC GAC CTC AAG CTC CCT GGC TTA 8825
   Lys Met Glu Ala Gly Lys Val Leu Ser Asp Leu Lys Leu Pro Gly Leu
   2775 2780 2785
   GCA GTC CAC CGA AAG AAG GCC GGG GCG TTG CGA ACA CGC ATG CTC CGC 8873
   Ala Val His Arg Lys Lys Ala Gly Ala Leu Arg Thr Arg Met Leu Arg
   2790 2795 2800 2805
   TCG CGC GGT TGG GCT GAG TTG GCT AGG GGC TTG TTG TGG CAT CCA GGC 8921
   Ser Arg Gly Trp Ala Glu Leu Ala Arg Gly Leu Leu Trp His Pro Gly
   2810 2815 2820
   CTA CGG CTT CCT CCC CCT GAG ATT GCT GGT ATC CCG GGG GGT TTC CCT 8969
   Leu Arg Leu Pro Pro Pro Glu Ile Ala Gly Ile Pro Gly Gly Phe Pro
   2825 2830 2835
   CTC TCC CCC CCC TAT ATG GGG GTG GTA CAT CAA TTG GAT TTC ACA AGC      9017
   Leu Ser Pro Pro Tyr Met Gly Val Val His Gln Leu Asp Phe Thr Ser
           2840                2845                2850
   CAG AGG AGT CGC TGG CGG TGG TTG GGG TTC TTA GCC CTG CTC ATC GTA      9065
   Gln Arg Ser Arg Trp Arg Trp Leu Gly Phe Leu Ala Leu Leu Ile Val
       2855                2860                2865
   GCC CTC TTC GGG TGAACTAAAT TCATCTGTTG CGGCAAGGTC TGGTGACTGA          9117
   Ala Leu Phe Gly
   2870
   TCATCACCGG AGGAGGTTCC CGCCCTCCCC GCCCCAGGGG TCTCCCCGCT GGGTAAAAAG    9177
   GGCCCGGCCT TGGGAGGCAT GGTGGTTACT AACCCCCTGG CAGGGTCAAA GCCTGATGGT    9237
   GCTAATGCAC TGCCACTTCG GTGGCGGGTC GCTACCTTAT AGCGTAATCC GTGACTACGG    9297
   GCTGCTCGCA GAGCCCTCCC CGGATGGGGC ACAGTGCACT GTGATCTGAA GGGGTGCACC    9357
   CCGGGAAGAG CTCGGCCCGA AGGCCGGSTT CTACT                               9392
(2) INFORMATION FOR SEQ ID NO:15:
   (i) SEQUENCE CHARACTERISTICS:
      (A) LENGTH: 2873 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Met Gly Pro Pro Ser Ser Ala Ala Ala Cys Ser Arg Gly Ser Pro Arg
    1               5                   10                  15
   Ile Leu Arg Val Arg Ala Gly Gly Ile Ser Phe Phe Tyr Thr Ile Met
               20                  25                  30
   Ala Val Leu Leu Leu Leu Leu Val Val Glu Ala Gly Ala Ile Leu Ala
           35                  40                  45
   Pro Ala Thr His Ala Cys Arg Ala Asn Gly Gln Tyr Phe Leu Thr Asn
       50                  55                  60
   Cys Cys Ala Pro Glu Asp Ile Gly Phe Cys Leu Glu Gly Gly Cys Leu
   65 70 75 80
   Val Ala Leu Gly Cys Thr Ile Cys Thr Asp Gln Cys Trp Pro Leu Tyr
   85 90 95
   Gln Ala Gly Leu Ala Val Arg Pro Gly Lys Ser Ala Ala Gln Leu Val
   100 105 110
   Gly Glu Leu Gly Ser Leu Tyr Gly Pro Leu Ser Val Ser Ala Tyr Val
   115 120 125
   Ala Gly Ile Leu Gly Leu Gly Glu Val Tyr Ser Gly Val Leu Thr Val
   130 135 140
   Gly Val Ala Leu Thr Arg Arg Val Tyr Pro Val Pro Asn Leu Thr Cys
   145 150 155 160
   Ala Val Ala Cys Glu Leu Lys Trp Glu Ser Glu Phe Trp Arg Trp Thr
   165 170 175
   Glu Gln Leu Ala Ser Asn Tyr Trp Ile Leu Glu Tyr Leu Trp Lys Val
   180 185 190
   Pro Phe Asp Phe Trp Arg Gly Val Ile Ser Leu Thr Pro Leu Leu Val
   195 200 205
   Cys Val Ala Ala Leu Leu Leu Leu Glu Gln Arg Ile Val Met Val Phe
   210 215 220
   Leu Leu Val Thr Met Ala Gly Met Ser Gln Gly Ala Pro Ala Ser Val
   225 230 235 240
   Leu Gly Ser Arg Pro Phe Asp Tyr Gly Leu Thr Trp Gln Thr Cys Ser
   245 250 255
   Cys Arg Ala Asn Gly Ser Arg Phe Ser Thr Gly Glu Lys Val Trp Asp
   260 265 270
   Arg Gly Asn Val Thr Leu Gln Cys Asp Cys Pro Asn Gly Pro Trp Val
   275 280 285
   Trp Leu Pro Ala Phe Cys Gln Ala Ile Gly Trp Gly Asp Pro Ile Thr
   290 295 300
   Tyr Trp Ser His Gly Gln Asn Gln Trp Pro Leu Ser Cys Pro Gln Tyr
   305 310 315 320
   Val Tyr Gly Ser Ala Thr Val Thr Cys Val Trp Gly Ser Ala Ser Trp
   325 330 335
   Phe Ala Ser Thr Ser Gly Arg Asp Ser Lys Ile Asp Val Trp Ser Leu
   340 345 350
   Val Pro Val Gly Ser Ala Thr Cys Thr Ile Ala Ala Leu Gly Ser Ser
   355 360 365
   Asp Arg Asp Thr Val Pro Gly Leu Ser Glu Trp Gly Ile Pro Cys Val
   370 375 380
   Thr Cys Val Leu Asp Arg Arg Pro Ala Ser Cys Gly Thr Cys Val Arg
   385 390 395 400
   Asp Cys Trp Pro Glu Thr Gly Ser Val Arg Phe Pro Phe His Arg Cys
   405 410 415
   Gly Val Gly Pro Arg Leu Thr Lys Asp Leu Glu Ala Val Pro Phe Val
   420 425 430
   Asn Arg Thr Thr Pro Phe Thr Ile Arg Gly Pro Leu Gly Asn Gln Gly
   435 440 445
   Arg Gly Asn Pro Val Arg Ser Pro Leu Gly Phe Gly Ser Tyr Ala Met
   450 455 460
   Thr Arg Ile Arg Asp Thr Leu His Leu Val Glu Cys Pro Thr Pro Ala
   465 470 475 480
   Ile Glu Pro Pro Thr Gly Thr Phe Gly Phe Phe Pro Gly Thr Pro Pro
   485 490 495
   Leu Asn Asn Cys Met Leu Leu Gly Thr Glu Val Ser Glu Ala Leu Gly
   500 505 510
   Gly Ala Gly Leu Thr Gly Gly Phe Tyr Glu Pro Leu Val Arg Arg Cys
   515 520 525
   Ser Lys Leu Met Gly Ser Arg Asn Pro Val Cys Pro Gly Phe Ala Trp
   530 535 540
   Leu Ser Ser Gly Arg Pro Asp Gly Phe Ile His Val Gln Gly His Leu
   545 550 555 560
   Gln Glu Val Asp Ala Gly Asn Phe Ile Pro Pro Pro Arg Trp Leu Leu
   565 570 575
   Leu Asp Phe Val Phe Val Leu Leu Tyr Leu Met Lys Leu Ala Glu Ala
   580 585 590
   Arg Leu Val Pro Leu Ile Leu Leu Leu Leu Trp Trp Trp Val Asn Gln
   595 600 605
   Leu Ala Val Leu Gly Leu Pro Ala Val Glu Ala Ala Val Ala Gly Glu
   610 615 620
   Val Phe Ala Gly Pro Ala Leu Ser Trp Cys Leu Gly Leu Pro Val Val
   625 630 635 640
   Ser Met Ile Leu Gly Leu Ala Asn Leu Val Leu Tyr Phe Arg Trp Leu
   645 650 655
   Gly Pro Gln Arg Leu Met Phe Leu Val Leu Trp Lys Leu Ala Arg Gly
   660 665 670
   Ala Phe Pro Leu Ala Leu Leu Met Gly Ile Ser Ala Thr Arg Gly Arg
   675 680 685
   Thr Ser Val Leu Gly Ala Glu Phe Cys Phe Asp Ala Thr Phe Glu Val
   690 695 700
   Asp Thr Ser Val Leu Gly Trp Val Val Ala Ser Val Val Ala Trp Ala
   705 710 715 720
   Ile Ala Leu Leu Ser Ser Met Ser Ala Gly Gly Trp Arg His Lys Ala
   725 730 735
   Val Ile Tyr Arg Thr Trp Cys Lys Gly Tyr Gln Ala Ile Arg Gln Arg
   740 745 750
   Val Val Arg Ser Pro Leu Gly Glu Gly Arg Pro Ala Lys Pro Leu Thr
   755 760 765
   Phe Ala Trp Cys Leu Ala Ser Tyr Ile Trp Pro Asp Ala Val Met Met
   770 775 780
   Val Val Val Ala Leu Val Leu Leu Phe Gly Leu Phe Asp Ala Leu Asp
   785 790 795 800
   Trp Ala Leu Glu Glu Ile Leu Val Ser Arg Pro Ser Leu Arg Arg Leu
   805 810 815
   Ala Arg Val Val Glu Cys Cys Val Met Ala Gly Glu Lys Ala Thr Thr
   820 825 830
   Val Arg Leu Val Ser Lys Met Cys Ala Arg Gly Ala Tyr Leu Phe Asp
   835 840 845
   His Met Gly Ser Phe Ser Arg Ala Val Lys Glu Arg Leu Leu Glu Trp
   850 855 860
   Asp Ala Ala Leu Glu Pro Leu Ser Phe Thr Arg Thr Asp Cys Arg Ile
   865 870 875 880
   Ile Arg Asp Ala Ala Arg Thr Leu Ser Cys Gly Gln Cys Val Met Gly
   885 890 895
   Leu Pro Val Val Ala Arg Arg Gly Asp Glu Val Leu Ile Gly Val Phe
   900 905 910
   Gln Asp Val Asn His Leu Pro Pro Gly Phe Val Pro Thr Ala Pro Val
   915 920 925
   Val Ile Arg Arg Cys Gly Lys Gly Phe Leu Gly Val Thr Lys Ala Ala
   930 935 940
   Leu Thr Gly Arg Asp Pro Asp Leu His Pro Gly Asn Val Met Val Leu
   945 950 955 960
   Gly Thr Ala Thr Ser Arg Ser Met Gly Thr Cys Leu Asn Gly Leu Leu
   965 970 975
   Phe Thr Thr Phe His Gly Ala Ser Ser Arg Thr Ile Ala Thr Pro Val
   980 985 990
   Gly Ala Leu Asn Pro Arg Trp Trp Ser Ala Ser Asp Asp Val Thr Val
   995 1000 1005
   Tyr Pro Leu Pro Asp Gly Ala Thr Ser Leu Thr Pro Cys Thr Cys Gln
   1010 1015 1020
   Ala Glu Ser Cys Trp Val Ile Arg Ser Asp Gly Ala Leu Cys His Gly
   1025 1030 1035 1040
   Leu Ser Lys Gly Asp Lys Val Glu Leu Asp Val Ala Met Glu Val Ser
   1045 1050 1055
   Asp Phe Arg Gly Ser Ser Gly Ser Pro Val Leu Cys Asp Glu Gly His
   1060 1065 1070
   Ala Val Gly Met Leu Val Ser Val Leu His Ser Gly Gly Arg Val Thr
   1075 1080 1085
   Ala Ala Arg Phe Thr Arg Pro Trp Thr Gln Val Pro Thr Asp Ala Lys
   1090 1095 1100
   Thr Thr Thr Glu Pro Pro Pro Val Pro Ala Lys Gly Val Phe Lys Glu
   1105 1110 1115 1120
   Ala Pro Leu Phe Met Pro Thr Gly Ala Gly Lys Ser Thr Arg Val Pro
   1125 1130 1135
   Leu Glu Tyr Asp Asn Met Gly His Lys Val Leu Ile Leu Asn Pro Ser
   1140 1145 1150
   Val Ala Thr Val Arg Ala Met Gly Pro Tyr Met Glu Arg Leu Ala Gly
   1155 1160 1165
   Lys His Pro Ser Ile Tyr Cys Gly His Asp Thr Thr Ala Phe Thr Arg
   1170 1175 1180
   Ile Thr Asp Ser Pro Leu Thr Tyr Ser Thr Tyr Gly Arg Phe Leu Ala
   1185 1190 1195 1200
   Asn Pro Arg Gln Met Leu Arg Gly Val Ser Val Val Ile Cys Asp Glu
   1205 1210 1215
   Cys His Ser His Asp Ser Thr Val Leu Leu Gly Ile Gly Arg Val Arg
   1220 1225 1230
   Glu Leu Ala Arg Gly Cys Gly Val Gln Leu Val Leu Tyr Ala Thr Ala
   1235 1240 1245
   Thr Pro Pro Gly Ser Pro Met Thr Gln His Pro Ser Ile Ile Glu Thr
   1250 1255 1260
   Lys Leu Asp Val Gly Glu Ile Pro Phe Tyr Gly His Gly Ile Pro Leu
   1265 1270 1275 1280
   Glu Arg Met Arg Thr Gly Arg His Leu Val Phe Cys His Ser Lys Ala
   1285 1290 1295
   Glu Cys Glu Arg Leu Ala Gly Gln Phe Ser Ala Arg Gly Val Asn Ala
   1300 1305 1310
   Ile Ala Tyr Tyr Arg Gly Lys Asp Ser Ser Ile Ile Lys Asp Gly Asp
   1315 1320 1325
   Leu Val Val Cys Ala Thr Asp Ala Leu Ser Thr Gly Tyr Thr Gly Asn
   1330 1335 1340
   Phe Asp Ser Val Thr Asp Cys Gly Leu Val Val Glu Glu Val Val Glu
   1345 1350 1355 1360
   Val Thr Leu Asp Pro Thr Ile Thr Ile Ser Leu Arg Thr Val Pro Ala
   1365 1370 1375
   Ser Ala Glu Leu Ser Met Gln Arg Arg Gly Arg Thr Gly Arg Gly Arg
   1380 1385 1390
   Ser Gly Arg Tyr Tyr Tyr Ala Gly Val Gly Lys Ala Pro Ala Gly Val
   1395 1400 1405
   Val Arg Ser Gly Pro Val Trp Ser Ala Val Glu Ala Gly Val Thr Trp
   1410 1415 1420
   Tyr Gly Met Glu Pro Asp Leu Thr Ala Asn Leu Leu Arg Leu Tyr Asp
   1425 1430 1435 1440
   Asp Cys Pro Tyr Thr Ala Ala Val Ala Ala Asp Ile Gly Glu Ala Ala
   1445 1450 1455
   Val Phe Phe Ser Gly Leu Ala Pro Leu Arg Met His Pro Asp Val Ser
   1460 1465 1470
   Trp Ala Lys Val Arg Gly Val Asn Trp Pro Leu Leu Val Gly Val Gln
   1475 1480 1485
   Arg Thr Met Cys Arg Glu Thr Leu Ser Pro Gly Pro Ser Asp Asp Pro
   1490 1495 1500
   Gln Trp Ala Gly Leu Lys Gly Pro Asn Pro Val Pro Leu Leu Leu Arg
   1505 1510 1515 1520
   Trp Gly Asn Asp Leu Pro Ser Lys Val Ala Gly His His Ile Val Asp
   1525 1530 1535
   Asp Leu Val Arg Arg Leu Gly Val Ala Glu Gly Tyr Val Arg Cys Asp
   1540 1545 1550
   Ala Gly Pro Ile Leu Met Ile Gly Leu Ala Ile Ala Gly Gly Met Ile
   1555 1560 1565
   Tyr Ala Ser Tyr Thr Gly Ser Leu Val Val Val Thr Asp Trp Asp Val
   1570 1575 1580
   Lys Gly Gly Gly Ala Pro Leu Tyr Arg His Gly Asp Gln Ala Thr Pro
   1585 1590 1595 1600
   Gln Pro Val Val Gln Val Pro Pro Val Asp His Arg Pro Gly Gly Glu
   1605 1610 1615
   Ser Ala Pro Ser Asp Ala Lys Thr Val Thr Asp Ala Val Ala Ala Ile
   1620 1625 1630
   Gln Val Asp Cys Asp Trp Thr Ile Met Thr Leu Ser Ile Gly Glu Val
   1635 1640 1645
   Leu Ser Leu Ala Gln Ala Lys Thr Ala Glu Ala Tyr Thr Ala Thr Ala
   1650 1655 1660
   Lys Trp Leu Ala Gly Cys Tyr Thr Gly Thr Arg Ala Val Pro Thr Val
   1665 1670 1675 1680
   Ser Ile Val Asp Lys Leu Phe Ala Gly Gly Trp Ala Ala Val Val Gly
   1685 1690 1695
   His Cys His Ser Val Ile Ala Ala Ala Val Ala Ala Tyr Gly Ala Ser
   1700 1705 1710
   Arg Ser Pro Pro Leu Ala Ala Ala Ala Ser Tyr Leu Met Gly Leu Gly
   1715 1720 1725
   Val Gly Gly Asn Ala Gln Thr Arg Leu Ala Ser Ala Leu Leu Leu Gly
   1730 1735 1740
   Ala Ala Gly Thr Ala Leu Gly Thr Pro Val Val Gly Leu Thr Met Ala
   1745 1750 1755 1760
   Gly Ala Phe Met Gly Gly Ala Ser Val Ser Pro Ser Leu Val Thr Ile
   1765 1770 1775
   Leu Leu Gly Ala Val Gly Gly Trp Glu Gly Val Val Asn Ala Ala Ser
   1780 1785 1790
   Leu Val Phe Asp Phe Met Ala Gly Lys Leu Ser Ser Glu Asp Leu Trp
   1795 1800 1805
   Tyr Ala Ile Pro Val Leu Thr Ser Pro Gly Ala Gly Leu Ala Gly Ile
   1810 1815 1820
   Ala Leu Gly Leu Val Leu Tyr Ser Ala Asn Asn Ser Gly Thr Thr Thr
   1825 1830 1835 1840
   Trp Leu Asn Arg Leu Leu Thr Thr Leu Pro Arg Ser Ser Cys Ile Pro
   1845 1850 1855
   Asp Ser Tyr Phe Gln Gln Val Asp Tyr Cys Asp Lys Val Ser Ala Val
   1860 1865 1870
   Leu Arg Arg Leu Ser Leu Thr Arg Thr Val Val Ala Leu Val Asn Arg
   1875 1880 1885
   Glu Pro Lys Val Asp Glu Val Gln Val Gly Tyr Val Trp Asp Leu Trp
   1890 1895 1900
   Glu Trp Ile Met Arg Gln Val Arg Val Val Met Ala Arg Leu Arg Ala
   1905 1910 1915 1920
   Leu Cys Pro Val Val Ser Leu Pro Leu Trp His Cys Gly Glu Gly Trp
   1925 1930 1935
   Ser Gly Glu Trp Leu Leu Asp Gly His Val Glu Ser Arg Cys Leu Cys
   1940 1945 1950
   Gly Cys Val Ile Thr Gly Asp Val Leu Asn Gly Gln Leu Lys Glu Pro
   1955 1960 1965
   Val Tyr Ser Thr Lys Leu Cys Arg His Tyr Trp Met Gly Thr Val Pro
   1970 1975 1980
   Val Asn Met Leu Gly Tyr Gly Glu Thr Ser Pro Leu Leu Ala Ser Asp
   1985 1990 1995 2000
   Thr Pro Lys Val Val Pro Phe Gly Thr Ser Gly Trp Ala Glu Val Val
   2005 2010 2015
   Val Thr Thr Thr His Val Val Ile Arg Arg Thr Ser Ala Tyr Lys Leu
   2020 2025 2030
   Leu Arg Gln Gln Ile Leu Ser Ala Ala Val Ala Glu Pro Tyr Tyr Val
   2035 2040 2045
   Asp Gly Ile Pro Val Ser Trp Asp Ala Asp Ala Arg Ala Pro Ala Met
   2050 2055 2060
   Val Tyr Gly Pro Gly Gln Ser Val Thr Ile Asp Gly Glu Arg Tyr Thr
   2065 2070 2075 2080
   Leu Pro His Gln Leu Arg Leu Arg Asn Val Ala Pro Ser Glu Val Ser
   2085 2090 2095
   Ser Glu Val Ser Ile Asp Ile Gly Thr Glu Thr Glu Asp Ser Glu Leu
   2100 2105 2110
   Thr Glu Ala Asp Leu Pro Pro Ala Ala Ala Ala Leu Gln Ala Ile Glu
   2115 2120 2125
   Asn Ala Ala Arg Ile Leu Glu Pro His Ile Asp Val Ile Met Glu Asp
   2130 2135 2140
   Cys Ser Thr Pro Ser Leu Cys Gly Ser Ser Arg Glu Met Pro Val Trp
   2145 2150 2155 2160
   Gly Glu Asp Ile Pro Arg Thr Pro Ser Pro Ala Leu Ile Ser Val Thr
   2165 2170 2175
   Glu Ser Ser Ser Asp Glu Lys Thr Pro Ser Val Ser Ser Ser Gln Glu
   2180 2185 2190
   Asp Thr Pro Ser Ser Asp Ser Phe Glu Val Ile Gln Glu Ser Glu Thr
   2195 2200 2205
   Ala Glu Gly Glu Glu Ser Val Phe Asn Val Ala Leu Ser Val Leu Lys
   2210 2215 2220
   Ala Leu Phe Pro Gln Ser Asp Ala Thr Arg Lys Leu Thr Val Lys Met
   2225 2230 2235 2240
   Ser Cys Cys Val Glu Lys Ser Val Thr Arg Phe Phe Ser Leu Gly Leu
   2245 2250 2255
   Thr Val Ala Asp Val Ala Ser Leu Cys Glu Met Glu Ile Gln Asn His
   2260 2265 2270
   Thr Ala Tyr Cys Asp Gln Val Arg Thr Pro Leu Glu Leu Gln Val Gly
   2275 2280 2285
   Cys Leu Val Gly Asn Glu Leu Thr Phe Glu Cys Asp Lys Cys Glu Ala
   2290 2295 2300
   Arg Gln Glu Thr Leu Ala Ser Phe Ser Tyr Ile Trp Ser Gly Val Pro
   2305 2310 2315 2320
   Leu Thr Arg Ala Thr Pro Ala Lys Pro Pro Val Val Arg Pro Val Gly
   2325 2330 2335
   Ser Leu Leu Val Ala Asp Thr Thr Lys Val Tyr Val Thr Asn Pro Asp
   2340 2345 2350
   Asn Val Gly Arg Arg Val Asp Lys Val Thr Phe Trp Arg Ala Pro Arg
   2355 2360 2365
   Val His Asp Lys Tyr Leu Val Asp Ser Ile Glu Arg Ala Lys Arg Ala
   2370 2375 2380
   Ala Gln Ala Cys Leu Ser Met Gly Tyr Thr Tyr Glu Glu Ala Ile Arg
   2385 2390 2395 2400
   Thr Val Arg Pro His Ala Ala Met Gly Trp Gly Ser Lys Val Ser Val
   2405 2410 2415
   Lys Asp Leu Ala Thr Pro Ala Gly Lys Met Ala Val His Asp Arg Leu
   2420 2425 2430
   Gln Glu Ile Leu Glu Gly Thr Pro Val Pro Phe Thr Leu Thr Val Lys
   2435 2440 2445
   Lys Glu Val Phe Phe Lys Asp Arg Lys Glu Glu Lys Ala Pro Arg Leu
   2450 2455 2460
   Ile Val Phe Pro Pro Leu Asp Phe Arg Ile Ala Glu Lys Leu Ile Leu
   2465 2470 2475 2480
   Gly Asp Pro Gly Arg Val Ala Lys Ala Val Leu Gly Gly Ala Tyr Ala
   2485 2490 2495
   Phe Gln Tyr Thr Pro Asn Gln Arg Val Lys Glu Met Leu Lys Leu Trp
   2500 2505 2510
   Glu Ser Lys Lys Thr Pro Cys Ala Ile Cys Val Asp Ala Thr Cys Phe
   2515 2520 2525
   Asp Ser Ser Ile Thr Glu Glu Asp Val Ala Leu Glu Thr Glu Leu Tyr
   2530 2535 2540
   Ala Leu Ala Ser Asp His Pro Glu Trp Val Arg Ala Leu Gly Lys Tyr
   2545 2550 2555 2560
   Tyr Ala Ser Gly Thr Met Val Thr Pro Glu Gly Val Pro Val Gly Glu
   2565 2570 2575
   Arg Tyr Cys Arg Ser Ser Gly Val Leu Thr Thr Ser Ala Ser Asn Cys
   2580 2585 2590
   Leu Thr Cys Tyr Ile Lys Val Lys Ala Ala Cys Glu Arg Val Gly Leu
   2595 2600 2605
   Lys Asn Val Ser Leu Leu Ile Ala Gly Asp Asp Cys Leu Ile Ile Cys
   2610 2615 2620
   Glu Arg Pro Val Cys Asp Pro Ser Asp Ala Leu Gly Arg Ala Leu Ala
   2625 2630 2635 2640
   Ser Tyr Gly Tyr Ala Cys Glu Pro Ser Tyr His Ala Ser Leu Asp Thr
   2645 2650 2655
   Ala Pro Phe Cys Ser Thr Trp Leu Ala Glu Cys Asn Ala Asp Gly Lys
   2660 2665 2670
   Arg His Phe Phe Leu Thr Thr Asp Phe Arg Arg Pro Leu Ala Arg Met
   2675 2680 2685
   Ser Ser Glu Tyr Ser Asp Pro Met Ala Ser Ala Ile Gly Tyr Ile Leu
   2690 2695 2700
   Leu Tyr Pro Trp His Pro Ile Thr Arg Trp Val Ile Ile Pro His Val
   2705 2710 2715 2720
   Leu Thr Cys Ala Phe Arg Gly Gly Gly Thr Pro Ser Asp Pro Val Trp
   2725 2730 2735
   Cys Gln Val His Gly Asn Tyr Tyr Lys Phe Pro Leu Asp Lys Leu Pro
   2740 2745 2750
   Asn Ile Ile Val Ala Leu His Gly Pro Ala Ala Leu Arg Val Thr Ala
   2755 2760 2765
   Asp Thr Thr Lys Thr Lys Met Glu Ala Gly Lys Val Leu Ser Asp Leu
   2770 2775 2780
   Lys Leu Pro Gly Leu Ala Val His Arg Lys Lys Ala Gly Ala Leu Arg
   2785 2790 2795 2800
   Thr Arg Met Leu Arg Ser Arg Gly Trp Ala Glu Leu Ala Arg Gly Leu
   2805 2810 2815
   Leu Trp His Pro Gly Leu Arg Leu Pro Pro Pro Glu Ile Ala Gly Ile
   2820 2825 2830
   Pro Gly Gly Phe Pro Leu Ser Pro Pro Tyr Met Gly Val Val His Gln
   2835 2840 2845
   Leu Asp Phe Thr Ser Gln Arg Ser Arg Trp Arg Trp Leu Gly Phe Leu
   2850 2855 2860
   Ala Leu Leu Ile Val Ala Leu Phe Gly
   2865 2870
(2) INFORMATION FOR SEQ ID NO:16:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PROBE 470-20-1-152F
   (xi) SEQUENCE DESCRIPTION: SEQ ID NO:16:
   TCGGTTACTG AGAGCAGCTC AGATGAG 27
(2) INFORMATION FOR SEQ ID NO:17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: JML-A, PRIMER
   (xi) SEQUENCE DESCRIPTION: SEQ ID NO:17:
   AGGAATTCAG CGGCCGCGAG 20
(2) INFORMATION FOR SEQ ID NO:18:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (vi) ORIGINAL SOURCE:
      (C) INDIVIDUAL ISOLATE: JML-B, PRIMER
   (xi) SEQUENCE DESCRIPTION: SEQ ID NO:18:
   CTCGCGGCCG CTGAATTCCT TT 22
(2) INFORMATION FOR SEQ ID NO:19:
   (i) SEQUENCE CHARACTERISTICS:
      (A) LENGTH: 203 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (vi) ORIGINAL SOURCE:
      (C) INDIVIDUAL ISOLATE: 470-20-1 CLONE, WITHOUT SISPA LINKERS
   (ix) FEATURE:
      (A) NAME/KEY: CDS
      (B) LOCATION: 2..203
   (xi) SEQUENCE DESCRIPTION: SEQ ID NO:19:
   G GCT GTC TCG GAC TCT TGG ATG ACC TCG AAT GAG TCA GAG GAC GGG      46
     Ala Val Ser Asp Ser Trp Met Thr Ser Asn Glu Ser Glu Asp Gly
       1               5                  10                  15
   GTA TCC TCC TGC GAG GAG GAC ACC GGC GGG GTC TTC TCA TCT GAG CTG    94
   Val Ser Ser Cys Glu Glu Asp Thr Gly Gly Val Phe Ser Ser Glu Leu
                    20                  25                  30
   CTC TCA GTA ACC GAG ATA AGT GCT GGC GAT GGA GTA CGG GGG ATG TCT   142
   Leu Ser Val Thr Glu Ile Ser Ala Gly Asp Gly Val Arg Gly Met Ser
                35                  40                  45
   TCT CCC CAT ACA GGC ATC TCT CGG CTA CTA CCA CAA AGA GAG GGT GTA   190
   Ser Pro His Thr Gly Ile Ser Arg Leu Leu Pro Gln Arg Glu Gly Val
                50                  55                  60
   CTG CAG TCC TCC A                                                  203
   Leu Gln Ser Ser
        65
(2) INFORMATION FOR SEQ ID NO:20:
   (i) SEQUENCE CHARACTERISTICS:
      (A) LENGTH: 67 amino acids
      (B) TYPE: amino acid
      (D) TOPOLOGY: linear
   (ii) MOLECULE TYPE: protein
   (xi) SEQUENCE DESCRIPTION: SEQ ID NO:20:
   Ala Val Ser Asp Ser Trp Met Thr Ser Asn Glu Ser Glu Asp Gly Val
    1               5                  10                  15
   Ser Ser Cys Glu Glu Asp Thr Gly Gly Val Phe Ser Ser Glu Leu Leu
               20                  25                  30
   Ser Val Thr Glu Ile Ser Ala Gly Asp Gly Val Arg Gly Met Ser Ser
           35                  40                  45
   Pro His Thr Gly Ile Ser Arg Leu Leu Pro Gln Arg Glu Gly Val Leu
       50                  55                  60
   Gln Ser Ser
    65
(2) INFORMATION FOR SEQ ID NO:21:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470-20-1-152R
   (xi) SEQUENCE DESCRIPTION: SEQ ID NO:21:
   CTCATCTGAG CTGCTCTCAG TAACCGA                                    27
(2) INFORMATION FOR SEQ ID NO:22:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: OLIGONUCLEOTIDE B
   (xi) SEQUENCE DESCRIPTION: SEQ ID NO:22:
   CTGTCTCGGA CTCTTGGATG ACCT 24
(2) INFORMATION FOR SEQ ID NO:23:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: COGNATE OLIGONUCLEOTIDE 211R'
   (xi) SEQUENCE DESCRIPTION: SEQ ID NO:23:
   ATACCCCGTC CTCTGACTCA TTCG 24
(2) INFORMATION FOR SEQ ID NO:24:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: COGNATE OLIGONUCLEOTIDE B'
   (xi) SEQUENCE DESCRIPTION: SEQ ID NO:24:
   AGGTCATCCA AGAGTCCGAG ACAG 24
(2) INFORMATION FOR SEQ ID NO:25:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: LAMBDA GT 11 FORWARD PRIMER, 20mer
   (xi) SEQUENCE DESCRIPTION: SEQ ID NO:25:
   CACATGGCTG AATATCGACG 20
(2) INFORMATION FOR SEQ ID NO:26:
   (i) SEQUENCE CHARACTERISTICS:
      (A) LENGTH: 180 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 4E3
   (xi) SEQUENCE DESCRIPTION: SEQ ID NO:26:
   GCGAGCCTAG TCTTTGACTT CATGGCGGGG AAACTTTCAT CAGAAGATCT GTGGTATGCC 60
   ATCCCGGTAC TGACCAGCCC GGGGGCGGGC CTTGCGGGGA TCGCTCTCGG GTTGGTTTTG 120
   TATTCAGCTA ACAACTCTGG CACTACCACT TGGTTGAACC GTCTGCTGAC TACGTTACCA 180
(2) INFORMATION FOR SEQ ID NO:27:
   (i) SEQUENCE CHARACTERISTICS:
      (A) LENGTH: 430 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 3E3
   (xi) SEQUENCE DESCRIPTION: SEQ ID NO:27:
   GGCACTACCA CTTGGTTGAA CCGTCTGCTG ACTACGTTAC CAAGGTCTTC ATGTATCCCG 60
   GACAGTTACT TTCAGCAAGT TGACTATTGC GACAAGGTCT CAGCCGTGCT CCGGCGCCTG 120
   AGCCTCACCC GCACAGTGGT TGCCCTGGTC AACAGGGAGC CTAAGGTGGA TGAGGTACAG 180
   GTGGGGTATG TCTGGGACCT GTGGGAGTGG ATCATGCGCC AAGTGCGCGT GGTCATGGCC 240
   AGACTCAGGG CCCTCTGCCC CGTGGTGTCA CTACCCTTGT GGCATTGCGG GGAGGGGTGG 300
   TCCGGGGAAT GGTTGCTTGA CGGTCATGTT GAGAGTCGCT GCCTCTGTGG CTGCGTGATC 360
   ACTGGTGACG TTCTGAATGG GCAACTCAAA GAACCAGTTT ACTCTACCAA GCTGTGCCGG 420
   CACTATTGGA 430
(2) INFORMATION FOR SEQ ID NO:28:
   (i) SEQUENCE CHARACTERISTICS:
      (A) LENGTH: 180 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 2E5
   (xi) SEQUENCE DESCRIPTION: SEQ ID NO:28:
   CTTACCGTCA AGATGTCGTG CTGCGTTGAA AAGAGCGTCA CGCGCTTTTT CTCATTGGGG      60
   TTGACGGTGG CTGATGTTGC TAGCCTGTGT GAGATGGAAA TCCAGAACCA TACAGCCTAT     120
   TGTGACCAGG TGCGCACTCC GCTTGAATTG CAGGTTGGGT GCTTGGTGGG CAATGAACTT     180
(2) INFORMATION FOR SEQ ID NO:29:
   (i) SEQUENCE CHARACTERISTICS:
      (A) LENGTH: 344 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 1E5
   (xi) SEQUENCE DESCRIPTION: SEQ ID NO:29:
   CTTCTCTTTG TGGTAGTAGC CGAGAGATGC CTGTATGGGG AGAAGACATC CCCCGTACTC 60
   CATCGCCAGC ACTTATCTCG GTTACTGAGA GCAGCTCAGA TGAGAAGACC CCGTCGGTGT 120
   CCTCCTCGCA GGAGGATACC CCGTCCTCTG ACTCATTCGA GGTCATCCAA GAGTCCGAGA 180
   CAGCCGAAGG GGAGGAAAGT GTCTTCAACG TGGCTCTTTC CGTATTAAAA GCCTTATTTC 240
   CACAGAGCGA CGCGACCAGG AAGCTTACCG TCAAGATGTC GTGCTGCGTT GAAAAGAGCG 300
   TCACGCGCTT TTTCTCATTG GGGTTGACGG TGGCTGATGT TGCT 344
(2) INFORMATION FOR SEQ ID NO:30:
   (i) SEQUENCE CHARACTERISTICS:
      (A) LENGTH: 423 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 4E5
   (xi) SEQUENCE DESCRIPTION: SEQ ID NO:30:
   GTAAGGCCAC ATGCTGCCAT GGGCTGGGGA TCTAAGGTGT CGGTTAAGGA CTTAGCCACC 60
   CCCGCGGGGA AGATGGCCGT CCATGACCGG CTTCAGGAGA TACTTGAAGG GACTCCGGTC 120
   CCCTTTACTC TTACTGTGAA AAAGGAGGTG TTCTTCAAAG ACCGGAAGGA GGAGAAGGCC 180
   CCCCGCCTCA TTGTGTTCCC CCCCCTGGAC TTCCGGATAG CTGAAAAGCT CATCTTGGGA 240
   GACCCAGGCC GGGTAGCCAA GGCGGTGTTG GGGGGGGCCT ACGCCTTCCA GTACACCCCA 300
   AATCAGCGAG TTAAGGAGAT GCTCAAGCTA TGGGAGTCTA AGAAGACCCC TTGCGCCATC 360
   TGTGTGGACG CCACCTGCTT CGACAGTAGC ATAACTGAAG AGGACGTGGC TTTGGAGACA 420
   GAG 423
(2) INFORMATION FOR SEQ ID NO:31:
   (i) SEQUENCE CHARACTERISTICS:
      (A) LENGTH: 516 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 3E5
   (xi) SEQUENCE DESCRIPTION: SEQ ID NO:31:
   TACAGCCTAT TGTGACCAGG TGCGCACTCC GCTTGAATTG CAGGTTGGGT GCTTGGTGGG 60
   CAATGAACTT ACCTTTGAAT GTGACAAGTG TGAGGCTAGG CAAGAAACCT TGGCCTCCTT 120
   CTCTTACATT TGGTCTGGAG TGCCGCTGAC TAGGGCCACG CCGGCCAAGC CTCCCGTGGT 180
   GAGGCCGGTT GGCTCTTTGT TAGTGGCCGA CACTACTAAG GTGTATGTTA CCAATCCAGA 240
   CAATGTGGGA CGGAGGGTGG ACAAGGTGAC CTTCTGGCGT GCTCCTAGGG TTCATGATAA 300
   GTACCTCGTG GACTCTATTG AGCGCGCTAA GAGGGCCGCT CAAGCCTGCC TAAGCATGGG 360
   TTACACTTAT GAGGAAGCAA TAAGGACTGT AAGGCCACAT GCTGCCATGG GCTGGGGATC 420
   TAAGGTGTCG GTTAAGGACT TAGCCACCCC CGCGGGGAAG ATGGCCGTCC ATGACCGGCT 480
   TCAGGAGATA CTTGAAGGGA CTCCGGTCCC CTTTAC 516
(2) INFORMATION FOR SEQ ID NO:32:
   (i) SEQUENCE CHARACTERISTICS:
      (A) LENGTH: 518 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 2E3
   (xi) SEQUENCE DESCRIPTION: SEQ ID NO:32:
   GAATGGGCAA CTCAAAGAAC CAGTTTACTC TACCAAGCTG TGCCGGCACT ATTGGATGGG    60
   GACTGTCCCT GTGAACATGC TGGGTTACGG TGAAACGTCG CCTCTCCTGG CCTCCGACAC   120
   CCCGAAGGTT GTGCCCTTCG GGACGTCTGG CTGGGCTGAG GTGGTGGTGA CCACTACCCA   180
   CGTGGTAATC AGGAGGACCT CCGCCTATAA GCTGCTGCGC CAGCAAATCC TATCGGCTGC   240
   TGTAGCTGAG CCCTACTACG TCGACGGCAT TCCGGTCTCA TGGGACGCGG ACGCTCGTGC   300
   GCCCGCCATG GTCTATGGCC CTGGGCAAAG TGTTACCATT GACGGGGAGC GCTACACCTT   360
   GCCTCATCAA CTGAGGCTCA GGAATGTGGC ACCCTCTGAG GTTTCATCCG AGGTGTCCAT   420
   TGACATTGGG ACGGAGACTG AAGACTCAGA ACTGACTGAG GCCGATCTGC CGCCGGCGGC   480
   TGCTGCTCTC CAAGCGATCG AGAATGCTGC GAGGATTC                           518
(2) INFORMATION FOR SEQ ID NO:33:
   (i) SEQUENCE CHARACTERISTICS:
      (A) LENGTH: 268 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 1E3
   (xi) SEQUENCE DESCRIPTION: SEQ ID NO:33:
   CTTACTGAGG CCGATCTGCC GCCGGCGGCT GCTGCTCTCC AAGCGATCGA GAATGCTGCG 60
   AGGATTCTTG AACCGCACAT TGATGTCATC ATGGAGGACT GCAGTACACC CTCTCTTTGT 120
   GGTAGTAGCC GAGAGATGCC TGTATGGGGA GAAGACATCC CCCGTACTCC ATCGCCAGCA 180
   CTTATCTCGG TTACTGAGAG CAGCTCAGAT GAGAAGACCC CGTCGGTGTC CTCCTCGCAG 240
   GAGGATACCC CGTCCTCTGA CTCATTCG 268
(2) INFORMATION FOR SEQ ID NO:34:
   (i) SEQUENCE CHARACTERISTICS:
      (A) LENGTH: 781 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: INDIVIDUAL CLONE 4E5-20
   (xi) SEQUENCE DESCRIPTION: SEQ ID NO:34:
   GTAAGGCCAC ATGCTGCCAT GGGCTGGGGA TCTAAGGTGT CGGTTAAGGA CTTAGCCACC 60
   CCCGCGGGGA AGATGGCCGT CCATGACCGG CTTCAGGAGA TACTTGAAGG GACTCCGGTC 120
   CCCTTTACTC TTACTGTGAA AAAGGAGGTG TTCTTCAAAG ACCGGAAGGA GGAGAAGGCC 180
   CCCCGCCTCA TTGTGTTCCC CCCCCTGGAC TTCCGGATAG CTGAAAAGCT CATCTTGGGA 240
   GACCCAGGCC GGGTAGCCAA GGCGGTGTTG GGGGGGGCCT ACGCCTTCCA GTACACCCCA 300
   AATCAGCGAG TTAAGGAGAT GCTCAAGCTA TGGGAGTCTA AGAAGACCCC TTGCGCCATC 360
   TGTGTGGACG CCACCTGCTT CGACAGTAGC ATAACTGAAG AGGACGTGGC TTTGGAGACA 420
   GAGTTATACG CTCTGGCCTC TGACCATCCA GAATGGGTGC GGGCACCTGG GAAATACTAT 480
   GCCTCAGGCA CCATGGTCAC CCCGGAAGGG GTGCCCGTCG GTGAGAGGTA TTGCAGATCC 540
   TCGGGTGTCC TAACAACTAG CGCGAGCAAC TGCCTGACCT GCTACATCAA GGTGAAAGCT 600
   GCCTGTGAGA GAGTGGGGCT GAAAAATGTC TCTCTTCTCA TAGCCGGCGA TGACTGCTTG 660
   ATCATATGTG AGCGGCCAGT GTGCGACCCA AGCGACGCTT TGGGCAGAGC CCTAGCGAGC 720
   TATGGGTACG CGTGCGAGCC CTCATATCAT GCATCATTGG ACACGGCCCC CTTCTGCTCC 780
   A 781
(2) INFORMATION FOR SEQ ID NO:35:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PROBE 470-201-1-142R
   (xi) SEQUENCE DESCRIPTION: SEQ ID NO:35:
   TCGGTTACTG AGAGCAGCTC AGATGAG 27
(2) INFORMATION FOR SEQ ID NO:36:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PROBE 470-20-1-152F
   (xi) SEQUENCE DESCRIPTION: SEQ ID NO:36:
   TCGGTTACTG AGAGCAGCTC AGATGAG 27
(2) INFORMATION FOR SEQ ID NO:37:
   (i) SEQUENCE CHARACTERISTICS:
      (A) LENGTH: 570 base pairs
      (B) TYPE: nucleic acid
      (C) STRANDEDNESS: double
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Clone 470EXP1
   (ix) FEATURE:
      (A) NAME/KEY: CDS
      (B) LOCATION: 1..570
   (xi) SEQUENCE DESCRIPTION: SEQ ID NO:37:
   GCT GTA TGG TTC TGG ATT TCC ATC TCA CAC AGG CTA GCA ACA TCA GCC          48
   Ala Val Trp Phe Trp Ile Ser Ile Ser His Arg Leu Ala Thr Ser Ala
     1               5                  10                  15
   ACC GTC AAC CCC AAT GAG AAA AAG CGC GTG ACG CTC TTT TCA ACG CAG          96
   Thr Val Asn Pro Asn Glu Lys Lys Arg Val Thr Leu Phe Ser Thr Gln
                20                  25                  30
   CAC GAC ATC TTG ACG GTA AGC TTC CTG GTC GCG TCG CTC TGT GGA AAT         144
   His Asp Ile Leu Thr Val Ser Phe Leu Val Ala Ser Leu Cys Gly Asn
           35                  40                  45
   AAG GCT TTT AAT ACG GAA AGA GCC ACG TTG AAG ACA CTT TCC TCC CCT         192
   Lys Ala Phe Asn Thr Glu Arg Ala Thr Leu Lys Thr Leu Ser Ser Pro
        50                  55                  60
   TCG GCT GTC TCG GAC TCT TGG ATG ACC TCG AAT GAG TCA GAG GAC GGG         240
   Ser Ala Val Ser Asp Ser Trp Met Thr Ser Asn Glu Ser Glu Asp Gly
    65                  70                  75                  80
   GTA TCC TCC TGC GAG GAG GAC ACC GAC GGG GTC TTC TCA TCT GAG CTG         288
   Val Ser Ser Cys Glu Glu Asp Thr Asp Gly Val Phe Ser Ser Glu Leu
                    85                  90                  95
   CTC TCA GTA ACC GAG ATA AGT GCT GGC GAT GGA GTA CGG GGG ATG TCT         336
   Leu Ser Val Thr Glu Ile Ser Ala Gly Asp Gly Val Arg Gly Met Ser
               100                 105                 110
   TCT CCC CAT ACA GGC ATC TCT CGG CTA CTA CCA CAA AGA GAG GGT GTA         384
   Ser Pro His Thr Gly Ile Ser Arg Leu Leu Pro Gln Arg Glu Gly Val
           115                 120                 125
   CTG CAG TCC TCC ATG ATG ACA TCA ATG TGC GGT TCA AGA ATC CTC GCA         432
   Leu Gln Ser Ser Met Met Thr Ser Met Cys Gly Ser Arg Ile Leu Ala
       130                 135                 140
   GCA TTC TCG ATC GCT TGG AGA GCA GCA GCC GCC GGC GGC AGA TCG GCC         480
   Ala Phe Ser Ile Ala Trp Arg Ala Ala Ala Ala Gly Gly Arg Ser Ala
   145                 150                 155                 160
   TCA GTC AGT TCT GAG TCT TCA GTC TCC GTC CCA ATG TCA ATG GAC ACC         528
   Ser Val Ser Ser Glu Ser Ser Val Ser Val Pro Met Ser Met Asp Thr
                   165                 170                 175
   TCG GAT GAA ACC TCA GAG GGT GCC ACA TTC CTG AGC CTC AGT                 570
   Ser Asp Glu Thr Ser Glu Gly Ala Thr Phe Leu Ser Leu Ser
               180                 185                 190
(2) INFORMATION FOR SEQ ID NO:38:
   (i) SEQUENCE CHARACTERISTICS:
      (A) LENGTH: 190 amino acids
      (B) TYPE: amino acid
      (D) TOPOLOGY: linear
   (ii) MOLECULE TYPE: protein
   (xi) SEQUENCE DESCRIPTION: SEQ ID NO:38:
   Ala Val Trp Phe Trp Ile Ser Ile Ser His Arg Leu Ala Thr Ser Ala
     1               5                  10                  15
   Thr Val Asn Pro Asn Glu Lys Lys Arg Val Thr Leu Phe Ser Thr Gln
                20                  25                  30
   His Asp Ile Leu Thr Val Ser Phe Leu Val Ala Ser Leu Cys Gly Asn
            35                  40                  45
   Lys Ala Phe Asn Thr Glu Arg Ala Thr Leu Lys Thr Leu Ser Ser Pro
        50                  55                  60
   Ser Ala Val Ser Asp Ser Trp Met Thr Ser Asn Glu Ser Glu Asp Gly
    65                  70                  75                  80
   Val Ser Ser Cys Glu Glu Asp Thr Asp Gly Val Phe Ser Ser Glu Leu
                    85                  90                  95
   Leu Ser Val Thr Glu Ile Ser Ala Gly Asp Gly Val Arg Gly Met Ser
               100                 105                 110
   Ser Pro His Thr Gly Ile Ser Arg Leu Leu Pro Gln Arg Glu Gly Val
           115                 120                 125
   Leu Gln Ser Ser Met Met Thr Ser Met Cys Gly Ser Arg Ile Leu Ala
       130                 135                 140
   Ala Phe Ser Ile Ala Trp Arg Ala Ala Ala Ala Gly Gly Arg Ser Ala
   145                 150                 155                 160
   Ser Val Ser Ser Glu Ser Ser Val Ser Val Pro Met Ser Met Asp Thr
                   165                 170                 175
   Ser Asp Glu Thr Ser Glu Gly Ala Thr Phe Leu Ser Leu Ser
               180                 185                 190
(2) INFORMATION FOR SEQ ID NO:39:
   (i) SEQUENCE CHARACTERISTICS:
      (A) LENGTH: 1288 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 5E3
   (xi) SEQUENCE DESCRIPTION: SEQ ID NO:39:
   ACGGGTAGGG GCAGGTCTGG ACGCTACTAC TACGCGGGGG TGGGCAAAGC CCCTGCGGGT 60
   GTGGTGCGCT CAGGTCCTGT CTGGTCGGCG GTGGAAGCTG GAGTGACCTG GTACGGAATG 120
   GAACCTGACT TGACAGCTAA CCTACTGAGA CTTTACGACG ACTGCCCTTA CACCGCAGCC 180
   GTCGCGGCTG ATATCGGAGA AGCCGCGGTG TTCTTCTCTG GGCTCGCCCC ATTGAGGATG 240
   CACCCTGATG TCAGCTGGGC AAAAGTTCGC GGCGTCAACT GGCCCCTCTT GGTGGGTGTT 300
   CAGCGGACCA TGTGTCGGGA AACACTGTCT CCCGGCCCAT CGGATGACCC CCAATGGGCA 360
   GGTCTGAAGG GCCCAAATCC TGTCCCACTC CTGCTGAGGT GGGGCAATGA TTTACCATCT 420
   AAAGTGGCCG GCCACCACAT AGTGGACGAC CTGGTCCGGA GACTCGGTGT GGCGGAGGGT 480
   TACGTCCGCT GCGACGCTGG GCCGATCTTG ATGATCGGTC TAGCTATCGC GGGGGGAATG 540
   ATCTACGCGT CATACACCGG GTCGCTAGTG GTGGTGACAG ACTGGGATGT GAAGGGGGGT 600
   GGCGCCCCCC TTTATCGGCA TGGAGACCAG GCCACGCCTC AGCCGGTGGT GCAGGTTCCT 660
   CCGGTAGACC ATCGGCCGGG GGGTGAATCA GCACCATCGG ATGCCAAGAC AGTGACAGAT 720
   GCGGTGGCAG CCATCCAGGT GGACTGCGAT TGGACTATCA TGACTCTGTC GATCGGAGAA 780
   GTGTTGTCCT TGGCTCAGGC TAAGACGGCC GAGGCCTACA CAGCAACCGC CAAGTGGCTC 840
   GCTGGCTGCT ATACGGGGAC GCGGGCCGTT CCCACTGTAT CCATTGTTGA CAAGCTCTTC 900
   GCCGGAGGGT GGGCGGCTGT GGTGGGCCAT TGCCACAGCG TGATTGCTGC GGCGGTGGCG 960
   GCCTACGGGG CTTCAAGGAG CCCGCCGTTG GCAGCCGCGG CTTCCTACCT GATGGGGTTG 1020
   GGCGTTGGAG GCAACGCTCA GACGCGCCTG GCGTCTGCCC TCCTATTGGG GGCTGCTGGA 1080
   ACCGCCTTGG GCACTCCTGT CGTGGGCTTG ACCATGGCAG GTGCGTTCAT GGGGGGGGCC 1140
   AGTGTCTCCC CCTCCTTGGT CACCATTTTA TTGGGGGCCG TCGGAGGTTG GGAGGGTGTT 1200
   GTCAACGCGG CGAGCCTAGT CTTTGACTTC ATGGCGGGGA AACTTTCATC AGAAGATCTG 1260
   TGGTATGCCA TCCCGGTACT GACCAGCC 1288
(2) INFORMATION FOR SEQ ID NO:40:
   (i) SEQUENCE CHARACTERISTICS:
      (A) LENGTH: 862 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 6E3
   (xi) SEQUENCE DESCRIPTION: SEQ ID NO:40:
   ACGGCAACAT GGGGCACAAG GTCTTAATCT TGAACCCCTC AGTGGCCACT GTGCGGGCCA 60
   TGGGCCCGTA CATGGAGCGG CTGGCGGGTA AACATCCAAG TATATACTGT GGGCATGATA 120
   CAACTGCTTT CACAAGGATC ACTGACTCCC CCCTGACGTA TTCAACCTAT GGGAGGTTTT 180
   TGGCCAACCC TAGGCAGATG CTACGGGGCG TTTCGGTGGT CATTTGTGAT GAGTGCCACA 240
   GTCATGACTC AACCGTGCTG TTAGGCATTG GGAGAGTTCG GGAGCTGGCG CGTGGGTGCG 300
   GAGTGCAACT AGTGCTCTAC GCCACCGCTA CACCTCCCGG ATCCCCTATG ACGCAGCACC 360
   CTTCCATAAT TGAGACAAAA TTGGACGTGG GCGAGATTCC CTTTTATGGG CATGGAATAC 420
   CCCTCGAGCG GATGCGAACC GGAAGGCACC TCGTGTTCTG CCATTCTAAG GCTGAGTGCG 480
   AGCGCCTTGC TGGCCAGTTC TCCGCTAGGG GGGTCAATGC CATTGCCTAT TATAGGGGTA 540
   AAGACAGTTC TATCATCAAG GATGGGGACC TGGTGGTCTG TGCTACAGAC GCGCTTTCCA 600
   CTGGGTACAC TGGAAATTTC GACTCCGTCA CCGACTGTGG ATTAGTGGTG GAGGAGGTCG 660
   TTGAGGTGAC CCTTGATCCC ACCATTACCA TCTCCCTGCG GACAGTGCCT GCGTCGGCTG 720
   AACTGTCGAT GCAAAGACGA GGACGCACGG GTAGGGGCAG GTCTGGACGC TACTACTACG 780
   CGGGGGTGGG CAAAGCCCCT GCGGGTGTGG TGCGCTCAGG TCCTGTCTGG TCGGCGGTGG 840
   AAGCTGGAGT GACCTCGTAC GG 862
(2) INFORMATION FOR SEQ ID NO:41:
   (i) SEQUENCE CHARACTERISTICS:
      (A) LENGTH: 865 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Individual Clone GE3L-11
   (xi) SEQUENCE DESCRIPTION: SEQ ID NO:41:
   AGTACGGCAA CATGGGGCAC AAGGTCTTAA TCTTGAACCC CTCAGTGGCC ACTGTGCGGG 60
   CCATGGGCCC GTACATGGAG CGGCTGGCGG GTAAACATCC AAGTATATAC TGTGGGCATG 120
   ATACAACTGC TTTCACAAGG ATCACTGACT CCCCCCTGAC GTATTCAACC TATGGGAGGT 180
   TTTTGGCCAA CCCTAGGCAG ATGCTACGGG GCGTTTCGGT GGTCATTTGT GATGAGTGCC 240
   ACAGTCATGA CTCAACCGTG CTGTTAGGCA TTGGGAGAGT CCGGGAGCTG GCGCGTGGGT 300
   GCGGGGTGCA ACTAGTGCTC TACGCCACCG CTACACCTCC CGGATCCCCT ATGACGCAGC 360
   ACCCTTCCAT AATTGAGACA AAATTGGACG TGGGCGAGAT TCCCTTTTAT GGACATGGAA 420
   TACCCCTCGA GCGGATGCGA ACCGGAAGGC ACCTCGTGTT CTGCCATTCT AAGGCTGAGT 480
   GCGAGCGCCT TGCTGGCCAG TTCTCCGCTA GGGGGGTCAA TGCCATTGCC TATTATAGGG 540
   GTAAAGACAG TTCTATCATC AAGGATGGGG ACCTGGTGGT CTGTGCTACA GACGCGCTTT 600
   CCACTGGGTA CACTGGAAAT TTCGACTCCG TCACCGACTG TGGATTAGTG GTGGAGGAGG 660
   TCGTTGAGGT GACCCTTGAT CCCACCATTA CCATCTCCCT GCGGACAGTG CCTGCGTCGG 720
   CTGAACTGTC GATGCAAAGA CGAGGACGCA CGGGTAGGGG CAGGTCTGGA CGCTACTACT 780
   ACGCGGGGGT GGGCAAAGCC CCTGCGGGTG TGGTGCGCTC AGGTCCTGTC TGGTCGGCGG 840
   TGGAAGCTGG AGTGACCTCG TACGG 865
(2) INFORMATION FOR SEQ ID NO:42:
   (i) SEQUENCE CHARACTERISTICS:
      (A) LENGTH: 596 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 7E3
   (xi) SEQUENCE DESCRIPTION: SEQ ID NO:42:
   AGCATGGGAA CATGCTTGAA CGGCCTGCTG TTCACGACCT TCCATGGGGC TTCATCCCGA 60
   ACCATCGCCA CACCCGTGGG GGCCCTTAAT CCCAGATGGT GGTCAGCCAG TGATGATGTC 120
   ACGGTGTATC CACTCCCGGA TGGGGCTACT TCGTTAACAC CTTGTACTTG CCAGGCTGAG 180
   TCCTGTTGGG TCATCAGATC CGACGGGGCC CTATGCCATG GCTTGAGCAA GGGGGACAAG 240
   GTGGAGCTGG ATGTGGCCAT GGAGGTCTCT GACTTCCGTG GCTCGTCTGG CTCACCGGTC 300
   CTATGTGACG AAGGGCACGC AGTAGGAATG CTCGTGTCTG TGCTTCACTC CGGTGGTAGG 360
   GTCACCGCGG CACGGTTCAC TAGGCCGTGG ACCCAAGTGC CAACAGATGC CAAAACCACT 420
   ACTGAACCCC CTCCGGTGCC GGCCAAAGGA GTTTTCAAAG AGGCCCCGTT GTTTATGCCT 480
   ACGGGAGCGG GAAAGAGCAC TCGCGTCCCG TTGGAGTACG ATAACATGGG GCACAAGGTC 540
   TTAATCTTGA ACCCCTCAGT GGCCACTGTG CGGGCCATGG GCCCGTACAT GGAGCG 596
(2) INFORMATION FOR SEQ ID NO:43:
   (i) SEQUENCE CHARACTERISTICS:
      (A) LENGTH: 586 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 5E5
   (xi) SEQUENCE DESCRIPTION: SEQ ID NO:43:
   GAGCTATGGG TACGCGTGCG AGCCCTCATA TCATGCATCA TTGGACACGG CCCCCTTCTG 60
   CTCCACTTGG CTTGCTGAGT GCAATGCAGA TGGGAAGCGC CATTTCTTCC TGACCACGGA 120
   CTTCCGGAGG CCGCTCGCTC GCATGTCGAG TGAGTATAGT GACCCGATGG CTTCGGCGAT    180
   CGGTTACATC CTCCTTTATC CTTGGCACCC CATCACACGG TGGGTCATCA TCCCTCATGT    240
   GCTAACGTGC GCATTCAGGG GTGGAGGCAC ACCGTCTGAT CCGGTTTGGT GCCAGGTGCA    300
   TGGTAACTAC TACAAGTTTC CACTGGACAA ACTGCCTAAC ATCATCGTGG CCCTCCACGG    360
   ACCAGCAGCG TTGAGGGTTA CCGCAGACAC AACTAAAACA AAGATGGAGG CTGGTAAGGT    420
   TCTGAGCGAC CTCAAGCTCC CTGGCTTAGC AGTCCACCGA AAGAAGGCCG GGGCGTTGCG    480
   AACACGCATG CTCCGCTCGC GCGGTTGGGC TGAGTTGGCT AGGGGCTTGT TGTGGCATCC    540
   AGGCCTACGG CTTCCTCCCC CTGAGATTGC TGGTATCCCG GGGGGT                   586
(2) INFORMATION FOR SEQ ID NO:44:
   (i) SEQUENCE CHARACTERISTICS:
      (A) LENGTH: 242 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 6E5 (44F)
   (xi) SEQUENCE DESCRIPTION: SEQ ID NO:44:
   CGAACGCGCA TGCTCCGCTC GCGCGGTTGG GCTGAGTTGG CTAGGGGCTT GTTGTGGCAT     60
   CCAGGCCTAC GGCTTCCTCC CCCTGAGATT GCTGGTATCC CGGGGGGTTT CCCTCTCTCC    120
   CCCCCCTATA TGGGGGTGGT ACACCAATTG GATTTCACAA GCCAGAGGAG TCGCTGGCGG    180
   TGGTTGGGGT TCTTAGCCCT GCTCATCGTA GCCCTCTTCG GGTGAACTAA ATTCATCTGT    240
   TG                                                                   242
(2) INFORMATION FOR SEQ ID NO:45:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer Gtll rev-JL
   (xi) SEQUENCE DESCRIPTION: SEQ ID NO:45:
   TGGTAATGGT AGCGACCGGC GCTCAGC 27
(2) INFORMATION FOR SEQ ID NO:46:
   (i) SEQUENCE CHARACTERISTICS:
      (A) LENGTH: 45 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE-3F
   (xi) SEQUENCE DESCRIPTION: SEQ ID NO:46:
   GCCGCCATGG TCTCATGGGA CGCGGACGCT CGTGCGCCCG CGATG 45
(2) INFORMATION FOR SEQ ID NO:47:
   (i) SEQUENCE CHARACTERISTICS:
      (A) LENGTH: 34 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE-3R
   (xi) SEQUENCE DESCRIPTION: SEQ ID NO:47:
   GCGCGGATCC GATAAGTGCT GGCGATGGAG TACG 34
(2) INFORMATION FOR SEQ ID NO:48:
   (i) SEQUENCE CHARACTERISTICS:
      (A) LENGTH: 22 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE-9F
   (xi) SEQUENCE DESCRIPTION: SEQ ID NO:48:
   GGCACCATGG TCACCCCGGA AG 22
(2) INFORMATION FOR SEQ ID NO:49:
   (i) SEQUENCE CHARACTERISTICS:
      (A) LENGTH: 28 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE-9R
   (xi) SEQUENCE DESCRIPTION: SEQ ID NO:49:
   GCTCGGATCC GGAGCAGAAG GGGGCCGT 28
(2) INFORMATION FOR SEQ ID NO:50:
   (i) SEQUENCE CHARACTERISTICS:
      (A) LENGTH: 364 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: GE3-2
   (ix) FEATURE:
      (A) NAME/KEY: CDS
      (B) LOCATION: 2..364
   (xi) SEQUENCE DESCRIPTION: SEQ ID NO:50:
   G GTC TCA TGG GAC GCG GAC GCT CGT GCG CCC GCG ATG GTC TAT GGC          46
     Val Ser Trp Asp Ala Asp Ala Arg Ala Pro Ala Met Val Tyr Gly
       1               5                  10                  15
   CCT GGG CAA AGT GTT ACC ATT GAC GGG GAG CGC TAC ACC TTG CCT CAT        94
   Pro Gly Gln Ser Val Thr Ile Asp Gly Glu Arg Tyr Thr Leu Pro His
                       20                  25                  30
   CAA CTG AGG CTC AGG AAT GTG GCA CCC TCT GAG GTT TCA TCC GAG GTG       142
   Gln Leu Arg Leu Arg Asn Val Ala Pro Ser Glu Val Ser Ser Glu Val
                   35                  40                  45
   TCC ATT GAC ATT GGG ACG GAG ACT GAA GAC TCA GAA CTG ACT GAG GCC       190
   Ser Ile Asp Ile Gly Thr Glu Thr Glu Asp Ser Glu Leu Thr Glu Ala
               50                  55                  60
   GAT CTG CCG CCG GCG GCT GCT GCT CTC CAA GCG ATC GAG AAT GCT GCG       238
   Asp Leu Pro Pro Ala Ala Ala Ala Leu Gln Ala Ile Glu Asn Ala Ala
           65                  70                  75
   AGG ATT CTT GAA CCG CAC ATT GAT GTC ATC ATG GAG GAC TGC AGT ACA       286
   Arg Ile Leu Glu Pro His Ile Asp Val Ile Met Glu Asp Cys Ser Thr
    80                  85                  90                  95
   CCC TCT CTT TGT GGT AGT AGC CGA GAG ATG CCT GTA TGG GGA GAA GAC       334
   Pro Ser Leu Cys Gly Ser Ser Arg Glu Met Pro Val Trp Gly Glu Asp
                      100                 105                 110
   ATC CCC CGT ACT CCA TCG CCA GCA CTT ATC                               364
   Ile Pro Arg Thr Pro Ser Pro Ala Leu Ile
                  115                 120
(2) INFORMATION FOR SEQ ID NO:51:
   (i) SEQUENCE CHARACTERISTICS:
      (A) LENGTH: 121 amino acids
      (B) TYPE: amino acid
      (D) TOPOLOGY: linear
   (ii) MOLECULE TYPE: protein
   (xi) SEQUENCE DESCRIPTION: SEQ ID NO:51:
   Val Ser Trp Asp Ala Asp Ala Arg Ala Pro Ala Met Val Tyr Gly Pro
     1               5                  10                  15
   Gly Gln Ser Val Thr Ile Asp Gly Glu Arg Tyr Thr Leu Pro His Gln
                20                  25                  30
   Leu Arg Leu Arg Asn Val Ala Pro Ser Glu Val Ser Ser Glu Val Ser
            35                  40                  45
   Ile Asp Ile Gly Thr Glu Thr Glu Asp Ser Glu Leu Thr Glu Ala Asp
        50                  55                  60
   Leu Pro Pro Ala Ala Ala Ala Leu Gln Ala Ile Glu Asn Ala Ala Arg
    65                  70                  75                  80
   Ile Leu Glu Pro His Ile Asp Val Ile Met Glu Asp Cys Ser Thr Pro
                    85                  90                  95
   Ser Leu Cys Gly Ser Ser Arg Glu Met Pro Val Trp Gly Glu Asp Ile
               100                 105                 110
   Pro Arg Thr Pro Ser Pro Ala Leu Ile
           115                 120
(2) INFORMATION FOR SEQ ID NO:52:
   (i) SEQUENCE CHARACTERISTICS:
      (A) LENGTH: 290 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone GE9-2
   (ix) FEATURE:
      (A) NAME/KEY: CDS
      (B) LOCATION: 3..290
   (xi) SEQUENCE DESCRIPTION: SEQ ID NO:52:
   CC ATG GTC ACC CCG GAA GGG GTG CCC GTT GGT GAG AGG TAT TGC AGA          47
      Met Val Thr Pro Glu Gly Val Pro Val Gly Glu Arg Tyr Cys Arg
        1               5                  10                  15
   TCC TCG GGT GTC CTA ACA ACT AGC GCG AGC AAC TGC TTG ACC TGC TAC          95
   Ser Ser Gly Val Leu Thr Thr Ser Ala Ser Asn Cys Leu Thr Cys Tyr
                     20                  25                  30
   ATC AAG GTG AAA GCC GCC TGT GAG AGG GTG GGG CTG AAA AAT GTC TCT         143
   Ile Lys Val Lys Ala Ala Cys Glu Arg Val Gly Leu Lys Asn Val Ser
                 35                  40                  45
   CTT CTC ATA GCC GGC GAT GAC TGC TTG ATC ATA TGT GAG CGG CCA GTG         191
   Leu Leu Ile Ala Gly Asp Asp Cys Leu Ile Ile Cys Glu Arg Pro Val
             50                  55                  60
   TGC GAC CCA AGC GAC GCT TTG GGC AGA GCC CTA GCG AGC TAT GGG TAC         239
   Cys Asp Pro Ser Asp Ala Leu Gly Arg Ala Leu Ala Ser Tyr Gly Tyr
        65                  70                  75
   GCG TGC GAG CCC TCA TAT TAT GCA TGC TCG GAC ACG GCC CCC TTC TGC         287
   Ala Cys Glu Pro Ser Tyr Tyr Ala Cys Ser Asp Thr Ala Pro Phe Cys
    80                  85                  90                  95
   TCC                                                                     290
   Ser
(2) INFORMATION FOR SEQ ID NO:53:
   (i) SEQUENCE CHARACTERISTICS:
      (A) LENGTH: 96 amino acids
      (B) TYPE: amino acid
      (D) TOPOLOGY: linear
   (ii) MOLECULE TYPE: protein
   (xi) SEQUENCE DESCRIPTION: SEQ ID NO:53:
   Met Val Thr Pro Glu Gly Val Pro Val Gly Glu Arg Tyr Cys Arg Ser
    1               5                  10                  15
   Ser Gly Val Leu Thr Thr Ser Ala Ser Asn Cys Leu Thr Cys Tyr Ile
               20                  25                  30
   Lys Val Lys Ala Ala Cys Glu Arg Val Gly Leu Lys Asn Val Ser Leu
           35                  40                  45
   Leu Ile Ala Gly Asp Asp Cys Leu Ile Ile Cys Glu Arg Pro Val Cys
       50                  55                  60
   Asp Pro Ser Asp Ala Leu Gly Arg Ala Leu Ala Ser Tyr Gly Tyr Ala
   65                  70                  75                  80
   Cys Glu Pro Ser Tyr Tyr Ala Cys Ser Asp Thr Ala Pro Phe Cys Ser
                   85                  90                  95
(2) INFORMATION FOR SEQ ID NO:54:
   (i) SEQUENCE CHARACTERISTICS:
      (A) LENGTH: 20 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: JML-A SISPA Primer
   (xi) SEQUENCE DESCRIPTION: SEQ ID NO:54:
   AGGAATTCAG CGGCCGCGAG                                          20
(2) INFORMATION FOR SEQ ID NO:55:
   (i) SEQUENCE CHARACTERISTICS:
      (A) LENGTH: 22 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: JML-B SISPA Primer
   (xi) SEQUENCE DESCRIPTION: SEQ ID NO:55:
   CTCGCGGCCG CTGAATTCCT TT 22
(2) INFORMATION FOR SEQ ID NO:56:
   (i) SEQUENCE CHARACTERISTICS:
      (A) LENGTH: 32 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470ep-f1 Primer
   (xi) SEQUENCE DESCRIPTION: SEQ ID NO:56:
   GCGAATTCGC CATGGCGGGG AGACTTTCAT CA 32
(2) INFORMATION FOR SEQ ID NO:57:
   (i) SEQUENCE CHARACTERISTICS:
      (A) LENGTH: 35 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470ep-R1 Primer
   (xi) SEQUENCE DESCRIPTION: SEQ ID NO:57:
   GCGAATTCGG ATCCAGGGCC ATAGACCATC GCGGG 35
(2) INFORMATION FOR SEQ ID NO:58:
   (i) SEQUENCE CHARACTERISTICS:
      (A) LENGTH: 26 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470ep-f2 Primer
   (xi) SEQUENCE DESCRIPTION: SEQ ID NO:58:
   GCGAATTCCG TGCGCCCGCC ATGGTC 26
(2) INFORMATION FOR SEQ ID NO:59:
   (i) SEQUENCE CHARACTERISTICS:
      (A) LENGTH: 32 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470ep-R3 Primer
   (xi) SEQUENCE DESCRIPTION: SEQ ID NO:59:
   GCGAATTCGG ATCCCAAGGT TTCTTGCCTA GC 32
(2) INFORMATION FOR SEQ ID NO:60:
   (i) SEQUENCE CHARACTERISTICS:
      (A) LENGTH: 26 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470ep-f4 Primer
   (xi) SEQUENCE DESCRIPTION: SEQ ID NO:60:
   GCGAATTCAA GTGTGAGGCT AGGCAA 26
(2) INFORMATION FOR SEQ ID NO:61:
   (i) SEQUENCE CHARACTERISTICS:
      (A) LENGTH: 35 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 470ep-R4 Primer
   (xi) SEQUENCE DESCRIPTION: SEQ ID NO:61:
   GCGAATTCGG ATCCCCACAC AGATGGCGCA AGGGG 35
(2) INFORMATION FOR SEQ ID NO:62:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: KL-1 SISPA Primer
   (xi) SEQUENCE DESCRIPTION: SEQ ID NO:62:
   GCAGGATCCG AATTCGCATC TAGAGAT 27
(2) INFORMATION FOR SEQ ID NO:63:
   (i) SEQUENCE CHARACTERISTICS:
      (A) LENGTH: 29 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: KL-2 SISPA Primer
   (xi) SEQUENCE DESCRIPTION: SEQ ID NO:63:
   ATCTCTAGAT GCGAATTCGG ATCCTGCGA 29
(2) INFORMATION FOR SEQ ID NO:64:
   (i) SEQUENCE CHARACTERISTICS:
      (A) LENGTH: 186 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-10
   (ix) FEATURE:
      (A) NAME/KEY: CDS
      (B) LOCATION: 1..186
   (xi) SEQUENCE DESCRIPTION: SEQ ID NO:64:
   CGT GCG CCC GCC ATG GTC TAT GGC CCT GGG CAA AGT GTT GCC ATT GAC          48
   Arg Ala Pro Ala Met Val Tyr Gly Pro Gly Gln Ser Val Ala Ile Asp
     1               5                  10                  15
   GGG GAG CGC TAC ACC TTG CCT CAT CAA CTG AGG CTC AGG AAT GTG GCA          96
   Gly Glu Arg Tyr Thr Leu Pro His Gln Leu Arg Leu Arg Asn Val Ala
                20                  25                  30
   CCC TCT GAG GTT TCA TCC GAG GTG TCC ATT GAC ATT GGG ACG GAG GCT         144
   Pro Ser Glu Val Ser Ser Glu Val Ser Ile Asp Ile Gly Thr Glu Ala
            35                  40                  45
   GAA AAC TCA GAA CTG ACT GAG GCC GAT CTG CCG CCG GCG GCT                 186
   Glu Asn Ser Glu Leu Thr Glu Ala Asp Leu Pro Pro Ala Ala
        50                  55                  60
(2) INFORMATION FOR SEQ ID NO:65:
   (i) SEQUENCE CHARACTERISTICS:
      (A) LENGTH: 62 amino acids
      (B) TYPE: amino acid
      (D) TOPOLOGY: linear
   (ii) MOLECULE TYPE: protein
   (xi) SEQUENCE DESCRIPTION: SEQ ID NO:65:
   Arg Ala Pro Ala Met Val Tyr Gly Pro Gly Gln Ser Val Ala Ile Asp
     1               5                  10                  15
   Gly Glu Arg Tyr Thr Leu Pro His Gln Leu Arg Leu Arg Asn Val Ala
                20                  25                  30
   Pro Ser Glu Val Ser Ser Glu Val Ser Ile Asp Ile Gly Thr Glu Ala
            35                  40                  45
   Glu Asn Ser Glu Leu Thr Glu Ala Asp Leu Pro Pro Ala Ala
        50                  55                  60
(2) INFORMATION FOR SEQ ID NO:66:
   (i) SEQUENCE CHARACTERISTICS:
      (A) LENGTH: 282 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-12
   (ix) FEATURE:
      (A) NAME/KEY: CDS
      (B) LOCATION: 1..282
   (xi) SEQUENCE DESCRIPTION: SEQ ID NO:66:
   CGT GCG CCC GCC ATG GTC TAT GGC CCT GGG CAA AGT GTT ACC ATT GAC        48
   Arg Ala Pro Ala Met Val Tyr Gly Pro Gly Gln Ser Val Thr Ile Asp
     1               5                  10                  15
   GGG GAG CGC TAC ACC TTG CCT CAT CAA CTG AGG CTC AGG AAT GTG GCA        96
   Gly Glu Arg Tyr Thr Leu Pro His Gln Leu Arg Leu Arg Asn Val Ala
                20                  25                  30
   CCC TCT GAG GTT TCA TCC GAG GTG TCC ATT GAC ATT GGG ACG GAG ACT       144
   Pro Ser Glu Val Ser Ser Glu Val Ser Ile Asp Ile Gly Thr Glu Thr
            35                  40                  45
   GAA GAC TCA GAA CTG ACT GAG GCC GAT CTG CCG CCG GCG GCT GCT GCT       192
   Glu Asp Ser Glu Leu Thr Glu Ala Asp Leu Pro Pro Ala Ala Ala Ala
        50                  55                  60
   CTC CAA GCG ATC GAG AAT GCT GCG AGG ATT CTT GAA CCG CAC ATT GAT       240
   Leu Gln Ala Ile Glu Asn Ala Ala Arg Ile Leu Glu Pro His Ile Asp
    65                  70                  75                  80
   GTC ATC ATG GAG GAC TGC AGT ACA CCC TCT CTT TGT GGT AGT               282
   Val Ile Met Glu Asp Cys Ser Thr Pro Ser Leu Cys Gly Ser
                    85                  90
(2) INFORMATION FOR SEQ ID NO:67:
   (i) SEQUENCE CHARACTERISTICS:
      (A) LENGTH: 94 amino acids
      (B) TYPE: amino acid
      (D) TOPOLOGY: linear
   (ii) MOLECULE TYPE: protein
   (xi) SEQUENCE DESCRIPTION: SEQ ID NO:67:
   Arg Ala Pro Ala Met Val Tyr Gly Pro Gly Gln Ser Val Thr Ile Asp
     1               5                  10                      15
   Gly Glu Arg Tyr Thr Leu Pro His Gln Leu Arg Leu Arg Asn Val Ala
                20                  25                  30
   Pro Ser Glu Val Ser Ser Glu Val Ser Ile Asp Ile Gly Thr Glu Thr
            35                  40                  45
   Glu Asp Ser Glu Leu Thr Glu Ala Asp Leu Pro Pro Ala Ala Ala Ala
        50                  55                  60
   Leu Gln Ala Ile Glu Asn Ala Ala Arg Ile Leu Glu Pro His Ile Asp
    65                  70                  75                  80
   Val Ile Met Glu Asp Cys Ser Thr Pro Ser Leu Cys Gly Ser
                    85                  90
(2) INFORMATION FOR SEQ ID NO:68:
   (i) SEQUENCE CHARACTERISTICS:
      (A) LENGTH: 279 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-26
   (ix) FEATURE:
      (A) NAME/KEY: CDS
      (B) LOCATION: 1..279
   (xi) SEQUENCE DESCRIPTION: SEQ ID NO:68:
   CGT GCG CCC GCC ATG GTC TAT GGC CCT GGG CAA AGT GTT TCC ATT GAC     48
   Arg Ala Pro Ala Met Val Tyr Gly Pro Gly Gln Ser Val Ser Ile Asp
     1               5                  10                  15
   GGG GAG CGC TAC ACC TTG CCT CAT CAA CTG AGG CTC AGG AAT GTG GCA     96
   Gly Glu Arg Tyr Thr Leu Pro His Gln Leu Arg Leu Arg Asn Val Ala
                20                  25                  30
   CCC TCT GAG GTT TCA TCC GAG GTG TCC ATT GAC ATT GGG ACG GAG ACT    144
   Pro Ser Glu Val Ser Ser Glu Val Ser Ile Asp Ile Gly Thr Glu Thr
            35                  40                  45
   GAA GAC TCA GAA CTG ACT GAG GCC GAC CTG CCG CCG GCG GCT GCT GCT    192
   Glu Asp Ser Glu Leu Thr Glu Ala Asp Leu Pro Pro Ala Ala Ala Ala
        50                  55                  60
   CTC CAA GCG ATC GAG AAT GCT GCG AGG ATT CTT GAA CCG CAC ATC GAT    240
   Leu Gln Ala Ile Glu Asn Ala Ala Arg Ile Leu Glu Pro His Ile Asp
    65                  70                  75                  80
   GTC ATC ATG GAG GAC TGC AGT ACA CCC TCT CTT TGT GGT                279
   Val Ile Met Glu Asp Cys Ser Thr Pro Ser Leu Cys Gly
                    85                  90
(2) INFORMATION FOR SEQ ID NO:69:
   (i) SEQUENCE CHARACTERISTICS:
      (A) LENGTH: 93 amino acids
      (B) TYPE: amino acid
      (D) TOPOLOGY: linear
   (ii) MOLECULE TYPE: protein
   (xi) SEQUENCE DESCRIPTION: SEQ ID NO:69:
   Arg Ala Pro Ala Met Val Tyr Gly Pro Gly Gln Ser Val Ser Ile Asp
    1               5                  10                  15
   Gly Glu Arg Tyr Thr Leu Pro His Gln Leu Arg Leu Arg Asn Val Ala
                20                  25                  30
   Pro Ser Glu Val Ser Ser Glu Val Ser Ile Asp Ile Gly Thr Glu Thr
            35                  40                  45
   Glu Asp Ser Glu Leu Thr Glu Ala Asp Leu Pro Pro Ala Ala Ala Ala
        50                  55                  60
   Leu Gln Ala Ile Glu Asn Ala Ala Arg Ile Leu Glu Pro His Ile Asp
    65                  70                  75                  80
   Val Ile Met Glu Asp Cys Ser Thr Pro Ser Leu Cys Gly
                    85                  90
(2) INFORMATION FOR SEQ ID NO:70:
   (i) SEQUENCE CHARACTERISTICS:
      (A) LENGTH: 108 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-5
   (ix) FEATURE:
      (A) NAME/KEY: CDS
      (B) LOCATION: 1..108
   (xi) SEQUENCE DESCRIPTION: SEQ ID NO:70:
   GCC TAT TGT GAC AAG GTG CGC ACT CCG CTT GAA TTG CAG GTT GGG TGC        48
   Ala Tyr Cys Asp Lys Val Arg Thr Pro Leu Glu Leu Gln Val Gly Cys
     1               5                   10                  15
   TTG GTG GGC AAT GAA CTT ACC TTT GAA TGT GAC AAG TGT GAG GCT AGG        96
   Leu Val Gly Asn Glu Leu Thr Phe Glu Cys Asp Lys Cys Glu Ala Arg
               20                  25                  30
   CAA GAA ACC TTG                                                       108
   Gln Glu Thr Leu
           35
(2) INFORMATION FOR SEQ ID NO:71:
   (i) SEQUENCE CHARACTERISTICS:
      (A) LENGTH: 36 amino acids
      (B) TYPE: amino acid
      (D) TOPOLOGY: linear
   (ii) MOLECULE TYPE: protein
   (xi) SEQUENCE DESCRIPTION: SEQ ID NO:71:
   Ala Tyr Cys Asp Lys Val Arg Thr Pro Leu Glu Leu Gln Val Gly Cys
     1               5                  10                  15
   Leu Val Gly Asn Glu Leu Thr Phe Glu Cys Asp Lys Cys Glu Ala Arg
               20                  25                  30
   Gln Glu Thr Leu
           35
(2) INFORMATION FOR SEQ ID NO:72:
   (i) SEQUENCE CHARACTERISTICS:
      (A) LENGTH: 132 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-3
   (ix) FEATURE:
      (A) NAME/KEY: CDS
      (B) LOCATION: 1..132
   (xi) SEQUENCE DESCRIPTION: SEQ ID NO:72:
   GAG ATG GAA ATC CAG AAC CAT ACA GCC TAT TGT GAC AAG GTG CGC ACT        48
   Glu Met Glu Ile Gln Asn His Thr Ala Tyr Cys Asp Lys Val Arg Thr
     1               5                  10                  15
   CCG CTT GAA TTG CAG GTT GGG TGC TTG GTG GGC AAT GAA CTT ACC TTT        96
   Pro Leu Glu Leu Gln Val Gly Cys Leu Val Gly Asn Glu Leu Thr Phe
                20                  25                  30
   GAA TGT GAC AAG TGT GAG GCT AGG CAA GAA ACC TTG                       132
   Glu Cys Asp Lys Cys Glu Ala Arg Gln Glu Thr Leu
            35                  40
(2) INFORMATION FOR SEQ ID NO:73:
   (i) SEQUENCE CHARACTERISTICS:
      (A) LENGTH: 44 amino acids
      (B) TYPE: amino acid
      (D) TOPOLOGY: linear
   (ii) MOLECULE TYPE: protein
   (xi) SEQUENCE DESCRIPTION: SEQ ID NO:73:
   Glu Met Glu Ile Gln Asn His Thr Ala Tyr Cys Asp Lys Val Arg Thr
     1               5                  10                  15
   Pro Leu Glu Leu Gln Val Gly Cys Leu Val Gly Asn Glu Leu Thr Phe
                20                  25                  30
   Glu Cys Asp Lys Cys Glu Ala Arg Gln Glu Thr Leu
            35                  40
(2) INFORMATION FOR SEQ ID NO:74:
   (i) SEQUENCE CHARACTERISTICS:
      (A) LENGTH: 258 base pairs
      (B) TYPE: nucleic acid
      (C) STRANDEDNESS: both
      (D) TOPOLOGY: linear
   (ii) MOLECULE TYPE: CDNA
   (iii) HYPOTHETICAL: NO
   (iv) ANTI-SENSE: NO
   (vi) ORIGINAL SOURCE:
      (C) INDIVIDUAL ISOLATE: Clone Y5-27
   (ix) FEATURE:
      (A) NAME/KEY: CDS
      (B) LOCATION: 1..258
   (xi) SEQUENCE DESCRIPTION: SEQ ID NO:74:
   AAA GCC TTA TTT CCA CAG AGC GAC GCG ACC AGG AAG CTT ACC GTC AAG          48
   Lys Ala Leu Phe Pro Gln Ser Asp Ala Thr Arg Lys Leu Thr Val Lys
     1               5                  10                  15
   ATG TCA TGC TGC GTT GAA AAG AGC GTC ACG CGC TTT TTC TCA TTG GGG          96
   Met Ser Cys Cys Val Glu Lys Ser Val Thr Arg Phe Phe Ser Leu Gly
                    20                  25                  30
   TTG ACG GTG GCT GAT GTT GCT AGC CTG TGT GAG ATG GAA ATC CAG AAC         144
   Leu Thr Val Ala Asp Val Ala Ser Leu Cys Glu Met Glu Ile Gln Asn
                35                  40                  45
   CAT ATA GCC TAT TGT GAC AAG GTG CGC ACT CCG CTT GAA TTG CAG GTT         192
   His Ile Ala Tyr Cys Asp Lys Val Arg Thr Pro Leu Glu Leu Gln Val
            50                  55                  60
   GGG TGC TTG GTG GGC AAT GAA CTC ACC TTT GAA TGT GAC AAG TGT GAG         240
   Gly Cys Leu Val Gly Asn Glu Leu Thr Phe Glu Cys Asp Lys Cys Glu
    65                  70                  75                  80
   GCT AGG CAA GAA ACC TTG                                                 258
   Ala Arg Gln Glu Thr Leu
                    85
(2) INFORMATION FOR SEQ ID NO:75:
   (i) SEQUENCE CHARACTERISTICS:
      (A) LENGTH: 86 amino acids
      (B) TYPE: amino acid
      (D) TOPOLOGY: linear
   (ii) MOLECULE TYPE: protein
   (xi) SEQUENCE DESCRIPTION: SEQ ID NO:75:
   Lys Ala Leu Phe Pro Gln Ser Asp Ala Thr Arg Lys Leu Thr Val Lys
   1 5 10 15
   Met Ser Cys Cys Val Glu Lys Ser Val Thr Arg Phe Phe Ser Leu Gly
   20 25 30
   Leu Thr Val Ala Asp Val Ala Ser Leu Cys Glu Met Glu Ile Gln Asn
   35 40 45
   His Ile Ala Tyr Cys Asp Lys Val Arg Thr Pro Leu Glu Leu Gln Val
   50 55 60
   Gly Cys Leu Val Gly Asn Glu Leu Thr Phe Glu Cys Asp Lys Cys Glu
   65 70 75 80
   Ala Arg Gln Glu Thr Leu
   85
(2) INFORMATION FOR SEQ ID NO:76:
   (i) SEQUENCE CHARACTERISTICS:
      (A) LENGTH: 108 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-25
   (ix) FEATURE:
      (A) NAME/KEY: CDS
      (B) LOCATION: 1..108
   (xi) SEQUENCE DESCRIPTION: SEQ ID NO:76:
   ACC TAT TGT GAC AAG GTG CGC ACT CCG CTT GAA TTG CAG GTT GGG TGC          48
   Thr Tyr Cys Asp Lys Val Arg Thr Pro Leu Glu Leu Gln Val Gly Cys
     1               5                  10                  15
   TTG GTG GGC AAT GAA CTT ACC TTT GAA TGT GAC AAG TGT GAG GCT AGG          96
   Leu Val Gly Asn Glu Leu Thr Phe Glu Cys Asp Lys Cys Glu Ala Arg
                20                  25                  30
   CAA GAA ACC TTG                                                         108
   Gln Glu Thr Leu
            35
(2) INFORMATION FOR SEQ ID NO:77:
   (i) SEQUENCE CHARACTERISTICS:
      (A) LENGTH: 36 amino acids
      (B) TYPE: amino acid
      (D) TOPOLOGY: linear
   (ii) MOLECULE TYPE: protein
   (xi) SEQUENCE DESCRIPTION: SEQ ID NO:77:
   Thr Tyr Cys Asp Lys Val Arg Thr Pro Leu Glu Leu Gln Val Gly Cys
     1               5                  10                  15
   Leu Val Gly Asn Glu Leu Thr Phe Glu Cys Asp Lys Cys Glu Ala Arg
                20                  25                  30
   Gln Glu Thr Leu
            35
(2) INFORMATION FOR SEQ ID NO:78:
   (i) SEQUENCE CHARACTERISTICS:
      (A) LENGTH: 108 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-20
   (ix) FEATURE:
      (A) NAME/KEY: CDS
      (B) LOCATION: 52..108
   (xi) SEQUENCE DESCRIPTION: SEQ ID NO:78:
   GCCGACACTA CTAAGGTGTA TGTTACCAAT CCAGACAATG TGGGACGAAG G GTG GGC        57
                                                            Val Gly
                                                              1
   AAT GAA CTT ACC TTT GAA TGT GAC AAG TGT GAG GCT AGG CAA GAA ACC        105
   Asn Glu Leu Thr Phe Glu Cys Asp Lys Cys Glu Ala Arg Gln Glu Thr
           5                  10                  15
   TTG                                                                    108
   Leu
(2) INFORMATION FOR SEQ ID NO:79:
   (i) SEQUENCE CHARACTERISTICS:
      (A) LENGTH: 19 amino acids
      (B) TYPE: amino acid
      (D) TOPOLOGY: linear
   (ii) MOLECULE TYPE: protein
   (xi) SEQUENCE DESCRIPTION: SEQ ID NO:79:
   Val Gly Asn Glu Leu Thr Phe Glu Cys Asp Lys Cys Glu Ala Arg Gln
     1               5                  10                  15
   Glu Thr Leu
(2) INFORMATION FOR SEQ ID NO:80:
   (i) SEQUENCE CHARACTERISTICS:
      (A) LENGTH: 168 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-16
   (ix) FEATURE:
      (A) NAME/KEY: CDS
      (B) LOCATION: 1..168
   (xi) SEQUENCE DESCRIPTION: SEQ ID NO:80:
   TTG GGG TTG ACG GTG GCT GAT GTT GCT AGC CTG TGT GAG ATG GAA ATC        48
   Leu Gly Leu Thr Val Ala Asp Val Ala Ser Leu Cys Glu Met Glu Ile
     1               5                  10                  15
   CAG AAC CAT ACA GCC TAT TGT GAC AAG GTG CGC ACT CCG CTT GAA TTG        96
   Gln Asn His Thr Ala Tyr Cys Asp Lys Val Arg Thr Pro Leu Glu Leu
                20                  25                  30
   CAG GTT GGG TGC TTG GTG GGC AAT GAA CTT ACC TTT GAA TGT GAC AAG       144
   Gln Val Gly Cys Leu Val Gly Asn Glu Leu Thr Phe Glu Cys Asp Lys
            35                  40                  45
   TGT GAG GCT AGG CAA GAA ACC TTG                                       168
   Cys Glu Ala Arg Gln Glu Thr Leu
        50                  55
(2) INFORMATION FOR SEQ ID NO:81:
   (i) SEQUENCE CHARACTERISTICS:
      (A) LENGTH: 56 amino acids
      (B) TYPE: amino acid
      (D) TOPOLOGY: linear
   (ii) MOLECULE TYPE: protein
   (xi) SEQUENCE DESCRIPTION: SEQ ID NO:81:
   Leu Gly Leu Thr Val Ala Asp Val Ala Ser Leu Cys Glu Met Glu Ile
     1               5                  10                  15
   Gln Asn His Thr Ala Tyr Cys Asp Lys Val Arg Thr Pro Leu Glu Leu
                20                  25                  30
   Gln Val Gly Cys Leu Val Gly Asn Glu Leu Thr Phe Glu Cys Asp Lys
            35                  40                  45
   Cys Glu Ala Arg Gln Glu Thr Leu
        50                  55
(2) INFORMATION FOR SEQ ID NO:82:
   (i) SEQUENCE CHARACTERISTICS:
      (A) LENGTH: 313 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-50
   (ix) FEATURE:
      (A) NAME/KEY: CDS
      (B) LOCATION: 1..313
   (xi) SEQUENCE DESCRIPTION: SEQ ID NO:82:
   ATC ACC GTC AAC CCC AAT GAG AAA AAG CGC GTG ACG CTC TTT TCA ACG       48
   Ile Thr Val Asn Pro Asn Glu Lys Lys Arg Val Thr Leu Phe Ser Thr
    1               5                  10                  15
   CAG CAC GAC ATC TTG ACG GTA AGC TTC CTG GTC GCG TCG CTC TGT GGA       96
   Gln His Asp Ile Leu Thr Val Ser Phe Leu Val Ala Ser Leu Cys Gly
               20                  25                  30
   AAT AAG GCT TTT AAT ACG GAA AGA GCC ACG TTG AAG ACA CTT TCC TCC      144
   Asn Lys Ala Phe Asn Thr Glu Arg Ala Thr Leu Lys Thr Leu Ser Ser
           35                  40                  45
   CCT TCG GCT GTC TCG GAC TCT TGG ATG ACC TCG AAT GAG TCA GAG GAC      192
   Pro Ser Ala Val Ser Asp Ser Trp Met Thr Ser Asn Glu Ser Glu Asp
       50                  55                  60
   GGG GTA TCC TCC TGC GAG GAG GAC ACC GAC GGG GTC TTC TCA TCT GAG      240
   Gly Val Ser Ser Cys Glu Glu Asp Thr Asp Gly Val Phe Ser Ser Glu
   65                  70                  75                  80
   CTG CTC TCA GTA ACC GAG ATA AGT GCT GGC GAT GGA GTA CGG GGG ATG      288
   Leu Leu Ser Val Thr Glu Ile Ser Ala Gly Asp Gly Val Arg Gly Met
                   85                  90                  95
   TCT TCT CCC CAT ACA GGC ATC TCT C                                    313
   Ser Ser Pro His Thr Gly Ile Ser
               100
(2) INFORMATION FOR SEQ ID NO:83:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:83:
   Ile Thr Val Asn Pro Asn Glu Lys Lys Arg Val Thr Leu Phe Ser Thr
    1               5                  10                  15
   Gln His Asp Ile Leu Thr Val Ser Phe Leu Val Ala Ser Leu Cys Gly
                   20                  25                  30
   Asn Lys Ala Phe Asn Thr Glu Arg Ala Thr Leu Lys Thr Leu Ser Ser
               35                  40                  45
   Pro Ser Ala Val Ser Asp Ser Trp Met Thr Ser Asn Glu Ser Glu Asp
       50                  55                  60
   Gly Val Ser Ser Cys Glu Glu Asp Thr Asp Gly Val Phe Ser Ser Glu
   65                  70                  75                  80
   Leu Leu Ser Val Thr Glu Ile Ser Ala Gly Asp Gly Val Arg Gly Met
                       85                  90                  95
   Ser Ser Pro His Thr Gly Ile Ser
                   100
(2) INFORMATION FOR SEQ ID NO:84:
   (i) SEQUENCE CHARACTERISTICS:
      (A) LENGTH: 89 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-52
   (ix) FEATURE:
      (A) NAME/KEY: CDS
      (B) LOCATION: 28..87
   (xi) SEQUENCE DESCRIPTION: SEQ ID NO:84:
   ACTGAGAGCA GCTCAGATGA GAAGACC CCT TCG GCT GTC TCG GAC TCT TGG           51
                                 Pro Ser Ala Val Ser Asp Ser Trp
                                      1                   5
   ATG ACC TCG AAT GAG TCA GAG GAC GGG GTA TCC TCG CA                          89
   Met Thr Ser Asn Glu Ser Glu Asp Gly Val Ser Ser
        10                  15                  20
(2) INFORMATION FOR SEQ ID NO:85:
   (i) SEQUENCE CHARACTERISTICS:
      (A) LENGTH: 20 amino acids
      (B) TYPE: amino acid
      (D) TOPOLOGY: linear
   (ii) MOLECULE TYPE: protein
   (xi) SEQUENCE DESCRIPTION: SEQ ID NO:85:
   Pro Ser Ala Val Ser Asp Ser Trp Met Thr Ser Asn Glu Ser Glu Asp
     1               5                  10                  15
   Gly Val Ser Ser
                20
(2) INFORMATION FOR SEQ ID NO:86:
   (i) SEQUENCE CHARACTERISTICS:
      (A) LENGTH: 214 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-53
   (ix) FEATURE:
      (A) NAME/KEY: CDS
      (B) LOCATION: 1..100
   (xi) SEQUENCE DESCRIPTION: SEQ ID NO:86:
   AAT AAG GCT TTT AAT ACG GAA AGA GCC ACG TTG AAG ACA CTT TCC TCC        48
   Asn Lys Ala Phe Asn Thr Glu Arg Ala Thr Leu Lys Thr Leu Ser Ser
     1               5                  10                  15
   CCT TCG GCT GTC TCG GAC TCT TGG ATG ACC TCG AAT GAG TCA GAG GAC        96
   Pro Ser Ala Val Ser Asp Ser Trp Met Thr Ser Asn Glu Ser Glu Asp
                20                  25                  30
   GGG G ATCTCTAGAT GCGAATTCAA GTGTGAGGCT AGGCAAGAAA CCTTGGCCTC          150
   Gly
   CTTCTCTTAC ATTTGGTCTG GAGTGCCGCT GACTAGGGCC ACGCCGGCCA AGCCTCCCGT     210
   GGTG                                                                  214
(2) INFORMATION FOR SEQ ID NO:87:
   (i) SEQUENCE CHARACTERISTICS:
      (A) LENGTH: 33 amino acids
      (B) TYPE: amino acid
      (D) TOPOLOGY: linear
   (ii) MOLECULE TYPE: protein
   (xi) SEQUENCE DESCRIPTION: SEQ ID NO:87:
   Asn Lys Ala Phe Asn Thr Glu Arg Ala Thr Leu Lys Thr Leu Ser Ser
     1               5                  10                  15
   Pro Ser Ala Val Ser Asp Ser Trp Met Thr Ser Asn Glu Ser Glu Asp
                20                  25                  30
   Gly
(2) INFORMATION FOR SEQ ID NO:88:
   (i) SEQUENCE CHARACTERISTICS:
      (A) LENGTH: 113 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-55
   (ix) FEATURE:
      (A) NAME/KEY: CDS
      (B) LOCATION: 52..113
   (xi) SEQUENCE DESCRIPTION: SEQ ID NO:88:
   CCATCGCCAG CACTTATCTC GGTTACTGAG AGCAGCTCAG ATCAGAAGAC C CCT TCG       57
                                                           Pro Ser
                                                             1
   GCT GTC TCG GAC TCT TGG ATG ACC TCG AAT GAG TCA GAG GAC GGG GTA       105
   Ala Val Ser Asp Ser Trp Met Thr Ser Asn Glu Ser Glu Asp Gly Val
             5                  10                  15
   TCC TCG CA                                                            113
   Ser Ser
        20
(2) INFORMATION FOR SEQ ID NO:89:
   (i) SEQUENCE CHARACTERISTICS:
      (A) LENGTH: 20 amino acids
      (B) TYPE: amino acid
      (D) TOPOLOGY: linear
   (ii) MOLECULE TYPE: protein
   (xi) SEQUENCE DESCRIPTION: SEQ ID NO:89:
   Pro Ser Ala Val Ser Asp Ser Trp Met Thr Ser Asn Glu Ser Glu Asp
     1               5                  10                  15
   Gly Val Ser Ser
                20
(2) INFORMATION FOR SEQ ID NO:90:
   (i) SEQUENCE CHARACTERISTICS:
      (A) LENGTH: 330 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-56
   (ix) FEATURE:
      (A) NAME/KEY: CDS
      (B) LOCATION: 1..330
   (xi) SEQUENCE DESCRIPTION: SEQ ID NO:90:
   ACG TTG AAG ACA CTT TCC TCC CCT TCG GCT GTC TCG GAC TCT TGG ATG        48
   Thr Leu Lys Thr Leu Ser Ser Pro Ser Ala Val Ser Asp Ser Trp Met
     1               5                  10                  15
   ACC TCG AAT GAG TCA GAG GAC GGG GTA TCC TCC TGC GAG GAG GAC ACC        96
   Thr Ser Asn Glu Ser Glu Asp Gly Val Ser Ser Cys Glu Glu Asp Thr
                20                  25                  30
   GAC GGG GTC TTC TCA TCT GAG CTG CTC TCA GTA ACC GAG ATA AGT GCT       144
   Asp Gly Val Phe Ser Ser Glu Leu Leu Ser Val Thr Glu Ile Ser Ala
            35                  40                  45
   GGC GAT GGA GTA CGG GGG ATG TCT TCT CCC CAT ACA GGC ATC TCT CGG       192
   Gly Asp Gly Val Arg Gly Met Ser Ser Pro His Thr Gly Ile Ser Arg
        50                  55                  60
   CTA CTA CCA CAA AGA GAG GGT GTA CTG CAG TCC TCC ATG ATG ACA TCA       240
   Leu Leu Pro Gln Arg Glu Gly Val Leu Gln Ser Ser Met Met Thr Ser
    65                  70                  75                  80
   ATG TGC GGT TCA AGA ATC CTC GCA GCA TTC TCG ATC GCT TGG AGA GCA       288
   Met Cys Gly Ser Arg Ile Leu Ala Ala Phe Ser Ile Ala Trp Arg Ala
                    85                  90                  95
   GCA GCC GCC GGC GGC AGA TCG GCC TCA GTC AGT TCT GAG TCT               330
   Ala Ala Ala Gly Gly Arg Ser Ala Ser Val Ser Ser Glu Ser
               100                 105                 110
(2) INFORMATION FOR SEQ ID NO:91:
   (i) SEQUENCE CHARACTERISTICS:
      (A) LENGTH: 110 amino acids
      (B) TYPE: amino acid
      (D) TOPOLOGY: linear
   (ii) MOLECULE TYPE: protein
   (xi) SEQUENCE DESCRIPTION: SEQ ID NO:91:
   Thr Leu Lys Thr Leu Ser Ser Pro Ser Ala Val Ser Asp Ser Trp Met
     1               5                  10                  15
   Thr Ser Asn Glu Ser Glu Asp Gly Val Ser Ser Cys Glu Glu Asp Thr
                20                  25                  30
   Asp Gly Val Phe Ser Ser Glu Leu Leu Ser Val Thr Glu Ile Ser Ala
            35                  40                  45
   Gly Asp Gly Val Arg Gly Met Ser Ser Pro His Thr Gly Ile Ser Arg
        50                  55                  60
   Leu Leu Pro Gln Arg Glu Gly Val Leu Gln Ser Ser Met Met Thr Ser
    65                  70                  75                  80
   Met Cys Gly Ser Arg Ile Leu Ala Ala Phe Ser Ile Ala Trp Arg Ala
                    85                  90                  95
   Ala Ala Ala Gly Gly Arg Ser Ala Ser Val Ser Ser Glu Ser
               100                 105                 110
(2) INFORMATION FOR SEQ ID NO:92:
   (i) SEQUENCE CHARACTERISTICS:
      (A) LENGTH: 195 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-57
   (ix) FEATURE:
      (A) NAME/KEY: CDS
      (B) LOCATION: 1..195
   (xi) SEQUENCE DESCRIPTION: SEQ ID NO:92:
   ACG GAA AGA GCC ACG TTG AAG ACA CTT TCC TCC CCT TCG GCT GCC TCG        48
   Thr Glu Arg Ala Thr Leu Lys Thr Leu Ser Ser Pro Ser Ala Ala Ser
     1               5                  10                  15
   GAC TCT TGG ATG ACC TCG AAT GAG TCG GAG GAC GGG GTA TCC TCC TGC        96
   Asp Ser Trp Met Thr Ser Asn Glu Ser Glu Asp Gly Val Ser Ser Cys
                20                  25                  30
   GAA GAG GAC ACC GAC GGG GTC TTC TCA TCT GAG CTG CTC TCA GTA ACC       144
   Glu Glu Asp Thr Asp Gly Val Phe Ser Ser Glu Leu Leu Ser Val Thr
            35                  40                  45
   GAG ATA AGT GCT GGC GGT GGA GTA CGG GGG ATG TCT TCT CCC CAT ACG       192
   Glu Ile Ser Ala Gly Gly Gly Val Arg Gly Met Ser Ser Pro His Thr
        50                  55                  60
   GGC                                                                   195
   Gly
    65
(2) INFORMATION FOR SEQ ID NO:93:
   (i) SEQUENCE CHARACTERISTICS:
      (A) LENGTH: 65 amino acids
      (B) TYPE: amino acid
      (D) TOPOLOGY: linear
   (ii) MOLECULE TYPE: protein
   (xi) SEQUENCE DESCRIPTION: SEQ ID NO:93:
   Thr Glu Arg Ala Thr Leu Lys Thr Leu Ser Ser Pro Ser Ala Ala Ser
    1               5                  10                  15
   Asp Ser Trp Met Thr Ser Asn Glu Ser Glu Asp Gly Val Ser Ser Cys
               20                  25                  30
   Glu Glu Asp Thr Asp Gly Val Phe Ser Ser Glu Leu Leu Ser Val Thr
           35                  40                  45
   Glu Ile Ser Ala Gly Gly Gly Val Arg Gly Met Ser Ser Pro His Thr
       50                  55                  60
   Gly
    65
(2) INFORMATION FOR SEQ ID NO:94:
   (i) SEQUENCE CHARACTERISTICS:
      (A) LENGTH: 115 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-60
   (ix) FEATURE:
      (A) NAME/KEY: CDS
      (B) LOCATION: 1..115
   (xi) SEQUENCE DESCRIPTION: SEQ ID NO:94:
   AAG ACA CTT TCC TCC CCT TCG GCT GTC TCG GAC TCT TGG ATG ACC TCG          48
   Lys Thr Leu Ser Ser Pro Ser Ala Val Ser Asp Ser Trp Met Thr Ser
     1               5                  10                  15
   AAT GAG TCA GAG GAC GGG GTA TCC TCC TGC GAG GAG GAC ACC GAC TGG          96
   Asn Glu Ser Glu Asp Gly Val Ser Ser Cys Glu Glu Asp Thr Asp Trp
                20                  25                  30
   GTC TTC TCA TCT GAG CTG C                                               115
   Val Phe Ser Ser Glu Leu
            35
(2) INFORMATION FOR SEQ ID NO:95:
   (i) SEQUENCE CHARACTERISTICS:
      (A) LENGTH: 38 amino acids
      (B) TYPE: amino acid
      (D) TOPOLOGY: linear
   (ii) MOLECULE TYPE: protein
   (xi) SEQUENCE DESCRIPTION: SEQ ID NO:95:
   Lys Thr Leu Ser Ser Pro Ser Ala Val Ser Asp Ser Trp Met Thr Ser
     1               5                  10                  15
   Asn Glu Ser Glu Asp Gly Val Ser Ser Cys Glu Glu Asp Thr Asp Trp
                20                  25                  30
   Val Phe Ser Ser Glu Leu
            35
(2) INFORMATION FOR SEQ ID NO:96:
   (i) SEQUENCE CHARACTERISTICS:
      (A) LENGTH: 93 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone Y5-63
   (ix) FEATURE:
      (A) NAME/KEY: CDS
      (B) LOCATION: 19..93
   (xi) SEQUENCE DESCRIPTION: SEQ ID NO:96:
   GAGAGCAGCT CAGATGAG AAG ACA CTT TCC TCC CCT TCG GCT GTC TCG GAC         51
                       Lys Thr Leu Ser Ser Pro Ser Ala Val Ser Asp
                        1               5                  10
   TCT TGG ATG ACC TCG AAT GAG TCA GAG GAC GGG GTA TCC TCG                 93
   Ser Trp Met Thr Ser Asn Glu Ser Glu Asp Gly Val Ser Ser
            15                  20                  25
(2) INFORMATION FOR SEQ ID NO:97:
   (i) SEQUENCE CHARACTERISTICS:
      (A) LENGTH: 25 amino acids
      (B) TYPE: amino acid
      (D) TOPOLOGY: linear
   (ii) MOLECULE TYPE: protein
   (xi) SEQUENCE DESCRIPTION: SEQ ID NO:97:
   Lys Thr Leu Ser Ser Pro Ser Ala Val Ser Asp Ser Trp Met Thr Ser
    1               5                  10                  15
   Asn Glu Ser Glu Asp Gly Val Ser Ser
               20                  25
(2) INFORMATION FOR SEQ ID NO:98:
   (i) SEQUENCE CHARACTERISTICS:
      (A) LENGTH: 1181 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 8E3
   (xi) SEQUENCE DESCRIPTION: SEQ ID NO:98:
   GCTGGCTGAG GCACGGTTGG TCCCGCTGAT CTTGCTGCTG CTATGGTGGT GGGTGAACCA     60
   GCTGGCAGTC CTAGGGCTGC CGGCTGTGGA AGCCGCCGTG GCAGGTGAGG TCTTCGCGGG    120
   CCCTGCCCTG TCCTGGTGTC TGGGACTCCC GGTCGTCAGT ATGATATTGG GTTTGGCAAA    180
   CCTGGTGCTG TACTTTAGAT GGTTGGGACC CCAACGCCTG ATGTTCCTCG TGTTGTGGAA    240
   GCTTGCTCGG GGAGCTTTCC CGCTGGCCCT CTTGATGGGG ATTTCGGCGA CCCGCGGGCG    300
   CACCTCAGTG CTCGGGGCCG AGTTCTGCTT CGATGCTACA TTCGAGGTGG ACACTTCGGT    360
   GTTGGGCTGG GTGGTGGCCA GTGTGGTAGC TTGGGCCATT GCGCTCCTGA GCTCGATGAG    420
   CGCAGGGGGG TGGAGGCACA AAGCCGTGAT CTATAGGACG TGGTGTAAGG GGTACCAGGC    480
   AATCCGTCAA AGGGTGGTGA GGAGCCCCCT CGGGGAGGGG CGGCCTGCCA AACCCCTGAC    540
   CTTTGCCTGG TGCTTGGCCT CGTACATCTG GCCAGATGCT GTGATGATGG TGGTGGTTGC    600
   CTTGGTCCTT CTCTTTGGCC TGTTCGACGC GTTGGATTGG GCCTTGGAGG AGATCTTGGT    660
   GTCCCGGCCC TCGTTGCGGC GTTTGGCTCG GGTGGTTGAG TGCTGTGTGA TGGCGGGTGA    720
   GAAGGCCACA ACCGTCCGGC TGGTCTCCAA GATGTGTGCG AGAGGAGCTT ATTTGTTCGA    780
   TCATATGGGC TCTTTTTCGC GTGCTGTCAA GGAGCGCCTG TTGGAATGGG ACGCAGCTCT    840
   TGAACCTCTG TCATTCACTA GGACGGACTG TCGCATCATA CGGGATGCCG CGAGGACTTT    900
   GTCCTGCGGG CAGTGCGTCA TGGGTTTACC CGTGGTTGCG CGCCGTGGTG ATGAGGTTCT    960
   CATCGGCGTC TTCCAGGATG TGAATCATTT GCCTCCCGGG TTTGTTCCGA CCGCGCCTGT   1020
   TGTCATCCGA CGGTGCGGAA AGGGCTTCTT GGGGGTCACA AAGGCTGCCT TGACAGGTCG   1080
   GGATCCTGAC TTACATCCAG GGAACGTCAT GGTGTTGGGG ACGGCTACGT CGCGAAGCAT   1140
   GGGAACATGC TTGAACGGCC TGCTGTTCAC GACCTTCCAT G                        1181
(2) INFORMATION FOR SEQ ID NO:99:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer Y5-10-F1
   (xi) SEQUENCE DESCRIPTION: SEQ ID NO:99:
   TCAGCCATGG CTCGTGCGCC CGCGATGGTC 30
(2) INFORMATION FOR SEQ ID NO:100:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer Y5-10-R1
   (xi) SEQUENCE DESCRIPTION: SEQ ID NO:100:
   CGAGGATCCA GCCGCCGGCG GCAGATC 27
(2) INFORMATION FOR SEQ ID NO:101:
   (i) SEQUENCE CHARACTERISTICS:
      (A) LENGTH: 32 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer Y5-16F1
   (xi) SEQUENCE DESCRIPTION: SEQ ID NO:101:
   GATTCCATGG GTTTGGGGTT GACGGTGGCT GA 32
(2) INFORMATION FOR SEQ ID NO:102:
   (i) SEQUENCE CHARACTERISTICS:
      (A) LENGTH: 32 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470EP-R3
   (xi) SEQUENCE DESCRIPTION: SEQ ID NO:102:
   GCGAATTCGG ATCCCAAGGT TTCTTGCCTA GC 32
(2) INFORMATION FOR SEQ ID NO:103:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer Y5-5-F1
   (xi) SEQUENCE DESCRIPTION: SEQ ID NO:103:
   GAGGCCATGG CCTATTGTGA CAAGGTG 27
(2) INFORMATION FOR SEQ ID NO:104:
   (i) SEQUENCE CHARACTERISTICS:
      (A) LENGTH: 17 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer PGEX-R
   (xi) SEQUENCE DESCRIPTION: SEQ ID NO:104:
   GACCGTCTCC GGGAGCT 17
(2) INFORMATION FOR SEQ ID NO:105:
   (i) SEQUENCE CHARACTERISTICS:
      (A) LENGTH: 326 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone GE15
   (ix) FEATURE:
      (A) NAME/KEY: CDS
      (B) LOCATION: 3..326
   (xi) SEQUENCE DESCRIPTION: SEQ ID NO:105:
   CC ATG GAG GTC TCT GAC TTC CGT GGC TCG TCT GGC TCA CCG GTC CTA          47
      Met Glu Val Ser Asp Phe Arg Gly Ser Ser Gly Ser Pro Val Leu
        1               5                  10                  15
   TGT GAC GAA GGG CAC GCA GTA GGA ATG CTC GTG TCT GTG CTT CAC TCC          95
   Cys Asp Glu Gly His Ala Val Gly Met Leu Val Ser Val Leu His Ser
                      20                  25                  30
   GGT GGT AGG GTC ACC GCG GCA CGG TTC ACT AGG CCG TGG ACC CAA GTG         143
   Gly Gly Arg Val Thr Ala Ala Arg Phe Thr Arg Pro Trp Thr Gln Val
                  35                  40                  45
   CCA ACA GAT GCC AAA ACC ACC ACT GAA CCC CCT CCG GTG CCG GCC AAA         191
   Pro Thr Asp Ala Lys Thr Thr Thr Glu Pro Pro Pro Val Pro Ala Lys
              50                  55                  60
   GGA GTT TTC AAA GAG GCC CCG TTG TTT ATG CCT ACG GGA GCG GGA AAG         239
   Gly Val Phe Lys Glu Ala Pro Leu Phe Met Pro Thr Gly Ala Gly Lys
          65                  70                  75
   AGC ACT CGC GTC CCG TTG GAG TAC GGC AAC ATG GGG CAC AAG GTC TTA         287
   Ser Thr Arg Val Pro Leu Glu Tyr Gly Asn Met Gly His Lys Val Leu
    80                  85                  90                  95
   ATC TTG AAC CCC TCA GTG GCC ACT GTG CGG GCG ATG GGC                     326
   Ile Leu Asn Pro Ser Val Ala Thr Val Arg Ala Met Gly
                   100                 105
(2) INFORMATION FOR SEQ ID NO:106:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:106:
   Met Glu Val Ser Asp Phe Arg Gly Ser Ser Gly Ser Pro Val Leu Cys
     1               5                  10                  15
   Asp Glu Gly His Ala Val Gly Met Leu Val Ser Val Leu His Ser Gly
                20                  25                  30
   Gly Arg Val Thr Ala Ala Arg Phe Thr Arg Pro Trp Thr Gln Val Pro
            35                  40                  45
   Thr Asp Ala Lys Thr Thr Thr Glu Pro Pro Pro Val Pro Ala Lys Gly
        50                  55                  60
   Val Phe Lys Glu Ala Pro Leu Phe Met Pro Thr Gly Ala Gly Lys Ser
    65                  70                  75                  80
   Thr Arg Val Pro Leu Glu Tyr Gly Asn Met Gly His Lys Val Leu Ile
                    85                  90                  95
   Leu Asn Pro Ser Val Ala Thr Val Arg Ala Met Gly
               100                 105
(2) INFORMATION FOR SEQ ID NO:107:
   (i) SEQUENCE CHARACTERISTICS:
      (A) LENGTH: 138 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Clone GE17
   (ix) FEATURE:
      (A) NAME/KEY: CDS
      (B) LOCATION: 1..138
   (xi) SEQUENCE DESCRIPTION: SEQ ID NO:107:
   GGT GAT GAG GTT CTC ATC GGC GTC TTC CAG GAT GTG AAT CAT TTG CCT        48
   Gly Asp Glu Val Leu Ile Gly Val Phe Gln Asp Val Asn His Leu Pro
     1               5                  10                  15
   CCC GGG TTT GTT CCG ACC GCG CCT GTT GTC ATC CGA CGG TGC GGA AAG        96
   Pro Gly Phe Val Pro Thr Ala Pro Val Val Ile Arg Arg Cys Gly Lys
               20                  25                  30
   GGC TTC TTG GGG GTC ACA AAG GCT GCC TTG ACA GGT CGG GAT               138
   Gly Phe Leu Gly Val Thr Lys Ala Ala Leu Thr Gly Arg Asp
           35                  40                  45
(2) INFORMATION FOR SEQ ID NO:108:
   (i) SEQUENCE CHARACTERISTICS:
      (A) LENGTH: 46 amino acids
      (B) TYPE: amino acid
      (D) TOPOLOGY: linear
   (ii) MOLECULE TYPE: protein
   (xi) SEQUENCE DESCRIPTION: SEQ ID NO:108:
   Gly Asp Glu Val Leu Ile Gly Val Phe Gln Asp Val Asn His Leu Pro
     1               5                  10                  15
   Pro Gly Phe Val Pro Thr Ala Pro Val Val Ile Arg Arg Cys Gly Lys
               20                  25                  30
   Gly Phe Leu Gly Val Thr Lys Ala Ala Leu Thr Gly Arg Asp
           35                  40                  45
(2) INFORMATION FOR SEQ ID NO:109:
   (i) SEQUENCE CHARACTERISTICS:
      (A) LENGTH: 395 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 9E3
   (xi) SEQUENCE DESCRIPTION: SEQ ID NO:109:
   TGTATTTGTC CTGTTATACC TGATGAAGCT GGCTGAGGCA CGGTTGGTCC CGCTGATCTT 60
   GCTGCTGCTA TGGTGGTGGG TGAACCAGCT GGCAGTCCTA GGGCTGCCGG CTGTGGAAGC 120
   CGCCGTGGCA GGTGAGGTCT TCGCGGGCCC TGCCCTGTCC TGGTGTCTGG GACTCCCGGT 180
   CGTCAGTATG ATATTGGGTT TGGCAAACCT AGTGCTGTAC TTTAGATGGT TGGGACCCCA 240
   ACGCCTGATG TTCCTCGTGT TGTGGAAGCT TGCTCGGGGA GCTTTCCCGC TGGCCCTCTT 300
   GATGGGGATT TCGGCGACCC GCGGGCGCAC CTCAGTGCTC GGGGCCGAGT TCTGCTTCGA 360
   TGCTACATTC GAGGTGGACA CTTCGGTGTT GGGCT 395
(2) INFORMATION FOR SEQ ID NO:110:
   (i) SEQUENCE CHARACTERISTICS:
      (A) LENGTH: 460 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 10E3
   (xi) SEQUENCE DESCRIPTION: SEQ ID NO:110:
   GCCCCTGGGC AACCAGGGCC GAGGCAACCC GGTGCGGTCG CCCTTGGGTT TTGGGTCCTA 60
   CGCCATGACC AGGATCCGAG ATACCCTACA TCTGGTGGAG TGTCCCACAC CAGCCATTGA 120
   GCCTCCCACC GGGACGTTTG GGTTCTTCCC CGGGACGCCG CCTCTCAACA ACTGCATGCT 180
   CTTGGGCACG GAAGTGTCCG AGGCACTTGG GGGGGCTGGC CTCACGGGGG GGTTCTATGA 240
   ACCCCTGGTG CGCAGGTGTT CGAAGCTGAT GGGAAGCCGA AATCCGGTTT GTCCGGGGTT 300
   TGCATGGCTC TCTTCGGGCA GGCCTGATGG GTTTATACAT GTCCAGGGTC ACTTGCAGGA 360
   GGTGGATGCA GGCAACTTCA TCCCGCCCCC GCGCTGGTTG CTCTTGGACT TTGTATTTGT 420
   CCTGTTATAC CTGATGAAGC TGGCTGAGGC ACGGTTGGTC 460
(2) INFORMATION FOR SEQ ID NO:111:
   (i) SEQUENCE CHARACTERISTICS:
      (A) LENGTH: 28 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE15F
   (xi) SEQUENCE DESCRIPTION: SEQ ID NO:111:
   GCCGCCATGG AGGTCTCTGA CTTCCGTG 28
(2) INFORMATION FOR SEQ ID NO:112
   (i) SEQUENCE CHARACTERISTICS:
      (A) LENGTH: 31 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE15R
   (xi) SEQUENCE DESCRIPTION: SEQ ID NO:112:
   GCGCGGATCC GCCCATCGCC CGCACAGTGG C 31
(2) INFORMATION FOR SEQ ID NO:113:
   (i) SEQUENCE CHARACTERISTICS:
      (A) LENGTH: 31 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE17F
   (xi) SEQUENCE DESCRIPTION: SEQ ID NO:113:
   CGCTCCATGG GTGATGAGGT TCTCATCGGC G 31
(2) INFORMATION FOR SEQ ID NO:114:
   (i) SEQUENCE CHARACTERISTICS:
      (A) LENGTH: 28 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE17R
   (xi) SEQUENCE DESCRIPTION: SEQ ID NO:114:
   GTAAGTCAGG ATCCCGACCT GTCAAGGC 28
(2) INFORMATION FOR SEQ ID NO:115:
   (i) SEQUENCE CHARACTERISTICS:
      (A) LENGTH: 452 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: NcoI/EcoRI-containing fragment of pGEX-HISb-GE3-s HGV plasmid
   (xi) SEQUENCE DESCRIPTION: SEQ ID NO:115:
   CAAAATCGGA TCTGGTTCCG CGTGGTTCCA TGGTCTCATG GGACGCGGAC GCTCGTGCGC 60
   CCGCGATGGT CTATGGCCCT GGGCAAAGTG TTACCATTGA CGGGGAGCGC TACACCTTGC 120
   CTCATCAACT GAGGCTCAGG AATGTGGCAC CCTCTGAGGT TTCATCCGAG GTGTCCATTG 180
   ACATTGGGAC GGAGACTGAA GACTCAGAAC TGACTGAGGC CGATCTGCCG CCGGCGGCTG 240
   CTGCTCTCCA AGCGATCGAG AATGCTGCGA GGATTCTTGA ACCGCACATT GATGTCATCA 300
   TGGAGGACTG CAGTACACCC TCTCTTTGTG GTAGTAGCCG AGAGATGCCT GTATGGGGAG 360
   AAGACATCCC CCGTACTCCA TCGCCAGCAC TTATCGGATC CCACCATCAC CATCACCATT 420
   AGAATTCATC GTGACTGACT GACGATCTAC CT 452
(2) INFORMATION FOR SEQ ID NO:116:
   (i) SEQUENCE CHARACTERISTICS:
      (A) LENGTH: 590 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 11E3
   (xi) SEQUENCE DESCRIPTION: SEQ ID NO:116:
   AGCAATCGGC TGGGGTGACC CCATCACTTA TTGGAGCCAC GGGCAAAATC AGTGGCCCCT    60
   TTCATGCCCC CAGTATGTCT ATGGGTCTGC TACAGTCACT TGCGTGTGGG GTTCCGCTTC   120
   TTGGTTTGCC TCCACCAGTG GTCGCGACTC GAAGATAGAT GTGTGGAGTT TAGTGCCAGT   180
   TGGCTCTGCC ACCTGCACCA TAGCCGCACT TGGATCATCG GATCGCGACA CGGTGCCTGG   240
   GCTCTCCGAG TGGGGAATCC CGTGCGTGAC GTGTGTTCTG GACCGTCGGC CTGCCTCCTG   300
   CGGCACCTGT GTGAGGGACT GCTGGCCCGA GACCGGGTCG GTTAGGTTCC CATTCCATCG   360
   GTGCGGCGTG GGGCCTCGGC TGACAAAGGA CTTGGAAGCT GTGCCCTTCG TCAACAGGAC   420
   AACTCCCTTC ACCATTAGGG GGCCCCTGGG CAACCAGGGC CGAGGCAACC CGGTGCGGTC   480
   GCCCTTGGGT TTTGGGTCCT ACGCCATGAC CAGGATCCGA GATACCCTAC ATCTGGTGGA   540
   GTGTCCCACA CCAGCCATCG AGCCTCCCAC CGGGACGTTT GGGTTCTTCC              590
(2) INFORMATION FOR SEQ ID NO:117:
   (i) SEQUENCE CHARACTERISTICS:
      (A) LENGTH: 29 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Probe E3-111PROB
   (xi) SEQUENCE DESCRIPTION: SEQ ID NO:117:
   TGGTGAAGGG AGTTGTCCTA TTGACGAAG    29
(2) INFORMATION FOR SEQ ID NO:118:
   (i) SEQUENCE CHARACTERISTICS:
      (A) LENGTH: 735 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 12E3
   (xi) SEQUENCE DESCRIPTION: SEQ ID NO:118:
   ATTGTTGTGC CCCGGAGGAC ATCGGGTTCT GCCTGGAGGG TGGATGCCTG GTGGCCCTGG    60
   GGTGCACGAT TTGCACTGAC CAATGCTGGC CACTGTATCA GGCGGGTTTG GCTGTGCGGC   120
   CTGGCAAGTC CGCGGCCCAA CTGGTGGGGG AGCTGGGTAG CCTATACGGG CCCCTGTCGG   180
   TCTCGGCCTA TGTGGCTGGG ATCCTGGGCC TGGGTGAGGT GTACTCGGGT GTCCTAACGG   240
   TGGGAGTCGC GTTGACGCGC CGGGTCTACC CGGTGCCTAA CCTGACGTGT GCAGTCGCGT   300
   GTGAGCTAAA GTGGGAAAGT GAGTTTTGGA GATGGACTGA ACAGCTGGCC TCCAACTACT   360
   GGATTCTGGA ATACCTCTGG AAGGTCCCAT TTGATTTCTG GAGAGGCGTG ATAAGCCTGA   420
   CCCCCTTGTT GGTTTGCGTG GCCGCATTGC TGCTGCTTGA GCAACGGATT GTCATGGTCT   480
   TCCTGTTGGT GACGATGGCC GGGATGTCGC AAGGCGCCCC TGCCTCCGTT TTGGGGTCAC   540
   GCCCCTTTGA CTACGGGTTG ACTTGGCAGA CCTGCTCTTG CAGGGCCAAC GGTTCGCGTT   600
   TTTCGACTGG GGAGAAGGTG TGGGACCGTG GGAACGTTAC GCTTCAGTGT GACTGCCCTA   660
   ACGGCCCCTG GGTGTGGTTG CCAGCCTTTT GCCAAGCAAT CGGCTGGGGT GACCCCATCA   720
   CTTATTGGAG CCACG                                                    735
(2) INFORMATION FOR SEQ ID NO:119:
   (i) SEQUENCE CHARACTERISTICS:
      (A) LENGTH: 22 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470EXT4-2189R
   (xi) SEQUENCE DESCRIPTION: SEQ ID NO:119:
   ATCTGTGGTA TGCCATCCCG GT 22
(2) INFORMATION FOR SEQ ID NO:120:
   (i) SEQUENCE CHARACTERISTICS:
      (A) LENGTH: 23 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470EXT4-29F
   (xi) SEQUENCE DESCRIPTION: SEQ ID NO:120:
   GTTATGCTAC TGTCGAAGCA GGT 23
(2) INFORMATION FOR SEQ ID NO:121:
   (i) SEQUENCE CHARACTERISTICS:
      (A) LENGTH: 24 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: NS5 Primer GV57-4512 MF
   (xi) SEQUENCE DESCRIPTION: SEQ ID NO:121:
   GGACTTCCGG ATAGCTGARA AGCT 24
(2) INFORMATION FOR SEQ ID NO:122:
   (i) SEQUENCE CHARACTERISTICS:
      (A) LENGTH: 20 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: NS5 Primer GV57-4657 MR
   (xi) SEQUENCE DESCRIPTION: SEQ ID NO:122:
   GCRTCCACAC AGATGGCGCA 20
(2) INFORMATION FOR SEQ ID NO:123:
   (i) SEQUENCE CHARACTERISTICS:
      (A) LENGTH: 28 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: NS5 Probe GV22dc-89 MF
   (xi) SEQUENCE DESCRIPTION: SEQ ID NO:123:
   CYCGCTGRTT TGGGGTGTAC TGGAAGGC 28
(2) INFORMATION FOR SEQ ID NO:124:
   (i) SEQUENCE CHARACTERISTICS:
      (A) LENGTH: 31 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 5'-UTR Primer FV94-22F
   (xi) SEQUENCE DESCRIPTION: SEQ ID NO:124:
   GAAAGCCCCA GAAACCGACG CCTATCTAAG T 31
(2) INFORMATION FOR SEQ ID NO:125:
   (i) SEQUENCE CHARACTERISTICS:
      (A) LENGTH: 26 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 5'UTR Primer FV94-724R
   (xi) SEQUENCE DESCRIPTION: SEQ ID NO:125:
   GCACAGCCAA ACCCGCCTGA TACAGT 26
(2) INFORMATION FOR SEQ ID NO:126:
   (i) SEQUENCE CHARACTERISTICS:
      (A) LENGTH: 28 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 5'-UTR Primer FV94-94F
   (xi) SEQUENCE DESCRIPTION: SEQ ID NO:126:
   GTGGTGGATG GGTGATGACA GGGTTGGT                              28
(2) INFORMATION FOR SEQ ID NO:127:
   (i) SEQUENCE CHARACTERISTICS:
      (A) LENGTH: 29 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: 5'-UTR Primer FV94-912R
   (xi) SEQUENCE DESCRIPTION: SEQ ID NO:127:
   TAACTCACAC GCGACTGCAC ACGTCAGGT                             29
(2) INFORMATION FOR SEQ ID NO:128:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: ENV Library Primer GEP-F15
   (xi) SEQUENCE DESCRIPTION: SEQ ID NO:128:
   GCGGCCATGG TGCCCTTCGT CAATAGGACA 30
(2) INFORMATION FOR SEQ ID NO:129:
   (i) SEQUENCE CHARACTERISTICS:
      (A) LENGTH: 29 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: ENV Library Primer GEP-R15
   (xi) SEQUENCE DESCRIPTION: SEQ ID NO:129:
   CTTGCCATGG CCAGCTGGTT CACCCACCA 29
(2) INFORMATION FOR SEQ ID NO:130:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-F17
   (xi) SEQUENCE DESCRIPTION: SEQ ID NO:130:
   GCAGGATCCC CTCTGGAAGG TCCCATTTGA 30
(2) INFORMATION FOR SEQ ID NO:131:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-R16
   (xi) SEQUENCE DESCRIPTION: SEQ ID NO:131:
   TGCGAATCCT CGGCCCTGGT TGCCCAG 27
(2) INFORMATION FOR SEQ ID NO:132:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470ep-F9
   (xi) SEQUENCE DESCRIPTION: SEQ ID NO:132:
   GCTAGATCTG GCAACATGGG GCACAAGGTC 30
(2) INFORMATION FOR SEQ ID NO:133:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470ep-R9
   (xi) SEQUENCE DESCRIPTION: SEQ ID NO:133:
   CACAGATCTC GCGTAGTAGT AGCGTCCAGA 30
(2) INFORMATION FOR SEQ ID NO:134:
   (i) SEQUENCE CHARACTERISTICS:
      (A) LENGTH: 38 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: AP Primer for Race PCR
   (xi) SEQUENCE DESCRIPTION: SEQ ID NO:134:
   CTGGTTCGGC CCACCTCTGA AGGTTCCAGA ATCGATAG 38
(2) INFORMATION FOR SEQ ID NO:135:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-F10
   (xi) SEQUENCE DESCRIPTION: SEQ ID NO:135:
   GCTGGATCCA GCATGGGAAC ATGCTTGAAC 30
(2) INFORMATION FOR SEQ ID NO:136:
   (i) SEQUENCE CHARACTERISTICS:
      (A) LENGTH: 30 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-R10
   (xi) SEQUENCE DESCRIPTION: SEQ ID NO:136:
   CGCGGATCCC ACAGTGGCCA CTGAGGGGTT 30
(2) INFORMATION FOR SEQ ID NO:137:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer EXY10-F1
   (xi) SEQUENCE DESCRIPTION: SEQ ID NO:137:
   GCCCATATGG TGATCACTGG TGACGTT 27
(2) INFORMATION FOR SEQ ID NO:138:
   (i) SEQUENCE CHARACTERISTICS:
      (A) LENGTH: 24 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer EXY10-F2
   (xi) SEQUENCE DESCRIPTION: SEQ ID NO:138:
   GCCCATATGC TGGGTTACGG TGAA 24
(2) INFORMATION FOR SEQ ID NO:139:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer EXY10-F3
   (xi) SEQUENCE DESCRIPTION: SEQ ID NO:139:
   GCCCATATGA CCTCCGCCTA TAAGCTG 27
(2) INFORMATION FOR SEQ ID NO:140:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer EXY10-R1
   (xi) SEQUENCE DESCRIPTION: SEQ ID NO:140:
   GCCCATATGA GCCGCCGGCG GCAGATC 27
(2) INFORMATION FOR SEQ ID NO:141:
   (i) SEQUENCE CHARACTERISTICS:
      (A) LENGTH: 24 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer EXY5-R1
   (xi) SEQUENCE DESCRIPTION: SEQ ID NO:141:
   TGCGGATCCC ACATTGTCTG GATT 24
(2) INFORMATION FOR SEQ ID NO:142:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer Y5-5-F1
   (xi) SEQUENCE DESCRIPTION: SEQ ID NO:142:
   TCGGCCATGG CCTATTGTGA CAAGGTG                                27
(2) INFORMATION FOR SEQ ID NO:143:
   (i) SEQUENCE CHARACTERISTICS:
      (A) LENGTH: 219 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone Q7-12-1
   (ix) FEATURE:
      (A) NAME/KEY: CDS
      (B) LOCATION: 1..219
   (xi) SEQUENCE DESCRIPTION: SEQ ID NO:143:
   GTG CCC TTC GTC AAT AGG ACA ACT CTC TTC ACC ATT AGG GGG CCC CTG      48
   Val Pro Phe Val Asn Arg Thr Thr Leu Phe Thr Ile Arg Gly Pro Leu
     1               5                  10                  15
   GGC AAC CAG GGC CGA GGC AAC CCG GTG CGG TCG CCC TTG GGT TTT GGG      96
   Gly Asn Gln Gly Arg Gly Asn Pro Val Arg Ser Pro Leu Gly Phe Gly
                20                  25                  30
   TCC TAC GCC ATG ACC AGG ATC CGA GAT ACC CTA CAT CTG GTG GAG TGT     144
   Ser Tyr Ala Met Thr Arg Ile Arg Asp Thr Leu His Leu Val Glu Cys
            35                  40                  45
   CCC ACA CCA GCC ATC GAG CCT CCC ACC GGG ACG TCT GGG TTC TTC CCC        192
   Pro Thr Pro Ala Ile Glu Pro Pro Thr Gly Thr Ser Gly Phe Phe Pro
        50                  55                  60
   GGG ACG CCG CCT CTC AAC AGC TGC ATG                                    219
   Gly Thr Pro Pro Leu Asn Ser Cys Met
    65                  70
(2) INFORMATION FOR SEQ ID NO:144:
   (i) SEQUENCE CHARACTERISTICS:
      (A) LENGTH: 73 amino acids
      (B) TYPE: amino acid
      (D) TOPOLOGY: linear
   (ii) MOLECULE TYPE: protein
   (xi) SEQUENCE DESCRIPTION: SEQ ID NO:144:
   Val Pro Phe Val Asn Arg Thr Thr Leu Phe Thr Ile Arg Gly Pro Leu
     1               5                  10                  15
   Gly Asn Gln Gly Arg Gly Asn Pro Val Arg Ser Pro Leu Gly Phe Gly
                20                  25                  30
   Ser Tyr Ala Met Thr Arg Ile Arg Asp Thr Leu His Leu Val Glu Cys
            35                  40                  45
   Pro Thr Pro Ala Ile Glu Pro Pro Thr Gly Thr Ser Gly Phe Phe Pro
        50                  55                  60
   Gly Thr Pro Pro Leu Asn Ser Cys Met
    65                  70
(2) INFORMATION FOR SEQ ID NO:145:
   (i) SEQUENCE CHARACTERISTICS:
      (A) LENGTH: 264 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone Y12-10-3
   (ix) FEATURE:
      (A) NAME/KEY: CDS
      (B) LOCATION: 1..264
   (xi) SEQUENCE DESCRIPTION: SEQ ID NO:145:
   CCC CTC GAG CGG ATG CGA ACC GGA AGG CAC CTC GTG TTC TGC CAT TCT        48
   Pro Leu Glu Arg Met Arg Thr Gly Arg His Leu Val Phe Cys His Ser
     1               5                  10                  15
   AAG GCT GAG TGC GAG CGC CTT GCT GGC CAG TTC TCC GCT AGG GGG GTC        96
   Lys Ala Glu Cys Glu Arg Leu Ala Gly Gln Phe Ser Ala Arg Gly Val
                20                  25                  30
   AAT GCC ATT GCC TAT TAT AGG GGT AAA GAC AGC TCT ATC ATC AAG GAT       144
   Asn Ala Ile Ala Tyr Tyr Arg Gly Lys Asp Ser Ser Ile Ile Lys Asp
            35                  40                  45
   GGG GAC CTG GTG GTC TGT GCT ACA GAC GCG CTT TCC ACT GGG TAC ACT       192
   Gly Asp Leu Val Val Cys Ala Thr Asp Ala Leu Ser Thr Gly Tyr Thr
        50                  55                  60
   GGA AAT TTC GAC TCC GTC ACC GAC TGT GGA TTA GTG GTG GAG GAG GTC       240
   Gly Asn Phe Asp Ser Val Thr Asp Cys Gly Leu Val Val Glu Glu Val
    65                  70                  75                  80
   GTT GAG GTG ACC CTT GAT CCC ACC                                       264
   Val Glu Val Thr Leu Asp Pro Thr
                    85
(2) INFORMATION FOR SEQ ID NO:146:
   (i) SEQUENCE CHARACTERISTICS:
      (A) LENGTH: 88 amino acids
      (B) TYPE: amino acid
      (D) TOPOLOGY: linear
   (ii) MOLECULE TYPE: protein
   (xi) SEQUENCE DESCRIPTION: SEQ ID NO:146:
   Pro Leu Glu Arg Met Arg Thr Gly Arg His Leu Val Phe Cys His Ser
    1               5                  10                  15
   Lys Ala Glu Cys Glu Arg Leu Ala Gly Gln Phe Ser Ala Arg Gly Val
               20                  25                  30
   Asn Ala Ile Ala Tyr Tyr Arg Gly Lys Asp Ser Ser Ile Ile Lys Asp
           35                  40                  45
   Gly Asp Leu Val Val Cys Ala Thr Asp Ala Leu Ser Thr Gly Tyr Thr
       50                  55                  60
   Gly Asn Phe Asp Ser Val Thr Asp Cys Gly Leu Val Val Glu Glu Val
   65                  70                  75                  80
   Val Glu Val Thr Leu Asp Pro Thr
                   85
(2) INFORMATION FOR SEQ ID NO:147:
   (i) SEQUENCE CHARACTERISTICS:
      (A) LENGTH: 205 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone Y12-15-1
   (ix) FEATURE:
      (A) NAME/KEY: CDS
      (B) LOCATION: 1..205
   (xi) SEQUENCE DESCRIPTION: SEQ ID NO:147:
   GCT AGA TCT GGC AAC ATG GGG CAC AAG GTC TTA ATC TTG AAC CCC TCA        48
   Ala Arg Ser Gly Asn Met Gly His Lys Val Leu Ile Leu Asn Pro Ser
     1               5                  10                  15
   GTG GCC ACT GTG CGG GCC ATG GGC CCG TAC ATG GAG CGG CTG GCG GGT        96
   Val Ala Thr Val Arg Ala Met Gly Pro Tyr Met Glu Arg Leu Ala Gly
                20                  25                  30
   AAA CAT CCA AGT ATA TAC TGT GGG CAT GAT ACA ACT GCT TTC ACA AGG       144
   Lys His Pro Ser Ile Tyr Cys Gly His Asp Thr Thr Ala Phe Thr Arg
            35                  40                  45
   ATC ACT GAC TCC CCC CTG ACG TAT TCA ACC TAT GGG AGG TTT TTG GCC       192
   Ile Thr Asp Ser Pro Leu Thr Tyr Ser Thr Tyr Gly Arg Phe Leu Ala
        50                  55                  60
   AAC CCT AGG CAG A                                                     205
   Asn Pro Arg Gln
    65
(2) INFORMATION FOR SEQ ID NO:148:
   (i) SEQUENCE CHARACTERISTICS:
      (A) LENGTH: 68 amino acids
      (B) TYPE: amino acid
      (D) TOPOLOGY: linear
   (ii) MOLECULE TYPE: protein
   (xi) SEQUENCE DESCRIPTION: SEQ ID NO:148:
   Ala Arg Ser Gly Asn Met Gly His Lys Val Leu Ile Leu Asn Pro Ser
     1               5                  10                  15
   Val Ala Thr Val Arg Ala Met Gly Pro Tyr Met Glu Arg Leu Ala Gly
                20                  25                  30
   Lys His Pro Ser Ile Tyr Cys Gly His Asp Thr Thr Ala Phe Thr Arg
            35                  40                  45
   Ile Thr Asp Ser Pro Leu Thr Tyr Ser Thr Tyr Gly Arg Phe Leu Ala
   50 55 60
   Asn Pro Arg Gln
   65
(2) INFORMATION FOR SEQ ID NO:149:
   (i) SEQUENCE CHARACTERISTICS:
      (A) LENGTH: 32 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE4F
   (xi) SEQUENCE DESCRIPTION: SEQ ID NO:149:
   GCCGCCATGG CTCTCCAAGC GATCGAGAAT GC 32
(2) INFORMATION FOR SEQ ID NO:150:
   (i) SEQUENCE CHARACTERISTICS:
      (A) LENGTH: 31 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE4R
   (xi) SEQUENCE DESCRIPTION: SEQ ID NO:150:
   GCGCGGATCC CAACCCCAAT GAGAAAAAGC G 31
(2) INFORMATION FOR SEQ ID NO:151:
   (i) SEQUENCE CHARACTERISTICS:
      (A) LENGTH: 23 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470EXP3F
   (xi) SEQUENCE DESCRIPTION: SEQ ID NO:151:
   CCGCCATGGG ACGCGGACGC TCG 23
(2) INFORMATION FOR SEQ ID NO:152:
   (i) SEQUENCE CHARACTERISTICS:
      (A) LENGTH: 28 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470EXP3R
   (xi) SEQUENCE DESCRIPTION: SEQ ID NO:152:
   CGCGGATCCT TACTGTCTTA TTGCTTCC 28
(2) INFORMATION FOR SEQ ID NO:153:
   (i) SEQUENCE CHARACTERISTICS:
      (A) LENGTH: 34 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer FV94-2888F
   (xi) SEQUENCE DESCRIPTION: SEQ ID NO:153:
   GCGGAATTCT TGGCTCGGGT GGTTGAGTGC TGTG 34
(2) INFORMATION FOR SEQ ID NO:154:
   (i) SEQUENCE CHARACTERISTICS:
      (A) LENGTH: 32 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer FV94-3216R
   (xi) SEQUENCE DESCRIPTION: SEQ ID NO:154:
   GCGAAGCTTC CGTCGGATGA CAACAGGCGC GG 32
(2) INFORMATION FOR SEQ ID NO:155:
   (i) SEQUENCE CHARACTERISTICS:
      (A) LENGTH: 36 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer FV94-6521F
   (xi) SEQUENCE DESCRIPTION: SEQ ID NO:155:
   GCGGAATTCA CCTCCGCCTA TAAGCTGCTG CGCCAG 36
(2) INFORMATION FOR SEQ ID NO:156:
   (i) SEQUENCE CHARACTERISTICS:
      (A) LENGTH: 42 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer FV94-7483R
   (xi) SEQUENCE DESCRIPTION: SEQ ID NO:156:
   GCTGCGGCCG CCCTCCGTCC CACATTGTCT GGATTGGTAA CA 42
(2) INFORMATION FOR SEQ ID NO:157:
   (i) SEQUENCE CHARACTERISTICS:
      (A) LENGTH: 22 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer T7F
   (xi) SEQUENCE DESCRIPTION: SEQ ID NO:157:
   ATTAATACGA CTCACTATAG GG 22
(2) INFORMATION FOR SEQ ID NO:158:
   (i) SEQUENCE CHARACTERISTICS:
      (A) LENGTH: 22 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer T7R
   (xi) SEQUENCE DESCRIPTION: SEQ ID NO:158:
   CAAGGGGTTA TGCTAGTTAT TG 22
(2) INFORMATION FOR SEQ ID NO:159:
   (i) SEQUENCE CHARACTERISTICS:
      (A) LENGTH: 402 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone GE4-8
   (ix) FEATURE:
      (A) NAME/KEY: CDS
      (B) LOCATION: 1..402
   (xi) SEQUENCE DESCRIPTION: SEQ ID NO:159:
   GCT CTC CAA GCG ATC GAG AAT GCT GCG AGG ATT CTT GAA CCG CAC ATT        48
   Ala Leu Gln Ala Ile Glu Asn Ala Ala Arg Ile Leu Glu Pro His Ile
    1               5                  10                  15
   GAT GTC ATC ATG GAG GAC TGC AGT ACA CCC TCT CTT TGT GGT AGT AGC        96
   Asp Val Ile Met Glu Asp Cys Ser Thr Pro Ser Leu Cys Gly Ser Ser
                   20                  25                  30
   CGA GAG ATG CCT GTA TGG GGA GAA GAC ATC CCC CGT ACT CCA TCG CCA       144
   Arg Glu Met Pro Val Trp Gly Glu Asp Ile Pro Arg Thr Pro Ser Pro
               35                  40                  45
   GCA CTT ATC TCG GTT ACT GAG AGC AGC TCA GAT GAG AAG ACC CCG TCG       192
   Ala Leu Ile Ser Val Thr Glu Ser Ser Ser Asp Glu Lys Thr Pro Ser
           50                  55                  60
   GTG TCC TCC TCG CAG GAG GAT ACC CCG TCC TCT GAC TCA TTC GAG GTC       240
   Val Ser Ser Ser Gln Glu Asp Thr Pro Ser Ser Asp Ser Phe Glu Val
    65                  70                  75                  80
   ATC CAA GAG TCC GAG ACA GCC GAA GGG GAG GAA AGT GTC TTC AAC GTG       288
   Ile Gln Glu Ser Glu Thr Ala Glu Gly Glu Glu Ser Val Phe Asn Val
                       85                  90                  95
   GCT CTT TCC GTA TTA AAA GCC TTA TTT CCA CAG AGC GAC GCG ACC AGG       336
   Ala Leu Ser Val Leu Lys Ala Leu Phe Pro Gln Ser Asp Ala Thr Arg
                   100                 105                 110
   AAG CTT ACC GTC AAG ATG TCG TGC TGC GTT GAA AAG AGC GTC ACG CGC       384
   Lys Leu Thr Val Lys Met Ser Cys Cys Val Glu Lys Ser Val Thr Arg
               115                 120                 125
   TTT TTC TCA TTG GGG TTG                                               402
   Phe Phe Ser Leu Gly Leu
       130
(2) INFORMATION FOR SEQ ID NO:160:
   (i) SEQUENCE CHARACTERISTICS:
      (A) LENGTH: 134 amino acids
      (B) TYPE: amino acid
      (D) TOPOLOGY: linear
   (ii) MOLECULE TYPE: protein
   (xi) SEQUENCE DESCRIPTION: SEQ ID NO:160:
   Ala Leu Gln Ala Ile Glu Asn Ala Ala Arg Ile Leu Glu Pro His Ile
    1               5                  10                  15
   Asp Val Ile Met Glu Asp Cys Ser Thr Pro Ser Leu Cys Gly Ser Ser
               20                  25                  30
   Arg Glu Met Pro Val Trp Gly Glu Asp Ile Pro Arg Thr Pro Ser Pro
           35                  40                  45
   Ala Leu Ile Ser Val Thr Glu Ser Ser Ser Asp Glu Lys Thr Pro Ser
       50                  55                  60
   Val Ser Ser Ser Gln Glu Asp Thr Pro Ser Ser Asp Ser Phe Glu Val
   65                  70                  75                  80
   Ile Gln Glu Ser Glu Thr Ala Glu Gly Glu Glu Ser Val Phe Asn Val
                   85                  90                  95
   Ala Leu Ser Val Leu Lys Ala Leu Phe Pro Gln Ser Asp Ala Thr Arg
               100                 105                 110
   Lys Leu Thr Val Lys Met Ser Cys Cys Val Glu Lys Ser Val Thr Arg
           115                 120                 125
   Phe Phe Ser Leu Gly Leu
       130
(2) INFORMATION FOR SEQ ID NO:161:
   (i) SEQUENCE CHARACTERISTICS:
      (A) LENGTH: 1011 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone EXP3-7
   (ix) FEATURE:
      (A) NAME/KEY: CDS
      (B) LOCATION: 1..1011
   (xi) SEQUENCE DESCRIPTION: SEQ ID NO:161:
   ATG GTC TAT GGC CCT GGG CAA AGT GTT ACC ATT GAC GGG GAG CGC TAC        48
   Met Val Tyr Gly Pro Gly Gln Ser Val Thr Ile Asp Gly Glu Arg Tyr
     1               5                  10                  15
   ACC TTG CCT CAT CAA CTG AGG CTC AGG AAT GTG GCA CCC TCT GAG GTT        96
   Thr Leu Pro His Gln Leu Arg Leu Arg Asn Val Ala Pro Ser Glu Val
                    20                  25                  30
   TCA TCC GAG GTG TCC ATT GAC ATT GGG ACG GAG ACT GAA GAC TCA GAA         144
   Ser Ser Glu Val Ser Ile Asp Ile Gly Thr Glu Thr Glu Asp Ser Glu
               35                  40                  45
   CTG ACT GAG GCC GAT CTG CCG CCG GCG GCT GCT GCT CTC CAA GCG ATC         192
   Leu Thr Glu Ala Asp Leu Pro Pro Ala Ala Ala Ala Leu Gln Ala Ile
           50                  55                  60
   GAG AAT GCT GCG AGG ATT CTT GAA CCG CAC ATT GAT GTC ATC ATG GAG         240
   Glu Asn Ala Ala Arg Ile Leu Glu Pro His Ile Asp Val Ile Met Glu
    65                  70                  75                  80
   GAC TGC AGT ACA CCC TCT CTT TGT GGT AGT AGC CGA GAG ATG CCT GTA         288
   Asp Cys Ser Thr Pro Ser Leu Cys Gly Ser Ser Arg Glu Met Pro Val
                    85                  90                  95
   TGG GGA GAA GAC ATC CCC CGT ACT CCA TCG CCA GCA CTT ATC TCG GTT         336
   Trp Gly Glu Asp Ile Pro Arg Thr Pro Ser Pro Ala Leu Ile Ser Val
               100                 105                 110
   ACT GAG AGC AGC TCA GAT GAG AAG ACC CCG TCG GTG TCC TCC TCG CAG         384
   Thr Glu Ser Ser Ser Asp Glu Lys Thr Pro Ser Val Ser Ser Ser Gln
           115                 120                 125
   GAG GAT ACC CCG TCC TCT GAC TCA TTC GAG GTC ATC CAA GAG TCC GAG         432
   Glu Asp Thr Pro Ser Ser Asp Ser Phe Glu Val Ile Gln Glu Ser Glu
       130                 135                 140
   ACA GCC GAA GGG GAG GAA AGT GTC TTC AAC GTG GCT CTT TCC GTA TTA         480
   Thr Ala Glu Gly Glu Glu Ser Val Phe Asn Val Ala Leu Ser Val Leu
   145                 150                 155                 160
   AAA GCC TTA TTT CCA CAG AGC GAC GCG ACC AGG AAG CTT ACC GTC AAG         528
   Lys Ala Leu Phe Pro Gln Ser Asp Ala Thr Arg Lys Leu Thr Val Lys
                   165                 170                 175
   ATG TCG TGC TGC GTT GAA AAG AGC GTC ACG CGC TTT TTC TCA TTG GGG         576
   Met Ser Cys Cys Val Glu Lys Ser Val Thr Arg Phe Phe Ser Leu Gly
               180                 185                 190
   TTG ACG GTG GCT GAT GTT GCT AGC CTG TGT GAG ATG GAA ATC CAG AAC         624
   Leu Thr Val Ala Asp Val Ala Ser Leu Cys Glu Met Glu Ile Gln Asn
           195                 200                 205
   CAT ACA GCC TAT TGT GAC CAG GTG CGC ACT CCG CTT GAA TTG CAG GTT        672
   His Thr Ala Tyr Cys Asp Gln Val Arg Thr Pro Leu Glu Leu Gln Val
       210                 215                 220
   GGG TGC TTG GTG GGC AAT GAA CTT ACC TTT GAA TGT GAC AAG TGT GAG        720
   Gly Cys Leu Val Gly Asn Glu Leu Thr Phe Glu Cys Asp Lys Cys Glu
   225                 230                 235                 240
   GCT AGG CAA GAA ACC TTG GCC TCC TTC TCT TAC ATT TGG TCT GGA GTG        768
   Ala Arg Gln Glu Thr Leu Ala Ser Phe Ser Tyr Ile Trp Ser Gly Val
                   245                 250                 255
   CCG CTG ACT AGG GCC ACG CCG GCC AAG CCT CCC GTG GTG AGG CCG GTT        816
   Pro Leu Thr Arg Ala Thr Pro Ala Lys Pro Pro Val Val Arg Pro Val
               260                 265                 270
   GGC TCT TTG TTA GTG GCC GAC ACT ACT AAG GTG TAT GTT ACC AAT CCA        864
   Gly Ser Leu Leu Val Ala Asp Thr Thr Lys Val Tyr Val Thr Asn Pro
           275                 280                 285
   GAC AAT GTG GGA CGG AGG GTG GAC AAG GTG ACC TTC TGG CGT GCT CCT        912
   Asp Asn Val Gly Arg Arg Val Asp Lys Val Thr Phe Trp Arg Ala Pro
       290                 295                 300
   AGG GTT CAT GAT AAG TAC CTC GTG GAC TCT ATT GAG CGC GCT AAG AGG        960
   Arg Val His Asp Lys Tyr Leu Val Asp Ser Ile Glu Arg Ala Lys Arg
   305                 310                 315                 320
   GCC GCT CAA GCC TGC CTA AGC ATG GGT TAC ACT TAT GAG GAA GCA ATA       1008
   Ala Ala Gln Ala Cys Leu Ser Met Gly Tyr Thr Tyr Glu Glu Ala Ile
                   325                 330                 335
   AGG                                                                   1011
   Arg
(2) INFORMATION FOR SEQ ID NO:162:
   (i) SEQUENCE CHARACTERISTICS:
      (A) LENGTH: 337 amino acids
      (B) TYPE: amino acid
      (D) TOPOLOGY: linear
   (ii) MOLECULE TYPE: protein
   (xi) SEQUENCE DESCRIPTION: SEQ ID NO:162:
   Met Val Tyr Gly Pro Gly Gln Ser Val Thr Ile Asp Gly Glu Arg Tyr
    1               5                  10                  15
   Thr Leu Pro His Gln Leu Arg Leu Arg Asn Val Ala Pro Ser Glu Val
               20                  25                  30
   Ser Ser Glu Val Ser Ile Asp Ile Gly Thr Glu Thr Glu Asp Ser Glu
           35                  40                  45
   Leu Thr Glu Ala Asp Leu Pro Pro Ala Ala Ala Ala Leu Gln Ala Ile
       50                  55                  60
   Glu Asn Ala Ala Arg Ile Leu Glu Pro His Ile Asp Val Ile Met Glu
   65                  70                  75                  80
   Asp Cys Ser Thr Pro Ser Leu Cys Gly Ser Ser Arg Glu Met Pro Val
                   85                  90                  95
   Trp Gly Glu Asp Ile Pro Arg Thr Pro Ser Pro Ala Leu Ile Ser Val
               100                 105                 110
   Thr Glu Ser Ser Ser Asp Glu Lys Thr Pro Ser Val Ser Ser Ser Gln
           115                 120                 125
   Glu Asp Thr Pro Ser Ser Asp Ser Phe Glu Val Ile Gln Glu Ser Glu
       130                 135                 140
   Thr Ala Glu Gly Glu Glu Ser Val Phe Asn Val Ala Leu Ser Val Leu
   145                 150                 155                 160
   Lys Ala Leu Phe Pro Gln Ser Asp Ala Thr Arg Lys Leu Thr Val Lys
                   165                 170                 175
   Met Ser Cys Cys Val Glu Lys Ser Val Thr Arg Phe Phe Ser Leu Gly
               180                 185                 190
   Leu Thr Val Ala Asp Val Ala Ser Leu Cys Glu Met Glu Ile Gln Asn
           195                 200                 205
   His Thr Ala Tyr Cys Asp Gln Val Arg Thr Pro Leu Glu Leu Gln Val
       210                 215                 220
   Gly Cys Leu Val Gly Asn Glu Leu Thr Phe Glu Cys Asp Lys Cys Glu
   225 230 235 240
   Ala Arg Gln Glu Thr Leu Ala Ser Phe Ser Tyr Ile Trp Ser Gly Val
   245 250 255
   Pro Leu Thr Arg Ala Thr Pro Ala Lys Pro Pro Val Val Arg Pro Val
   260 265 270
   Gly Ser Leu Leu Val Ala Asp Thr Thr Lys Val Tyr Val Thr Asn Pro
   275 280 285
   Asp Asn Val Gly Arg Arg Val Asp Lys Val Thr Phe Trp Arg Ala Pro
   290 295 300
   Arg Val His Asp Lys Tyr Leu Val Asp Ser Ile Glu Arg Ala Lys Arg
   305 310 315 320
   Ala Ala Gln Ala Cys Leu Ser Met Gly Tyr Thr Tyr Glu Glu Ala Ile
   325 330 335
   Arg
(2) INFORMATION FOR SEQ ID NO:163:
   (i) SEQUENCE CHARACTERISTICS:
      (A) LENGTH: 351 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone GENS2b-1
   (ix) FEATURE:
      (A) NAME/KEY: CDS
      (B) LOCATION: 1..351
   (xi) SEQUENCE DESCRIPTION: SEQ ID NO:163:
   TTG GCT CGG GTG GTT GAG TGC TGT GTG ATG GCG GGT GAG AAG GCC ACA      48
   Leu Ala Arg Val Val Glu Cys Cys Val Met Ala Gly Glu Lys Ala Thr
     1               5                  10                  15
   ACC GTC CGG CTG GTC TCC AAG ATG TGT GCG AGA GGA GCT TAT TTG TTC      96
   Thr Val Arg Leu Val Ser Lys Met Cys Ala Arg Gly Ala Tyr Leu Phe
                20                  25                  30
   GAT CAT ATG GGC TCT TTT TCG CGT GCT GTC AAG GAG CGC CTG TTG GAA     144
   Asp His Met Gly Ser Phe Ser Arg Ala Val Lys Glu Arg Leu Leu Glu
            35                  40                  45
   TGG GAC GCA GCT CTT GAA CCT CTG TCA TTC ACT AGG ACG GAC TGT CGC     192
   Trp Asp Ala Ala Leu Glu Pro Leu Ser Phe Thr Arg Thr Asp Cys Arg
        50                  55                  60
   ATC ATA CGG GAT GCC GCG AGG ACT TTG TCC TGC GGG CAG TGC GTC ATG     240
   Ile Ile Arg Asp Ala Ala Arg Thr Leu Ser Cys Gly Gln Cys Val Met
    65                  70                  75                  80
   GGT TTA CCC GTG GTT GCG CGC CGT GGT GAT GAG GTT CTC ATC GGC GTC     288
   Gly Leu Pro Val Val Ala Arg Arg Gly Asp Glu Val Leu Ile Gly Val
                    85                  90                  95
   TTC CAG GAT GTG AAT CAT TTG CCT CCC GGG TTT GTT CCG ACC GCG CCT     336
   Phe Gln Asp Val Asn His Leu Pro Pro Gly Phe Val Pro Thr Ala Pro
               100                 105                 110
   GTT GTC ATC CGA CGG                                                 351
   Val Val Ile Arg Arg
           115
(2) INFORMATION FOR SEQ ID NO:164:
   (i) SEQUENCE CHARACTERISTICS:
      (A) LENGTH: 117 amino acids
      (B) TYPE: amino acid
      (D) TOPOLOGY: linear.
   (ii) MOLECULE TYPE: protein
   (xi) SEQUENCE DESCRIPTION: SEQ ID NO:164:
   Leu Ala Arg Val Val Glu Cys Cys Val Met Ala Gly Glu Lys Ala Thr
   1               5                   10                  15
   Thr Val Arg Leu Val Ser Lys Met Cys Ala Arg Gly Ala Tyr Leu Phe
               20                  25                  30
   Asp His Met Gly Ser Phe Ser Arg Ala Val Lys Glu Arg Leu Leu Glu
           35                  40                  45
   Trp Asp Ala Ala Leu Glu Pro Leu Ser Phe Thr Arg Thr Asp Cys Arg
       50                  55                  60
   Ile Ile Arg Asp Ala Ala Arg Thr Leu Ser Cys Gly Gln Cys Val Met
   65                  70                  75                  80
   Gly Leu Pro Val Val Ala Arg Arg Gly Asp Glu Val Leu Ile Gly Val
                   85                  90                  95
   Phe Gln Asp Val Asn His Leu Pro Pro Gly Phe Val Pro Thr Ala Pro
               100                 105                 110
   Val Val Ile Arg Arg
           115
(2) INFORMATION FOR SEQ ID NO:165:
   (i) SEQUENCE CHARACTERISTICS:
      (A) LENGTH: 993 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Antigen Clone GENS5a-3
   (ix) FEATURE:
      (A) NAME/KEY: CDS
      (B) LOCATION: 1..993
   (xi) SEQUENCE DESCRIPTION: SEQ ID NO:165:
   ACC TCC GCC TAT AAG CTG CTG CGC CAG CAA ATC CTA TCG GCT GCT GTA 48
   Thr Ser Ala Tyr Lys Leu Leu Arg Gln Gln Ile Leu Ser Ala Ala Val
   1 5 10 15
   GCT GAG CCC TAC TAC GTC GAC GGC ATT CCG GTC TCA TGG GAC GCG GAC 96
   Ala Glu Pro Tyr Tyr Val Asp Gly Ile Pro Val Ser Trp Asp Ala Asp
   20 25 30
   GCT CGT GCG CCC GCC ATG GTC TAT GGC CCT GGG CAA AGT GTT ACC ATT 144
   Ala Arg Ala Pro Ala Met Val Tyr Gly Pro Gly Gln Ser Val Thr Ile
   35 40 45
   GAC GGG GAG CGC TAC ACC TTG CCT CAT CAA CTG AGG CTC AGG AAT GTG 192
   Asp Gly Glu Arg Tyr Thr Leu Pro His Gln Leu Arg Leu Arg Asn Val
   50 55 60
   GCA CCC TCT GAG GTT TCA TCC GAG GTG TCC ATT GAC ATT GGG ACG GAG 240
   Ala Pro Ser Glu Val Ser Ser Glu Val Ser Ile Asp Ile Gly Thr Glu
   65 70 75 80
   ACT GAA GAC TCA GAA CTG ACT GAG GCC GAT CTG CCG CCG GCG GCT GCT 288
   Thr Glu Asp Ser Glu Leu Thr Glu Ala Asp Leu Pro Pro Ala Ala Ala
   85 90 95
   GCT CTC CAA GCG ATC GAG AAT GCT GCG AGG ATT CTT GAA CCG CAC ATT 336
   Ala Leu Gln Ala Ile Glu Asn Ala Ala Arg Ile Leu Glu Pro His Ile
   100 105 110
   GAT GTC ATC ATG GAG GAC TGC AGT ACA CCC TCT CTT TGT GGT AGT AGC 384
   Asp Val Ile Met Glu Asp Cys Ser Thr Pro Ser Leu Cys Gly Ser Ser
   115 120 125
   CGA GAG ATG CCT GTA TGG GGA GAA GAC ATC CCC CGT ACT CCA TCG CCA 432
   Arg Glu Met Pro Val Trp Gly Glu Asp Ile Pro Arg Thr Pro Ser Pro
   130 135 140
   GCA CTT ATC TCG GTT ACT GAG AGC AGC TCA GAT GAG AAG ACC CCG TCG 480
   Ala Leu Ile Ser Val Thr Glu Ser Ser Ser Asp Glu Lys Thr Pro Ser
   145 150 155 160
   GTG TCC TCC TCG CAG GAG GAT ACC CCG TCC TCT GAC TCA TTC GAG GTC 528
   Val Ser Ser Ser Gln Glu Asp Thr Pro Ser Ser Asp Ser Phe Glu Val
   165 170 175
   ATC CAA GAG TCC GAG ACA GCC GAA GGG GAG GAA AGT GTC TTC AAC GTG      576
   Ile Gln Glu Ser Glu Thr Ala Glu Gly Glu Glu Ser Val Phe Asn Val
               180                 185                 190
   GCT CTT TCC GTA TTA AAA GCC TTA TTT CCA CAG AGC GAC GCG ACC AGG      624
   Ala Leu Ser Val Leu Lys Ala Leu Phe Pro Gln Ser Asp Ala Thr Arg
           195                 200                 205
   AAG CTT ACC GTC AAG ATG TCG TGC TGC GTT GAA AAG AGC GTC ACG CGC      672
   Lys Leu Thr Val Lys Met Ser Cys Cys Val Glu Lys Ser Val Thr Arg
       210                 215                 220
   TTT TTC TCA TTG GGG TTG ACG GTG GCT GAT GTT GCT AGC CTG TGT GAG      720
   Phe Phe Ser Leu Gly Leu Thr Val Ala Asp Val Ala Ser Leu Cys Glu
   225                 230                 235                 240
   ATG GAA ATC CAG AAC CAT ACA GCC TAT TGT GAC CAG GTG CGC ACT CCG      768
   Met Glu Ile Gln Asn His Thr Ala Tyr Cys Asp Gln Val Arg Thr Pro
                   245                 250                 255
   CTT GAA TTG CAG GTT GGG TGC TTG GTG GGC AAT GAA CTT ACC TTT GAA      816
   Leu Glu Leu Gln Val Gly Cys Leu Val Gly Asn Glu Leu Thr Phe Glu
               260                 265                 270
   TGT GAC AAG TGT GAG GCT AGG CAA GAA ACC TTG GCC TCC TTC TCT TAC      864
   Cys Asp Lys Cys Glu Ala Arg Gln Glu Thr Leu Ala Ser Phe Ser Tyr
           275                 280                 285
   ATT TGG TCT GGA GTG CCG CTG ACT AGG GCC ACG CCG GCC AAG CCT CCC      912
   Ile Trp Ser Gly Val Pro Leu Thr Arg Ala Thr Pro Ala Lys Pro Pro
       290                 295                 300
   GTG GTG AGG CCG GTT GGC TCT TTG TTA GTG GCC GAC ACT ACT AAG GTG      960
   Val Val Arg Pro Val Gly Ser Leu Leu Val Ala Asp Thr Thr Lys Val
   305                 310                 315                 320
   TAT GTT ACC AAT CCA GAC AAT GTG GGA CGG AGG                          993
   Tyr Val Thr Asn Pro Asp Asn Val Gly Arg Arg
                   325                 330
(2) INFORMATION FOR SEQ ID NO:166:
   (i) SEQUENCE CHARACTERISTICS:
      (A) LENGTH: 331 amino acids
      (B) TYPE: amino acid
      (D) TOPOLOGY: linear
   (ii) MOLECULE TYPE: protein
   (xi) SEQUENCE DESCRIPTION: SEQ ID NO:166:
   Thr Ser Ala Tyr Lys Leu Leu Arg Gln Gln Ile Leu Ser Ala Ala Val
     1               5                  10                  15
   Ala Glu Pro Tyr Tyr Val Asp Gly Ile Pro Val Ser Trp Asp Ala Asp
                20                  25                  30
   Ala Arg Ala Pro Ala Met Val Tyr Gly Pro Gly Gln Ser Val Thr Ile
            35                  40                  45
   Asp Gly Glu Arg Tyr Thr Leu Pro His Gln Leu Arg Leu Arg Asn Val
        50                  55                  60
   Ala Pro Ser Glu Val Ser Ser Glu Val Ser Ile Asp Ile Gly Thr Glu
    65                  70                  75                  80
   Thr Glu Asp Ser Glu Leu Thr Glu Ala Asp Leu Pro Pro Ala Ala Ala
                    85                  90                  95
   Ala Leu Gln Ala Ile Glu Asn Ala Ala Arg Ile Leu Glu Pro His Ile
               100                 105                 110
   Asp Val Ile Met Glu Asp Cys Ser Thr Pro Ser Leu Cys Gly Ser Ser
           115                 120                 125
   Arg Glu Met Pro Val Trp Gly Glu Asp Ile Pro Arg Thr Pro Ser Pro
       130                 135                 140
   Ala Leu Ile Ser Val Thr Glu Ser Ser Ser Asp Glu Lys Thr Pro Ser
   145                 150                 155                 160
   Val Ser Ser Ser Gln Glu Asp Thr Pro Ser Ser Asp Ser Phe Glu Val
                   165                 170                 175
   Ile Gln Glu Ser Glu Thr Ala Glu Gly Glu Glu Ser Val Phe Asn Val
               180                 185                 190
   Ala Leu Ser Val Leu Lys Ala Leu Phe Pro Gln Ser Asp Ala Thr Arg
           195                 200                 205
   Lys Leu Thr Val Lys Met Ser Cys Cys Val Glu Lys Ser Val Thr Arg
       210                 215                 220
   Phe Phe Ser Leu Gly Leu Thr Val Ala Asp Val Ala Ser Leu Cys Glu
   225                 230                 235                 240
   Met Glu Ile Gln Asn His Thr Ala Tyr Cys Asp Gln Val Arg Thr Pro
                   245                 250                 255
   Leu Glu Leu Gln Val Gly Cys Leu Val Gly Asn Glu Leu Thr Phe Glu
               260                 265                 270
   Cys Asp Lys Cys Glu Ala Arg Gln Glu Thr Leu Ala Ser Phe Ser Tyr
           275                 280                 285
   Ile Trp Ser Gly Val Pro Leu Thr Arg Ala Thr Pro Ala Lys Pro Pro
       290                 295                 300
   Val Val Arg Pro Val Gly Ser Leu Leu Val Ala Asp Thr Thr Lys Val
   305                 310                 315                 320
   Tyr Val Thr Asn Pro Asp Asn Val Gly Arg Arg
                   325                 330
(2) INFORMATION FOR SEQ ID NO:167:
   (i) SEQUENCE CHARACTERISTICS:
      (A) LENGTH: 536 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 3'-end
   (xi) SEQUENCE DESCRIPTION: SEQ ID NO:167:
   CTGAGCGACC TCAAGCTCCC TGGCTTAGCA GTCCACCGAA AGAAGGCCGG GGCGTTGCGA      60
   ACACGCATGC TCCGCTCGCG CGGTTGGGCT GAGTTGGCTA GGGGCTTGTT GTGGCATCCA     120
   GGCCTACGGC TTCCTCCCCC TGAGATTGCT GGTATCCCGG GGGGTTTCCC TCTCTCCCCC     180
   CCCTATATGG GGGTGGTACA TCAATTGGAT TTCACAAGCC AGAGGAGTCG CTGGCGGTGG     240
   TTGGGGTTCT TAGCCCTGCT CATCGTAGCC CTCTTCGGGT GAACTAAATT CATCTGTTGC     300
   GGCAAGGTCT GGTGACTGAT CATCACCGGA GGAGGTTCCC GCCCTCCCCG CCCCAGGGGT     360
   CTCCCCGCTG GGTAAAAAGG GCCCGGCCTT GGGAGGCATG GTGGTTACTA ACCCCCTGGC     420
   AGGGTCAAAG CCTGATGGTG CTAATGCACT GCCACTTCGG TGGCGGGTCG CTACCTTATA     480
   GCGTAATCCG TGACTACGGG CTGCTCGCAG AGCCCTCCCC GGATGGGGCA CAGTGC         536
(2) INFORMATION FOR SEQ ID NO:168:
   (i) SEQUENCE CHARACTERISTICS:
      (A) LENGTH: 594 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Individual Clone MP3-3
   (xi) SEQUENCE DESCRIPTION: SEQ ID NO:168:
   CTGAGCGACC TCAAGCTCCC TGGCTTAGCA GTCCACCGAA AGAAGGCCGG GGCGTTGCGA      60
   ACACGCATGC TCCGCTCGCG CGGTTGGGCT GAGTTGGCTA GGGGCTTGTT GTGGCATCCA     120
   GGCCTACGGC TTCCTCCCCC TGAGATTGCT GGTATCCCGG GGGGTTTCCC TCTCTCCCCC     180
   CCCTATATGG GGGTGGTACA CCAATTGGAT TTCACAAGCC AGAGGAGTCG CTGGCGGTGG 240
   TTGGGGTTCT TAGCCCTGCT CATCGTAGCC CTCTTCGGGT GAACTAAATT CATCTGTTGC 300
   GGCAAGGTCT GGTGACTGAT CATCACCGGA GGAGGTTCCC GCCCTCCCCG CCCCAGGGGT 360
   CTCCCCGCTG GGTAAAAAGG GCCCGGCCTT GGGAGGCATG GTGGTTACTA ACCCCCTGGC 420
   AGGGTCAAAG CCTGATGGTG CTAATGCACT GCCACTTCGG TGGCGGGTCG CTACCTTATA 480
   GCGTAATCCG TGACTACGGG CTGCTCGCAG AGCCCTCCCC GGATGGGGCA CAGTGCACTG 540
   TGATCTGAAG GGGTGCACCC CGGGAAGAGC TCGGCCCGAA GGCCGGCTTC TACT 594
(2) INFORMATION FOR SEQ ID NO:169:
   (i) SEQUENCE CHARACTERISTICS:
      (A) LENGTH: 594 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Individual Clone MP3-7
   (xi) SEQUENCE DESCRIPTION: SEQ ID NO:169:
   CTGAGCGACC TCAAGCTCCC TGGCTTAGCA GTCCACCGAA AGAAGGCCGG GGCGTTGCGA 60
   ACACGCATGC TCCGCTCGCG CGGTTGGGCT GAGTTGGCTA GGGGCTTGTT GTGGCATCCA 120
   GGCCTACGGC TTCCTCCCCC TGAGATTGCT GGTGTCCCGG GGGGTTTCCC TCTCTCCCCC 180
   CCCTATATGG GGGTGGTACA CCAATTGGAT TTCACAAGCC AGAGGAGTCG CTGGCGGTGG 240
   TTGGGGTTCT TAGCCCTGCT CATCGTAGCC CTCTTCGGGT GAACTAAATT CATCTGTTGC 300
   GGCAAGGTCT GGTGACTGAT CATCACCGGA GGAGGTTCCC GCCCTCCCCG CCCCAGGGGT 360
   CTCCCCGCTG GGTAAAAAGG GCCCGGCCTT GGGAGGCATG GTGGTTACTA ACCCCCTGGC 420
   AGGGTCAAAG CCTGATGGTG CTAATGCACT GCCACTTCGG TGGCGGGTCG CTACCTTATA 480
   GCGTAATCCG TGACTACGGG CTGCTCGCAG AGCCCTCCCC GGATGGGGCA CAGTGCACTG 540
   TGATCTGAAG GGGTGCACCC CGGTAAGAGC TCGGCCCGAA GGCCGGGTTC TACT 594
(2) INFORMATION FOR SEQ ID NO:170:
   (i) SEQUENCE CHARACTERISTICS:
      (A) LENGTH: 39 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GV5446IRT
   (xi) SEQUENCE DESCRIPTION: SEQ ID NO:170:
   CGGTCCCTCG AACTCCAGCG AGTCTTTTTT TTTTTTTTT 39
(2) INFORMATION FOR SEQ ID NO:171:
   (i) SEQUENCE CHARACTERISTICS:
      (A) LENGTH: 24 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GV59-5446F
   (xi) SEQUENCE DESCRIPTION: SEQ ID NO:171:
   CTGAGCGACC TCAAGCTCCC TGGC 24
(2) INFORMATION FOR SEQ ID NO:172:
   (i) SEQUENCE CHARACTERISTICS:
      (A) LENGTH: 24 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GV-5446IR
   (xi) SEQUENCE DESCRIPTION: SEQ ID NO:172:
   CGGTCCCTCG AACTCCAGCG AGTC 24
(2) INFORMATION FOR SEQ ID NO:173:
   (i) SEQUENCE CHARACTERISTICS:
      (A) LENGTH: 23 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Probe E5-7-PRB
   (xi) SEQUENCE DESCRIPTION: SEQ ID NO:173:
   CGTAGCCCTC GGGTGAACTA AAT 23
(2) INFORMATION FOR SEQ ID NO:174:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Race Anchor Sequence
   (xi) SEQUENCE DESCRIPTION: SEQ ID NO:174:
   CACGAATTCA CTATCGATTC TGGAACCTTC AGAGG 35
(2) INFORMATION FOR SEQ ID NO:175:
   (i) SEQUENCE CHARACTERISTICS:
      (A) LENGTH: 736 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: Consensus Sequence 5'-end
   (xi) SEQUENCE DESCRIPTION: SEQ ID NO:175:
   ACGTGGGGGA GTTGATCCCC CCCCCCCGGC ACTGGGTGCA AGCCCCAGAA ACCGACGCCT     60
   ATCTAAGTAG ACGCAATGAC TCGGCGCCGA CTCGGCGACC GGCCAAAAGG TGGTGGATGG    120
   GTGATGACAG GGTTGGTAGG TCGTAAATCC CGGTCACCTT GGTAGCCACT ATAGGTGGGT    180
   CTTAAGAGAA GGTTAAGATT CCTCTTGTGC CTGCGGCGAG ACCGCGCACG GTCCACAGGT    240
   GTTGGCCCTA CCGGTGGGAA TAAGGGCCCG ACGTCAGGCT CGTCGTTAAA CCGAGCCCGT    300
   TACCCACCTG GGCAAACGAC GCCCACGTAC GGTCCACGTC GCCCTTCAAT GTCTCTCTTG    360
   ACCAATAGGC GTAGCCGGCG AGTTGACAAG GACCAGTGGG GGCCGGGGGC TTGGAGAGGG    420
   ACTCCAAGTC CCGCCCTTCC CGGTGGGCCG GGAAATGCAT GGGGCCACCC AGCTCCGCGG    480
   CGGCCTGCAG CCGGGGTAGC CCAAGAATCC TTCGGGTGAG GGCGGGTGGC ATTTCCTTTT    540
   TCTATACCAT CATGGCAGTC CTTCTGCTCC TTCTCGTGGT TGAGGCCGGG GCCATTCTGG    600
   CCCCGGCCAC CCACGCTTGT CGAGCGAATG GGCAATATTT CCTCACAAAT TGTTGTGCCC    660
   CGGAGGACAT CGGGTTCTGC CTGGAGGGTG GATGCCTGGT GGCCCTGGGG TGCACGATTT    720
   GCACTGACCA ATGCTG                                                    736
(2) INFORMATION FOR SEQ ID NO:176:
   (i) SEQUENCE CHARACTERISTICS:
      (A) LENGTH: 688 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: HGV Variant BG34
   (ix) FEATURE:
      (A) NAME/KEY: CDS
      (B) LOCATION: 272..688
   (xi) SEQUENCE DESCRIPTION: SEQ ID NO:176:
   GACTCGGCGC CGACTCGGCG ACCGGCCAAA AGGTGGTGGA TGGGTGATGA CAGGGTTGGT      60
   AGGTCGTAAA TCCCGGTCAC CTTGGTAGCC ACTATAGGTG GGTCTTAAGA GAAGGTTAAG     120
   ATTCCTCTTG TGCCTGCGGC GAGACCGCGC ACGGTCCACA GGTGTTGGCC CTACCGGTGT     180
   GAATAAGGGC CCGACGTCAG GCTCGTCGTT AAACCGAGCC CGTCACCCAC CTGGGCAAAC     240
   GACGCCCACG TACGGTCCAC GTCGCCCTTC A ATG CCT CTC TTG GCC AAT AGG        292
                                      Met Pro Leu Leu Ala Asn Arg
                                        1               5
   AGT ATC CGG CGA GTT GAC AAG GAC CAG TGG GGG CCG GGA GTC ACG GGG       340
   Ser Ile Arg Arg Val Asp Lys Asp Gln Trp Gly Pro Gly Val Thr Gly
            10                  15                  20
   ATG GAC CCC GGG CTC TGC CCT TCC CGG TGG AAC GGG AAA CGC ATG GGG       388
   Met Asp Pro Gly Leu Cys Pro Ser Arg Trp Asn Gly Lys Arg Met Gly
        25                  30                  35
   CCA CCC AGC TCC GCG GCG GCC TGC AGC CGG GGT AGC CCA AGA ACC CTT       436
   Pro Pro Ser Ser Ala Ala Ala Cys Ser Arg Gly Ser Pro Arg Thr Leu
    40                  45                  50                  55
   CGG GTG AGG GCG GGT GGC ATT TCT CTT TTC TGT ATC ATC ATG GCA GTC       484
   Arg Val Arg Ala Gly Gly Ile Ser Leu Phe Cys Ile Ile Met Ala Val
                    60                  65                  70
   CTC CTG CTC CTT CTC GTG GTT GAG GCC GGG GCC ATT CTG GCC CCG GCC       532
   Leu Leu Leu Leu Leu Val Val Glu Ala Gly Ala Ile Leu Ala Pro Ala
                75                  80                  85
   ACC CAC GCT TGT CGA GCG AAT GGA CAA TAT TTC CTC ACA AAC TGT TGC      580
   Thr His Ala Cys Arg Ala Asn Gly Gln Tyr Phe Leu Thr Asn Cys Cys
            90                  95                  100
   GCC CTC GAG GAC ATC GGG TTC TGC CTG GAA GGC GGG TGC CTG GTG GCC      628
   Ala Leu Glu Asp Ile Gly Phe Cys Leu Glu Gly Gly Cys Leu Val Ala
       105                 110                 115
   TTA GGG TGC ACC ATT TGC ACT GAC CGT TGC TGG CCA CTG TAT CAG GCG      676
   Leu Gly Cys Thr Ile Cys Thr Asp Arg Cys Trp Pro Leu Tyr Gln Ala
   120                 125                 130                 135
   GGT TTG GCT GTG                                                      688
   Gly Leu Ala Val
(2) INFORMATION FOR SEQ ID NO:177:
   (i) SEQUENCE CHARACTERISTICS:
      (A) LENGTH: 139 amino acids
      (B) TYPE: amino acid
      (D) TOPOLOGY: linear
   (ii) MOLECULE TYPE: protein
   (xi) SEQUENCE DESCRIPTION: SEQ ID NO:177:
   Met Pro Leu Leu Ala Asn Arg Ser Ile Arg Arg Val Asp Lys Asp Gln
     1               5                  10                  15
   Trp Gly Pro Gly Val Thr Gly Met Asp Pro Gly Leu Cys Pro Ser Arg
                20                  25                  30
   Trp Asn Gly Lys Arg Met Gly Pro Pro Ser Ser Ala Ala Ala Cys Ser
            35                  40                  45
   Arg Gly Ser Pro Arg Thr Leu Arg Val Arg Ala Gly Gly Ile Ser Leu
        50                  55                  60
   Phe Cys Ile Ile Met Ala Val Leu Leu Leu Leu Leu Val Val Glu Ala
    65                  70                  75                  80
   Gly Ala Ile Leu Ala Pro Ala Thr His Ala Cys Arg Ala Asn Gly Gln
                    85                  90                  95
   Tyr Phe Leu Thr Asn Cys Cys Ala Leu Glu Asp Ile Gly Phe Cys Leu
               100                 105                 110
   Glu Gly Gly Cys Leu Val Ala Leu Gly Cys Thr Ile Cys Thr Asp Arg
           115                 120                 125
   Cys Trp Pro Leu Tyr Gln Ala Gly Leu Ala Val
       130                 135
(2) INFORMATION FOR SEQ ID NO:178:
   (i) SEQUENCE CHARACTERISTICS:
      (A) LENGTH: 663 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: HGV Variant T55806
   (ix) FEATURE:
      (A) NAME/KEY: CDS
      (B) LOCATION: 271..663
   (xi) SEQUENCE DESCRIPTION: SEQ ID NO:178:
   GACTCGGCGC CGACTCGGCG ACCGGCCAAA AGGTGGTGGA TGGGTGATGC CAGGGTTGGT      60
   AGGTCGTAAA TCCCGGTCAT CTTGGTAGCC ACTATAGGTG GGTCTTAAGA GAAGGTTAAG     120
   ATTCCTCTTG TGCCTGCGGC GAGACCGCGC ACGGTCCACA GGTGTTGGCC CTACCGGTGG     180
   AATAAGGGCC CGACGTCAGG CTCGTCGTTA AACCGAGCCC GTCACCCACC TGGGCAAACG     240
   ACGCTCACGT ACGGTCCACG TCGCCCTTCA ATG TCT CTC TTG ACC AAT AGG TTT      294
                                    Met Ser Leu Leu Thr Asn Arg Phe
                                      1               5
   ATC CGG CGA GTT GAC AAG GAC CAG TGG GGG CCG GGG GTT ACG GGG ACG        342
   Ile Arg Arg Val Asp Lys Asp Gln Trp Gly Pro Gly Val Thr Gly Thr
        10                  15                  20
   GAC CCC GAA CCC TGC CCT TCC CGG TGG GCC GGG AAA TGC ATG GGG CCA        390
   Asp Pro Glu Pro Cys Pro Ser Arg Trp Ala Gly Lys Cys Met Gly Pro
    25                  30                  35                  40
   CCC AGC TCC GCG GCG GCC TGC AGC CGG GGT AGC CCA AGA ATC CTT CGG        438
   Pro Ser Ser Ala Ala Ala Cys Ser Arg Gly Ser Pro Arg Ile Leu Arg
                    45                  50                  55
   GTG AGG GCG GGT GGC ATT TCT CTT TTC TAT ACC ATC ATG GCA GTC CTT        486
   Val Arg Ala Gly Gly Ile Ser Leu Phe Tyr Thr Ile Met Ala Val Leu
                60                  65                  70
   CTG CTC TTC TTC GTG GTT GAG GCC GGG GCG ATT CTC GCC CCG GCC ACC        534
   Leu Leu Phe Phe Val Val Glu Ala Gly Ala Ile Leu Ala Pro Ala Thr
            75                  80                  85
   CAC GCT TGT CGG GCG AAT GGG CAA TAT TTC CTC ACA AAT TGT TGC GCC        582
   His Ala Cys Arg Ala Asn Gly Gln Tyr Phe Leu Thr Asn Cys Cys Ala
        90                  95                 100
   CCA GAG GAT GTT GGG TTC TGC CTG GAG GGC GGA TGC CTG GTG GCT CTG        630
   Pro Glu Asp Val Gly Phe Cys Leu Glu Gly Gly Cys Leu Val Ala Leu
   105                 110                 115                 120
   GGG TGT ACG ATT TGC ACT GAC CGT TGC TGG CCA                            663
   Gly Cys Thr Ile Cys Thr Asp Arg Cys Trp Pro
                   125                 130
(2) INFORMATION FOR SEQ ID NO:179:
   (i) SEQUENCE CHARACTERISTICS:
      (A) LENGTH: 131 amino acids
      (B) TYPE: amino acid
      (D) TOPOLOGY: linear
   (ii) MOLECULE TYPE: protein
   (xi) SEQUENCE DESCRIPTION: SEQ ID NO:179:
   Met Ser Leu Leu Thr Asn Arg Phe Ile Arg Arg Val Asp Lys Asp Gln
   1               5                   10                  15
   Trp Gly Pro Gly Val Thr Gly Thr Asp Pro Glu Pro Cys Pro Ser Arg
               20                  25                  30
   Trp Ala Gly Lys Cys Met Gly Pro Pro Ser Ser Ala Ala Ala Cys Ser
           35                  40                  45
   Arg Gly Ser Pro Arg Ile Leu Arg Val Arg Ala Gly Gly Ile Ser Leu
       50                  55                  60
   Phe Tyr Thr Ile Met Ala Val Leu Leu Leu Phe Phe Val Val Glu Ala
   65                  70                  75                  80
   Gly Ala Ile Leu Ala Pro Ala Thr His Ala Cys Arg Ala Asn Gly Gln
                   85                  90                  95
   Tyr Phe Leu Thr Asn Cys Cys Ala Pro Glu Asp Val Gly Phe Cys Leu
               100                 105                 110
   Glu Gly Gly Cys Leu Val Ala Leu Gly Cys Thr Ile Cys Thr Asp Arg
           115                 120                 125
   Cys Trp Pro
       130
(2) INFORMATION FOR SEQ ID NO:180:
   (i) SEQUENCE CHARACTERISTICS:
      (A) LENGTH: 632 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: HGV Variant EB20-2
   (ix) FEATURE:
      (A) NAME/KEY: CDS
      (B) LOCATION: 271..632
   (xi) SEQUENCE DESCRIPTION: SEQ ID NO:180:
   GACTCGGCGC CGACTCGGCG ACCGGCCAAA AGGTGGTGGA TGGGTGATGC CAGGGTTGGT       60
   AGGTCGTAAA TCCCGGTCAT CTTGGTAGCC ACTATAGGTG GGTCTTAAGA GAAGGTTAAG      120
   ATTCCTCTTG TGCCTGCGGC GAGACCGCGC ACGGTCCACA GGTGTTGGCC CTACCGGTGT      180
   AATAAGGGCC CGACGTCAGG CTCGTCGTTA AACCGAGCCC GTCACCCACC TGGGCAAACG      240
   ACGCCCACGT ACGGTCCACG TCGCCCTTCA ATG CCT CTC TTG GCC AAT AGG AGT      294
                                    Met Pro Leu Leu Ala Asn Arg Ser
                                      1               5
   TAT CTC CGG CGA GTT GGC AAG GAC CAG TGG GGG CCG GGG GTT ACG GGG        342
   Tyr Leu Arg Arg Val Gly Lys Asp Gln Trp Gly Pro Gly Val Thr Gly
        10                  15                  20
   AAG GAC CCC GAA CCC TGC CCT TCC CGG TGG GCC GGG AAA TGC ATG GGG        390
   Lys Asp Pro Glu Pro Cys Pro Ser Arg Trp Ala Gly Lys Cys Met Gly
    25                  30                  35                  40
   CCA CCC AGC TCC GCG GCG GCC TGC AGC CGG GGT AGC CCA AAA AAC CTT        438
   Pro Pro Ser Ser Ala Ala Ala Cys Ser Arg Gly Ser Pro Lys Asn Leu
                    45                  50                  55
   CGG GTG AGG GCG GGT GGC ATT TTC TTT TCC TAT ACC ATC ATG GCA GTC        486
   Arg Val Arg Ala Gly Gly Ile Phe Phe Ser Tyr Thr Ile Met Ala Val
                60                  65                  70
   CTT CTG CTC CTT CTC GTG GTT GAG GCC GGG GCC ATT TTG GCC CCG GCC        534
   Leu Leu Leu Leu Leu Val Val Glu Ala Gly Ala Ile Leu Ala Pro Ala
            75                  80                  85
   ACC CAC GCT TGC AGA GCT AAT GGG CAA TAT TTC CTC ACA AAC TGT TGT        582
   Thr His Ala Cys Arg Ala Asn Gly Gln Tyr Phe Leu Thr Asn Cys Cys
        90                  95                 100
   GCC TTG GAG GAC ATC GGG TTC TGC CTG GAA GGC GGA TGC TTG GTG GCG CT     632
   Ala Leu Glu Asp Ile Gly Phe Cys Leu Glu Gly Gly Cys Leu Val Ala
   105                 110                 115                 120
(2) INFORMATION FOR SEQ ID NO:181:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (xi) SEQUENCE DESCRIPTION: SEQ ID NO:181:
   Met Pro Leu Leu Ala Asn Arg Ser Tyr Leu Arg Arg Val Gly Lys Asp
    1               5                  10                   15
   Gln Trp Gly Pro Gly Val Thr Gly Lys Asp Pro Glu Pro Cys Pro Ser
               20                  25                  30
   Arg Trp Ala Gly Lys Cys Met Gly Pro Pro Ser Ser Ala Ala Ala Cys
           35                  40                  45
   Ser Arg Gly Ser Pro Lys Asn Leu Arg Val Arg Ala Gly Gly Ile Phe
       50                  55                  60
   Phe Ser Tyr Thr Ile Met Ala Val Leu Leu Leu Leu Leu Val Val Glu
    65                 70                  75                  80
   Ala Gly Ala Ile Leu Ala Pro Ala Thr His Ala Cys Arg Ala Asn Gly
                   85                  90                  95
   Gln Tyr Phe Leu Thr Asn Cys Cys Ala Leu Glu Asp Ile Gly Phe Cys
               100                 105                 110
   Leu Glu Gly Gly Cys Leu Val Ala
           115                 120
(2) INFORMATION FOR SEQ ID NO:182:
   (i) SEQUENCE CHARACTERISTICS:
      (A) LENGTH: 9103 base pairs
      (B) TYPE: nucleic acid
      (C) STRANDEDNESS: both
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: HGV-JC Variant
   (ix) FEATURE:
      (A) NAME/KEY: CDS
      (B) LOCATION: 276..9005
   (xi) SEQUENCE DESCRIPTION: SEQ ID NO:182:
   CAATGACTCG GCGCCGACTC GGCGACCGGC CAAAAGGTGG TGGATGGGTG ATGACAGGGT       60
   TGGTAGGTCG TAAATCCCGG TCACCTTGGT AGCCACTATA GGTGGGTCTT AAGAGAAGGT      120
   TAAGATTCCT CTTGTGCCTG CGGCGAGACC GCGCACGGTC CACAGGTGTT GGCCCTACCG      180
   GTGGGAATAA GGGCCCGACG TCAGGCTCGT CGTTAAACCG AGCCCGTAAC CCGCCTGGGC      240
   AAACGACGCC CACGTACGGT CCACGTCGCC CTTCA ATG TCG CTC TTG ACC AAT         293
                                          Met Ser Leu Leu Thr Asn
                                            1               5
   AGG CTT AGC CGG CGA GTT GAC AAG GAC CAG TGG GGG CCG GGG TTT ATG        341
   Arg Leu Ser Arg Arg Val Asp Lys Asp Gln Trp Gly Pro Gly Phe Met
                10                  15                  20
   GGG AAG GAC CCC AAA CCC TGC CCT TCC CGG CGG ACC GGG AAA TGC ATG        389
   Gly Lys Asp Pro Lys Pro Cys Pro Ser Arg Arg Thr Gly Lys Cys Met
            25                  30                  35
   GGG CCA CCC AGC TCC GCG GCG GCC TGC AGC CGG GGT AGC CCA AGA ATC        437
   Gly Pro Pro Ser Ser Ala Ala Ala Cys Ser Arg Gly Ser Pro Arg Ile
        40                  45                  50
   CTT CGG GTG AGG GCG GGT GGC ATT TCT CTT CCT TAT ACC ATC ATG GAA        485
   Leu Arg Val Arg Ala Gly Gly Ile Ser Leu Pro Tyr Thr Ile Met Glu
    55                  60                  65                  70
   GCC CTC CTG TTC CTC CTC GGG GTG GAG GCC GGG GCC ATT CTG GCC CCG        533
   Ala Leu Leu Phe Leu Leu Gly Val Glu Ala Gly Ala Ile Leu Ala Pro
                   75                  80                  85
   GCC ACC CAC GCT TGT CGA GCG AAT GGG CAA TAT TTC CTC ACA AAC TGT      581
   Ala Thr His Ala Cys Arg Ala Asn Gly Gln Tyr Phe Leu Thr Asn Cys
               90                  95                  100
   TGT GCT CCA GAG GAC ATT GGG TTC TGC CTC GAA GGC GGT TGC CTT GTG      629
   Cys Ala Pro Glu Asp Ile Gly Phe Cys Leu Glu Gly Gly Cys Leu Val
           105                 110                 115
   GCC CTG GGG TGC ACA GTT TGC ACT GAC CGA TGC TGG CCG CTG TAT CAG      677
   Ala Leu Gly Cys Thr Val Cys Thr Asp Arg Cys Trp Pro Leu Tyr Gln
       120                 125                 130
   GCG GGC TTG GCT GTG CGG CCT GGC AAG TCC GCA GCC CAG CTG GTG GGG      725
   Ala Gly Leu Ala Val Arg Pro Gly Lys Ser Ala Ala Gln Leu Val Gly
   135                 140                 145                 150
   CAA CTG GGT GGC CTC TAC GGG CCC TTG TCG GTG TCG GCC TAC GTG GCC      773
   Gln Leu Gly Gly Leu Tyr Gly Pro Leu Ser Val Ser Ala Tyr Val Ala
                   155                 160                 165
   GGC ATC CTG GGC CTG GGT GAG GTG TAC TCG GGT GTC CTA ACA GTT GGT      821
   Gly Ile Leu Gly Leu Gly Glu Val Tyr Ser Gly Val Leu Thr Val Gly
               170                 175                 180
   GTT GCG TTG ACG CGC CGG GTC TAC CCG ATG CCC AAC CTG ACG TGT GCA      869
   Val Ala Leu Thr Arg Arg Val Tyr Pro Met Pro Asn Leu Thr Cys Ala
           185                 190                 195
   GTA GAG TGT GAG CTT AAG TGG GAA AGT GAG TTT TGG AGA TGG ACT GAG      917
   Val Glu Cys Glu Leu Lys Trp Glu Ser Glu Phe Trp Arg Trp Thr Glu
       200                 205                 210
   CAG CTG GCC TCC AAT TAC TGG ATT CTG GAA TAC CTT TGG AAG GTC CCG      965
   Gln Leu Ala Ser Asn Tyr Trp Ile Leu Glu Tyr Leu Trp Lys Val Pro
   215                 220                 225                 230
   TTT GAC TTC TGG AGA GGC GTG CTA AGC CTG ACT CCC TTG CTG GTT TGC     1013
   Phe Asp Phe Trp Arg Gly Val Leu Ser Leu Thr Pro Leu Leu Val Cys
                   235                 240                 245
   GTG GCC GCG TTG CTG CTG CTG GAG CAA CGG ATT GTC ATG GTC TTC CTG     1061
   Val Ala Ala Leu Leu Leu Leu Glu Gln Arg Ile Val Met Val Phe Leu
   250 255 260
   TTG GTG ACG ATG GCC GGG ATG TCG CAA GGC GCT CCG GCC TCC GTT TTG 1109
   Leu Val Thr Met Ala Gly Met Ser Gln Gly Ala Pro Ala Ser Val Leu
   265 270 275
   GGG TCT CGC CCC TTT GAC TAC GGG TTG ACA TGG CAG TCT TGT TCC TGC 1157
   Gly Ser Arg Pro Phe Asp Tyr Gly Leu Thr Trp Gln Ser Cys Ser Cys
   280 285 290
   AGG GCT AAT GGG TCG CGC TAT ACT ACT GGG GAG AAG GTG TGG GAC CGT 1205
   Arg Ala Asn Gly Ser Arg Tyr Thr Thr Gly Glu Lys Val Trp Asp Arg
   295 300 305 310
   GGG AAC GTC ACG CTC CTG TGT GAC TGC CCC AAC GGC CCC TGG GTG TGG 1253
   Gly Asn Val Thr Leu Leu Cys Asp Cys Pro Asn Gly Pro Trp Val Trp
   315 320 325
   TTG CCG GCC TTT TGC CAA GCA ATC GGC TGG GGC GAT CCC ATC ACT CAT 1301
   Leu Pro Ala Phe Cys Gln Ala Ile Gly Trp Gly Asp Pro Ile Thr His
   330 335 340
   TGG AGC CAC GGC CAA AAT CGG TGG CCC CTC TCA TGC CCC CAG TAT GTC 1349
   Trp Ser His Gly Gln Asn Arg Trp Pro Leu Ser Cys Pro Gln Tyr Val
   345 350 355
   TAT GGG TCT GTT TCA GTC ACT TGC GTG TGG GGT TCC GTC TCT TGG TTT 1397
   Tyr Gly Ser Val Ser Val Thr Cys Val Trp Gly Ser Val Ser Trp Phe
   360 365 370
   GCC TCG ACT GGC GGT CGC GAC TCG AAG ATC GAT GTG TGG AGT CTG GTG 1445
   Ala Ser Thr Gly Gly Arg Asp Ser Lys Ile Asp Val Trp Ser Leu Val
   375 380 385 390
   CCG GTT GGT TCC GCC AGC TGC ACC ATA GCC GCT CTT GGA TCG TCG GAT 1493
   Pro Val Gly Ser Ala Ser Cys Thr Ile Ala Ala Leu Gly Ser Ser Asp
   395 400 405
   CGG GAC ACG GTA GTT GAG CTC TCC GAG TGG GGA GTC CCG TGC GCA ACG 1541
   Arg Asp Thr Val Val Glu Leu Ser Glu Trp Gly Val Pro Cys Ala Thr
   410 415 420
   TGC ATT CTG GAT CGT CGG CCG GCC TCG TGC GGC ACC TGT GTG AGA GAC 1589
   Cys Ile Leu Asp Arg Arg Pro Ala Ser Cys Gly Thr Cys Val Arg Asp
   425 430 435
   TGC TGG CCC GAA ACC GGG TCG GTT AGG TTT CCA TTC CAT CGG TGC GGC 1637
   Cys Trp Pro Glu Thr Gly Ser Val Arg Phe Pro Phe His Arg Cys Gly
   440 445 450
   GCG GGG CCT AAG CTG ACA AAG GAC TTG GAA GCT GTG CCC TTC GTC AAT 1685
   Ala Gly Pro Lys Leu Thr Lys Asp Leu Glu Ala Val Pro Phe Val Asn
   455 460 465 470
   AGG ACA ACT CCC TTC ACC ATA AGG GGC CCC CTG GGC AAC CAG GGG AGA 1733
   Arg Thr Thr Pro Phe Thr Ile Arg Gly Pro Leu Gly Asn Gln Gly Arg
   475 480 485
   GGC AAC CCG GTG CGG TCG CCC TTG GGT TTT GGG TCC TAC GCC ATG ACC 1781
   Gly Asn Pro Val Arg Ser Pro Leu Gly Phe Gly Ser Tyr Ala Met Thr
   490 495 500
   AAG ATC CGA GAC TCC TTA CAT TTG GTG AAA TGT CCC ACA CCA GCC ATT 1829
   Lys Ile Arg Asp Ser Leu His Leu Val Lys Cys Pro Thr Pro Ala Ile
   505 510 515
   GAG CCT CCC ACC GGG ACG TTT GGG TTC TTC CCC GGA GTG CCG CCT CTT 1877
   Glu Pro Pro Thr Gly Thr Phe Gly Phe Phe Pro Gly Val Pro Pro Leu
   520 525 530
   AAC AAC TGC CTG CTG TTG GGC ACG GAA GTG TCC GAA GCG CTG GGC GGG 1925
   Asn Asn Cys Leu Leu Leu Gly Thr Glu Val Ser Glu Ala Leu Gly Gly
   535 540 545 550
   GCC GGC CTC ACG GGG GGG TTC TAT GAA CCC CTG GTG CGC AGG CGT TCG 1973
   Ala Gly Leu Thr Gly Gly Phe Tyr Glu Pro Leu Val Arg Arg Arg Ser
   555 560 565
   GAG CTG ATG GGG CGC CGA AAT CCG GTT TGC CCG GGG TTT GCA TGG CTG 2021
   Glu Leu Met Gly Arg Arg Asn Pro Val Cys Pro Gly Phe Ala Trp Leu
   570 575 580
   TCC TCG GGT CGA CCT GAC GGG TTT ATA CAC GTC CAG GGC CAC TTG CAG 2069
   Ser Ser Gly Arg Pro Asp Gly Phe Ile His Val Gln Gly His Leu Gln
   585 590 595
   GAG GTC GAT GCT GGC AAC TTC ATC CCT CCA CCT CGC TGG TTG CTC TTG 2117
   Glu Val Asp Ala Gly Asn Phe Ile Pro Pro Pro Arg Trp Leu Leu Leu
   600 605 610
   GAC TTT GTG TTT GTC CTG TTA TAC CTG ATG AAG CTG GCT GAG GCA CGG 2165
   Asp Phe Val Phe Val Leu Leu Tyr Leu Met Lys Leu Ala Glu Ala Arg
   615                 620                 625                 630
   CTG GTC CCG TTG ATC TTG CTT CTG CTG TGG TGG TGG GTG AAC CAG TTG      2213
   Leu Val Pro Leu Ile Leu Leu Leu Leu Trp Trp Trp Val Asn Gln Leu
                   635                 640                 645
   GCA GTC CTT GGA CTG CCG GCT GTG GAC GCC GCC GTG GCT GGT GAG GTC      2261
   Ala Val Leu Gly Leu Pro Ala Val Asp Ala Ala Val Ala Gly Glu Val
               650                 655                 660
   TTC GCG GGC CCG GCC CTG TCG TGG TGT CTG GGC CTC CCC ACC GTT AGT      2309
   Phe Ala Gly Pro Ala Leu Ser Trp Cys Leu Gly Leu Pro Thr Val Ser
           665                 670                 675
   ATG ATC CTG GGC TTA GCA AAC CTG GTG TTG TAT TTC CGG TGG ATG GGT      2357
   Met Ile Leu Gly Leu Ala Asn Leu Val Leu Tyr Phe Arg Trp Met Gly
       680                 685                 690
   CCC CAA CGC CTC ATG TTC CTC GTG TTG TGG AAG CTC GCT CGG GGA GCC      2405
   Pro Gln Arg Leu Met Phe Leu Val Leu Trp Lys Leu Ala Arg Gly Ala
   695                 700                 705                 710
   TTC CCG CTG GCA CTT CTG ATG GGG ATC TCG GCA ACC CGC GGG CGC ACC      2453
   Phe Pro Leu Ala Leu Leu Met Gly Ile Ser Ala Thr Arg Gly Arg Thr
                   715                 720                 725
   TCG GTG CTC GGG GCC GAG TTC TGC TTC GAT GTC ACA TTC GAG GTG GAC      2501
   Ser Val Leu Gly Ala Glu Phe Cys Phe Asp Val Thr Phe Glu Val Asp
               730                 735                 740
   ACG TCG GTT TTG GGC TGG GTG GTG GCC AGT GTG GTA GCC TGG GCC ATT      2549
   Thr Ser Val Leu Gly Trp Val Val Ala Ser Val Val Ala Trp Ala Ile
           745                 750                 755
   GCG CTC CTG AGC TCG ATG AGC GCG GGA GGG TGG AGG CAC AAG GCC GTG      2597
   Ala Leu Leu Ser Ser Met Ser Ala Gly Gly Trp Arg His Lys Ala Val
       760                 765                 770
   ATC TAT AGG ACG TGG TGT AAG GGG TAC CAG GCA ATA CGC CAA CGG GTG      2645
   Ile Tyr Arg Thr Trp Cys Lys Gly Tyr Gln Ala Ile Arg Gln Arg Val
   775                 780                 785                 790
   GTG CGG AGC CCC CTC GGG GAG GGG CGG CCC ACC AAA CCC TTG ACG TTT      2693
   Val Arg Ser Pro Leu Gly Glu Gly Arg Pro Thr Lys Pro Leu Thr Phe
   795 800 805
   GCT TGG TGC TTG GCC TCA TAC ATC TGG CCG GAT GCT GTG ATG ATG GTG 2741
   Ala Trp Cys Leu Ala Ser Tyr Ile Trp Pro Asp Ala Val Met Met Val
   810 815 820
   GTG GTA GCC TTG GTG CTC CTC TTT GGC CTG TTC GAC GCG TTG GAC TGG 2789
   Val Val Ala Leu Val Leu Leu Phe Gly Leu Phe Asp Ala Leu Asp Trp
   825 830 835
   GCT TTG GAG GAG CTC TTG GTG TCC CGG CCC TCG TTA CGG CGT CTG GCC 2837
   Ala Leu Glu Glu Leu Leu Val Ser Arg Pro Ser Leu Arg Arg Leu Ala
   840 845 850
   CGG GTG GTT GAG TGC TGT GTG ATG GCG GGA GAG AAG GCC ACA ACC GTC 2885
   Arg Val Val Glu Cys Cys Val Met Ala Gly Glu Lys Ala Thr Thr Val
   855 860 865 870
   CGG CTG GTC TCC AAG ATG TGC GCG AGA GGG GCC TAT TTG TTT GAC CAT 2933
   Arg Leu Val Ser Lys Met Cys Ala Arg Gly Ala Tyr Leu Phe Asp His
   875 880 885
   ATG GGC TCT TTT TCG CGC GCT GTC AAG GAG CGC CTG CTG GAG TGG GAC 2981
   Met Gly Ser Phe Ser Arg Ala Val Lys Glu Arg Leu Leu Glu Trp Asp
   890 895 900
   GCG GCT TTG GAA CCC CTG TCA TTC ACT AGG ACG GAC TGT CGC ATC ATT 3029
   Ala Ala Leu Glu Pro Leu Ser Phe Thr Arg Thr Asp Cys Arg Ile Ile
   905 910 915
   AGA GAT GCT GCG AGG ACC TTG GCC TGC GGG CAG TGC GTC ATG GGC TTG 3077
   Arg Asp Ala Ala Arg Thr Leu Ala Cys Gly Gln Cys Val Met Gly Leu
   920 925 930
   CCT GTG GTA GCG CGC CGT GGT GAC GAG GTT CTT ATC GGT GTC TTT CAG 3125
   Pro Val Val Ala Arg Arg Gly Asp Glu Val Leu Ile Gly Val Phe Gln
   935 940 945 950
   GAT GTG AAC CAT TTG CCT CCC GGA TTC GTC CCG ACC GCA CCC GTT GTC 3173
   Asp Val Asn His Leu Pro Pro Gly Phe Val Pro Thr Ala Pro Val Val
   955 960 965
   ATC CGG CGG TGC GGG AAG GGG TTT CTG GGG GTC ACT AAG GCT GCC TTG 3221
   Ile Arg Arg Cys Gly Lys Gly Phe Leu Gly Val Thr Lys Ala Ala Leu
   970 975 980
   ACT GGT CGG GAT CCT GAC TTA CAT CCA GGG AAC GTC ATG GTG TTG GGG       3269
   Thr Gly Arg Asp Pro Asp Leu His Pro Gly Asn Val Met Val Leu Gly
           985                 990                 995
   ACG GCT ACG TCG CGA AGC ATG GGG ACA TGC CTG AAC GGC CTG CTG TTC       3317
   Thr Ala Thr Ser Arg Ser Met Gly Thr Cys Leu Asn Gly Leu Leu Phe
       1000                1005                1010
   ACG ACT TTC CAT GGG GCT TCA TCC CGA ACC ATC GCC ACG CCC GTG GGG       3365
   Thr Thr Phe His Gly Ala Ser Ser Arg Thr Ile Ala Thr Pro Val Gly
   1015                1020                1025                1030
   GCC CTT AAT CCC AGG TGG TGG TCC GCC AGT GAT GAC GTC ACG GTG TAC       3413
   Ala Leu Asn Pro Arg Trp Trp Ser Ala Ser Asp Asp Val Thr Val Tyr
                   1035                1040                1045
   CCG CTC CCG GAT GGG GCA ACC TCG TTG ACG CCC TGC ACT TGC CAG GCT       3461
   Pro Leu Pro Asp Gly Ala Thr Ser Leu Thr Pro Cys Thr Cys Gln Ala
               1050                1055                1060
   GAG TCC TGT TGG GTC ATA CGG TCC GAC GGG GCT TTG TGC CAT GGC TTG       3509
   Glu Ser Cys Trp Val Ile Arg Ser Asp Gly Ala Leu Cys His Gly Leu
           1065                1070                1075
   AGT AAG GGA GAC AAG GTG GAG CTA GAT GTG GCC ATG GAG GTC TCA GAT       3557
   Ser Lys Gly Asp Lys Val Glu Leu Asp Val Ala Met Glu Val Ser Asp
       1080                1085                1090
   TTC CGT GGC TCG TCC GGC TCA CCT GTC CTG TGC GAC GAG GGG CAC GCA       3605
   Phe Arg Gly Ser Ser Gly Ser Pro Val Leu Cys Asp Glu Gly His Ala
   1095                1100                1105                1110
   GTA GGA ATG CTC GTG TCG GTG CTC CAC TCG GGT GGT CGG GTC ACC GCG       3653
   Val Gly Met Leu Val Ser Val Leu His Ser Gly Gly Arg Val Thr Ala
                   1115                1120                1125
   GCT CGA TTC ACC AGG CCG TGG ACC CAG GTC CCA ACA GAT GCT AAG ACC       3701
   Ala Arg Phe Thr Arg Pro Trp Thr Gln Val Pro Thr Asp Ala Lys Thr
               1130                1135                1140
   ACC ACT GAA CCC CCT CCG GTG CCG GCA AAG GGA GTT TTC AAG GAA GCC       3749
   Thr Thr Glu Pro Pro Pro Val Pro Ala Lys Gly Val Phe Lys Glu Ala
           1145                1150                1155
   CCA CTG TTT ATG CCC ACG GGC GCA GGA AAG AGC ACG CGC GTC CCG TTG       3797
   Pro Leu Phe Met Pro Thr Gly Ala Gly Lys Ser Thr Arg Val Pro Leu
       1160                1165                1170
   GAG TAT GGC AAC ATG GGG CAC AAG GTC CTG ATT TTG AAC CCC TCG GTG      3845
   Glu Tyr Gly Asn Met Gly His Lys Val Leu Ile Leu Asn Pro Ser Val
   1175                1180                1185                1190
   GCG ACA GTG AGG GCC ATG GGC CCT TAC ATG GAG CGA CTG GCG GGA AAA      3893
   Ala Thr Val Arg Ala Met Gly Pro Tyr Met Glu Arg Leu Ala Gly Lys
                   1195                1200                1205
   CAT CCA AGT ATC TAC TGT GGC CAT GAC ACC ACT GCC TTC ACA AGG ATC      3941
   His Pro Ser Ile Tyr Cys Gly His Asp Thr Thr Ala Phe Thr Arg Ile
               1210                1215                1220
   ACT GAT TCC CCC TTA ACG TAC TCT ACC TAT GGG AGG TTT CTG GCC AAC      3989
   Thr Asp Ser Pro Leu Thr Tyr Ser Thr Tyr Gly Arg Phe Leu Ala Asn
           1225                1230                1235
   CCT AGG CAG ATG CTG CGA GGT GTG TCG GTG GTC ATT TGC GAT GAA TGC      4037
   Pro Arg Gln Met Leu Arg Gly Val Ser Val Val Ile Cys Asp Glu Cys
       1240                1245                1250
   CAC AGT CAT GAT TCC ACT GTG TTG TTG GGG ATT GGA CGG GTC CGG GAG      4085
   His Ser His Asp Ser Thr Val Leu Leu Gly Ile Gly Arg Val Arg Glu
   1255                1260                1265                1270
   CTG GCA CGA GAG TGT GGG GTG CAG CTT GTG CTC TAC GCC ACT GCC ACG      4133
   Leu Ala Arg Glu Cys Gly Val Gln Leu Val Leu Tyr Ala Thr Ala Thr
                   1275                1280                1285
   CCT CCT GGG TCC CCC ATG ACT CAG CAT CCG TCA ATC ATT GAG ACC AAA      4181
   Pro Pro Gly Ser Pro Met Thr Gln His Pro Ser Ile Ile Glu Thr Lys
               1290                1295                1300
   TTG GAT GTG GGT GAG ATT CCC TTC TAT GGG CAT GGC ATA CCC CTC GAG      4229
   Leu Asp Val Gly Glu Ile Pro Phe Tyr Gly His Gly Ile Pro Leu Glu
           1305                1310                1315
   CGG ATG CGG ACC GGT AGG CAC CTC GTA TTC TGC TAC TCT AAG GCA GAG      4277
   Arg Met Arg Thr Gly Arg His Leu Val Phe Cys Tyr Ser Lys Ala Glu
       1320                1325                1330
   TGT GAG CGG CTA GCC GGT CAG TTT TCT GCT AGG GGA GTT AAC GCC ATA      4325
   Cys Glu Arg Leu Ala Gly Gln Phe Ser Ala Arg Gly Val Asn Ala Ile
   1335                1340                1345                1350
   GCC TAT TAC AGG GGA AAA GAC AGT TCT ATC ATC AAG GAC GGA GAT CTG      4373
   Ala Tyr Tyr Arg Gly Lys Asp Ser Ser Ile Ile Lys Asp Gly Asp Leu
                   1355                1360                1365
   GTG GTG TGC GCG ACC GAC GCG CTA TCC ACT GGA TAC ACT GGG AAC TTC      4421
   Val Val Cys Ala Thr Asp Ala Leu Ser Thr Gly Tyr Thr Gly Asn Phe
               1370                1375                1380
   GAT TCT GTC ACC GAC TGT GGG TTA GTG GTG GAG GAG GTC GTC GAG GTG      4469
   Asp Ser Val Thr Asp Cys Gly Leu Val Val Glu Glu Val Val Glu Val
           1385                1390                1395
   ACC CTT GAT CCC ACC ATT ACC ATC TCC CTG CGG ACA GTG CCC GCG TCG      4517
   Thr Leu Asp Pro Thr Ile Thr Ile Ser Leu Arg Thr Val Pro Ala Ser
       1400                1405                1410
   GCA GAA CTG TCG ATG CAG AGA CGA GGA CGC ACG GGT AGA GGC AGG TCT      4565
   Ala Glu Leu Ser Met Gln Arg Arg Gly Arg Thr Gly Arg Gly Arg Ser
   1415                1420                1425                1430
   GGG CGC TAC TAC TAC GCC GGG GTC GGA AAG GCC CCC GCG GGT GTG GTG      4613
   Gly Arg Tyr Tyr Tyr Ala Gly Val Gly Lys Ala Pro Ala Gly Val Val
                   1435                1440                1445
   CGC TCG GGT CCT GTC TGG TCG GCG GTG GAG GCC GGA GTG ACC TGG TAT      4661
   Arg Ser Gly Pro Val Trp Ser Ala Val Glu Ala Gly Val Thr Trp Tyr
               1450                1455                1460
   GGA ATG GAA CCT GAC TTG ACA GCT AAC CTA TTG AGA CTT TAC GAC GAC      4709
   Gly Met Glu Pro Asp Leu Thr Ala Asn Leu Leu Arg Leu Tyr Asp Asp
           1465                1470                1475
   TGC CCT TAC ACC GCA GCC GTC GCA GCT GAC ATC GGT GAA GCC GCG GTG      4757
   Cys Pro Tyr Thr Ala Ala Val Ala Ala Asp Ile Gly Glu Ala Ala Val
       1480                1485                1490
   TTT TTC TCC GGG CTA GCC CCG TTG AGG ATG CAT CCC GAT GTT AGC TGG      4805
   Phe Phe Ser Gly Leu Ala Pro Leu Arg Met His Pro Asp Val Ser Trp
   1495                1500                1505                1510
   GCA AAA GTG CGC GGC GTC AAC TGG CCC CTC TTG GTG GGT GTT CAG CGG      4853
   Ala Lys Val Arg Gly Val Asn Trp Pro Leu Leu Val Gly Val Gln Arg
                   1515                1520                1525
   ACC ATG TGC CGG GAA ACA CTG TCT CCC GGA CCA TCG GAC GAC CCC CAA 4901
   Thr Met Cys Arg Glu Thr Leu Ser Pro Gly Pro Ser Asp Asp Pro Gln
   1530 1535 1540
   TGG GCA GGT CTG AAG GGC CCG AAT CCT GTT CCA CTA CTG CTG AGG TGG 4949
   Trp Ala Gly Leu Lys Gly Pro Asn Pro Val Pro Leu Leu Leu Arg Trp
   1545 1550 1555
   GGC AAT GAT TTA CCA TCA AAA GTG GCC GGC CAC CAC ATT GTT GAC GAC 4997
   Gly Asn Asp Leu Pro Ser Lys Val Ala Gly His His Ile Val Asp Asp
   1560 1565 1570
   CTG GTT CGT AGG CTT GGT GTG GCG GAG GGT TAT GTC CGC TGC GAT GCG 5045
   Leu Val Arg Arg Leu Gly Val Ala Glu Gly Tyr Val Arg Cys Asp Ala
   1575 1580 1585 1590
   GGG CCG ATC TTA ATG GTC GGC CTC GCT ATC GCG GGG GGG ATG ATC TAC 5093
   Gly Pro Ile Leu Met Val Gly Leu Ala Ile Ala Gly Gly Met Ile Tyr
   1595 1600 1605
   GCA TCT TAC ACC GGG TCT TTA GTG GTG GTG ACA GAC TGG GAT GTA AAG 5141
   Ala Ser Tyr Thr Gly Ser Leu Val Val Val Thr Asp Trp Asp Val Lys
   1610 1615 1620
   GGG GGT GGC AGC CCT CTT TAT CGG CAT GGA GAC CAG GCC ACG CCA CAG 5189
   Gly Gly Gly Ser Pro Leu Tyr Arg His Gly Asp Gln Ala Thr Pro Gln
   1625 1630 1635
   CCG GTT GTG CAG GTC CCC CCG GTA GAC CAT CGG CCG GGG GGG GAG TCT 5237
   Pro Val Val Gln Val Pro Pro Val Asp His Arg Pro Gly Gly Glu Ser
   1640 1645 1650
   GCG CCT TCG GAT GCC AAG ACA GTG ACA GAT GCG GTG GCG GCC ATC CAG 5285
   Ala Pro Ser Asp Ala Lys Thr Val Thr Asp Ala Val Ala Ala Ile Gln
   1655 1660 1665 1670
   GTG GAT TGC GAT TGG TCA GTC ATG ACC CTG TCG ATC GGG GAA GTG CTG 5333
   Val Asp Cys Asp Trp Ser Val Met Thr Leu Ser Ile Gly Glu Val Leu
   1675 1680 1685
   TCC TTG GCT CAG GCT AAA ACA GCT GAG GCC TAC ACG GCA ACC GCC AAG 5381
   Ser Leu Ala Gln Ala Lys Thr Ala Glu Ala Tyr Thr Ala Thr Ala Lys
   1690 1695 1700
   TGG CTC GCT GGC TGC TAC ACG GGG ACG CGG GCC GTT CCC ACT GTT TCA 5429
   Trp Leu Ala Gly Cys Tyr Thr Gly Thr Arg Ala Val Pro Thr Val Ser
           1705                1710                1715
   ATT GTT GAC AAG CTC TTT GCC GGA GGG TGG GCG GCT GTG GTT GGC CAC       5477
   Ile Val Asp Lys Leu Phe Ala Gly Gly Trp Ala Ala Val Val Gly His
       1720                1725                1730
   TGT CAC AGC GTC ATA GCT GCG GCG GTG GCT GCC TAC GGG GCT TCC AGG       5525
   Cys His Ser Val Ile Ala Ala Ala Val Ala Ala Tyr Gly Ala Ser Arg
   1735                1740                1745                1750
   AGT CCG CCG TTG GCA GCC GCG GCT TCC TAC CTG ATG GGA CTG GGC GTC       5573
   Ser Pro Pro Leu Ala Ala Ala Ala Ser Tyr Leu Met Gly Leu Gly Val
                   1755                1760                1765
   GGA GGC AAC GCT CAG ACG CGT TTG GCG TCT GCC CTC CTG TTG GGG GCC       5621
   Gly Gly Asn Ala Gln Thr Arg Leu Ala Ser Ala Leu Leu Leu Gly Ala
               1770                1775                1780
   GCT GGC ACC GCC CTG GGC ACT CCC GTC GTG GGT TTA ACC ATG GCG GGG       5669
   Ala Gly Thr Ala Leu Gly Thr Pro Val Val Gly Leu Thr Met Ala Gly
           1785                1790                1795
   GCG TTC ATG GGG GGT GCT AGC GTC TCT CCC TCC TTG GTC ACC ATC TTG       5717
   Ala Phe Met Gly Gly Ala Ser Val Ser Pro Ser Leu Val Thr Ile Leu
       1800                1805                1810
   TTG GGG GCC GTG GGA GGC TGG GAG GGC GTC GTC AAC GCT GCT AGC CTT       5765
   Leu Gly Ala Val Gly Gly Trp Glu Gly Val Val Asn Ala Ala Ser Leu
   1815                1820                1825                1830
   GTC TTT GAC TTC ATG GCG GGG AAA CTA TCG TCA GAA GAT CTG TGG TAC       5813
   Val Phe Asp Phe Met Ala Gly Lys Leu Ser Ser Glu Asp Leu Trp Tyr
                   1835                1840                1845
   GCC ATC CCA GTG CTC ACC AGC CCG GGG GCG GGC CTT GCG GGG ATC GCC       5861
   Ala Ile Pro Val Leu Thr Ser Pro Gly Ala Gly Leu Ala Gly Ile Ala
               1850                1855                1860
   CTT GGG TTG GTG CTG TAC TCA GCT AAC AAC TCT GGT ACT ACC ACT TGG       5909
   Leu Gly Leu Val Leu Tyr Ser Ala Asn Asn Ser Gly Thr Thr Thr Trp
           1865                1870                1875
   TTG AAC CGT CTG CTG ACT ACG TTA CCT AGG TCT TCT TGC ATC CCT GAC       5957
   Leu Asn Arg Leu Leu Thr Thr Leu Pro Arg Ser Ser Cys Ile Pro Asp
   1880 1885 1890
   AGC TAT TTC CAA CAG GCC GAT TAC TGT GAC AAG GTC TCG GCC GTG CTT 6005
   Ser Tyr Phe Gln Gln Ala Asp Tyr Cys Asp Lys Val Ser Ala Val Leu
   1895 1900 1905 1910
   CGC CGA CTG AGC CTC ACC CGC ACT GTG GTG GCC CTA GTC AAT AGG GAA 6053
   Arg Arg Leu Ser Leu Thr Arg Thr Val Val Ala Leu Val Asn Arg Glu
   1915 1920 1925
   CCC AAG GTG GAC GAG GTA CAG GTG GGG TAC GTC TGG GAT CTC TGG GAG 6101
   Pro Lys Val Asp Glu Val Gln Val Gly Tyr Val Trp Asp Leu Trp Glu
   1930 1935 1940
   TGG ATC ATG CGT CAA GTG CGC ATG GTC ATG GCC AGG CTC CGG GCT CTC 6149
   Trp Ile Met Arg Gln Val Arg Met Val Met Ala Arg Leu Arg Ala Leu
   1945 1950 1955
   TGC CCC GTG GTG TCA CTG CCT TTG TGG CAC TGC GGG GAG GGG TGG TCC 6197
   Cys Pro Val Val Ser Leu Pro Leu Trp His Cys Gly Glu Gly Trp Ser
   1960 1965 1970
   GGA GAG TGG TTG TTG GAC GGC CAT GTG GAG AGT CGC TGT CTT TGC GGG 6245
   Gly Glu Trp Leu Leu Asp Gly His Val Glu Ser Arg Cys Leu Cys Gly
   1975 1980 1985 1990
   TGC GTG ATC ACC GGC GAT GTT TTC AAT GGG CAA CTC AAA GAG CCA GTT 6293
   Cys Val Ile Thr Gly Asp Val Phe Asn Gly Gln Leu Lys Glu Pro Val
   1995 2000 2005
   TAC TCT ACA AAG TTG TGC CGG CAC TAT TGG ATG GGG ACC GTT CCT GTG 6341
   Tyr Ser Thr Lys Leu Cys Arg His Tyr Trp Met Gly Thr Val Pro Val
   2010 2015 2020
   AAC ATG CTG GGT TAC GGC GAA ACA TCA CCC CTC TTG GCC TCT GAC ACC 6389
   Asn Met Leu Gly Tyr Gly Glu Thr Ser Pro Leu Leu Ala Ser Asp Thr
   2025 2030 2035
   CCG AAG GTG GTG CCT TTT GGG ACG TCG GGC TGG GCT GAG GTG GTG GTG 6437
   Pro Lys Val Val Pro Phe Gly Thr Ser Gly Trp Ala Glu Val Val Val
   2040 2045 2050
   ACC CCT ACC CAC GTG GTG ATC AGG AGA ACC TCT CCC TAC GAG TTG CTG 6485
   Thr Pro Thr His Val Val Ile Arg Arg Thr Ser Pro Tyr Glu Leu Leu
   2055 2060 2065 2070
   CGC CAA CAA ATC CTA TCA GCT GCA GTT GCT GAG CCC TAT TAT GTC GAC      6533
   Arg Gln Gln Ile Leu Ser Ala Ala Val Ala Glu Pro Tyr Tyr Val Asp
                   2075                2080                2085
   GGC ATA CCG GTC TCA TGG GAC GCG GAC GCT CGT GCG CCT GCT ATG GTT      6581
   Gly Ile Pro Val Ser Trp Asp Ala Asp Ala Arg Ala Pro Ala Met Val
               2090                2095                2100
   TAT GGC CCT GGG CAA AGT GTT ACC ATT GAC GGG GAG CGC TAC ACC CTG      6629
   Tyr Gly Pro Gly Gln Ser Val Thr Ile Asp Gly Glu Arg Tyr Thr Leu
           2105                2110                2115
   CCG CAT CAA CTG CGG CTC AGG AAT GTA GCG CCC TCT GAG GTT TCA TCC      6677
   Pro His Gln Leu Arg Leu Arg Asn Val Ala Pro Ser Glu Val Ser Ser
       2120                2125                2130
   GAG GTG TCC ATA GAC ATT GGG ACG GAG ACT GAA GAC TCA GAA CTG ACT      6725
   Glu Val Ser Ile Asp Ile Gly Thr Glu Thr Glu Asp Ser Glu Leu Thr
   2135                2140                2145                2150
   GAG GCC GAC CTG CCG CCG GCA GCT GCA GCC CTC CAG GCT ATC GAG AAT      6773
   Glu Ala Asp Leu Pro Pro Ala Ala Ala Ala Leu Gln Ala Ile Glu Asn
                   2155                2160                2165
   GCT GCG AGG ATT CTT GAG CCT CAT ATT GAT GTC ATC ATG GAG GAT TGC      6821
   Ala Ala Arg Ile Leu Glu Pro His Ile Asp Val Ile Met Glu Asp Cys
               2170                2175                2180
   AGT ACA CCC TCT CTT TGT GGT AGT AGC CGA GAG ATG CCT GTG TGG GGA      6869
   Ser Thr Pro Ser Leu Cys Gly Ser Ser Arg Glu Met Pro Val Trp Gly
           2185                2190                2195
   GAA GAC ATC CCC CGC ACT CCA TCG CCA GCA CTT ATC TCG GTT ACC GAG      6917
   Glu Asp Ile Pro Arg Thr Pro Ser Pro Ala Leu Ile Ser Val Thr Glu
       2200                2205                2210
   AGC AGC TCA GAT GAG AAG ACC CCG TCG GTG TCC TCC TCG CAG GAG GAT      6965
   Ser Ser Ser Asp Glu Lys Thr Pro Ser Val Ser Ser Ser Gln Glu Asp
   2215                2220                2225                2230
   ACC CCG TCC TCT GAC TCA TTC GAA GTC ATC CAA GAG TCT GAG ACA GCT      7013
   Thr Pro Ser Ser Asp Ser Phe Glu Val Ile Gln Glu Ser Glu Thr Ala
                   2235                2240                2245
   GAA GGA GAG GAA AGT GTC TTC AAC GTG GCT CTT TCC GTA CTA GAA GCC      7061
   Glu Gly Glu Glu Ser Val Phe Asn Val Ala Leu Ser Val Leu Glu Ala
   2250 2255 2260
   TTG TTT CCA CAG AGT GAT GCC ACT AGA AAG CTT ACC GTC AGG ATG AAT 7109
   Leu Phe Pro Gln Ser Asp Ala Thr Arg Lys Leu Thr Val Arg Met Asn
   2265 2270 2275
   TGC TGC GTT GAG AAG AGC GTC ACG CGC TTC TTT TCT TTG GGG CTG ACG 7157
   Cys Cys Val Glu Lys Ser Val Thr Arg Phe Phe Ser Leu Gly Leu Thr
   2280 2285 2290
   GTG GCT GAT GTG GCC AGT CTG TGT GAG ATG GAG ATC CAG AAC CAT ACA 7205
   Val Ala Asp Val Ala Ser Leu Cys Glu Met Glu Ile Gln Asn His Thr
   2295 2300 2305 2310
   GCC TAT TGT GAC AAG GTG CGC ACT CCG CTC GAA TTG CAA GTT GGG TGC 7253
   Ala Tyr Cys Asp Lys Val Arg Thr Pro Leu Glu Leu Gln Val Gly Cys
   2315 2320 2325
   TTG GTG GGC AAT GAA CTT ACC TTT GAA TGT GAT AAG TGT GAG GCT AGG 7301
   Leu Val Gly Asn Glu Leu Thr Phe Glu Cys Asp Lys Cys Glu Ala Arg
   2330 2335 2340
   CAA GAG ACT TTG GCC TCC TTC TCC TAT ATT TGG TCT GGG GTG CCA TTG 7349
   Gln Glu Thr Leu Ala Ser Phe Ser Tyr Ile Trp Ser Gly Val Pro Leu
   2345 2350 2355
   ACT AGG GCC ACA CCG GCT AAA CCA CCT GTG GTG AGG CCG GTG GGG TCC 7397
   Thr Arg Ala Thr Pro Ala Lys Pro Pro Val Val Arg Pro Val Gly Ser
   2360 2365 2370
   TTG TTG GTG GCT GAC ACC ACG AAA GTG TAT GTC ACA AAC CCG GAC AAT 7445
   Leu Leu Val Ala Asp Thr Thr Lys Val Tyr Val Thr Asn Pro Asp Asn
   2375 2380 2385 2390
   GTT GGG AGA AGA GTG GAC AAG GTG ACC TTC TGG CGC GCC CCC AGG GTC 7493
   Val Gly Arg Arg Val Asp Lys Val Thr Phe Trp Arg Ala Pro Arg Val
   2395 2400 2405
   CAT GAC AAA TAT CTC GTG GAC TCC ATC GAG CGT GCC AGG AGG GCG GCT 7541
   His Asp Lys Tyr Leu Val Asp Ser Ile Glu Arg Ala Arg Arg Ala Ala
   2410 2415 2420
   CAA GCC TGC CAA AGC ATG GGT TAC ACT TAT GAG GAA GCA ATA AGG ACT 7589
   Gln Ala Cys Gln Ser Met Gly Tyr Thr Tyr Glu Glu Ala Ile Arg Thr
   2425 2430 2435
   GTT AGG CCA CAT GCT GCC ATG GGC TGG GGA TCT AAG GTG TCG GTC AAG 7637
   Val Arg Pro His Ala Ala Met Gly Trp Gly Ser Lys Val Ser Val Lys
   2440 2445 2450
   GAC TTG GCC ACC CCT GCG GGG AAG ATG GCC GTC CAC GAC CGA CTT CAG 7685
   Asp Leu Ala Thr Pro Ala Gly Lys Met Ala Val His Asp Arg Leu Gln
   2455 2460 2465 2470
   GAG ATA CTT GAG GGG ACT CCG GTC CCT TTT ACT CTT ACT GTG AAA AAG 7733
   Glu Ile Leu Glu Gly Thr Pro Val Pro Phe Thr Leu Thr Val Lys Lys
   2475 2480 2485
   GAG GTG TTC TTC AAA GAC CGT AAG GAG GAG AAG GCC CCC CGC CTC ATT 7781
   Glu Val Phe Phe Lys Asp Arg Lys Glu Glu Lys Ala Pro Arg Leu Ile
   2490 2495 2500
   GTG TTC CCC CCC CTG GAC TTC CGG ATA GCT GAG AAG CTT ATC CTG GGA 7829
   Val Phe Pro Pro Leu Asp Phe Arg Ile Ala Glu Lys Leu Ile Leu Gly
   2505 2510 2515
   GAC CCG GGG CGG GTG GCC AAG GCG GTG TTG GGG GGG GCT TAC GCC TTC 7877
   Asp Pro Gly Arg Val Ala Lys Ala Val Leu Gly Gly Ala Tyr Ala Phe
   2520 2525 2530
   CAG TAC ACC CCA AAT CAG CGA GTT AAG GAG ATG CTC AAA CTG TGG GAG 7925
   Gln Tyr Thr Pro Asn Gln Arg Val Lys Glu Met Leu Lys Leu Trp Glu
   2535 2540 2545 2550
   TCA AAG AAA ACA CCT TGC GCC ATC TGT GTG GAC GCC ACT TGC TTC GAC 7973
   Ser Lys Lys Thr Pro Cys Ala Ile Cys Val Asp Ala Thr Cys Phe Asp
   2555 2560 2565
   AGT AGC ATT ACT GAA GAG GAC GTG GCG CTG GAG ACA GAG CTG TAC GCT 8021
   Ser Ser Ile Thr Glu Glu Asp Val Ala Leu Glu Thr Glu Leu Tyr Ala
   2570 2575 2580
   CTG GCC TCT GAC CAT CCA GAG TGG GTG CGA GCT TTG GGG AAG TAC TAT 8069
   Leu Ala Ser Asp His Pro Glu Trp Val Arg Ala Leu Gly Lys Tyr Tyr
   2585 2590 2595
   GCC TCA GGA ACC ATG GTC ACC CCT GAG GGG GTT CCC GTA GGT GAG AGG 8117
   Ala Ser Gly Thr Met Val Thr Pro Glu Gly Val Pro Val Gly Glu Arg
   2600 2605 2610
   TAT TGT AGA TCC TCA GGC GTT TTG ACT ACC AGC GCG AGT AAC TGC CTG 8165
   Tyr Cys Arg Ser Ser Gly Val Leu Thr Thr Ser Ala Ser Asn Cys Leu
   2615 2620 2625 2630
   ACC TGC TAC ATC AAG GTG AAA GCC GCT TGT GAG AGA GTG GGG CTG AAA 8213
   Thr Cys Tyr Ile Lys Val Lys Ala Ala Cys Glu Arg Val Gly Leu Lys
   2635 2640 2645
   AAT GTC TCG CTT CTC ATA GCC GGC GAT GAC TGT TTG ATC ATA TGC GAA 8261
   Asn Val Ser Leu Leu Ile Ala Gly Asp Asp Cys Leu Ile Ile Cys Glu
   2650 2655 2660
   CGG CCA GTG TGC GAC CCT TGT GAC GCC TTG GGC AGA GCC CTG GCG AGC 8309
   Arg Pro Val Cys Asp Pro Cys Asp Ala Leu Gly Arg Ala Leu Ala Ser
   2665 2670 2675
   TAT GGG TAT GCT TGC GAG CCT TCG TAT CAT GCA TCA CTG GAC ACG GCC 8357
   Tyr Gly Tyr Ala Cys Glu Pro Ser Tyr His Ala Ser Leu Asp Thr Ala
   2680 2685 2690
   CCC TTC TGC TCC ACT TGG CTC GCT GAG TGC AAC GCA GAT GGG AAA CGC 8405
   Pro Phe Cys Ser Thr Trp Leu Ala Glu Cys Asn Ala Asp Gly Lys Arg
   2695 2700 2705 2710
   CAT TTC TTC CTG ACC ACG GAC TTT CGG AGG CCG CTT GCT CGC ATG TCG 8453
   His Phe Phe Leu Thr Thr Asp Phe Arg Arg Pro Leu Ala Arg Met Ser
   2715 2720 2725
   AGC GAG TAT AGT GAC CCA ATG GCT TCG GCC ATA GGT TAC ATC CTC CTG 8501
   Ser Glu Tyr Ser Asp Pro Met Ala Ser Ala Ile Gly Tyr Ile Leu Leu
   2730 2735 2740
   TAT CCC TGG CAT CCC ATC ACA CGG TGG GTC ATC ATC CCT CAT GTG CTA 8549
   Tyr Pro Trp His Pro Ile Thr Arg Trp Val Ile Ile Pro His Val Leu
   2745 2750 2755
   ACG TGC GCA TTC AGG GGT GGT GGT ACA CCG TCT GAT CCG GTT TGG TGT 8597
   Thr Cys Ala Phe Arg Gly Gly Gly Thr Pro Ser Asp Pro Val Trp Cys
   2760 2765 2770
   CAG GTG CAT GGT AAC TAC TAC AAG TTT CCA CTG GAC AAA CTG CCT AAC 8645
   Gln Val His Gly Asn Tyr Tyr Lys Phe Pro Leu Asp Lys Leu Pro Asn
   2775 2780 2785 2790
   ATC ATC GTG GCC CTC CAC GGA CCA GCA GCG TTG AGG GTT ACC GCA GAC 8693
   Ile Ile Val Ala Leu His Gly Pro Ala Ala Leu Arg Val Thr Ala Asp
                   2795                2800                2805
   ACA ACT AAG ACA AAA ATG GAA GCT GGG AAG GTG CTG AGT GAC CTC AAG      8741
   Thr Thr Lys Thr Lys Met Glu Ala Gly Lys Val Leu Ser Asp Leu Lys
               2810                2815                2820
   CTC CCT GGC CTA GCG GTC CAC CGA AAG AAG GCC GGA GCA CTG CGA ACA      8789
   Leu Pro Gly Leu Ala Val His Arg Lys Lys Ala Gly Ala Leu Arg Thr
           2825                2830                2835
   CGC ATG CTT CGG TCG CGC GGT TGG GCC GAG TTG GCG AGG GGC CTG TTG      8837
   Arg Met Leu Arg Ser Arg Gly Trp Ala Glu Leu Ala Arg Gly Leu Leu
       2840                2845                2850
   TGG CAT CCA GGC CTC CGG CTC CCT CCC CCT GAG ATT GCT GGT ATC CCG      8885
   Trp His Pro Gly Leu Arg Leu Pro Pro Pro Glu Ile Ala Gly Ile Pro
   2855                2860                2865                2870
   GGG GGT TTC CCC CTC TCC CCC CCC TAC ATG GGG GTG GTG CAT CAA TTG      8933
   Gly Gly Phe Pro Leu Ser Pro Pro Tyr Met Gly Val Val His Gln Leu
                   2875                2880                2885
   GAT TTT ACA AGC CAG AGG AGT CGC TGG CGG TGG CTG GGG TTC TTA GCC      8981
   Asp Phe Thr Ser Gln Arg Ser Arg Trp Arg Trp Leu Gly Phe Leu Ala
               2890                2895                2900
   CTG CTC ATC GTA GCC CTC TTC GGG TGAACTAAAT TCATCTGTTG CGGCAAGGTC     9035
   Leu Leu Ile Val Ala Leu Phe Gly
           2905                2910
   CAGTGACTGA TCATCACTGG AGGAGGTTCC CGCCCTCCCC GCCCCAGGGG TCTCCCCGCT    9095
   GGGTAAAA                                                             9103
(2) INFORMATION FOR SEQ ID NO:183:
   (i) SEQUENCE CHARACTERISTICS:
      (A) LENGTH: 2910 amino acids
      (B) TYPE: amino acid
      (D) TOPOLOGY: linear
   (ii) MOLECULE TYPE: protein
   (xi) SEQUENCE DESCRIPTION: SEQ ID NO:183:
   Met Ser Leu Leu Thr Asn Arg Leu Ser Arg Arg Val Asp Lys Asp Gln
   1 5 10 15
   Trp Gly Pro Gly Phe Met Gly Lys Asp Pro Lys Pro Cys Pro Ser Arg
   20 25 30
   Arg Thr Gly Lys Cys Met Gly Pro Pro Ser Ser Ala Ala Ala Cys Ser
   35 40 45
   Arg Gly Ser Pro Arg Ile Leu Arg Val Arg Ala Gly Gly Ile Ser Leu
   50 55 60
   Pro Tyr Thr Ile Met Glu Ala Leu Leu Phe Leu Leu Gly Val Glu Ala
   65 70 75 80
   Gly Ala Ile Leu Ala Pro Ala Thr His Ala Cys Arg Ala Asn Gly Gln
   85 90 95
   Tyr Phe Leu Thr Asn Cys Cys Ala Pro Glu Asp Ile Gly Phe Cys Leu
   100 105 110
   Glu Gly Gly Cys Leu Val Ala Leu Gly Cys Thr Val Cys Thr Asp Arg
   115 120 125
   Cys Trp Pro Leu Tyr Gln Ala Gly Leu Ala Val Arg Pro Gly Lys Ser
   130 135 140
   Ala Ala Gln Leu Val Gly Gln Leu Gly Gly Leu Tyr Gly Pro Leu Ser
   145 150 155 160
   Val Ser Ala Tyr Val Ala Gly Ile Leu Gly Leu Gly Glu Val Tyr Ser
   165 170 175
   Gly Val Leu Thr Val Gly Val Ala Leu Thr Arg Arg Val Tyr Pro Met
   180 185 190
   Pro Asn Leu Thr Cys Ala Val Glu Cys Glu Leu Lys Trp Glu Ser Glu
   195 200 205
   Phe Trp Arg Trp Thr Glu Gln Leu Ala Ser Asn Tyr Trp Ile Leu Glu
   210 215 220
   Tyr Leu Trp Lys Val Pro Phe Asp Phe Trp Arg Gly Val Leu Ser Leu
   225 230 235 240
   Thr Pro Leu Leu Val Cys Val Ala Ala Leu Leu Leu Leu Glu Gln Arg
   245 250 255
   Ile Val Met Val Phe Leu Leu Val Thr Met Ala Gly Met Ser Gln Gly
   260 265 270
   Ala Pro Ala Ser Val Leu Gly Ser Arg Pro Phe Asp Tyr Gly Leu Thr
   275 280 285
   Trp Gln Ser Cys Ser Cys Arg Ala Asn Gly Ser Arg Tyr Thr Thr Gly
   290 295 300
   Glu Lys Val Trp Asp Arg Gly Asn Val Thr Leu Leu Cys Asp Cys Pro
   305 310 315 320
   Asn Gly Pro Trp Val Trp Leu Pro Ala Phe Cys Gln Ala Ile Gly Trp
   325 330 335
   Gly Asp Pro Ile Thr His Trp Ser His Gly Gln Asn Arg Trp Pro Leu
   340 345 350
   Ser Cys Pro Gln Tyr Val Tyr Gly Ser Val Ser Val Thr Cys Val Trp
   355 360 365
   Gly Ser Val Ser Trp Phe Ala Ser Thr Gly Gly Arg Asp Ser Lys Ile
   370 375 380
   Asp Val Trp Ser Leu Val Pro Val Gly Ser Ala Ser Cys Thr Ile Ala
   385 390 395 400
   Ala Leu Gly Ser Ser Asp Arg Asp Thr Val Val Glu Leu Ser Glu Trp
   405 410 415
   Gly Val Pro Cys Ala Thr Cys Ile Leu Asp Arg Arg Pro Ala Ser Cys
   420 425 430
   Gly Thr Cys Val Arg Asp Cys Trp Pro Glu Thr Gly Ser Val Arg Phe
   435 440 445
   Pro Phe His Arg Cys Gly Ala Gly Pro Lys Leu Thr Lys Asp Leu Glu
   450 455 460
   Ala Val Pro Phe Val Asn Arg Thr Thr Pro Phe Thr Ile Arg Gly Pro
   465 470 475 480
   Leu Gly Asn Gln Gly Arg Gly Asn Pro Val Arg Ser Pro Leu Gly Phe
   485 490 495
   Gly Ser Tyr Ala Met Thr Lys Ile Arg Asp Ser Leu His Leu Val Lys
   500 505 510
   Cys Pro Thr Pro Ala Ile Glu Pro Pro Thr Gly Thr Phe Gly Phe Phe
   515 520 525
   Pro Gly Val Pro Pro Leu Asn Asn Cys Leu Leu Leu Gly Thr Glu Val
   530 535 540
   Ser Glu Ala Leu Gly Gly Ala Gly Leu Thr Gly Gly Phe Tyr Glu Pro
   545 550 555 560
   Leu Val Arg Arg Arg Ser Glu Leu Met Gly Arg Arg Asn Pro Val Cys
   565 570 575
   Pro Gly Phe Ala Trp Leu Ser Ser Gly Arg Pro Asp Gly Phe Ile His
   580 585 590
   Val Gln Gly His Leu Gln Glu Val Asp Ala Gly Asn Phe Ile Pro Pro
   595 600 605
   Pro Arg Trp Leu Leu Leu Asp Phe Val Phe Val Leu Leu Tyr Leu Met
   610 615 620
   Lys Leu Ala Glu Ala Arg Leu Val Pro Leu Ile Leu Leu Leu Leu Trp
   625 630 635 640
   Trp Trp Val Asn Gln Leu Ala Val Leu Gly Leu Pro Ala Val Asp Ala
   645 650 655
   Ala Val Ala Gly Glu Val Phe Ala Gly Pro Ala Leu Ser Trp Cys Leu
   660 665 670
   Gly Leu Pro Thr Val Ser Met Ile Leu Gly Leu Ala Asn Leu Val Leu
   675 680 685
   Tyr Phe Arg Trp Met Gly Pro Gln Arg Leu Met Phe Leu Val Leu Trp
   690 695 700
   Lys Leu Ala Arg Gly Ala Phe Pro Leu Ala Leu Leu Met Gly Ile Ser
   705 710 715 720
   Ala Thr Arg Gly Arg Thr Ser Val Leu Gly Ala Glu Phe Cys Phe Asp
   725 730 735
   Val Thr Phe Glu Val Asp Thr Ser Val Leu Gly Trp Val Val Ala Ser
   740 745 750
   Val Val Ala Trp Ala Ile Ala Leu Leu Ser Ser Met Ser Ala Gly Gly
   755 760 765
   Trp Arg His Lys Ala Val Ile Tyr Arg Thr Trp Cys Lys Gly Tyr Gln
   770 775 780
   Ala Ile Arg Gln Arg Val Val Arg Ser Pro Leu Gly Glu Gly Arg Pro
   785 790 795 800
   Thr Lys Pro Leu Thr Phe Ala Trp Cys Leu Ala Ser Tyr Ile Trp Pro
   805 810 815
   Asp Ala Val Met Met Val Val Val Ala Leu Val Leu Leu Phe Gly Leu
   820 825 830
   Phe Asp Ala Leu Asp Trp Ala Leu Glu Glu Leu Leu Val Ser Arg Pro
   835 840 845
   Ser Leu Arg Arg Leu Ala Arg Val Val Glu Cys Cys Val Met Ala Gly
   850 855 860
   Glu Lys Ala Thr Thr Val Arg Leu Val Ser Lys Met Cys Ala Arg Gly
   865 870 875 880
   Ala Tyr Leu Phe Asp His Met Gly Ser Phe Ser Arg Ala Val Lys Glu
   885 890 895
   Arg Leu Leu Glu Trp Asp Ala Ala Leu Glu Pro Leu Ser Phe Thr Arg
   900 905 910
   Thr Asp Cys Arg Ile Ile Arg Asp Ala Ala Arg Thr Leu Ala Cys Gly
   915 920 925
   Gln Cys Val Met Gly Leu Pro Val Val Ala Arg Arg Gly Asp Glu Val
   930 935 940
   Leu Ile Gly Val Phe Gln Asp Val Asn His Leu Pro Pro Gly Phe Val
   945 950 955 960
   Pro Thr Ala Pro Val Val Ile Arg Arg Cys Gly Lys Gly Phe Leu Gly
   965 970 975
   Val Thr Lys Ala Ala Leu Thr Gly Arg Asp Pro Asp Leu His Pro Gly
   980 985 990
   Asn Val Met Val Leu Gly Thr Ala Thr Ser Arg Ser Met Gly Thr Cys
   995 1000 1005
   Leu Asn Gly Leu Leu Phe Thr Thr Phe His Gly Ala Ser Ser Arg Thr
   1010 1015 1020
   Ile Ala Thr Pro Val Gly Ala Leu Asn Pro Arg Trp Trp Ser Ala Ser
   1025 1030 1035 1040
   Asp Asp Val Thr Val Tyr Pro Leu Pro Asp Gly Ala Thr Ser Leu Thr
   1045 1050 1055
   Pro Cys Thr Cys Gln Ala Glu Ser Cys Trp Val Ile Arg Ser Asp Gly
   1060 1065 1070
   Ala Leu Cys His Gly Leu Ser Lys Gly Asp Lys Val Glu Leu Asp Val
   1075 1080 1085
   Ala Met Glu Val Ser Asp Phe Arg Gly Ser Ser Gly Ser Pro Val Leu
   1090 1095 1100
   Cys Asp Glu Gly His Ala Val Gly Met Leu Val Ser Val Leu His Ser
   1105 1110 1115 1120
   Gly Gly Arg Val Thr Ala Ala Arg Phe Thr Arg Pro Trp Thr Gln Val
   1125 1130 1135
   Pro Thr Asp Ala Lys Thr Thr Thr Glu Pro Pro Pro Val Pro Ala Lys
   1140 1145 1150
   Gly Val Phe Lys Glu Ala Pro Leu Phe Met Pro Thr Gly Ala Gly Lys
   1155 1160 1165
   Ser Thr Arg Val Pro Leu Glu Tyr Gly Asn Met Gly His Lys Val Leu
   1170 1175 1180
   Ile Leu Asn Pro Ser Val Ala Thr Val Arg Ala Met Gly Pro Tyr Met
   1185 1190 1195 1200
   Glu Arg Leu Ala Gly Lys His Pro Ser Ile Tyr Cys Gly His Asp Thr
   1205 1210 1215
   Thr Ala Phe Thr Arg Ile Thr Asp Ser Pro Leu Thr Tyr Ser Thr Tyr
   1220 1225 1230
   Gly Arg Phe Leu Ala Asn Pro Arg Gln Met Leu Arg Gly Val Ser Val
   1235 1240 1245
   Val Ile Cys Asp Glu Cys His Ser His Asp Ser Thr Val Leu Leu Gly
   1250 1255 1260
   Ile Gly Arg Val Arg Glu Leu Ala Arg Glu Cys Gly Val Gln Leu Val
   1265 1270 1275 1280
   Leu Tyr Ala Thr Ala Thr Pro Pro Gly Ser Pro Met Thr Gln His Pro
   1285 1290 1295
   Ser Ile Ile Glu Thr Lys Leu Asp Val Gly Glu Ile Pro Phe Tyr Gly
   1300 1305 1310
   His Gly Ile Pro Leu Glu Arg Met Arg Thr Gly Arg His Leu Val Phe
   1315 1320 1325
   Cys Tyr Ser Lys Ala Glu Cys Glu Arg Leu Ala Gly Gln Phe Ser Ala
   1330 1335 1340
   Arg Gly Val Asn Ala Ile Ala Tyr Tyr Arg Gly Lys Asp Ser Ser Ile
   1345 1350 1355 1360
   Ile Lys Asp Gly Asp Leu Val Val Cys Ala Thr Asp Ala Leu Ser Thr
   1365 1370 1375
   Gly Tyr Thr Gly Asn Phe Asp Ser Val Thr Asp Cys Gly Leu Val Val
   1380 1385 1390
   Glu Glu Val Val Glu Val Thr Leu Asp Pro Thr Ile Thr Ile Ser Leu
   1395 1400 1405
   Arg Thr Val Pro Ala Ser Ala Glu Leu Ser Met Gln Arg Arg Gly Arg
   1410 1415 1420
   Thr Gly Arg Gly Arg Ser Gly Arg Tyr Tyr Tyr Ala Gly Val Gly Lys
   1425 1430 1435 1440
   Ala Pro Ala Gly Val Val Arg Ser Gly Pro Val Trp Ser Ala Val Glu
   1445 1450 1455
   Ala Gly Val Thr Trp Tyr Gly Met Glu Pro Asp Leu Thr Ala Asn Leu
   1460 1465 1470
   Leu Arg Leu Tyr Asp Asp Cys Pro Tyr Thr Ala Ala Val Ala Ala Asp
   1475 1480 1485
   Ile Gly Glu Ala Ala Val Phe Phe Ser Gly Leu Ala Pro Leu Arg Met
   1490 1495 1500
   His Pro Asp Val Ser Trp Ala Lys Val Arg Gly Val Asn Trp Pro Leu
   1505 1510 1515 1520
   Leu Val Gly Val Gln Arg Thr Met Cys Arg Glu Thr Leu Ser Pro Gly
   1525 1530 1535
   Pro Ser Asp Asp Pro Gln Trp Ala Gly Leu Lys Gly Pro Asn Pro Val
   1540 1545 1550
   Pro Leu Leu Leu Arg Trp Gly Asn Asp Leu Pro Ser Lys Val Ala Gly
   1555 1560 1565
   His His Ile Val Asp Asp Leu Val Arg Arg Leu Gly Val Ala Glu Gly
   1570 1575 1580
   Tyr Val Arg Cys Asp Ala Gly Pro Ile Leu Met Val Gly Leu Ala Ile
   1585 1590 1595 1600
   Ala Gly Gly Met Ile Tyr Ala Ser Tyr Thr Gly Ser Leu Val Val Val
   1605 1610 1615
   Thr Asp Trp Asp Val Lys Gly Gly Gly Ser Pro Leu Tyr Arg His Gly
   1620 1625 1630
   Asp Gln Ala Thr Pro Gln Pro Val Val Gln Val Pro Pro Val Asp His
   1635 1640 1645
   Arg Pro Gly Gly Glu Ser Ala Pro Ser Asp Ala Lys Thr Val Thr Asp
   1650 1655 1660
   Ala Val Ala Ala Ile Gln Val Asp Cys Asp Trp Ser Val Met Thr Leu
   1665 1670 1675 1680
   Ser Ile Gly Glu Val Leu Ser Leu Ala Gln Ala Lys Thr Ala Glu Ala
   1685 1690 1695
   Tyr Thr Ala Thr Ala Lys Trp Leu Ala Gly Cys Tyr Thr Gly Thr Arg
   1700 1705 1710
   Ala Val Pro Thr Val Ser Ile Val Asp Lys Leu Phe Ala Gly Gly Trp
   1715 1720 1725
   Ala Ala Val Val Gly His Cys His Ser Val Ile Ala Ala Ala Val Ala
   1730 1735 1740
   Ala Tyr Gly Ala Ser Arg Ser Pro Pro Leu Ala Ala Ala Ala Ser Tyr
   1745 1750 1755 1760
   Leu Met Gly Leu Gly Val Gly Gly Asn Ala Gln Thr Arg Leu Ala Ser
   1765 1770 1775
   Ala Leu Leu Leu Gly Ala Ala Gly Thr Ala Leu Gly Thr Pro Val Val
   1780 1785 1790
   Gly Leu Thr Met Ala Gly Ala Phe Met Gly Gly Ala Ser Val Ser Pro
   1795 1800 1805
   Ser Leu Val Thr Ile Leu Leu Gly Ala Val Gly Gly Trp Glu Gly Val
   1810 1815 1820
   Val Asn Ala Ala Ser Leu Val Phe Asp Phe Met Ala Gly Lys Leu Ser
   1825 1830 1835 1840
   Ser Glu Asp Leu Trp Tyr Ala Ile Pro Val Leu Thr Ser Pro Gly Ala
   1845 1850 1855
   Gly Leu Ala Gly Ile Ala Leu Gly Leu Val Leu Tyr Ser Ala Asn Asn
   1860 1865 1870
   Ser Gly Thr Thr Thr Trp Leu Asn Arg Leu Leu Thr Thr Leu Pro Arg
   1875 1880 1885
   Ser Ser Cys Ile Pro Asp Ser Tyr Phe Gln Gln Ala Asp Tyr Cys Asp
   1890 1895 1900
   Lys Val Ser Ala Val Leu Arg Arg Leu Ser Leu Thr Arg Thr Val Val
   1905 1910 1915 1920
   Ala Leu Val Asn Arg Glu Pro Lys Val Asp Glu Val Gln Val Gly Tyr
   1925 1930 1935
   Val Trp Asp Leu Trp Glu Trp Ile Met Arg Gln Val Arg Met Val Met
   1940 1945 1950
   Ala Arg Leu Arg Ala Leu Cys Pro Val Val Ser Leu Pro Leu Trp His
   1955 1960 1965
   Cys Gly Glu Gly Trp Ser Gly Glu Trp Leu Leu Asp Gly His Val Glu
   1970 1975 1980
   Ser Arg Cys Leu Cys Gly Cys Val Ile Thr Gly Asp Val Phe Asn Gly
   1985 1990 1995 2000
   Gln Leu Lys Glu Pro Val Tyr Ser Thr Lys Leu Cys Arg His Tyr Trp
   2005 2010 2015
   Met Gly Thr Val Pro Val Asn Met Leu Gly Tyr Gly Glu Thr Ser Pro
   2020 2025 2030
   Leu Leu Ala Ser Asp Thr Pro Lys Val Val Pro Phe Gly Thr Ser Gly
   2035 2040 2045
   Trp Ala Glu Val Val Val Thr Pro Thr His Val Val Ile Arg Arg Thr
   2050 2055 2060
   Ser Pro Tyr Glu Leu Leu Arg Gln Gln Ile Leu Ser Ala Ala Val Ala
   2065 2070 2075 2080
   Glu Pro Tyr Tyr Val Asp Gly Ile Pro Val Ser Trp Asp Ala Asp Ala
   2085 2090 2095
   Arg Ala Pro Ala Met Val Tyr Gly Pro Gly Gln Ser Val Thr Ile Asp
   2100 2105 2110
   Gly Glu Arg Tyr Thr Leu Pro His Gln Leu Arg Leu Arg Asn Val Ala
   2115 2120 2125
   Pro Ser Glu Val Ser Ser Glu Val Ser Ile Asp Ile Gly Thr Glu Thr
   2130 2135 2140
   Glu Asp Ser Glu Leu Thr Glu Ala Asp Leu Pro Pro Ala Ala Ala Ala
   2145 2150 2155 2160
   Leu Gln Ala Ile Glu Asn Ala Ala Arg Ile Leu Glu Pro His Ile Asp
   2165 2170 2175
   Val Ile Met Glu Asp Cys Ser Thr Pro Ser Leu Cys Gly Ser Ser Arg
   2180 2185 2190
   Glu Met Pro Val Trp Gly Glu Asp Ile Pro Arg Thr Pro Ser Pro Ala
   2195 2200 2205
   Leu Ile Ser Val Thr Glu Ser Ser Ser Asp Glu Lys Thr Pro Ser Val
   2210 2215 2220
   Ser Ser Ser Gln Glu Asp Thr Pro Ser Ser Asp Ser Phe Glu Val Ile
   2225 2230 2235 2240
   Gln Glu Ser Glu Thr Ala Glu Gly Glu Glu Ser Val Phe Asn Val Ala
   2245 2250 2255
   Leu Ser Val Leu Glu Ala Leu Phe Pro Gln Ser Asp Ala Thr Arg Lys
   2260 2265 2270
   Leu Thr Val Arg Met Asn Cys Cys Val Glu Lys Ser Val Thr Arg Phe
   2275 2280 2285
   Phe Ser Leu Gly Leu Thr Val Ala Asp Val Ala Ser Leu Cys Glu Met
   2290 2295 2300
   Glu Ile Gln Asn His Thr Ala Tyr Cys Asp Lys Val Arg Thr Pro Leu
   2305 2310 2315 2320
   Glu Leu Gln Val Gly Cys Leu Val Gly Asn Glu Leu Thr Phe Glu Cys
   2325 2330 2335
   Asp Lys Cys Glu Ala Arg Gln Glu Thr Leu Ala Ser Phe Ser Tyr Ile
   2340 2345 2350
   Trp Ser Gly Val Pro Leu Thr Arg Ala Thr Pro Ala Lys Pro Pro Val
   2355 2360 2365
   Val Arg Pro Val Gly Ser Leu Leu Val Ala Asp Thr Thr Lys Val Tyr
   2370 2375 2380
   Val Thr Asn Pro Asp Asn Val Gly Arg Arg Val Asp Lys Val Thr Phe
   2385 2390 2395 2400
   Trp Arg Ala Pro Arg Val His Asp Lys Tyr Leu Val Asp Ser Ile Glu
   2405 2410 2415
   Arg Ala Arg Arg Ala Ala Gln Ala Cys Gln Ser Met Gly Tyr Thr Tyr
   2420 2425 2430
   Glu Glu Ala Ile Arg Thr Val Arg Pro His Ala Ala Met Gly Trp Gly
   2435 2440 2445
   Ser Lys Val Ser Val Lys Asp Leu Ala Thr Pro Ala Gly Lys Met Ala
   2450 2455 2460
   Val His Asp Arg Leu Gln Glu Ile Leu Glu Gly Thr Pro Val Pro Phe
   2465 2470 2475 2480
   Thr Leu Thr Val Lys Lys Glu Val Phe Phe Lys Asp Arg Lys Glu Glu
   2485 2490 2495
   Lys Ala Pro Arg Leu Ile Val Phe Pro Pro Leu Asp Phe Arg Ile Ala
   2500 2505 2510
   Glu Lys Leu Ile Leu Gly Asp Pro Gly Arg Val Ala Lys Ala Val Leu
   2515 2520 2525
   Gly Gly Ala Tyr Ala Phe Gln Tyr Thr Pro Asn Gln Arg Val Lys Glu
   2530 2535 2540
   Met Leu Lys Leu Trp Glu Ser Lys Lys Thr Pro Cys Ala Ile Cys Val
   2545 2550 2555 2560
   Asp Ala Thr Cys Phe Asp Ser Ser Ile Thr Glu Glu Asp Val Ala Leu
   2565 2570 2575
   Glu Thr Glu Leu Tyr Ala Leu Ala Ser Asp His Pro Glu Trp Val Arg
   2580 2585 2590
   Ala Leu Gly Lys Tyr Tyr Ala Ser Gly Thr Met Val Thr Pro Glu Gly
   2595 2600 2605
   Val Pro Val Gly Glu Arg Tyr Cys Arg Ser Ser Gly Val Leu Thr Thr
   2610 2615 2620
   Ser Ala Ser Asn Cys Leu Thr Cys Tyr Ile Lys Val Lys Ala Ala Cys
   2625 2630 2635 2640
   Glu Arg Val Gly Leu Lys Asn Val Ser Leu Leu Ile Ala Gly Asp Asp
   2645 2650 2655
   Cys Leu Ile Ile Cys Glu Arg Pro Val Cys Asp Pro Cys Asp Ala Leu
   2660 2665 2670
   Gly Arg Ala Leu Ala Ser Tyr Gly Tyr Ala Cys Glu Pro Ser Tyr His
   2675 2680 2685
   Ala Ser Leu Asp Thr Ala Pro Phe Cys Ser Thr Trp Leu Ala Glu Cys
   2690 2695 2700
   Asn Ala Asp Gly Lys Arg His Phe Phe Leu Thr Thr Asp Phe Arg Arg
   2705 2710 2715 2720
   Pro Leu Ala Arg Met Ser Ser Glu Tyr Ser Asp Pro Met Ala Ser Ala
   2725 2730 2735
   Ile Gly Tyr Ile Leu Leu Tyr Pro Trp His Pro Ile Thr Arg Trp Val
   2740 2745 2750
   Ile Ile Pro His Val Leu Thr Cys Ala Phe Arg Gly Gly Gly Thr Pro
   2755 2760 2765
   Ser Asp Pro Val Trp Cys Gln Val His Gly Asn Tyr Tyr Lys Phe Pro
   2770 2775 2780
   Leu Asp Lys Leu Pro Asn Ile Ile Val Ala Leu His Gly Pro Ala Ala
   2785 2790 2795 2800
   Leu Arg Val Thr Ala Asp Thr Thr Lys Thr Lys Met Glu Ala Gly Lys
   2805 2810 2815
   Val Leu Ser Asp Leu Lys Leu Pro Gly Leu Ala Val His Arg Lys Lys
   2820 2825 2830
   Ala Gly Ala Leu Arg Thr Arg Met Leu Arg Ser Arg Gly Trp Ala Glu
   2835 2840 2845
   Leu Ala Arg Gly Leu Leu Trp His Pro Gly Leu Arg Leu Pro Pro Pro
   2850 2855 2860
   Glu Ile Ala Gly Ile Pro Gly Gly Phe Pro Leu Ser Pro Pro Tyr Met
   2865                2870                2875                2880
   Gly Val Val His Gln Leu Asp Phe Thr Ser Gln Arg Ser Arg Trp Arg
                   2885                2890                2895
   Trp Leu Gly Phe Leu Ala Leu Leu Ile Val Ala Leu Phe Gly
               2900                2905                2910
(2) INFORMATION FOR SEQ ID NO:184:
   (i) SEQUENCE CHARACTERISTICS:
      (A) LENGTH: 39 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GV5446IRT
   (xi) SEQUENCE DESCRIPTION: SEQ ID NO:184:
   CGGTCCCTCG AACTCCAGCG AGTCTTTTTT TTTTTTTTT                    39
(2) INFORMATION FOR SEQ ID NO:185:
   (i) SEQUENCE CHARACTERISTICS:
      (A) LENGTH: 70 amino acids
      (B) TYPE: amino acid
      (C) STRANDEDNESS: double
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (C) INDIVIDUAL ISOLATE: GE-CAP from T55806
   (xi) SEQUENCE DESCRIPTION: SEQ ID NO:185:
   Met Ser Leu Leu Thr Asn Arg Phe Ile Arg Arg Val Asp Lys Asp Gln
   1 5 10 15
   Trp Gly Pro Gly Val Thr Gly Thr Asp Pro Glu Pro Cys Pro Ser Arg
   20 25 30
   Trp Ala Gly Lys Cys Met Gly Pro Pro Ser Ser Ala Ala Ala Cys Ser
   35 40 45
   Arg Gly Ser Pro Arg Ile Leu Arg Val Arg Ala Gly Gly Ile Ser Leu
   50 55 60
   Phe Tyr Thr Ile Met Ala
   65 70
(2) INFORMATION FOR SEQ ID NO:186:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-S59 Variant
   (xi) SEQUENCE DESCRIPTION: SEQ ID NO:186:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC GTTACCCACC 240
   TGGGCAAACG ACGCCCACGT ACGGTCCACG TCGCCCTTCA ATGCCTCTCT TGGCCAATAG 300
   GTTTATCCGG CGAGTTGACA AGGACCAGTG GGGGCCGGGG GCTTGGGGAA GGACCTCAAG 360
   CCCTGCCCTT CCCGGTGGGG CGGGAAATGC ATGGGGCCAC C 401
(2) INFORMATION FOR SEQ ID NO:187:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-5368 Variant
   (xi) SEQUENCE DESCRIPTION: SEQ ID NO:187:
   AGACGCAATG ACTCGGCGCC AACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC GTTACCCACC 240
   CGGGCAAACG ACGCCCACGT ACGGTCCACG TCGCCCTTCA ATGTCTCTCT TGACCAATAG 300
   GCTTAGCCGG CGAGTTGACA AGGACCAGTG GGGGCCGGGG GCTTGGAGAG GGACTCCAAG 360
   TCCTGCCCTT CCCGGTGGGC CGGGAAATGC ATGGGGCCAC C 401
(2) INFORMATION FOR SEQ ID NO:188:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-S309 VARIANT
   (xi) SEQUENCE DESCRIPTION: SEQ ID NO:188:
   AGACGCAATG ACTCGGCGCC GACTCGGCGA CCGGCCAAAA GGTGGTGGAT GGGTGATGAC    60
   AGGGTTGGTA GGTCGTAAAT CCCGGTCATC CTGGTAGCCA CTATAGGTGG GTCTTAAGAG   120
   AAGGTTAAGA TTCCTCTTGT GCATGCGGCG AGAACGCGCA CGGTCCACAG GTGTTGGCCC   180
   TACCGGTGTG AATAAGGGCC CGACGTCAGG CTCGTCGTTA AACCGAGCCC GTTACCCACC   240
   TGGGCAAACG ACGCCCACGT ACGGTCCACG TCGCCCTTCA ATGTCTCTCT TGACCAATAG   300
   GTTTATCCGG CGAGTTGACA AGGACCAGTG GGGGCCGGGG GTCACGGGGA AGGACCCCGG   360
   ATCCTGCCCT TCCCGGTGGG CCGGGAAATG CATGGGGCCA CC                      402
(2) INFORMATION FOR SEQ ID NO:189:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-FZ VARIANT
   (xi) SEQUENCE DESCRIPTION: SEQ ID NO:189:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGGCG AGACCGCGCA CGGTCCACAG GTGTTGGCCC     180
   TACCGGTGTG AATAAGGGCC CGACGTCAGG CTCGTCGTTA AACCGAGCCC GCTACCCACC     240
   TGGGCAAACG ACGCCCATGT ACGGTCCACG TCGCCCTTCA ATGTCTCTCT TGACCAATAG     300
   GATTCGTCCG GCGAGTTGAC AAGGACCAGT GGGGGCCGGG GGCCTGGGGA AGGACCCCAG     360
   ACCCTGCCCT TCCCGGTGGG ACGGGAAATG CATGGGGCCA CC                        402
(2) INFORMATION FOR SEQ ID NO:190:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G21 VARIANT
   (xi) SEQUENCE DESCRIPTION: SEQ ID NO:190:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGTCC 180
   TACCGGTGTG AATAAGGACC CGACGTCAGG CTCGTCGTTA AACCGAGCCC GTTACCCACC 240
   TGGGCAAACG ACGCCCACGT ACGGTCCACG TCGCCCTTCA ATGTCTCTCT TGACCAATAG 300
   GCTTAGCCGG CGAGTTGACA AGGACCAGTG GGGGCCGGGG GCTTGGGGAA GGACCCCAAG 360
   CCCTGCCCTT CCCGGTGGGC CGGGAAATGC ATGGGGCCAC C 401
(2) INFORMATION FOR SEQ ID NO:191:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G23 VARIANT
   (xi) SEQUENCE DESCRIPTION: SEQ ID NO:191:
   AGACGCAATG ACTCGGCGCC AACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCGCAG GTGTTGGCCC 180
   TACCGGTGTG AATAAGGGCC CGACATCAGG CATGTCGTTA AACCGAGCCC GTTACCCGCC 240
   TGGGCTAACG ACGCCCACGT ACGGTCCACG TCGCCCTTCA ATGTCTCTCT TGACCAATAG 300
   GTTTATCCGG CGAGTTGACA AGGACCAGTG GGGGCCGGGG GTTACGGGGA AGGACCCCGA 360
   ACCCTGCCCT TCCCGGCGGA CCGGGAAATG CATGGGGCCA CC 402
(2) INFORMATION FOR SEQ ID NO:192:
   (i) SEQUENCE CHARACTERISTICS:
      (A) LENGTH: 405 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G59 VARIANT
   (xi) SEQUENCE DESCRIPTION: SEQ ID NO:192:
   AGACGCAATG ACTCGGCGCC GACTCGGCGA CCGGCCAAAA GGTGGTGGAT GGGTGATGCC 60
   AGGGTTGGTA GGTCGTAAAT CCCGGTCATC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGGG AATAAGGGCC CGACGTCAGG CTCGTCGTTA AACTGAGCCC GTAACCCACC 240
   TGGGCAAACG ACGCCCACGT ACGGTCCACG TCGCCCTTCA ATGCCTCTCT TGGCCAATAG 300
   GGATTATTCC CGGCGAGTTG GCAAGGACCA GTGGGGGCCG GGAGCTACAG AGAAGGACTC 360
   TGAGCTCTGC CCTTCCCGGT GGAACGGGAA ATGCATGGGG CCACC 405
(2) INFORMATION FOR SEQ ID NO:193:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-E36 VARIANT
   (xi) SEQUENCE DESCRIPTION: SEQ ID NO:193:
   AGACGCAATG ACTCGGCGCC GACTCGGCGA CCGGCCAAAA GGTGGTGGAT GGGTGATGAC     60
   AGGGTTGGTA GGTCGTAAAT CCCGGTCACC TTGGTAGCCA CTATAGGTGG GTCTTAAGAG    120
   AAGGTTAAGA TTCCTCTTGT GCCTGCGGCG AGACCGCGCA CGGCCCACAG GTGTTGGCCC    180
   TACCGGTGTG AATAAGGGCC CGACGTCAGG CTCGTCGTTA AACCGAGCCC ACTACCCACC    240
   TGGGCAAACG ACGCCCACGT ACGGTCTACG TCGCCCTTCA ATGTCTCTCT TGACCAATAG    300
   GCTAAGCCGG CGAGTTGACA AAGACCAGTG GGGGCCGGGG GTCACAGGGA TGGACCCTGG    360
   ACCCTGCCCT TCCCGGTGGA GTGGGAAATG CATGGGGCCA CC                       402
(2) INFORMATION FOR SEQ ID NO:194:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-R38730 VARIANT
   (xi) SEQUENCE DESCRIPTION: SEQ ID NO:194:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGG ATCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC GTATCCCACC 240
   TGGGCAAACG ACGCCCACGT ACGGTCCACG TCGCCCTTCA ATGTCTCTCT TGACCAATAG 300
   GTTCGTCCGG CGAGTTGACA AGGACCAGTG GGGGCCGGGG GTTGCGGGGA AGGACCCCGA 360
   ACTCTGCCCT TCCCGGTGGG CCGGGAAATG CATGGGGCCA CC 402
(2) INFORMATION FOR SEQ ID NO:195:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G281 VARIANT
   (xi) SEQUENCE DESCRIPTION: SEQ ID NO:195:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGTCC 180
   TACCGGTGTG AATAAGGACC CGACGTCAGG CTCGTCGTTA AACCGAGCCC GTTACCCACC 240
   TGGGCAAACG ACGCCCACGT ACGGTCCACG TCGCCCTTCA ATGTCTCTCT TGACCAATAG 300
   GCTTAGCCGG CGAGTTGACA AGGACCAGTG GGGGCCGGGG GCTTGGGGAA GGACCCCAAG 360
   CCCTGCCCTT CCCGGTGGGC CGGGAAATGC ATGGGGCCAC C 401
(2) INFORMATION FOR SEQ ID NO:196:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G157 VARIANT
   (xi) SEQUENCE DESCRIPTION: SEQ ID NO:196:
   AGACGCAATG ACTCGGCGCC GACCCGGCGA CCGGCCAAAA GGTGGTGGAT GGGTGATGCC 60
   AGGGTTGGTA GGTCGTAAAT CCCGGTCATC TTGGTAGCCA CTATAGGTGG GTCTTAAGGG 120
   AAGGTTAAGA TTCCTCTTGT GCCTGTGGCG AGACAGCGCA CGGTCCACAG GTGTTGGCCC 180
   TACCGGTGTG AATAAGGGCC CGACGTCAGG CTCGTCGTTA AACCGAGACC GACACCCACC 240
   TGGGCAAACG ACGCCCACGT ACGGTCCACG TCGCCCTTCA ATGTCTCTCT TGACCAATAG 300
   GCTTTGCCGG CGAGTTGACA AGGACCAGTG GGGGCCGGGT GCTGGGGGAA GGACCCCCTT 360
   GCACCGCCCT TCCCGGTGGG ACGGGAAATG CATGGGGCCA CC 402
(2) INFORMATION FOR SEQ ID NO:197:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G154 VARIANT
   (xi) SEQUENCE DESCRIPTION: SEQ ID NO:197:
   AGACGCAATG ACTCGGCGCC GACTCGGCGA CCGGCCAAAA GGTGGTGGAT GGGTGATGAC     60
   AGGGTTGGTA GGTCGTAAAT CCCGGTCATC CTGGTAGCCA CTATAGGTGG GTCTTAAGAG    120
   AAGGTTAAGA TTCCTCTTAC GCCTGCGGCG AGACCGCGCA CGGTCCACAG GTGCTGGCCT    180
   TACCGGTGTG AATAAAGGCC CGACGTCAGG CTCGTCGTTA AACCGAGCCC GTCACCCACC    240
   TGGGCAAACG ACGCCCACGT ACGGTCCACG TCGCCCTTCA ATGTCTCTCT TGACCAGTAG    300
   GTTTAACCGG CGAGTTGACA AGGACCAGTG GGGGCCGGGG CCTTGGAGAT GGACTCCAAG    360
   TCCTGCCCTT CCCGGTGGGC CGGGAAATGC ATGGGGCCAC C                        401
(2) INFORMATION FOR SEQ ID NO:198:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G213 VARIANT
   (xi) SEQUENCE DESCRIPTION: SEQ ID NO:198:
   AGACGCAATG ACTCGGCGCC AACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGTCC 180
   TACCGGTGGG AATAAGGGCC CGACGTCAGG CTCGTCGTTA AACCGAGCCC GTCACCCACC 240
   TGGGCAAACG ACGCCCACGT ATGGTCCACG TCGCCCTTCA ATGCCTCTCT TGGCCAATAG 300
   GTTTATCCGG CGAGTTGACA AGGACCAGTG GGGGCCGGGG GTTCGGGGAA GGACCCCGTA 360
   CCCTGCCCTT CCCGGTGGAA CGGGAAATGC ATGGGGCCAC C 401
(2) INFORMATION FOR SEQ ID NO:199:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G204 VARIANT
   (xi) SEQUENCE DESCRIPTION: SEQ ID NO:199:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTT AATAAGGGCC CGACGTCAGG CTCGTCGTTA AACCGAGCCC GTCACCCACC 240
   TGGGCAAACG ACGCCCACGT ACGGTCCACG TCGCCCTTCA ATGTCTCTCT TGACCAATAG 300
   GCTTAGCCGG CGAGTTGACA AGGACCAGTG GGGGCCGGGG GCCTGGAGAG GGACTCCAGG 360
   TCCTGCCCTT CCCGGTGGGC CGGGAAATGC ATGGGGCCAC C 401
(2) INFORMATION FOR SEQ ID NO:200:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G191 VARIANT
   (xi) SEQUENCE DESCRIPTION: SEQ ID NO:200:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCATC CTGGTAGCCA CTATAGGTGG GTCTTAAGAG 120
   AAGGTTAAGG ATCCTCTTGT GCCTGCGGCG AGACCGCGCA CGGTCCACAG GTGTTGGCCC 180
   TACCGGTGTG AATAAGGGCC CGACGTCAGG CTCGTCGCTA AACCGAGCCC GTATCCCACC 240
   TGGGCAAACG ACGCCCACGT ACGGTCCACG TCGCCCTTCA ATGTCTCTCT TGACCAATAG 300
   GTTTATCCGG CGAGTTGACA AGGACCAGTG GGGGCCGGAG GTTACGGGGA AGGACCCCGA 360
   GCCTCGCCCT TCCCGGTGGG CCGGGAAATG CATGGGGCCA CC 402
(2) INFORMATION FOR SEQ ID NO:201:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-G299 VARIANT
   (xi) SEQUENCE DESCRIPTION: SEQ ID NO:201:
   AGACGCAATG ACTCGGCGCC GACTCGGCGA CCGGCCAAAA GGTGGTGGAT GGGTGATGAC     60
   AGGGTTGGTA GGTCGTAAAT CCCGGTCACC TTGGTAGCCA CTATAGGTGG GTCTTAAGAG    120
   AAGGTTAAGA TTCCTCTTGT GCCTGCGGCG AGACCGCGCA CGGTCCACAG GTGTTGGCCC    180
   TACCGGTGTG AATAAGGGCC CGACGTCAGG CTCGTCGTTA AACCGAGCCC GTCACCCACC    240
   TGGGCAAACG ACGCCCACGC ACGGTCCACG TCGCCCTTCA ATGCCTCTCT TGGCCAATAG    300
   GAGTATCCGG CGAGTTGACA AGGACCAGTG GGGGCCGGGA GTCACGGGGA TGGACCCCGG    360
   GCTCTGCCCT TCCCGGTGGA ACGGGAAATG CATGGGGCCA CC                       402
(2) INFORMATION FOR SEQ ID NO:202:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-T56957 VARIANT
   (xi) SEQUENCE DESCRIPTION: SEQ ID NO:202:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC ATCACCCACC 240
   TGGGCAAACG ACGCCCACGT ACGGTCCACG TCGCCCTACA ATGTCTCTCT TGACCAATAG 300
   GCTTAGCCGG CGAGTTGACA AGGACCAGTG GGGGCCGGGG GTCACAGGGA TGGACCCTGG 360
   GCCCTGCCCT TCCCGGTGGG GTGGGAAATG CATGGGGCCA CC 402
(2) INFORMATION FOR SEQ ID NO:203:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-C01698 VARIANT
   (xi) SEQUENCE DESCRIPTION: SEQ ID NO:203:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC GTCACCCACC 240
   TGGGCAAACG ACGCCCACGT ACGGTCCACG TCGCCCTTCA ATGTCTCTCT TGACCAATAG 300
   GCTTAGCCGG CGAGTTGACA AGGACCAGTG GGGGCCGGGG GCTTGGAGAT GGACTCCAAG 360
   TCCTGCCCTT CCCGGTGGGC CGGGAAATGC ATGGGGCCAC C 401
(2) INFORMATION FOR SEQ ID NO:204:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-T27034 VARIANT
   (xi) SEQUENCE DESCRIPTION: SEQ ID NO:204:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGGCG AGACCGCGCA CGGTCCACAG GTGTTGGCCC     180
   TACCGGTGTG AATAAGGGCC CGACGTCAGG CTCGTCGTTA AACCGAGCCC ATTTCCCGCC     240
   TGGGCTAACG ACGCCCACGT ACGGTCCACG TCGCCCTTCA ATGTCTCTCT TGACCAATAG     300
   GTTTATCCGG CGAGTTGACA AGGACCAGTG GGGGCCGGGA GTCACTGGGA TGGACCCAGG     360
   GCTCTGCCCT TCCCGGCGGG GTGGGAAAAG CATGGGGCCA CC                        402
(2) INFORMATION FOR SEQ ID NO:205:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-E57963 VARIANT
   (xi) SEQUENCE DESCRIPTION: SEQ ID NO:205:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGGCG AGACCGCGCA CGGTCCGCAG GTGTTGGCCC     180
   TACCGGTGTG AATAAGGGCC CGACGTCAGG CTCGTCGTTA AACCGAGCCC GTCACCCACC     240
   TGGGCAAACG ACGCCCACGT ACGGTCCACG TCGCCCTTCA ATGTCTCTCT TGACCAATAG     300
   GCTTAGCCGG CGAGTTGACA AGGACCAGTG GGGGCCGGGG GCTTGGAGAA GGACTCCAAG     360
   TCCTGCCCTT CCCGGTGGGC CGGGAAATGC ATGGGGCCAC C                         401
(2) INFORMATION FOR SEQ ID NO:206:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-R37166 VARIANT
   (xi) SEQUENCE DESCRIPTION: SEQ ID NO:206:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC GTAACCCGCC 240
   TGGGCAAACG ACGCCCACGT ACGGTCCACG TCGCCCTTCA ATGTCTCTCT TGACCAATAG 300
   GTTTAACCGG CGAGTTGACA AGGACCAGTG GGGGCCGGGG CCTTGGAGAT GGACTCCAAG 360
   TCCTGCCCTT CCCGGCGGGC CGGGAAATGC ATGGGGCCAC C 401
(2) INFORMATION FOR SEQ ID NO:207:
   (i) SEQUENCE CHARACTERISTICS:
      (A) LENGTH: 404 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-B5 VARIANT
   (xi) SEQUENCE DESCRIPTION: SEQ ID NO:207:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCATC CTGGTAGCCA CTATAGGTGG GTCTTAAGGG 120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC GTCACCCACC 240
   TGGGCTAACG ACGCCCACGT ACGGTCCACG TCGCCCTTCA ATGTCTCTCT TGACCAATAG 300
   GCTTTTTGCC GGCGAGTTGA CAAGGACCAG TGGGGGCCGG GGGTTATGGG GAAGGACCCC 360
   AAACCCTGCC CTTCCCGGTG GGCCGGGAAA TGCATGGGGC CACC 404
(2) INFORMATION FOR SEQ ID NO:208:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-B33 VARIANT
   (xi) SEQUENCE DESCRIPTION: SEQ ID NO:208:
   AGACGCAATG ACTCGGCGCC GACTCGGCGA CCGGCCAAAA GGTGGTGGAT GGGTGATGAC     60
   AGGGTTGGTA GGTCGTAAAT CCCGGTCATC CTGGTAGCCA CTATAGGTGG GTCTTAAGAG    120
   AAGGTTAAGA TTCCTCTTGT GCCTGCGGCG AGACCGCGCA CGGTCCACAG GTGTTGGCCC    180
   TACCGGTGTG AATAAGGGCC CGACGTCAGG CTCGTCGTTA AACCGAGCCC GTTCCCCGCC    240
   TGGGCAAACG ACGCCCACGT ACGGTCCACG TCGCCCTTCA ATGTCTCTCT TGACCAATAG    300
   GTTTATCCGG CGAGTTGACA AGGACCAGTG GGGGCCGGGG ATCATGGGGA AGGACCCCAG    360
   ATCCTGCCCT TCCCGGCGGG CCGGGAAATG CATGGGGCCA CC                       402
(2) INFORMATION FOR SEQ ID NO:209:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-FH010 VARIANT
   (xi) SEQUENCE DESCRIPTION: SEQ ID NO:209:
   AGACGCAATG ACTCGGCGCC GACCCGGCGA CCGGCCAAAA GGTGGTGGAT GGGTGATGCC      60
   AGGGTTGGTA GGTCGTAAAT CCCGGTCATC TTGGTAGCCA CTATAGGTGG GTCTTAAGGG     120
   AAGGTTAAGA TTCCTCTTGT GCCTGTGGCG AGACAGCGCA CGGTCCACAG GTGTTGGCCC     180
   TACCGGTGTG AATAAGGGCC CGACGTCAGG CTCGTCGTTA AACTGAGACC GACACCCACC     240
   TGGGCAAACG ACGCCCACGT ACGGTCCACG TCGCCCTTCA ATGTCTCTCT TGACCAATAG     300
   GCTTTGCCGG CGAGTTGACA AGGACCAGTG GGGGCCGGGG GCTGGGGGAA GGACCCCCAG     360
   TCCTGCCCTT CCCGGTGGGA CGGGAAATGC ATGGGGCCAC C                         401
(2) INFORMATION FOR SEQ ID NO:210:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-PNF2161 VARIANT
   (xi) SEQUENCE DESCRIPTION: SEQ ID NO:210:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGGG AATAAGGGCC CGACGTCAGG CTCGTCGTTA AACCGAGCCC GTTACCCACC 240
   TGGGCAAACG ACGCCCACGT ACGGTCCACG TCGCCCTTCA ATGTCTCTCT TGACCAATAG 300
   GCGTAGCCGG CGAGTTGACA AGGACCAGTG GGGGCCGGGG GCTTGGAGAG GGACTCCAAG 360
   TCCCGCCCTT CCCGGTGGGC CGGGAAATGC ATGGGGCCAC C 401
(2) INFORMATION FOR SEQ ID NO:211:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-JC VARIANT
   (xi) SEQUENCE DESCRIPTION: SEQ ID NO:211:
   AGACGCAATG ACTCGGCGCC GACTCGGCGA CCGGCCAAAA GGTGGTGGAT GGGTGATGAC 60
   AGGGTTGGTA GGTCGTAAAT CCCGGTCACC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGGG AATAAGGGCC CGACGTCAGG CTCGTCGTTA AACCGAGCCC GTAACCCGCC 240
   TGGGCAAACG ACGCCCACGT ACGGTCCACG TCGCCCTTCA ATGTCGCTCT TGACCAATAG 300
   GCTTAGCCGG CGAGTTGACA AGGACCAGTG GGGGCCGGGG TTTATGGGGA AGGACCCCAA 360
   ACCCTGCCCT TCCCGGCGGA CCGGGAAATG CATGGGGCCA CC 402
(2) INFORMATION FOR SEQ ID NO:212:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-7155 VARIANT
   (xi) SEQUENCE DESCRIPTION: SEQ ID NO:212:
   AGACGTTATG AACCGGCGCC GCCCCGGCGA CCGGCCAAAA GGTGGTGGAT GGGTGATGCC 60
   AGGGTTGGTA GGTCGTAAAT CCCGGTCATC TTGGTAGCCA CTATAGGTGG GTCTTAAGGG 120
   GTGGTCAAGG TCCCTCTAGC GCTTGTGGCG AGAAAGCGCA CGGTCCACAG GTGTTGGCCC 180
   TACCGGTGTG AATAAGGGCC CGACGTCAGG CTCGTCGTTA AACCGAGCCC ATTATCCTCC 240
   TGGGCAAACG ACGCCCACGT ACGGTCCACG TCGCCCTTCA ATGTCTCTCT TGACCAATAG 300
   GCTTTGCCGG CGAGTTGACA AGGACCAGTG GGGGCCGGGT GCCGGGGGAA GGACCCCCGG 360
   TACTGCCCCT CCCGGAGGAG TGGGAAATGC ATGGGGCCAC C 401
(2) INFORMATION FOR SEQ ID NO:213:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-7244 VARIANT
   (xi) SEQUENCE DESCRIPTION: SEQ ID NO:213:
   AGACGTTAAG AACCGGCGCC GCCCCGGCGA CCGGCCAAAA GGTGGTGGAT GGGTGATGCC      60
   AGGGTTGGTA GGTCGTAAAT CCCGGTCATC TTGGTAGCCA CTATAGGTGG GTCTTAAGGG     120
   GTGGTCAAGG TCCCTCTGGC GCTTGTGGCG AGAAAGCGCA CGGTCCACAG GTGTTGGCCC     180
   TACCGGTGTG AATAAGGGCC CGACGTCAGG CTCGTCGTTA AACCGAGCCC ATTACCCTCC     240
   TGGGCAAACG ACGCCCATGT ACGGTCCACG TCGCCCTTCA ATGTCTCTCT TGACCAATAG     300
   GCTTTGCCGG CGAGTTGACA AGGACCAGTG GGGGCCGGGT GGCGGGGGAA GGACCCCCGT     360
   CACTGCCCTT CCCGGAGGGG TGGGAAATGC ATGGGGCCAC C                         401
(2) INFORMATION FOR SEQ ID NO:214:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-K27 VARIANT
   (xi) SEQUENCE DESCRIPTION: SEQ ID NO:214:
   AGACGTTAAG TACCGGCGCC GACCCGGCGA CCGGCCAAAA GGTGGTGGAT GGGTGATGCC      60
   AGGGTTGGTA GGTCGTAAAT CCCGGTCATC TTGGTAGCCA CTATAGGTGG GTCTTAAGGG     120
   TTGGTCAAGG TCCCTCTGGC GCTTGTGGCG AGAAAGCGCA CGGTCCACAG GTGTTGGCCC     180
   TACCGGTGTG AATAAGGGCC CGACGTCAGG CTCGTCGTTA AACCGAGCCC ATTACCCACC     240
   TGGGCAAACA ACGCCCACGT ACGGTCCACG TCGCCCTACA ATGTCTCTCT TGACCAATAG     300
   GCTTTGCCGG CGAGTTGACA AGGACCAGTG GGGGCTGGGC GGCGAGGGAA GGACCCTCGT     360
   CGCTGCCCTT CCCGGCGGGG TGGGGAATGC ATGGGGCCAC C                         401
(2) INFORMATION FOR SEQ ID NO:215:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-K30 VARIANT
   (xi) SEQUENCE DESCRIPTION: SEQ ID NO:215:
   AGACGTTAAG AACCGGCGCC TTCCCGGCGA CCGGCCAAAA GGTGGTGGAT GGGTGATGCC      60
   AGGGTTGGTA GGTCGTAAGT CCCGGTCATC TTGGTAGCCA CTATAGGTGG GTCTTAAGGG     120
   AGGGTTAAGG TCCCTCTGGC GCTTGTGGCG AGAAAGCGCA CGGTCCACAG GTGTTGGCCC     180
   TACCGGTGTG AATAAGGGCC CGACGTCAGG CTCGTCGTTA AACCGAGCCC ATTACCCACC     240
   TGGGCAAACA ACGCCCACGT ACGGTCCACG TCGCCCTTCA ATGTCTCTCT TGACCAATAG     300
   GCTTTGCCGG CGAGTTGACA AGGACCAGTG GGGGCTGGGC GGTAGGGGAA GGACCCTTGC     360
   CGCTGCCCTT CCCGGTGGGG TGGGAAATGC ATGGGGCCAC C                         401
(2) INFORMATION FOR SEQ ID NO:216:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-T55875 VARIANT
   (xi) SEQUENCE DESCRIPTION: SEQ ID NO:216:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGACG AGACCGCGCA CGGTCCGCAG GTGTTGGCCC     180
   TACCGGTGTG AATAAGGGCC CGACGTCAGG CTCGTCGTTA AACCGAGCCC GTCACCCACC     240
   TGGGCAAACG ACGCCCACGT ACGGTCCACG TCGCCCTTCA ATGCCTCTCT TGGCCAATAG     300
   GTTTAACCGG CGAGTTGGCA AGGACCAGTG GGGGCCGGGG GCTTGGAGAG GGACTCCAAG     360
   TCCTGCCCTT CCCGGTGGGC CGGGAAATGC ATGGGGCCAC C                         401
(2) INFORMATION FOR SEQ ID NO:217:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-T56633 VARIANT
   (xi) SEQUENCE DESCRIPTION: SEQ ID NO:217:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGGCG AGACCGCGCA CGGTCCACAG GTGTTGGCCC 180
   TACCGGTGTG AATAAGGGCC CGACGTCAGG CTCGTCGTTA AACCGAGCCC ACTACCCACC 240
   TGGGCTAACG ACGCCCACGT ACGGTCCACG TCGCCCTTCA ATGTCTCTCT TGACCAATAG 300
   GCTAGTCCGG CGAGTTGACA AGGACCAGTG GGGGCCGGAG GTCACAGGGA TGGACCCTGG 360
   GCCTTGCCCT TCCCGGTGGA GTGGGAAAAG CATGGGGCCA CC 402
(2) INFORMATION FOR SEQ ID NO:218:
   (i) SEQUENCE CHARACTERISTICS:
      (A) LENGTH: 404 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-EB20 VARIANT
   (xi) SEQUENCE DESCRIPTION: SEQ ID NO:218:
   AGACGCAATG ACTCGGCGCC GACTCGGCGA CCGGCCAAAA GGTGGTGGAT GGGTGATGCC 60
   AGGGTTGGTA GGTCGTAAAT CCCGGTCATC TTGGTAGCCA CTATAGGTGG GTCTTAAGAG 120
   AAGGTTAAGA TTCCTCTTGT GCCTGCGGCG AGACCGCGCA CGGTCCACAG GTGTTGGCCC 180
   TACCGGTGTA ATAAGGGCCC GACGTCAGGC TCGTCGTTAA ACCGAGCCCG TCACCCACCT 240
   GGGCAAACGA CGCCCACGTA CGGTCCACGT CGCCCTTCAA TGCCTCTCTT GGCCAATAGG 300
   AGTTATCTCC GGCGAGTTGG CAAGGACCAG TGGGGGCCGG GGGTTACGGG GAAGGACCCC 360
   GAACCCTGCC CTTCCCGGTG GGCCGGGAAA TGCATGGGGC CACC 404
(2) INFORMATION FOR SEQ ID NO:219:
   (i) SEQUENCE CHARACTERISTICS:
      (A) LENGTH: 401 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-T55806 VARIANT
   (xi) SEQUENCE DESCRIPTION: SEQ ID NO:219:
   AGACGCAATG ACTCGGCGCC GACTCGGCGA CCGGCCAAAA GGTGGTGGAT GGGTGATGCC      60
   AGGGTTGGTA GGTCGTAAAT CCCGGTCATC TTGGTAGCCA CTATAGGTGG GTCTTAAGAG     120
   AAGGTTAAGA TTCCTCTTGT GCCTGCGGCG AGACCGCGCA CGGTCCACAG GTGTTGGCCC     180
   TACCGGTGGA ATAAGGGCCC GACGTCAGGC TCGTCGTTAA ACCGAGCCCG TCACCCACCT     240
   GGGCAAACGA CGCTCACGTA CGGTCCACGT CGCCCTTCAA TGTCTCTCTT GACCAATAGG     300
   TTTATCCGGC GAGTTGACAA GGACCAGTGG GGGCCGGGGG TTACGGGGAC GGACCCCGAA     360
   CCCTGCCCTT CCCGGTGGGC CGGGAAATGC ATGGGGCCAC C                         401
(2) INFORMATION FOR SEQ ID NO:220:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-BG34 VARIANT
   (xi) SEQUENCE DESCRIPTION: SEQ ID NO:220:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGGCG AGACCGCGCA CGGTCCACAG GTGTTGGCCC     180
   TACCGGTGTG AATAAGGGCC CGACGTCAGG CTCGTCGTTA AACCGAGCCC GTCACCCACC     240
   TGGGCAAACG ACGCCCACGT ACGGTCCACG TCGCCCTTCA ATGCCTCTCT TGGCCAATAG     300
   GAGTATCCGG CGAGTTGACA AGGACCAGTG GGGGCCGGGA GTCACGGGGA TGGACCCCGG     360
   GCTCTGCCCT TCCCGGTGGA ACGGGAAACG CATGGGGCCA CC                        402
(2) INFORMATION FOR SEQ ID NO:221:
   (i) SEQUENCE CHARACTERISTICS:
      (A) LENGTH: 402 base pairs
      (B) TYPE: nucleic acid
      (C) STRANDEDNESS: double
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-BE12 VARIANT
   (xi) SEQUENCE DESCRIPTION: SEQ ID NO:221:
   AGACGCAATG ACTCGGCGCC GACTCGGCGA CCGGCCAAAA GGTGGTGGAT GGGTGATGAC      60
   AGGGTTGGTA GGTCGTAAAT CCCGGTCACC TTGGTAGCCA CTATAGGTGG GTCTTAAGAG     120
   AAGGTTAAGA TTCCTCTTGT GCCTGCGGCG AGACCGCGCA CGGTCCGCAG GTGTTGGTCC     180
   TACCGGTGTG AATAAGGACC CGACGTCAGG CTCGTCGTTA AACCGAGCCC GCCACCCACC     240
   TGGGCAAACG ACGCCCACGT ACGGTCCACG TCGCCCTTCA ATGCCTCTCT TGGCCAATAG     300
   GTTTATCCGG CGAGTTGACA AGGACCAGTG GGGGCCGGGG GCTCCGGGGA AGAACCCCGA     360
   GCCCCGCCCT TCCCGGTGGG ACGGGAAATG CATGGGGCCA CC                        402
(2) INFORMATION FOR SEQ ID NO:222:
   (i) SEQUENCE CHARACTERISTICS:
      (A) LENGTH: 24 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-FORWARD PRIMER
   (xi) SEQUENCE DESCRIPTION: SEQ ID NO:222:
   CCAAAAGGTG GTGGATGGGT GATG 24
(2) INFORMATION FOR SEQ ID NO:223:
   (i) SEQUENCE CHARACTERISTICS:
      (A) LENGTH: 24 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-FORWARD PRIMER
   (xi) SEQUENCE DESCRIPTION: SEQ ID NO:223:
   GTGATGMCAG GGTTGGTAGG TCGT 24
(2) INFORMATION FOR SEQ ID NO:224:
   (i) SEQUENCE CHARACTERISTICS:
      (A) LENGTH: 26 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-FORWARD PRIMER
   (xi) SEQUENCE DESCRIPTION: SEQ ID NO:224:
   GGTAGCCACT ATAGGTGGGT CTTAAG 26
(2) INFORMATION FOR SEQ ID NO:225:
   (i) SEQUENCE CHARACTERISTICS:
      (A) LENGTH: 25 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-REVERSE PRIMER
   (xi) SEQUENCE DESCRIPTION: SEQ ID NO:225:
   GAGMGRCATT GWAGGGCGAC GTRGA 25
(2) INFORMATION FOR SEQ ID NO:226:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-REVERSE PRIMER
   (xi) SEQUENCE DESCRIPTION: SEQ ID NO:226:
   GRCATTGWAG GGCGACGTRG A 21
(2) INFORMATION FOR SEQ ID NO:227:
   (i) SEQUENCE CHARACTERISTICS:
      (A) LENGTH: 22 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: HGV-REVERSE PRIMER
   (xi) SEQUENCE DESCRIPTION: SEQ ID NO:227:
   CCCCACTGGT CYTTGYCAAC TC 22
(2) INFORMATION FOR SEQ ID NO:228:
   (i) SEQUENCE CHARACTERISTICS:
      (A) LENGTH: 29 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PRIMER GV75-36FE
   (xi) SEQUENCE DESCRIPTION: SEQ ID NO:228:
   GCGAGATCTA AAATGCAGGC CTGATGGGT 29
(2) INFORMATION FOR SEQ ID NO:229:
   (i) SEQUENCE CHARACTERISTICS:
      (A) LENGTH: 29 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PRIMER GV75-7064RLE
   (xi) SEQUENCE DESCRIPTION: SEQ ID NO:229:
   GCGAGATCTA AAATGTGGAC TGCTAAGCC 29
(2) INFORMATION FOR SEQ ID NO:230:
   (i) SEQUENCE CHARACTERISTICS:
      (A) LENGTH: 46 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PRIMER FV94-28F
   (xi) SEQUENCE DESCRIPTION: SEQ ID NO:230:
   GCGAGATCTA AAATGGCAAG CCCCAGAAAC CGACGCCTAT CTAAGT 46
(2) INFORMATION FOR SEQ ID NO:231:
   (i) SEQUENCE CHARACTERISTICS:
      (A) LENGTH: 39 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PRIMER FV94-2864R
   (xi) SEQUENCE DESCRIPTION: SEQ ID NO:231:
   GGCATGATGA ATTCGCAACG AGGGCCGGGA CACCAAGAT                39
(2) INFORMATION FOR SEQ ID NO:232:
   (i) SEQUENCE CHARACTERISTICS:
      (A) LENGTH: 39 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PRIMER FV94-6439F
   (xi) SEQUENCE DESCRIPTION: SEQ ID NO:232:
   GCGAGATCTA AAATGGGCCT CCGACACCCC GAAGGTTGT                39
(2) INFORMATION FOR SEQ ID NO:233:
   (i) SEQUENCE CHARACTERISTICS:
      (A) LENGTH: 39 base pairs
      (B) TYPE: nucleic acid
      (C) STRANDEDNESS: single
      (D) TOPOLOGY: unknown
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: PRIMER FV94-9331R
   (xi) SEQUENCE DESCRIPTION: SEQ ID NO:233:
   GCGAGATCTG AATTCTTCCC GGGGTGCACC CCTTCAGAT 39
(2) INFORMATION FOR SEQ ID NO:234:
   (i) SEQUENCE CHARACTERISTICS:
      (A) LENGTH: 9327 base pairs
      (B) TYPE: nucleic acid
      (C) STRANDEDNESS: double
      (D) TOPOLOGY: unknown
   (ii) MOLECULE TYPE: cDNA
   (iii) HYPOTHETICAL: NO
   (iv) ANTI-SENSE: NO
   (vi) ORIGINAL SOURCE:
      (C) INDIVIDUAL ISOLATE: 3ZHGV-6, HGV FROM PNF2161
   (xi) SEQUENCE DESCRIPTION: SEQ ID NO:234:
   GCAAGCCCCA GAAACCGACG CCTATCTAAG TAGACGCAAT GACTCGGCGC CGACTCGGCG 60
   ACCGGCCAAA AGGTGGTGGA TGGGTGATGA CAGGGTTGGT AGGTCGTAAA TCCCGGTCAC 120
   CTTGGTAGCC ACTATAGGTG GGTCTTAAGA GAAGGTTAAG ATTCCTCTTG TGCCTGCGGC 180
   GAGACCGCGC ACGGTCCACA GGTGTTGGCC CTACCGGTGG GAATAAGGGC CCGACGTCAG 240
   GCTCGTCGTT AAACCGAGCC CGTTACCCAC CTGGGCAAAC GACGCCCACG TACGGTCCAC 300
   GTCGCCCTTC AATGTCTCTC TTGACCAATA GGCGTAGCCG GCGAGTTGAC AAGGACCAGT 360
   GGGGGCCGGG GGCTTGGAGA GGGACTCCAA GTCCCGCCCT TCCCGGTGGG CCGGGAAATG 420
   CATGGGGCCA CCCAGCTCCG CGGCGGCCTG CAGCCGGGGT AGCCCAAGAA TCCTTCGGGT 480
   GAGGGCGGGT GGCATTTCCT TTTTCTATAC CATCATGGCA GTCCTTCTGC TCCTTCTCGT 540
   GGTTGAGGCC GGGGCCATTC TGGCCCCGGC CACCCACGCT TGTCGAGCGA ATGGGCAATA 600
   TTTCCTCACA AATTGTTGTG CCCCGGAGGA CATCGGGTTC TGCCTGGAGG GTGGATGCCT 660
   GGTGGCCCTG GGGTGCACGA TTTGCACTGA CCAATGCTGG CCACTGTATC AGGCGGGTTT 720
   GGCTGTGCGG CCTGGCAAGT CCGCGGCCCA ACTGGTGGGG GAGCTGGGTA GCCTATACGG 780
   GCCCCTGTCG GTCTCGGCCT ATGTGGCTGG GATCCTGGGC CTGGGTGAGG TGTACTCGGG 840
   TGTCCTAACG GTGGGAGTCG CGTTGACGCG CCGGATCTAC CCGGTGCCTA ACCTGACGTG 900
   TGCAGTCGCG TGTGAGTTAA AGTGGGAAAG TGAGTTTTGG AGATGGACTG AACAGCTGGC 960
   CTCCAACTAC TGGATTCTGG AATACCTCTG GAAGGTCCCA TTTGATTTCT GGAGAGGCGT 1020
   GATAAGCCTG ACCCCCTTGT TGGTTTGCGT GGCCGCATTG CTGCTGCTTG AGCAACGGGT 1080
   TGTCATGGTC TTCCTGTTGG TGACGATGGC CGGGATGTCG CAAGGCGCCC CTGCCTCCGT 1140
   TTTGGGGTCA CGCCCCTTTG ACTACGGGTT GACTTGGCAG ACCTGCTCTT GCAGGGCCAA 1200
   CGGTTCGCGT TTTTCGACTG GGGAGAAGGT GTGGGACCGT GGGAACGTTA CGCTTCAGTG 1260
   TGACTGCCCT AACGGCCCCT GGGTGTGGTT GCCAGCCTTT TGCCAAGCAA TCGGCTGGGG 1320
   TGACCCCATC ACTTATTGGA GCCACGGGCA AAATCAGTGG CCCCTTTCAT GCCCCCAGTA 1380
   TGTCTATGGG TCTGCTACAG TCACTTGCGT GTGGGGTTCC GCTTCTTGGT ATGCCTCCAC 1440
   CAGTGGTCGC GACTCGAAGA TAGATGTGTG GAGTTTAGTG CCAGTTGGCT CTGCCACCTG 1500
   CACCATAGCC GCACTTGGAT CATCGGATCG CGACACGGTG CCTGGGCTCT CCGAGTGGGG 1560
   AATCCCGTGC GTGACGTGTG TTCTGGACCG TCGGCCTGCT TCATGCGGCA CCTGTGTGAG 1620
   GGACTGCTGG CCCGAGACCG GGTCGGTTAG GTTCCCATTC CATCGGTGCG GCGTGGGGCC 1680
   TCGGCTGACA AAGGACTTGG AAGCTGTGCC CTTCGTCAAT AGGACAACTC CCTTCACCAT 1740
   TAGGGGGCCC CTGGGCAACC AGGGCCGAGG CAACCCGGTG CGGTCGCCCT TGGGTTTTGG 1800
   GTCCTACGCC ATGACCAGGA TCCGAGATAC CCTACATCTG GTGGAGTGTC CCACACCAGC 1860
   CATCGAGCCT CCCACCGGGA CGTTTGGGTT CTTCCCCGGG ACGCCGCCTC TCAACAACTG 1920
   CATGCTCTTG GGCACGGAAG TGTCCGAGGC ACTTGGGGGG GCTGGCCTCA CGGGGGGGTT 1980
   CTATGAACCC CTGGTGCGCA GGTGTTCGGA GCTGATGGGA AGCCGAAATC CGGTTTGTCC 2040
   GGGGTTTGCA TGGCTCTCTT CGGGCAGGCC TGATGGGTTT ATACATGTCC AGGGTCACTT 2100
   GCAGGAGGTG GATGCAGGCA ACTTCATCCC GCCCCCGCGC TGGTTGCTCT TGGACTTTGT 2160
   ATTTGTCCTG TTATACCTGA TGAAGCTGGC TGAGGCACGG TTGGTCCCGC TGATCTTGCT 2220
   GCTGCTATGG TGGTGGGTGA ACCAGCTGGC AGTCCTAGGG CTGCCGGCTG TGGAAGCCGC 2280
   CGTGGCAGGT GAGGTCTTCG CGGGCCCTGC CCTGTCCTGG TGTCTGGGAC TCCCGGTCGT 2340
   CAGTATGATA TTGGGTTTGG CAAACCTGGT GCTGTACTTT AGATGGTTGG GACCCCAACG 2400
   CCTGATGTTC CTCGTGTTGT GGAAGCTTGC TCGGGGAGCT TTCCCGCTGG CCCTCTTGAT 2460
   GGGGATTTCG GCGACCCGCG GGCGCACCTC AGTGCTCGGG GCCGAGTTCT GCTTCGATGC 2520
   TACATTCGAG GTGGACACTT CGGTGTTGGG CTGGGTGGTG GCCAATGTGG TAGCTTGGGC 2580
   CATTGCGCTC CTGAGCTCGA TGAGCGCAGG GGGGTGGAGG CACAAAGCCG TGATCTATAG 2640
   GACGTGGTGT AAGGGGTACC AGGCAATCCG TCAAAGGGTG GTGAGGAGCC CCCTCGGGGA 2700
   GGGGCGGCCT GCCAAACCCC TGACCTTTGC CTGGTGCTTG GCCTCGTACA TCTGGCCAGA 2760
   TGCTGTGATG ATGGTGGTGG TTGCCTTGGT TCTTCTCTTT GGCCTGTTCG ACGCGTTGGA 2820
   TTGGGCCTTG GAGGAGATCT TGGTGTCCCG GCCCTCGCTG CGGCGTTTGG CTCGGGTGGT 2880
   TGAGTGCTGT GTGATGGCGG GTGAGAAGGC CACAACCGTC CGGCTGGTCT CCAAGATGTG 2940
   TGCGAGAGGA GCTTATTTGT TCGATCATAT GGGCTCATTT TCGCGTGCTG TCAAGGAGCG 3000
   CCTGTTGGAA TGGGACGCGG CTCTTGAACC TCTGTCATTC ACTAGGACGG ACTGTCGCAT 3060
   CATACGGGAT GCCGCGAGGA CTTTGTCCTG CGGGCAATGC GTCATGGGTT TACCCGTGGT 3120
   TGCGCGCCGT GGTGATGAGG TTCTCATCGG CGTCTTCCAG GATGTGAATC ATTTGCCTCC 3180
   CGGGTTTGTT CCGACCGCGC CTGTTGTCAT CCGACGGTGC GGAAAGGGCT TCTTGGGGGT 3240
   CACAAAGGCT GCCTTGACAG GTCGGGATCC TGACTTACAT CCAGGGAACG TCATGGTGTT 3300
   GGGGACGGCT ACGTCGCGAA GCATGGGAAC ATGCTTGAAC GGCCTGCTGT TCACGACCTT 3360
   CCATGGGGCT TCATCCCGAA CCATCGCCAC ACCCGTGGGG GCCCTTAATC CCAGATGGTG 3420
   GTCAGCCAGT GATGATGTCA CGGTGTATCC ACTCCCGGAT GGGGCTACTT CGTTAACGCC 3480
   TTGTACTTGC CAGGCTGAGT CCTGTTGGGT CATCAGATCC GACGGGGCCC TATGCCATGG 3540
   CTTGAGCAAG GGGGACAAGG TGGAGCTGGA TGTGGCCATG GAGGTCCCTG ATTTCCGTGG 3600
   CTCGTCTGGC TCACCGGTCC TATGTGACGA GGGGCACGCA GTAGGAATGC TCGTGTCTGT 3660
   GCTTCACTCC GGTGGTAGGG TCACCGCGGC ACGGTTCACT AGGCCGTGGA CCCAAGTGCC 3720
   AACAGATGCC AAAACCACCA CTGAACCCCC TCCGGTGCCG GCCAAAGGAG TTTTCAAAGA 3780
   GGCCCCGTTG TTTATGCCTA CGGGAGCGGG AAAGAGCACT CGCGTCCCGT TGGAGTACGG 3840
   CAACATGGGG CACAAGGTCT TAGTCTTGAA CCCCTCAGTG GCCACTGTGC GGGCCATGGG 3900
   CCCGTACATG GAGCGGCTGG CGGGTAAACA TCCAAGTATA TACTGTGGGC ATGATACAAC 3960
   TGCTTTCACA AGGATCACTG ACTCCCCCCT GACGTATTCA ACCTATGGGA GGTTTTTGGC 4020
   CAACCCTAGG CAGATGCTAC GGGGCGTTTC GGTGGTCATT TGTGATGAGT GCCACAGTTA 4080
   TGACTCAACC GTGCTGTTAG GCATTGGGAG GGTTCGGGAG CTGGCGCGTG GGTGCGGAGT 4140
   GCAACTAGTG CTCTACGCCA CCGCTACGCC TCCCGGATCC CCTATGACGC AGCACCCTTC 4200
   CATAATTGAG ACAAAATTGG ACGTGGGCGA GATTCCCTTT TATGGGCACG GAATACCCCT 4260
   CGAGCGGATG CGAACCGGAA GGCACCTCGT GTTCTGCCAT TCTAAGGCTG AGTGCGAGCG 4320
   CCTTGCTGGC CAGTTCTCCG CTAGGGGGGT CAATGCCATT GCCTATTATA GGGGTAAAGA 4380
   CAGTTCTATC ATCAAGGATG GGGACCTGGT GGTCTGTGCC ACAGACGCGC TTTCCACTGG 4440
   GTACACTGGA AATTTCGACT CCGTCACCGA CTGTGGATTA GTGGTGGAGG AGGTCGTTGA 4500
   GGTGACCCTT GATCCTACCA TTACCATCTC CCTGCGGACA GTGCCTGCGT CGGCTGAACT 4560
   GTCGATGCAA AGACGAGGAC GCACGGGTAG GGGCAGGTCT GGACGCTACT ACTACGCGGG 4620
   GGTGGGCAAA GCCCCTGCGG GTGTGGTGCG CTCAGGTCCT GTCTGGTCGG CGGTGGAAGC 4680
   TGGAGTGACC TGGTACGGAA TGGAACCTGA CTTGACAGCT AACCTACTGA GACTTTACGA 4740
   CGACTGCCCT TACACCGCAG CCGTCGCGGC TGATATCGGA GAAGCCGCGG TGTTCTTCTC 4800
   TGGGCTCGCC CCATTGAGGA TGCACCCTGA TGTCAGCTGG GCAAAAGTTC GCGGCGTCAA 4860
   CTGGCCCCTC TTGGTGGGTG TTCAGCGGAC CATGTGTCGG GAAACACTGT CTCCCGGCCC 4920
   ATCGGATGAC CCCCAATGGG CAGGTCTGAA GGGCCCAAAT CCTGTCCCAC TCCTGCTGAG 4980
   GTGGGGCAAT GATTTACCAT CTAAAGTGGC CGGCCACCAC ATAGTGGACG ACCTGGTCCG 5040
   GAGACTCGGT GTGGCGGAGG GTTACGCCCG CTGCGACGCT GGGCCGATCT TGATGATCGG 5100
   TCTAGCTATC GCGGGGGGAA TGATCTACGC GTCGTACACC GGGTCGCTAG TGGTGGTGAC 5160
   AGACTGGGAT GTGAAGGGGG GTGGCGCCCC CCTTTATCGG CATGGAGACC AGGCCACGCC 5220
   TCAGCCGGTG GTGCAGGTTC CTCCGGTAGA CCATCGGCCG GGGGGTGAAT CAGCACCATC 5280
   GGATGCCAAG ACAGTGACAG ATGCGGTGGC AGCGATCCAG GTGGACTGCG ATTGGACTAT 5340
   CATGACTCTG TCGATCGGAG AAGTGTTGTC CTTGGCTCAG GCTAAGACGG CCGAGGCCTA 5400
   CACAGCAGCC ACCAAGTGGC TCGCTGGCTG CTATACGGGG ACGCGGGCCG TTCCCACTGT 5460
   ATCCATTGTT GACAAGCTCT TCGCCGGAGG GTGGGCGGCT GTGGTGGGCC ATTGCCACAA 5520
   CGTGATTGCT GCGGCGGTGG CGGCCTACGG GGCTTCAAAG AGCCCGCCGT TGGCAGCCGC 5580
   GGCTTCCTAC CTGATGGGGT TGGGCGTTGG AGGCAACGCT CAGACGCGTC TGGCATCTGC 5640
   CCTCCTATTG GGGGCTGCTG GAACCGCCTT GGGCACTCCT GTCGTGGGCT TGACCATGGC 5700
   AGGTGCGTTC ATGGGGGGCG CCAGTGTCTC CCCCTCCTTG GTCACCATTT TATTGGGGGC 5760
   CGTCGGAGGT TGGGAGGGTG TTGTCAACGC GGCGAGCCTA GTCTTTGACT TCATGGCGGG 5820
   GAAACTTTCA TCAGAAGATC TGTGGTATGC CATCCCGGTA CTGACCAGCC CGGGGGCGGG 5880
   CCTTGCGGGG ATCGCTCTCG GGTTGGTTTT GTATTCAGCT AACAACTCTG GCACTACCAC 5940
   TTGGTTGAAC CGTCTGCTGA CTACGTTACC AAGGTCTTCA TGTATCCCGG ACAGTTACTT 6000
   TCAGCAAGTT GACTATTGCG ACAAGGTCTC AGCCGTGCTC CGGCGCCTGA GCCTCACCCG 6060
   CACAGTGGTT GCCCTGGTCA ACAGGGAGCC TAAGGTGGAT GAGGTACAGG TGGGGTATGT 6120
   CTGGGACCTG TGGGAGTGGA TCATGCGCCA AGTGCGCGTG GTCATGGCCA GACTCAGGGC 6180
   CCTCTGCCCC GTGGTGTCAT TACCCTTGTG GCACTGCGGG GAGGGGTGGT CCGGGGAATG 6240
   GTTGCTTGAC GGTCATGTTG AGAGTCGCTG CCTCTGTGGC TGCGCGATCA CTGGTGACGT 6300
   TCTGAATGGG CAACTCAAAG AACCAGTTTA CTCTACCAAG CTGTGCCGGC ACTATTGGAT 6360
   GGGGACTGTC CCTGTGAACA TGCTGGGTTA CGGTGAAACG TCGCCTCTCC TGGCCTCCGA 6420
   CACCCCGAAG GTTGTGCCCT TCGGGACGTC TGGCTGGGCT GAGGTGGTGG TGACCACTAC 6480
   CCACGTGGTA ATCAGGAGAA CCTCCGCCTA TAAGCTGCTG CGCCAGCAAA TCCTATCGGC 6540
   TGCTGTAGCT GAGCCCTACT ACGTCGACGG CATTCCGGTC TCATGGGACG CGGACGCTCG 6600
   TGCGCCCGCC ATGGTCTATG GCCCTGGGCA AAGTGTTACC ATTGACGGGG AGCGCTACAC 6660
   CCTGCCTCAT CAACTGAGGC TCAGGAATGT GGCGCCCTCT GAGGTTTCAT CCGAGGTGTC 6720
   CATTGACATT GGGACGGAGA CTGGAGACTC AGAACTGACT GAGGCCGATC TGCCGCCGGC 6780
   GGCTGCTGCT CTCCAAGCGA TCGAGAATGC TGCGAGGATT CTTGAACCGC ACATTGATGC 6840
   CATCATGGAG GACTGCAGTA CACCCTCTCT TTGTGGTAGT AGCCGAGAGA TGCCTGTATG 6900
   GGGAGAAGAC ATCCCCCGTA CTCCATCGCC AGCACTTATC TCGGTTACTG AGAGCAGCTC 6960
   AGATGAGAAG ACCCCGTCGG TGTCCTCCTC GCAGGAGGAT ACCCCGTCCT CTGACTCATT 7020
   CGAGGTCATC CAAGAGTCCG AGACAGCCGA AGGGGAGGAA AGCGTCTTCA ACGTGGCTCT 7080
   TTCCGTATTA GAAGCCTCAT TTCCACAGAG CGACGCGACC AGGAAGCTTA CCGTCAAGAT 7140
   GTCGTGCTGC GTTGAAAAGA GCGTCACGCG CTTTTTCTCA TTGGGGTTGA CGGTGGCTGA 7200
   TGTTGCTAGC CTGTGTGAGA TGGAAATCCA GAACCATACA GCCTATTGTG ACAAGGTGCG 7260
   CACTCCGCTT GAATTGCAGG TTGGGTGCTT GGTGGGCAAT GAACTTACCT TTGAATGTGA 7320
   CAAGTGTGAG GCTAGGCAAG AAACCTTGGC CTCCTTCTCT TACATTTGGT CTGGAGTGCC 7380
   GCTGACTAGG GCCACGCCGG CCAAGCCTCC CGTGGTGAGG CCGGTTGGCT CTTTATTAGT 7440
   GGCCGACACT ACTAAGGTGT ATGTTACCAA TCCAGACAAT GTGGGACGGA GGGTGGACAA 7500
   GGTGACCTTC TGGCGTGCTC CTAGGGTTCA TGATAAGTAC CTCGTGGACT CTATTGAGCG 7560
   CGCTAAGAGG GCCGCTCAAG CCTGCCTAAG CATGGGTTAC ACTTATGAGG AAGCAATAAG 7620
   GACTGTAAGG CCACATGCTG CCATGGGCTG GGGATCTAAG GTGTCGGTTA AGGACTTAGC 7680
   CACCCCCGCG GGGAAGATGG CCGTCCATGA CCGGCTCCAG GAGATACTTG AAGGGACTCC 7740
   GGTCCCCTTT ACTCTTACTG TGAAAAAGGA GGTGTTCTTC AAAGACCGGA AGGAGGAGGA 7800
   GGCCCCCCGC CTCATTGTGT TCCCCCCCCT GGACTTCCGG ATAGCTGAAA AGCTCATCTT 7860
   GGGAGACCCA GACCGGGTAG CCAAGGCGGT GTTGGGGGGG GCCTACGCCT TCCAGTACAC 7920
   CCCAAATCAG CGAGTTAAGG AGATGCTCAA GCTATGGGAG TCTAAGAAGA CCCCTTGCGC 7980
   CATCTGTGTG GACGCCACCT GCTTCGACAG TAGCATAACT GAAGAGGACG TGGCTTTGGA 8040
   GACAGAGCTG TACGCTCTGG CCTCTGACCA TCCAGAATGG GTGCGGGCAC TTGGGAAATA 8100
   CTATGCCTCA GGCACCATGG TCACCCCGGA AGGGGTGCCC GTCGGTGAGA GGTATTGCAG 8160
   ATCCTCGGGT GTCCTAACAA CTAGCGCGAG CAACTGCTTG ACCTGCTACA TCAAGGTGAA 8220
   AGCCGCCTGT GAGAGGGTGG GGCTGAAGAA TGTCTCTCTT CTCATAGCCG GCGATGACTG 8280
   CTTGATCATA TGTGAGCGGC CAGTGTGCGA CCCAAGCGAC GCTTTGGGCA GAGCCCTAGC 8340
   GAGCTATGGG TACGCGTGCG AGCCCTCATA TCATGCATCC TTGGACACGG CCCCCTTCTG 8400
   CTCCACTTGG CTTGCTGAGT GCAATGCAGA TGGGAAGCGC CATTTCTTCC TGACCACGGA 8460
   CTTCCGGAGG CCGCTCGCTC GCATGTCGAG TGAGTATAGT GACCCGATGG CTTCGGCGAT 8520
   CGGTTACATC CTCCTTTATC CTTGGCACCC CATCACACGG TGGGTCATCA TCCCTCATGT 8580
   GCTAACGTGC GCATTCAGGG GTGGAGGCAC ACCGTCTGAT CCGGTTTGGT GCCAGGTACA 8640
   TGGTAACTAC TACAAGTTTC CACTGGACAA ACTGCCTAAC ATCATCGTGG CCCTCCACGG 8700
   ACCAGCAGCG TTGAGGGTTA CCGCAGACAC AACTAAAACA AAGATGGAGG CTGGTAAGGT 8760
   TCTGAGCGAC CTCAAGCTCC CTGGCTTAGC AGTCCACCGA AAGAAGGCCG GGGCGTTGCG 8820
   AACACGCATG CTCCGCTCGC GCGGTTGGGC TGAGTTGGCT AGGGGCTTGT TGTGGCATCC 8880
   AGGCCTACGG CTTCCTCCCC CTGAGATTGC TGGTATCCCG GGGGGTTTCC CTCTCTCCCC 8940
   CCCCTATATG GGGGTGGTAC ACCAATTGGA TTTTACAAGC CAGAGGAGTC GCTGGCGGTG 9000
   GTTGGGGTTC TTAGCCCTGC TCATCGTAGC CCTCTTCGGG TGAACTAAAT TCATCTGTTG 9060
   CGGCGAGGTC TGGTGACTGA TCGTCACCGG AGGAGGTTCC CGCCCTCCCC GCCCCAGGGG 9120
   TCTCCCCGCT GGGTAAAAAG GGCCCGGCCT TGGGAGGCAT GGTGGTTACT AACCCCCTGG 9180
   CAGGGTTAAA GCCTGATGGT GCTAATGCAC TGCCACTTCG GTGGCGGGTC GCTACCTTAT 9240
   AGCGTAATCC GTGACTACGG GCTGCTCGCA GAGCCCTCCC CGGATGGGGC ACAGTGCACT 9300
   GAGATCTGAA GGGGTGCACC CCGGGAA 9327
(2) INFORMATION FOR SEQ ID NO:235:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GLI-F
   (xi) SEQUENCE DESCRIPTION: SEQ ID NO:235:
   TAGCATGGCC TTTGCAGGGC TG 22
(2) INFORMATION FOR SEQ ID NO:236:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GLI-R
   (xi) SEQUENCE DESCRIPTION: SEQ ID NO:236:
   AAGCTGTGAC CGTCTCCG 18
(2) INFORMATION FOR SEQ ID NO:237:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE1-NF
   (xi) SEQUENCE DESCRIPTION: SEQ ID NO:237:
   GCCGCCATGG CGGGGAAACT TTCATCAGAA G 31
(2) INFORMATION FOR SEQ ID NO:238:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE1-NR
   (xi) SEQUENCE DESCRIPTION: SEQ ID NO:238:
   GCGCGGATCC TAGTGACACC ACGGGGCAGA GG 32
(2) INFORMATION FOR SEQ ID NO:239:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE57F
   (xi) SEQUENCE DESCRIPTION: SEQ ID NO:239:
   GCCGCCATGG CTCTCTTGAC CAATAGGTTT ATC 33
(2) INFORMATION FOR SEQ ID NO:240:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GE57R
   (xi) SEQUENCE DESCRIPTION: SEQ ID NO:240:
   GCGCGGATCC AGAAATGCCA CCCGCCCTCA C                                    31
(2) INFORMATION FOR SEQ ID NO:241:
   (i) SEQUENCE CHARACTERISTICS:
      (A) LENGTH: 61 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (C) INDIVIDUAL ISOLATE: GE57 amino acid sequence
   (xi) SEQUENCE DESCRIPTION: SEQ ID NO:241:
   Met Ser Leu Leu Thr Asn Arg Phe Ile Arg Arg Val Asp Lys Asp Gln
   1               5                   10                  15
   Trp Gly Pro Gly Val Thr Gly Thr Asp Pro Glu Pro Cys Pro Ser Arg
               20                  25                  30
   Trp Ala Gly Lys Cys Met Gly Pro Pro Ser Ser Ala Ala Ala Cys Ser
           35                  40                  45
   Arg Gly Ser Pro Arg Ile Leu Arg Val Arg Ala Gly Gly
       50                  55                  60
(2) INFORMATION FOR SEQ ID NO:242:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer for E1
   (xi) SEQUENCE DESCRIPTION: SEQ ID NO:242:
   GCGCAGATCT AAAATGAGCC GTGGTGGCAT TTCCTTTTTC TATACCATCA TG 52
(2) INFORMATION FOR SEQ ID NO:243:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer for E1
   (xi) SEQUENCE DESCRIPTION: SEQ ID NO:243:
   GCGCAGATCT CCAGAAATCA AATGGGACCT TCCAGAGG 38
(2) INFORMATION FOR SEQ ID NO:244:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer for E2 with insect signal sequence
   (xi) SEQUENCE DESCRIPTION: SEQ ID NO:244:
   CGCGAGATCT GTCGCAAGGC GCCCCT 26
(2) INFORMATION FOR SEQ ID NO:245:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer for E2 with insect signal sequence
   (xi) SEQUENCE DESCRIPTION: SEQ ID NO:245:
   GCGCAGATCT AGTTGCCTGC ATCCACCT 28
(2) INFORMATION FOR SEQ ID NO:246:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer for E2 with HGV signal sequence
   (xi) SEQUENCE DESCRIPTION: SEQ ID NO:246:
   CGCGAGATCT AAAATGAAAC TGCTTGTCAT GGTCTTCCTG TT 42
(2) INFORMATION FOR SEQ ID NO:247:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer for E2 with HGV signal sequence
   (xi) SEQUENCE DESCRIPTION: SEQ ID NO:247:
   GCGCAGATCT AGTTGCCTGC ATCCACCT 28
(2) INFORMATION FOR SEQ ID NO:248:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer for NS2a
   (xi) SEQUENCE DESCRIPTION: SEQ ID NO:248:
   GCGCAGATCT GGCCGTGGCA GGTGAGGTCT TCGC 34
(2) INFORMATION FOR SEQ ID NO:249:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer for NS2a
   (xi) SEQUENCE DESCRIPTION: SEQ ID NO:249:
   GCGCAGATCT TAACGCCGCA ACGAGGGCCG G                                   31
(2) INFORMATION FOR SEQ ID NO:250:
   (i) SEQUENCE CHARACTERISTICS:
      (A) LENGTH: 46 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer for NS2b
   (xi) SEQUENCE DESCRIPTION: SEQ ID NO:250:
   GCGCGGATCC AAAATGATCG CTCGGGTGGT TGAGTGCTGT GTGATG                   46
(2) INFORMATION FOR SEQ ID NO:251:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer for NS2b
   (xi) SEQUENCE DESCRIPTION: SEQ ID NO:251:
   GCGCGGATCC AGGCGCGGTC GGAACAAACC CG 32
(2) INFORMATION FOR SEQ ID NO:252:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer NS3
   (xi) SEQUENCE DESCRIPTION: SEQ ID NO:252:
   GCGAGATCTA AAATGTGCGG AAAGGGCTTC TTGGGGGTC 39
(2) INFORMATION FOR SEQ ID NO:253:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer NS3
   (xi) SEQUENCE DESCRIPTION: SEQ ID NO:253:
   GCGAGATCTC ATCTCCGGAC CAGGTCGTCC ACTATGTGG 39
(2) INFORMATION FOR SEQ ID NO:254:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer NS4a
   (xi) SEQUENCE DESCRIPTION: SEQ ID NO:254:
   GGCGGATCCA AAATGATCGG TGTGGCGGAG G 31
(2) INFORMATION FOR SEQ ID NO:255:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer NS4a
   (xi) SEQUENCE DESCRIPTION: SEQ ID NO:255:
   GGCGGGATCC ATGCGCCGGA GCACGG 26
(2) INFORMATION FOR SEQ ID NO:256:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer NS4b.
   (xi) SEQUENCE DESCRIPTION: SEQ ID NO:256:
   GCGGGATCCA AAATGATCAG CCTCACCCGC ACAG 34
(2) INFORMATION FOR SEQ ID NO:257:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer NS5a
   (xi) SEQUENCE DESCRIPTION: SEQ ID NO:257:
   GGCGGGATCC TACCTCCTGA TTACCACGT 29
(2) INFORMATION FOR SEQ ID NO:258:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer NS5a
   (xi) SEQUENCE DESCRIPTION: SEQ ID NO:258:
   GCGAGATCTA AAATGACCTC CGCCTATAAG CTGCTGCGCC AG 42
(2) INFORMATION FOR SEQ ID NO:259:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer NS5a
   (xi) SEQUENCE DESCRIPTION: SEQ ID NO:259:
   GGCAGATCTA CCTCCGTCCC ACATTGTCTG GATTGGTAAC 40
(2) INFORMATION FOR SEQ ID NO:260:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer NS5b
   (xi) SEQUENCE DESCRIPTION: SEQ ID NO:260:
   GCGAGATCTA AAATGGTGGA CAAGGTGACC TTCTGGCGTG CTC 43
(2) INFORMATION FOR SEQ ID NO:261:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer NS5b
   (xi) SEQUENCE DESCRIPTION: SEQ ID NO:261:
   GCGAGATCTC ACCCGAAGAG GGCTACGATG AGCAGG 36
(2) INFORMATION FOR SEQ ID NO:262:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Forward Primer E1-E2-NS2a
   (xi) SEQUENCE DESCRIPTION: SEQ ID NO:262:
   GCGCAGATCT AAAATGAGCC GTGGTGGCAT TTCCTTTTTC TATACCATCA TG 52
(2) INFORMATION FOR SEQ ID NO:263:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Reverse Primer E1-E2-NS2a
   (xi) SEQUENCE DESCRIPTION: SEQ ID NO:263:
   GCGCAGATCT TAACGCCGCA ACGAGGGCCG G 31
(2) INFORMATION FOR SEQ ID NO:264:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 9E3-REV
   (xi) SEQUENCE DESCRIPTION: SEQ ID NO:264:
   GCTGGCTGAG GCACGGTTGG TC 22
(2) INFORMATION FOR SEQ ID NO:265:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer E39-94PR
   (xi) SEQUENCE DESCRIPTION: SEQ ID NO:265:
   CACCATCATC ACAGCATCTG GC 22
(2) INFORMATION FOR SEQ ID NO:266:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-F12
   (xi) SEQUENCE DESCRIPTION: SEQ ID NO:266:
   GCAACCATGG AACCTGCCAA ACCCCTGACC TT 32
(2) INFORMATION FOR SEQ ID NO:267:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-R12
   (xi) SEQUENCE DESCRIPTION: SEQ ID NO:267:
   AGCCCCATGG AAGGTCGTGA A 21
(2) INFORMATION FOR SEQ ID NO:268:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-F14
   (xi) SEQUENCE DESCRIPTION: SEQ ID NO:268:
   TTGGGATCCC TCGTGTTCCG CCATTCTAAG 30
(2) INFORMATION FOR SEQ ID NO:269:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-R13
   (xi) SEQUENCE DESCRIPTION: SEQ ID NO:269:
   TATGGATCCT GGTAAATCAT TGCCCCACCT 30
(2) INFORMATION FOR SEQ ID NO:270:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer 470EP-F8
   (xi) SEQUENCE DESCRIPTION: SEQ ID NO:270:
   GCTGAATTCG CCATGGCGAC GTGCGCATTC AGGGGTGGA 39
(2) INFORMATION FOR SEQ ID NO:271:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Primer GEP-R14
   (xi) SEQUENCE DESCRIPTION: SEQ ID NO:271;
(2) INFORMATION FOR SEQ ID NO:272:
   (i) SEQUENCE CHARACTERISTICS:
      (A) LENGTH: 48 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (C) INDIVIDUAL ISOLATE: Y5 epitope
   (xi) SEQUENCE DESCRIPTION: SEQ ID NO:272:
   Ile Asp Gly Glu Arg Tyr Thr Leu Pro His Gln Leu Arg Leu Arg Asn
   1               5                   10                  15
   Val Ala Pro Ser Glu Val Ser Ser Glu Val Ser Ile Asp Ile Gly Thr
               20                  25                  30
   Glu Ala Glu Asn Ser Glu Leu Thr Glu Ala Asp Leu Pro Pro Ala Ala
           35                  40                  45
(2) INFORMATION FOR SEQ ID NO:273:
   (i) SEQUENCE CHARACTERISTICS:
      (A) LENGTH: 55 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (C) INDIVIDUAL ISOLATE: Q9 Epitope
   (xi) SEQUENCE DESCRIPTION: SEQ ID NO:273:
   Cys Gly Leu Leu Thr Arg His His Thr Ala Leu Asn His Pro Ser Gln
   1               5                   10                  15
   Thr Pro Gln Arg Gly Pro Gly His Gln Asp Leu Leu Gln Gly Pro Ile
               20                  25                  30
   Gln Arg Val Glu Gln Ala Lys Glu Lys Asp Gln Gly Asn His His His
           35                  40                  45
   His His Ser Ile Trp Pro Asp
   50 55
(2) INFORMATION FOR SEQ ID NO:274:
   (i) SEQUENCE CHARACTERISTICS:
      (A) LENGTH: 35 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (C) INDIVIDUAL ISOLATE: Q11 Epitope
   (xi) SEQUENCE DESCRIPTION: SEQ ID NO:274:
   Ala Ala Val Ala Glu Pro Tyr Tyr Val Asp Gly Ile Pro Val Ser Trp
   1 5 10 15
   Asp Ala Asp Ala Arg Ala Pro Ala Met Val Tyr Gly Pro Gly Gln Ser
   20 25 30
   Val Thr Ile
   35
(2) INFORMATION FOR SEQ ID NO:275:
   (i) SEQUENCE CHARACTERISTICS:
      (A) LENGTH: 225 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Q7-12-1 env clone
   (xi) SEQUENCE DESCRIPTION: SEQ ID NO:275:
   GTGCCCTTCG TCAACAGGAC AACTCTCTTC ACCATTAGGG GGCCCCTGGG CAACCAGGGC 60
   CGAGGCAACC CGGTGCGGTC GCCCTTGGGT TTTGGGTCCT ACGCCATGAC CAGGATCCGA 120
   GATACCCTAC ATCTGGTGGA GTGTCCCACA CCAGCCATCG AGCCTCCCAC CGGGACGTCT 180
   GGGTTCTTCC CCGGGACGCC GCCTCTCAAC AACTGCATGC ATATG 225
(2) INFORMATION FOR SEQ ID NO:276:
   (i) SEQUENCE CHARACTERISTICS:
      (A) LENGTH: 192 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Y12-15-1 NS3 clone DNA
   (xi) SEQUENCE DESCRIPTION: SEQ ID NO:276:
   AACATGGGGC ACAAGGTCTT AATCTTGAAC CCCTCAGTGG CCACTGTGCG GGCCATGGGC 60
   CCGTACATGG AGCGGCTGGC GGGTAAACAT CCAAGTATAT ACTGTGGGCA TGATACAACT 120
   GCTTTCACAA GGATCACTGA CTCCCCCCTG ACGTATTCAA CCTATGGGAG GTTTTTGGCC 180
   AACCCTAGGC AA 192
(2) INFORMATION FOR SEQ ID NO:277:
   (i) SEQUENCE CHARACTERISTICS:
      (A) LENGTH: 264 base pairs
      (B) TYPE: nucleic acid
      (C) STRANDEDNESS: both
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (C) INDIVIDUAL ISOLATE: Y12-10-2 NS3 clone
   (xi) SEQUENCE DESCRIPTION: SEQ ID NO:277:
   CCCCTCGAGC GGATGCGAAC CGGAAGGCAC CTCGTGTTCT GCCATTCTAA GGCTGAGTGC    60
   GAGCGCCTTG CTGGCCAGTT CTCCGCTAGG GGGGTCAATG CCATTGCCTA TTATAGGGGT   120
   AAAGACAGCT CTATCATCAA GGATGGGGAC CTGGTGGTCT GTGCTACAGA CGCGCTTTCC   180
   ACTGGGTACA CTGGAAATTT CGACTCCGTC ACCGACTGTG GATTAGTGGT GGAGGAGGTC   240
   GTTGAGGTGA CCCTTGATCC CACC                                          264